(19) Europäisches Patentamt
European Patent Office
Office européen des brevets

(11) Numéro de publication : **0 527 069 A1**

(12) **DEMANDE DE BREVET EUROPEEN**

(21) Numéro de dépôt : **92401904.5**

(22) Date de dépôt : **03.07.92**

(51) Int. Cl.[5] : **C07D 207/06,** C07K 5/06, A61K 31/40, A61K 37/02, C07B 57/00

(30) Priorité : **10.07.91 FR 9108675**

(43) Date de publication de la demande : **10.02.93 Bulletin 93/06**

(84) Etats contractants désignés : **PT**

(71) Demandeur : **RHONE-POULENC RORER SA**
**20, avenue Raymond Aron**
**F-92160 Antony (FR)**

(72) Inventeur : **Capet, Marc**
**10 rue de la Galaise**
**F-94320 Thiais (FR)**
Inventeur : **Cotrel, Claude**
**17 Avenue du Docteur Arnold Netter**
**F-75012 Paris (FR)**
Inventeur : **Guyon, Claude**
**17bis Avenue Henri Martin**
**F-94100 Saint Maur des Fosses (FR)**
Inventeur : **Joannic, Michel**
**88 rue du 11 Novembre**
**F-93330 Neuilly sur Marne (FR)**
Inventeur : **Manfre, Franco**
**3 place Chateaubriand**
**F-94450 Limeil-Brevannes (FR)**
Inventeur : **Roussel, Gérard**
**20 Ter rue des Carrières**
**F-91450 Soisy sur Seine (FR)**
Inventeur : **Dubroeucq, Marie-Christine**
**13 Villa de Malleville**
**F-95880 Enghien les Bains (FR)**
Inventeur : **Cheve, Michel**
**3 rue Paul Belmondo**
**F-91450 Soisy sur Seine (FR)**
Inventeur : **Dutruc-Rosset, Gilles**
**21 Avenue du Docteur Arnold Netter**
**F-75012 Paris (FR)**

(74) Mandataire : **Pilard, Jacques et al**
**RHONE-POULENC RORER S.A. 20 Avenue Raymond Aron**
**F-92165 Antony Cédex (FR)**

(54) **Dérivés de pyrrolidine et thiazolidine, leur préparation et les médicaments les contenants.**

(57) Composés de formule :

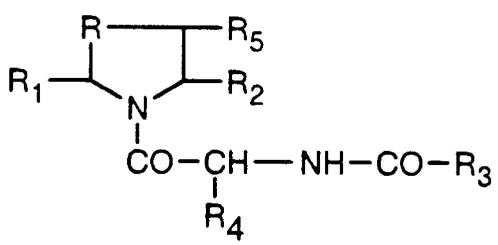

(I)

leurs sels, leur préparation et les médicaments les contenant.

EP 0 527 069 A1

La présente invention concerne des dérivés de formule:

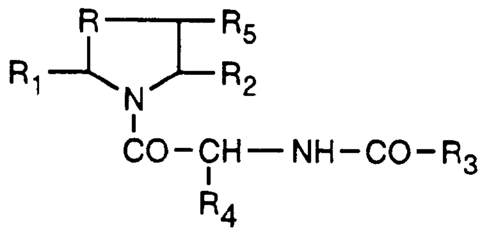

(I)

leurs sels, leur préparation et les médicaments les contenant.

Dans la formule (I),

- soit R représente un radical méthylène, éthylène, SO, $SO_2$, CHOH ou un atome de soufre, $R_1$ représente un radical pyridyle éventuellement substitué par un ou plusieurs radicaux alkyle, furyle éventuellement substitué par un ou plusieurs radicaux alkyle, thiènyle éventuellement substitué par un ou plusieurs radicaux alkyle, quinolyle éventuellement substitué par un ou plusieurs radicaux alkyle, naphtyle éventuellement substitué par un ou plusieurs radicaux alkyle, indolyle éventuellement substitué par un ou plusieurs radicaux alkyle ou phényle éventuellement substitué par un ou plusieurs substituants choisis parmi les atomes d'halogène et les radicaux alkyle, alcoxy, hydroxy, nitro, amino, monoalkylamino, dialkylamino, alcoxycarbonyle, $-CO-NR_7R_8$ $-NH-CO-CH_3$, trifluorométhyle ou trifluorométhoxy et $R_8$ représente un atome d'hydrogène,
- soit R représente un radical méthylène, $R_1$ représente un atome d'hydrogène et $R_5$ représente un radical phényle,
- soit R représente un radical $CHR_8$, $R_1$ et $R_5$ représentent chacun un atome d'hydrogène,
- $R_2$ représente un radical alcoxycarbonyle, cycloalkyloxycarbonyle, cycloalkylalkyloxycarbonyle, $-CONR_9R_{10}$ ou phényle éventuellement substitué par un ou plusieurs substituants choisis parmi les radicaux alkyle, alcoxy ou hydroxy,
- $R_3$ représente un radical phényle (éventuellement substitué par un ou plusieurs substituants choisis parmi les atomes d'halogène et les radicaux alkyle, alcoxy et alkylthio), naphtyle, indolyle, quinolyle ou phénylamino dont le noyau phényle est éventuellement substitué par un ou plusieurs substituants choisis parmi les atomes d'halogène et les radicaux alkyle, alcoxy, alkylthio, trifluorométhyle, carboxy, alcoxycarbonyle, hydroxy, nitro, amino, acyle, cyano, sulfamoyle, carbamoyle, hydroxyiminoalkyle, alcoxyiminoalkyle, hydroxyaminocarbonyle, alcoxyaminocarbonyle, tétrazolyl-5, tétrazolyl-5 alkyle, trifluorométhylsulfonamido, alkylsulfinyle, mono ou polyhydroxyalkyle, sulfo, -alk-O-CO-alk, -alk-COOX, -alk-O-alk, -alk'-COOX, -O-alk-COOX, -CH=CH-COOX, -CO-COOX, $-alk-SO_3H$, sous forme de sel, -CH=CH-alk', -C(=NOH)-COOX, -S-alk-COOX, $-O-CH_2-alk'-COOX$, -CX=N-O-alk-COOX, -alk-N(OH)-CO-alk ou diméthyl-2,2 dioxo-4,6 dioxanne-1,3-yle-5,
- $R_4$ représente un atome d'hydrogène ou un radical alkyle,
- $R_8$ représente un radical phényle,
- $R_7$ représente un atome d'hydrogène ou un radical alkyle, phénylalkyle ou phényle éventuellement substitué par un ou plusieurs substituants choisis parmi les atomes d'halogène et les radicaux alkyle, alcoxy et alkylthio,
- $R_8$ représente un radical alkyle, phénylalkyle ou phényle éventuellement substitué par un ou plusieurs substituants choisis parmi les atomes d'halogène et les radicaux alkyle, alcoxy et alkylthio,

ou bien $R_7$ et $R_8$ forment avec l'atome d'azote auquel ils sont rattachés un hétérocycle mono ou polycyclique saturé ou insaturé contenant 4 à 9 atomes de carbone et un ou plusieurs hétéroatomes (O, N) et éventuellement substitué par un ou plusieurs radicaux alkyle,

- $R_9$ représente un atome d'hydrogène ou un radical alkyle, cycloalkylalkyle, cycloalkyle, phénylalkyle ou phényle éventuellement substitué par un ou plusieurs substituants choisis parmi les atomes d'halogène et les radicaux alkyle, alcoxy et alkylthio,
- $R_{10}$ représente un radical alkyle, cycloalkylalkyle, cycloalkyle, phénylalkyle ou phényle éventuellement substitué par un ou plusieurs substituants choisis parmi les atomes d'halogène et les radicaux alkyle, alcoxy et alkylthio,

ou bien $R_9$ et $R_{10}$ forment ensemble avec l'atome d'azote auquel ils sont rattachés un hétérocycle mono ou polycyclique saturé ou insaturé contenant 4 à 9 atomes de carbone et un ou plusieurs hétéroatomes (O, N, S) et éventuellement substitué par un ou plusieurs radicaux alkyle,

- X représente un atome d'hydrogène, un radical alkyle ou phénylalkyle,

- alk représente un radical alkyle ou alkylène,
- alk′ représente un radical hydroxyalkyle, hydroxyalkylène, alkoxyalkyle ou alkoxyalkylène.

Dans les définitions qui précèdent et celles qui seront citées ci-après, sauf mention contraire, les radicaux alkyle, alkylène et alcoxy et les portions alkyle, alkylène et alcoxy contiennent 1 à 4 atomes de carbone en chaîne droite ou ramifiée, les radicaux ou portions acyle contiennent 2 à 4 atomes de carbone et les radicaux et portions cycloalkyle contiennent 3 à 6 atomes de carbone.

Lorsque $R_7$ et $R_8$ forment avec l'atome d'azote auquel ils sont rattachés un hétérocycle, celui-ci est de préférence un cycle pipéridino éventuellement substitué par un ou plusieurs radicaux alkyle ou un cycle tétrahydro-1,2,3,4 quinoléine.

Lorsque $R_9$ et $R_{10}$ forment avec l'atome d'azote auquel ils sont rattachés un hétérocycle, celui-ci est de préférence un cycle pipéridino, perhydroazépinyl-1, tétrahydro-1,2,3,6 pyridyl-1, tétrahydro-1,2,3,4 quinolyl-1, pyrrolidinyl-1, tétrahydro-1,2,3,4 isoquinolyl-2, thiomorpholino ou indolyl-1, ces cycles pouvant être éventuellement substitués par au moins un radical alkyle.

Les composés de formule (I) comportant un ou plusieurs centres asymétriques présentent des formes isomères. Les racémiques et les énantiomères de ces composés font également partie de l'invention.

Les composés de formule (I) pour lesquels R représente un radical méthylène, éthylène, CHOH, $CHR_6$ ou un atome de soufre et $R_3$ représente un radical phénylamino dont le noyau phényle est éventuellement substitué peuvent être préparés par action d'un dérivé réactif de l'acide carbamique, obtenu éventuellement in situ par action d'un dérivé réactif de l'acide carbonique choisi parmi le N,N′-diimidazole carbonyle, le phosgène, le diphosgène et le chloroformiate de p-nitrophényle sur un dérivé de formule :

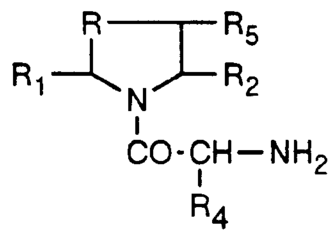

(II)

dans laquelle R représente un radical méthylène, éthylène, CHOH, $CHR_6$ ou un atome de soufre et $R_1$, $R_2$, $R_4$, $R_6$ et $R_6$ ont les mêmes significations que dans la formule (I), sur une aniline dont le noyau phényle est éventuellement substitué par un ou plusieurs substituants choisis parmi les atomes d'halogène et les radicaux alkyle, alcoxy, alkylthio, trifluorométhyle, carboxy, alcoxycarbonyle, hydroxy, nitro, amino, acyle, cyano, sulfamoyle, carbamoyle, hydroxyiminoalkyle, alcoxyiminoalkyle, hydroxyaminocarbonyle, alcoxyamino-carbonyle, tétrazolyl-5, tétrazolyl-5 alkyle, trifluorométhylsulfonamido, alkylsulfinyle, mono ou polyhydroxyalkyle, sulfo, -alk-O-CO-alk, -alk-COOX, -alk-O-alk, -alk′-COOX, -O-alk-COOX, -CH=CH-COOX, -CO-COOX, -alk-$SO_3H$, -CH=CH-alk′, -C(=NOH)-COOX, -S-alk-COOX, -O-$CH_2$-alk′-COOX, -CX=N-O-alk-COOX ou -alk-N(OH)-CO-alk ou diméthyl-2,2 dioxo-4,6 dioxanne-1,3-yl-5.

Cette réaction s'effectue généralement au sein d'un solvant inerte tel que le tétrahydrofuranne, le diméthylformamide, un solvant chloré (chloroforme, dichloro-1,2 éthane par exemple) ou un solvant aromatique (benzène, toluène par exemple), à une température comprise entre 20°C et la température d'ébullition du solvant.

Le dérivé réactif de l'acide carbamique peut être obtenu dans les mêmes conditions de solvant et de température.

Les dérivés de formule (II) peuvent être obtenus par déprotection d'un dérivé de formule :

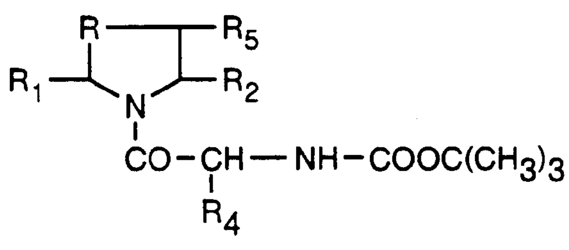

(III)

dans laquelle R représente un radical méthylène, éthylène, CHOH, $CHR_6$ ou un atome de soufre et $R_1$, $R_2$, $R_4$, $R_6$ et $R_6$ ont les mêmes significations que dans la formule (I).

Cette déprotection s'effectue de préférence au moyen d'iodotriméthylsilane, au sein d'un solvant inerte tel qu'un solvant chloré (chloroforme, dichloro-1,2 éthane par exemple), à une température comprise entre 15 et 40°C.

Les dérivés de formule (III) peuvent être obtenus par action d'un dérivé de formule :

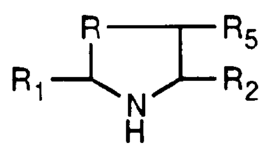

(IV)

dans laquelle R représente un radical méthylène, éthylene, CHOH, $CHR_6$ ou un atome de soufre et $R_1$, $R_2$, $R_5$ et $R_6$ ont les mêmes significations que dans la formule (I), sur un acide de formule :

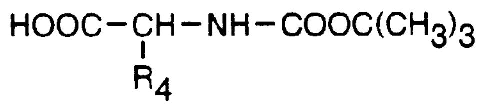

(V)

dans laquelle $R_4$ est défini comme dans la formule (I).

Cette réaction s'effectue au sein d'un solvant inerte tel que l'acétonitrile, le tétrahydrofuranne ou un solvant chloré, en présence d'un agent de condensation utilisé en chimie peptidique tel qu'un carbodiimide (N,N'-dicyclohexyl-carbodiimide par exemple) ou un chloroformiate d'alkyle, à une température comprise entre 10 et 40°C.

Les dérivés de formule (V) peuvent être obtenus selon les méthodes habituelles de protection des amino acides.

Les dérivés de formule (IV) peuvent être préparés par application ou adaptation des méthodes décrites dans la littérature et des méthodes décrites ci-dessous.

Les dérivés de formule (IV) pour lesquels $R_2$ représente un radical alcoxycarbonyle, cycloalkyloxycarbonyle ou cycloalkylalkyloxycarbonyle peuvent être obtenus par estérification d'un acide de formule :

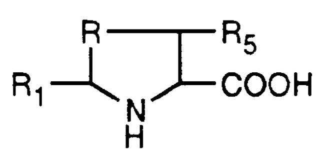

(VI)

dans laquelle R représente un radical méthylène, éthylène, CHOH, $CHR_5$ ou un atome de soufre et $R_1$, $R_5$ et $R_5$ ont les mêmes significations que dans la formule (I).

Cette estérification s'effectue généralement au moyen d'un alcool $R_{13}$-OH dans lequel $R_{13}$ représente un radical alkyle, cycloalkyle ou cycloalkylalkyle, en milieu acide, à la température d'ébullition du mélange réactionnel. Pour les composés de formule (IV) pour lesquels $R_2$ représente un radical tert-butoxycarbonyle on fait réagir l'isobutène, sur un produit de formule (VI) au sein d'un solvant inerte tel qu'un solvant chloré, en présence d'un acide tel que l'acide sulfurique, à une température voisine de 20°C.

Les dérivés de formule (VI) pour lesquels R représente un radical méthylène et $R_1$ et $R_5$ sont définis comme dans la formule (I) peuvent être préparés par application ou adaptation de la méthode décrite par H.GERSHON et coll., J. Org. Chem., 26, 2347 (1961).

Les dérivés de formule (VI) pour lesquels R représente un radical $CHR_6$ et $R_1$, $R_5$ et $R_5$ sont définis comme dans la formule (I) peuvent être préparés par application ou adaptation de la méthode décrite par J. K. THOTTATHIL et coll., Tetrahedron Letters, 27, 151 (1986) et D.R. KRONENTHAL et coll., Tetrahedron Letters, 31, 1241 (1990)

Les dérivés de formule (VI) pour lesquels R représente un radical méthylène, $R_1$ représente un atome d'hydrogène et $R_5$ représente un radical phényle peuvent être préparés par application ou adaptation de la méthode décrite par Y.N. BELOKON et coll., J. Chem. Soc. Perkin Trans 1, 2075 (1988) et J. RIVIER et G.R. MARSHALL, Peptides, Chemistry, Structure and biology, Proceedings of the Eleventh American Peptide Symposium, July 9-14, 1989 - La Jolla California USA - ESCOM Leiden 1990.

Les dérivés de formule (VI) pour lesquels R représente un atome de soufre $R_1$ est défini comme dans la formule (I) et $R_5$ représente un atome d'hydrogène peuvent être obtenus par action d'un dérivé de formule :

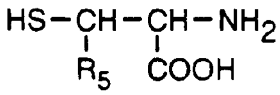

(VII)

dans laquelle $R_5$ représente un atome d'hydrogène, sur un aldéhyde de formule :

$R_1$-CHO (VIII)

dans laquelle $R_1$ a les mêmes significations que dans la formule (I).

Cette réaction s'effectue de préférence au sein d'un alcool, à la température d'ébullition du milieu réactionnel.

Les dérivés de formule (VI) pour lesquels R représente un radical éthylène, $R_1$ est défini comme dans la formule (I) et $R_5$ représente un atome d'hydrogène, peuvent être préparés par réduction des dérivés de formule :

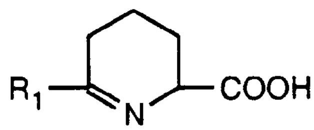

(IX)

dans laquelle $R_1$ a les mêmes significations que dans la formule (I).

Cette réduction s'effectue généralement au moyen d'hydrogène, au sein d'un solvant inerte tel qu'un alcool, en présence d'un catalyseur tel que l'oxyde de platine, à une température comprise entre 20 et 100°C, éventuellement sous pression ou au moyen de borohydrure de sodium et de carbonate de potassium au sein d'un mélange eau-alcool (éthanol, de préférence), à une température comprise entre 0 et 20°C.

Les dérivés de formule (IX) peuvent être obtenus par action d'un acétamidomalonate d'alkyle sur un dérivé de formule :

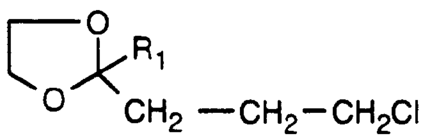

(X)

dans laquelle $R_1$ a les mêmes significations que dans la formule (I) suivi de l'hydrolyse, de la décarboxylation et de la déshydratation du produit obtenu en opérant par chauffage dans l'acide chlorhydrique aqueux, l'action de l'acétamidomalonate d'alkyle sur le produit de formule (X) étant effectuée au sein d'un alcool, en présence d'une base telle qu'un alcoolate de métal alcalin, à la température d'ébullition du solvant.

Les dérivés de formule (X) peuvent être obtenus par application ou adaptation de la méthode décrite par M. T. WILLS et coll., J. Org. Chem., 45 (12), 2495 (1980).

Les dérivés de formule (IV) peuvent également être obtenus par déprotection d'un dérivé de formule :

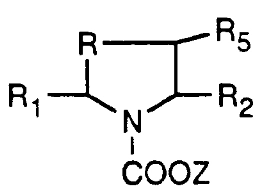

(XI)

dans laquelle R représente un radical méthylène, éthylène, CHOH, $CHR_6$ ou un atome de soufre, $R_1$ $R_2$, $R_5$ et $R_5$ ont les mêmes significations que dans la formule (I) et Z représente un radical alkyle et de préférence tert.butyle, étant entendu que lorsque $R_2$ représente un radical tert-butoxycarbonyle, Z ne peut pas être méthyle ou éthyle.

Cette réaction s'effectue au sein d'un solvant inerte tel qu'un solvant chloré, au moyen d'iodotriméthylsilane, à une température comprise entre 15°C et la température d'ébullition du milieu réactionnel.

Les dérivés de formule (XI) pour lesquels R représente un radical méthylène, $R_1$ représente un radical phényle, thiènyl-2 éventuellement substitué, furyl-2 éventuellement substitué ou indolyl-3 éventuellement substi-

tué, $R_2$ représente un radical alcoxycarbonyle, cycloalkyloxycarbonyle, cycloalkylalkyloxycarbonyle et $R_5$ représente un atome d'hydrogène, peuvent être obtenus par action d'un dérivé de formule :

$R_1H$ (XII)

dans laquelle $R_1$ représente un radical phényle, thiènyl-2 éventuellement substitué, furyl-2 éventuellement substitué ou indolyl-3 éventuellement substitué, sur un dérivé de formule :

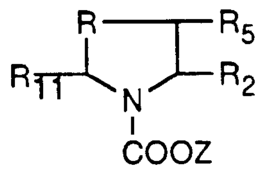

(XIII)

dans laquelle R représente un radical méthylène, $R_2$ et $R_5$ ont les mêmes significations que ci-dessus, $R_{11}$ représente un radical alcoxy contenant 1 ou 2 atomes de carbone et Z représente un radical alkyle.

Cette réaction s'effectue généralement en présence d'un acide fort tel que l'acide p-toluènesulfonique ou d'un acide de Lewis tel que le trichlorure d'aluminium, éventuellement au sein d'un solvant inerte tel qu'un solvant aromatique, à une température comprise entre 20°C et la température d'ébullition du milieu réactionnel.

Les dérivés de formule (XIII) peuvent être préparés par application ou adaptation de la méthode décrite par T. SHONO et coll., J. Am. Chem. Soc., 104, 6697 (1982).

Les dérivés de formule (XI) pour lesquels $R_2$ représente un radical alcoxycarbonyle, cycloalkyloxycarbonyle ou cycloalkylalkyloxycarbonyle et Z représente un radical tert.butyle peuvent être préparés par estérification d'un acide de formule :

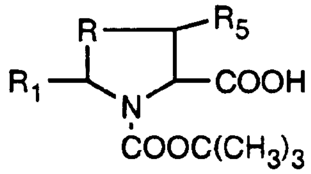

(XIV)

dans laquelle R représente un radical méthylène, éthylène, CHOH, $CHR_6$ ou un atome de soufre, $R_1$, $R_5$ et $R_5$ ont les mêmes significations que dans la formule (I).

Cette estérification s'effectue dans les conditions décrites précédemment pour l'estérification des acides de formule (VI) ou au moyen d'un alcool, en présence de chlorure de tosyle, au sein de la pyridine.

Les acides de formule (XIV) peuvent être obtenus par action de dicarbonate de ditert-butyle sur un acide de formule (VI).

Cette réaction s'effectue au sein d'un solvant inerte tel que l'eau, le dioxanne ou un mélange de ces solvants, en présence d'un carbonate de métal alcalin, à une température voisine de 20°C.

Les dérivés de formule (XI) pour lesquels $R_2$ représente un reste $-CONR_9R_{10}$ et Z représente un radical tert.butyle peuvent être obtenus par réaction d'un acide de formule (XIV) ou un dérivé réactif de cet acide sur une amine de formule :

$HNR_9R_{10}$ (XV)

dans laquelle $R_9$ et $R_{10}$ ont les mêmes significations que dans la formule (I).

Lorsque l'on met en oeuvre l'acide, on opère en présence d'un agent de condensation utilisé en chimie peptidique tel qu'un carbodiimide (par exemple le N,N'-dicyclohexylcarbodiimide) ou le N,N'-diimidazole carbonyle, dans un solvant inerte tel qu'un éther (tétrahydrofuranne, dioxanne par exemple), un amide (diméthylformamide) ou un solvant chloré (chlorure de méthylène, dichloro-1,2 éthane, chloroforme par exemple) à une température comprise entre 0°C et la température de reflux du mélange réactionnel.

Lorsque l'on met en oeuvre un dérivé réactif de l'acide, il est possible de faire réagir l'anhydride, un anhydride mixte ou un ester (qui peut être choisi parmi les esters activés ou non de l'acide).

On opère alors soit en milieu organique, éventuellement en présence d'un accepteur d'acide tel qu'une base organique azotée (trialkylamine, pyridine, diaza-1,8 bicyclo [5.4.0] undécène-7 ou diaza-1,5 bicyclo [4.3.0] nonène-5 par exemple), dans un solvant tel que cité ci-dessus, ou un mélange de ces solvants, à une température comprise entre 0°C et la température de reflux du mélange réactionnel, soit en milieu hydroorganique biphasique en présence d'une base alcaline ou alcalino-terreuse (soude, potasse) ou d'un carbonate ou bicarbonate d'un métal alcalin ou alcalinoterreux à une température comprise entre 0 et 40°C.

Les dérivés de formule (IV) pour lesquels R représente un radical méthylène, $R_1$ est défini comme dans

la formule générale (I) à l'exception des radicaux ou des substituants pouvant être altérés lors d'une réduction (par exemple radical quinolyl ou substituant nitro), $R_2$ représente un radical phényle éventuellement substitué par un ou plusieurs radicaux choisis parmi les radicaux alkyles, alcoxy et hydroxy et $R_5$ représente un atome d'hydrogène peuvent être obtenus par application ou adaptation des méthodes décrites par C.G. OVERBERGER et Coll., J. Amer. Chem. Soc., 91 887 (1969). Cette méthode met en jeu des réductions de pyrroles qui peuvent être obtenus par application ou adaptation des méthodes décrites dans Synthesis, 613 (1991), Tetrahedron Letters 4407-4410 (1986).

Les dérivés de formule (IV) pour lesquels R représente un radical méthylène, $R_1$ représente un radical phényle éventuellement substitué par un ou plusieurs radicaux choisis parmi les radicaux alkyle, alcoxy et hydroxy ou naphtyle éventuellement substitué et $R_2$ représente un radical phényle éventuellement substitué par un ou plusieurs radicaux choisis parmi les radicaux alkyle, alcoxy et hydroxy et $R_5$ représente un atome d'hydrogène peuvent également être obtenus par réaction d'éthylène sur un dérivé de formule :

$$R_1\text{-}CH\text{=}N\text{-}CH_2\text{-}R_2 \qquad (XVI)$$

dans laquelle $R_1$ et $R_2$ ont les mêmes significations que précédemment.

L'éthylène peut être formé in situ par décomposition de tétrahydrofuranne en présence d'une base telle que le butyllithium, à une température comprise entre 0 et 25°C. On peut également ajouter de l'éthylène, en présence de diisopropylamidure de lithium, au sein du tétrahydrofuranne, à une température voisine de 20°C.

Les dérivés de formule (XVI) peuvent être obtenus par action d'un aldéhyde de formule (VIII) dans laquelle $R_1$ a les mêmes significations que précédemment sur une amine de formule :

$$R_2\text{-}CH_2\text{-}NH_2 \qquad (XVII)$$

dans laquelle $R_2$ a les mêmes significations que précédemment.

Cette réaction s'effectue généralement au sein d'un solvant inerte tel qu'un hydrocarbure (benzène, toluène par exemple), un solvant chloré (dichlorométhane, chloroforme par exemple), éventuellement en présence d'acide p-toluène sulfonique, à la température d'ébullition du milieu réactionnel.

Les composés de formule (IV) pour lesquels R représente un radical méthylène ou CHOH, $R_1$ représente un radical pyridyle, naphtyle, quinolyle ou phényle, $R_2$ représente un radical alcoxycarbonyle, cycloalkyloxycarbonyle, cyclalkylalkyloxycarbonyle ou phényle éventuellement substitué par un ou plusieurs substituants choisis parmi les radicaux alkyle, alcoxy ou hydroxy et $R_5$ représente un atome d'hydrogène peuvent être obtenus par réduction d'un dérivé de formule :

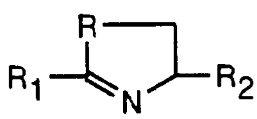

(XVIII)

dans laquelle R, $R_1$ et $R_2$ ont les mêmes significations que précédemment.

Cette réduction s'effectue de préférence au moyen d'hydrogène, en présence d'un catalyseur tel que l'oxyde de platine, au sein d'un solvant inerte tel que l'éthanol, à une température voisine de 20°C, ou au moyen de borohydrure de sodium et de carbonate de potassium au sein d'un mélange eau-alcool (éthanol de préférence), à une température comprise entre 0 et 20°C.

Les dérivés de formule XVIII peuvent être obtenus par application ou adaptation des méthodes décrites par A. MKAIRI et J. HAMELIN, Tetrahedron Letters. 28 1397 (1987), A. VANDER WERF et R.M. KELLOGG, Tetrahedron Letters. 32 3727 (1991), E. KATO et Coll., Chem. Pharm. Bull. 33 4836 (1985), J. ACKERMANN et Coll., Helv. Chim. Acta 73 122 (1990).

Les dérivés de formule (XVIII) peuvent également être obtenus par déprotection et déhydratation d'un dérivé de formule :

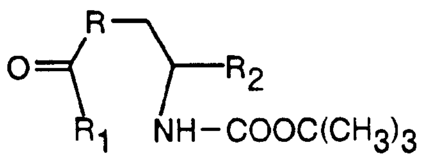

(XIX)

OU

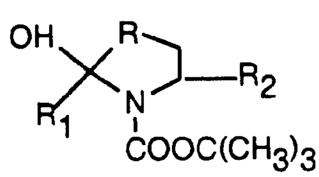

(X X)

dans lesquelles R, $R_1$ et $R_2$ ont les mêmes significations que précédemment, ou d'un mélange de ces dérivés.

Ces déprotection et déshydratation s'effectuent généralement au moyen d'acide trifluoroacétique ou d'iodotriméthylsilane, au sein d'un solvant inerte tel qu'un solvant chloré (dichlorométhane par exemple), à une température voisine de 20°C.

Les dérivés de formule (XIX) et (XX) peuvent être obtenus par action d'un dérivé de formule :

$R_1$-M (XXI)

dans laquelle $R_1$ a les mêmes significations que précédemment et $R_1$-M représente un dérivé organomagnesien, organolithien ou un cuprate, sur un dérivé carbonylé de formule :

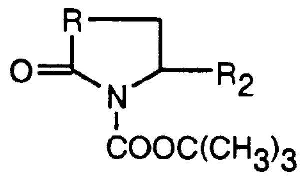

(XXII)

dans laquelle R et $R_2$ ont les mêmes significations que précédemment.

Cette réaction s'effectue au sein d'un solvant inerte tel que le tétrahydrofuranne, à une température comprise entre -78 et 20°C.

Les dérivés de formule (XXII) peuvent être obtenus par application ou adaptation des méthodes décrites par J. ACKERMANN et coll., Helv. Chim. Acta, 73, 122 (1990), T. OHTA, Chem. Lett., 2091 (1987) ou T. OHTA et Coll., Tetrahedron Letters 29 329 (1988). De préférence, on fait réagir le dicarbonate de ditert-butyle sur un dérivé de formule :

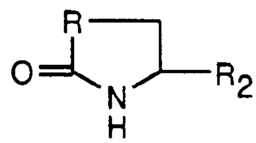

(XXIII)

dans laquelle R et $R_2$ ont les mêmes significations que précédemment.

Cette réaction s'effectue généralement en présence de triéthylamine et de diméthylamino-4 pyridine, au sein d'un solvant chloré tel que le dichlorométhane, à une température voisine de 20°C.

Les dérivés de formule (XXIII) peuvent être obtenus par application ou adaptation des méthodes décrites par T. KOLASA et coll., J. Org. Chem., 55, 1711 (1990), A. L. JOHNSON et coll., J. Med. Chem., 28, 1596 (1985) et B. RIGO et coll., J. Het. Chem., 25, 49 (1988), R.W. ROSENMUND et P. ENGELS, Arch. Pharm. 284, 16 (1951), C.F. KOELSCH et C.H. STRATTON, J. Am. Chem. Soc. 66 1883 (1944), S. WIDEQUIST, ArK. Kemi, Mineral. Geol. 26 1 (1948), J. SINNREICH et D. ELAD, Tetrahedron Letters, 24 4509 (1968), G.R. BROWN et Coll., J. Chem. Soc, Chem Commun, 1973 (1984).

Les dérivés de formule (IV), pour lesquels R représente un radical méthylène, $R_1$ représente un radical pyridyle éventuellement substitué, quinolyle éventuellement substitué, naphtyle éventuellement substitué ou phényle éventuellement substitué par un ou plusieurs substituants choisis parmi les atomes d'halogène et les radicaux alkyle, alcoxy, hydroxy, amino, monoalkylamino, dialkylamino, alcoxycarbonyle, -CO-$NR_7R_8$ ou -NH-CO-$CH_3$, $R_2$ représente un radical alcoxycarbonyle, cycloalkyloxycarbonyle ou cycloalkylalkyloxycarbonyle et $R_8$ représente un atome d'hydrogène peuvent également être obtenus par réduction d'un dérivé de formule :

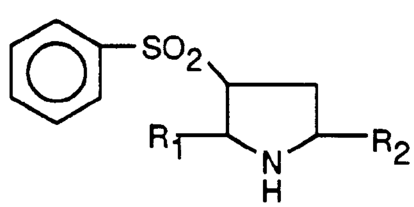

(XXIV)

dans laquelle $R_1$ et $R_2$ ont les mêmes significations que précédemment.

Cette réduction s'effectue généralement au moyen d'un amalgame mercure-sodium, en présence de dihydrogénophosphate de sodium ou d'hydrogénophosphate de sodium, au sein d'un solvant tel qu'un alcool (méthanol par exemple), le tétrahydrofuranne, l'eau ou un mélange de ces solvants, à une température comprise entre -10 et 40°C, ou au moyen de magnésium, au sein d'un solvant inerte tel qu'un alcool (méthanol par

exemple), à une température comprise entre 20°C et la température d'ébullition du milieu réactionnel.

Les dérivés de formule (XXIV) peuvent être obtenus par action d'un dérivé de formule (XVI) dans laquelle $R_1$ et $R_2$ ont les mêmes significations que précédemment sur la phényl vinylsulfone.

Cette réaction s'effectue généralement en présence d'un sel métallique tel que le bromure de lithium ou l'acétate d'argent et d'une trialkylamine telle que la triéthylamine, au sein d'un solvant inerte tel que l'acétonitrile, à une température voisine de 20°C.

Les dérivés de formule (IV) pour lesquels R représente un atome de soufre, $R_1$ est défini comme dans la formule (I), $R_2$ représente un radical phényle et $R_5$ représente un atome d'hydrogène peuvent être obtenus par action d'un dérivé de formule (VIII) sur un amino-2 phényl-2 éthanethiol dont le noyau phényle est éventuellement substitué par un ou plusieurs substituants choisis parmi les radicaux alkyle, alcoxy, hydroxy.

Cette réaction s'effectue généralement au sein d'un solvant inerte tel qu'un alcool , à la température d'ébullition du milieu réactionnel.

Les amino-2 phényl-2 éthanethiols dont le noyau phényle est éventuellement substitué peuvent être préparés par application ou adaptation de la méthode décrite dans le brevet JP 57197 447 qui met en oeuvre des amino-2 phényl-2 éthanols qui sont préparés par application ou adaptation des méthodes décrites par Z.L. KIS et J. MORLY, EP 258 191, J. PLESS, CH 590 820, S. MIYAMOTO et Coll. EP 432 661, J. SUZUKI et Coll. EP 345 775.

Les dérivés de formule (IV) pour lesquels $R_2$ représente un phényle éventuellement substitué par un ou plusieurs substituants choisis parmi les radicaux alkyle, alcoxy ou hydroxy, R représente un radical méthylène, $R_1$ représente un atome d'hydrogène et $R_5$ représente un radical phényle peuvent être préparés par application ou adaptation des méthodes décrites par W.H. PEARSON et Coll., J. Am. Chem. Soc. 114 1329 (1992), O. TSUGE et Coll., Bull. Chem. Soc. Japan 59 2537 (1986).

Ces dérivés peuvent également être préparés par réduction des pyrroles et des pyrrolines correspondants par application ou adaptation des méthodes décrites par C.G. OVERBERGER et Coll. J. Am. Chem. Soc. 91 687 (1969).

Ces pyrroles et ces pyrrolines peuvent être préparés par application ou adaptation des méthodes décrites par M. OHNO et Coll., Tetrahedron Letters 32 5093 (1991), S.C. CHERKOFSKY, US 4 267 184, S.C. CHERKOFSKY et G.A. BOSWELL Jr., EP 25884, O. TSUGE et Coll. Bull. Chem. Soc. Japan 59 1809 (1986).

Les dérivés de formule (IV) pour lesquels R représente un radical éthylène, $R_2$ représente un phényle éventuellement substitué par un ou plusieurs substituants choisis parmi les radicaux alkyle, alcoxy ou hydroxy, $R_5$ représente un atome d'hydrogène et $R_1$ a les mêmes significations que dans la formule (I) peuvent êtrepréparés par application ou adaptation des méthodes décrites par C.G. OVERBERGER et Coll., J. Am. Chem. Soc. 79 6430 (1957), J. THESING et H. MEYER, Ann. 609 46 (1957), D.Y. JACKSON et P.G. SCHULTZ, J. Am. Chem. Soc. 113 2319 (1991), C.G. OVERBERGER et L.P. HERIN, J. Org. Chem. 27 2423 (1962).

Certaines de ces méthodes mettent en oeuvre des réductions de pipéridéines qui peuvent aussi être obtenus par application ou adaptation des méthodes décrites par H. QUAST et B. MUELLER Chem. Ber. 116 3931 (1983), R. WEIL et N. COLLIGNON, C. Rend. Acad. Sci. Ser. C 275 299 (1972) et Bull. Soc. Chim. Fr. 258 (1974).

Les dérivés de formule (IV) pour lesquels $R_2$ représente un phényle éventuellement substitué par un ou plusieurs substituants choisis parmi les radicaux alkyle, alcoxy ou hydroxy, R représente un radical $CHR_5$, $R_1$ et $R_5$ représentent chacun un atome d'hydrogène et $R_6$ représente un radical phényle peuvent être préparés par application ou adaptation des méthodes décrites par M.C. KLOEZEL, J. Am. Chem. Soc. 69 2271 (1947), W. H. PEARSON et Coll., J. Am. Chem. Soc. 114 1329 (1992), O. TSUGE et Coll., Bull. Soc. Japan 59 2537 (1986), M. CARRIOU et Coll., Can. J. Chem. 61 2359 (1983), E. BREWER et D. MELUMAD, J. Org. Chem. 37 3949 (1972).

Certaines de ces méthodes mettent en oeuvre des réductions de pyrroles et de pyrrolines qui peuvent aussi être obtenues par application ou adaptation des méthodes décrites par C.F.H. ALLEN et C.V. WILSON, Org. Synth. Coll. Vol. III 358 (1955), W. DAVEY et D.J. TIVEY, J. Chem. Soc. 2276 (1958), W. CHEN et Coll., Chin. Chem. Lett. 2 439 (1991), S.M. BLOOM et P.P. GARCIA, US 3 883 555 et US 3 691 161.

Les dérivés de formule (III) pour lesquels $R_2$ représente un radical alcoxycarbonyle, cycloalkyloxycarbonyle ou cycloalkylalkyloxycarbonyle peuvent également être obtenus par estérification d'un acide de formule :

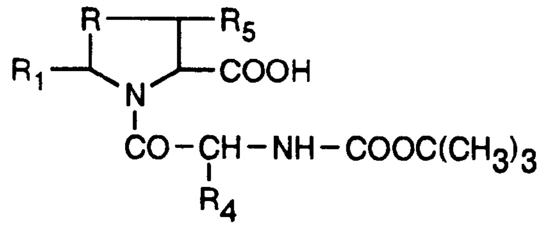

(XXV)

dans laquelle R, $R_1$, $R_4$, $R_5$ et $R_6$ ont les mêmes significations que dans la formule (I).

Cette réaction s'effectue de préférence dans les mêmes conditions que celles décrites précédemment pour l'estérification des composés de formule (V).

Les acides de formule (XXV) peuvent être obtenus par hydrolyse des esters méthyliques ou éthyliques correspondants de formule (III).

Cette hydrolyse s'effectue généralement au sein d'un solvant inerte tel que l'eau, le dioxanne ou un mélange de ces solvants, au moyen d'une base telle qu'un hydroxyde de métal alcalin (soude, potasse), à une température voisine de 20°C.

Les dérivés de formule (III) pour lesquels R représente un radical méthylène, $R_1$ représente un radical pyridyle éventuellement substitué, quinolyle éventuellement substitué, naphtyle éventuellement substitué ou phényle éventuellement substitué par un ou plusieurs substituants choisis parmi les atomes d'halogène et les radicaux alkyle, alcoxy, hydroxy, amino, monoalkylamino, dialkylamino, alcoxycarbonyle, -CO-$NR_7R_8$ ou -NH-CO-$CH_3$, $R_2$ représente un radical alcoxycarbonyle, cycloalkyloxycarbonyle ou cycloalkylalkyloxycarbonyle et $R_5$ représente un atome d'hydrogène peuvent être obtenus par réduction des dérivés de formule :

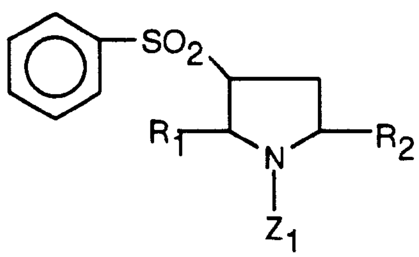

(XXVI)

dans laquelle $Z_1$ représente un radical tert-butoxycarbonyle ou un radical CO-CH($R_4$)-NH-COOC$(CH_3)_3$, $R_1$ et $R_2$ ont les mêmes significations que précédemment et $R_4$ a les mêmes significations que dans la formule (I).

Cette réaction s'effectue dans les mêmes conditions que celles décrites précédemment pour la réduction des dérivés de formule (XXIV).

Les dérivés de formule (XXVI) peuvent être obtenus par action d'un acide de formule (V) ou de di-carbonate de di-tert-butyle, selon le cas, sur un dérivé de formule (XXIV).

Cette réaction s'effectue au sein d'un solvant inerte tel que l'acétonitrile, le tétrahydrofuranne ou un solvant chloré, en présence d'un agent de condensation utilisé en chimie peptidique tel qu'un carbodiimide (N,N'-dicyclohexyl-carbodiimide par exemple) ou un chloroformiate d'alkyle, à une température comprise entre 10 et 40°C.

Les anilines éventuellement substituées sont commercialisées ou peuvent être obtenues par application ou adaptation des méthodes décrites par R. SCHRÖTER, Methoden der organischen Chemie, Houben Weil, Band XI/1, p 360; G.J. ESSELEN et coll., J. Am. Chem. Soc., 36, 322 (1914); G. ADRIANT et coll., Bull. Soc. Chim. FR, 1511 (1970); W.A. JACOBS et coll., J. Am. Chem. Soc., 39, 2438 (1917) et J. Am. Chem. Soc., 39, 1438 (1917) et dans les exemples.

Les composés de formule (I) pour lesquels R représente un radical méthylène, éthylène, CHOH, $CHR_6$ ou un atome de soufre et $R_3$ représente un radical phénylamino dont le noyau phényle est éventuellement substitué par un ou plusieurs substituants choisis parmi les atomes d'halogène et les radicaux alkyle, alcoxy, alkylthio, trifluorométhyle, nitro, acyle, cyano, sulfamoyle, alcoxycarbonyle, -alk-O-alk, trifluorométhylsulfonamido, alk-$SO_3H$, sous forme de sel, alk-COOX ou alk'-COOX dans lesquels X est différent d'un atome d'hydrogène peuvent également être préparés par action d'un dérivé de formule (II), sur un phénylisocyanate dont le noyau phényle est éventuellement substitué par un ou plusieurs substituants choisis parmi les atomes d'halogène et les radicaux alkyle, alcoxy, alkylthio, trifluorométhyle, nitro, acyle, cyano, sulfamoyle, alcoxycarbonyle, -alk-O-alk, trifluorométhylsulfonamido ou alk-$SO_3H$, sous forme de sel.

Cette réaction s'effectue généralement au sein d'un solvant inerte tel que le tétrahydrofuranne, le diméthylformamide, un solvant chloré (chloroforme, dichloro-1,2 éthane par exemple), un solvant aromatique (benzène, toluène par exemple), à une température comprise entre 10°C et la température d'ébullition du solvant.

Les phénylisocyanates sont commercialisés ou peuvent être obtenus par application ou adaptation des méthodes décrites par R. RICHTER et coll., The Chemistry of Cyanate and their thio derivatives, S. PATAI, part 2, Wiley New York (1977) et dans les exemples.

Les composés de formule (I) pour lesquels R représente un radical méthylène, éthylène, CHOH, $CHR_6$ ou un atome de soufre et $R_3$ représente un radical phénylamino dont le noyau phényle est substitué par un radical carboxy, -alk-COOH, -O-alk-COOH, -alk'-COOH, -CH=CH-COOH, -CO-COOH, -S-alk-COOH, -C(=NOH)-COOH, $OCH_2$alk'COOH ou CX = N-O-alk-COOH et $R_1$, $R_2$, $R_5$ et $R_5$ sont définis comme dans la formule (I) peuvent également être préparés par hydrolyse ou, selon le cas, hydrogénolyse des esters correspondants.

Lorsque l'on utilise les esters d'alkyle ou de phénylalkyle, il est avantageux d'effectuer l'hydrolyse au moyen d'une base telle que la soude, la potasse ou l'hydroxyde de lithium, au sein d'un solvant inerte tel que le tétrahydrofuranne, le dioxanne, l'eau ou un mélange de ces solvants, à une température comprise entre 20°C et 40°C. Lorsque l'on utilise un ester de triméthylsilyléthyle, il est avantageux d'opérer au sein d'un solvant inerte tel que le tétrahydrofuranne, au moyen d'un fluorure tel que le fluorure de tétrabutylammonium, à une température comprise entre 10 et 40°C. Lorsque l'on utilise des esters de phénylalkyle, il est peut être aussi avantageux d'effectuer une hydrogénolyse au moyen d'hydrogène ou de formiate d'ammonium en présence d'un catalyseur tel que le palladium sur charbon dans un solvant tel que le méthanol ou l'acétate d'éthyle.

Les esters de triméthylsilyléthyle peuvent être obtenus par application ou adaptation de la méthode décrite dans les exemples.

Les composés de formule (I) pour lesquels R représente un radical méthylène, éthylène, CHOH, $CHR_6$ ou un atome de soufre et $R_3$ représente un radical phénylamino dont le noyau phényle est éventuellement substitué par un radical hydroxyiminoalkyle ou alcoxyiminoalkyle, $R_1$, $R_2$, $R_5$ et $R_6$ sont définis comme dans la formule (I) peuvent également être préparés par action du dérivé acylé correspondant sur un dérivé de formule :

$$H_2N - OR_{12} \qquad (XXVII)$$

dans laquelle $R_{12}$ représente un atome d'hydrogène ou un radical alkyle.

Cette réaction s'effectue généralement au sein d'un solvant inerte tel qu'un alcool (méthanol, éthanol par exemple), l'eau ou un mélange de ces solvants, à la température d'ébullition du solvant et éventuellement en présence d'une base telle que la pyridine.

Les composés de formule (I) pour lesquels R représente un radical méthylène, éthylène, CHOH, $CHR_6$ ou un atome de soufre et $R_3$ représente un radical phényle éventuellement substitué, naphtyle, indolyle, quinolyle éventuellement substitué ou phénylamino dont le noyau phényle est éventuellement substitué par un ou plusieurs substituants choisis parmi les atomes d'halogène et les radicaux alkyle, alcoxy, alkylthio, trifluorométhyle, nitro, acyle, cyano, sulfamoyle, alcoxycarbonyle, -alk-O-alk, alk-COOX ou alk'-COOX dans lesquels X est différent d'un atome d'hydrogène trifluorométhylsulfonamido et $R_1$, $R_2$, $R_5$ et $R_6$ sont définis comme dans la formule (I) peuvent également être préparés par action d'un dérivé de formule (IV) sur un acide de formule :

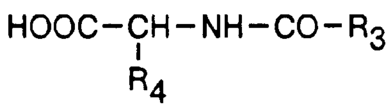

(XXVIII)

dans laquelle $R_3$ a les mêmes significations que ci-dessus ou un dérivé réactif de cet acide et $R_4$ a les mêmes significations que dans la formule (I).

Cette réaction s'effectue de préférence en présence d'un agent de condensation utilisé en chimie peptidique tel que le carbodimide au sein d'un solvant tel que l'acétonitrile, le tétrahydrofuranne ou un solvant chloré ou au moyen de chlorure thionyle dans le dichlorométhane à une température comprise entre 10° C et la température d'ébullition du solvant.

Les acides de formule (XXVIII) peuvent être obtenus par application ou adaptation de la méthode décrite par J.R. JOHNSON et coll., J. Am. Chem. Soc., 69, 2370 (1947) ou pour les composés pour lesquels R3 représente un radical phénylamino éventuellement substitué, par action d'un phénylisocyanate dont le noyau phényle est éventuellement substitué par un ou plusieurs substituants choisis parmi les atomes d'halogène et les radicaux alkyle, alcoxy, alkylthio, trifluorométhyle, nitro, acyle, cyano, sulfamoyle, alcoxycarbonyle, -alk-O-alk, alk-COOX ou alk'-COOX dans lesquels X est différent d'un atome d'hydrogène ou trifluorométhylsulfonamido sur un dérivé de formule :

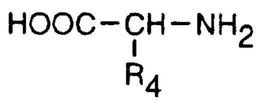 (XXIX)

dans laquelle $R_4$ a les mêmes significations que dans la formule (I).

Cette réaction s'effectue généralement en solution aqueuse en présence d'une base telle qu'un bicarbonate de métal alcalin ou dans le dioxanne aqueux, à une température voisine de 20°C.

Les composés de formule (I) pour lesquels R représente un radical SO ou $SO_2$, $R_1$, $R_2$ et $R_5$ sont définis comme dans la formule générale (I) peuvent être préparés par oxydation des composés de formule (I) correspondants pour lesquels R représente un atome de soufre, étant entendu que les autres radicaux et les autres substituants sont choisis de telle manière qu'ils soient insensibles aux conditions de la réaction.

Cette oxydation s'effectue généralement au moyen d'oxone[R] (peroxymonosulfate de potassium) commercialisé par Aldrich, au sein d'un alcool tel que le méthanol ou un mélange méthanol-eau, à une température voisine de 25°C.

Il est entendu pour l'homme de métier que, pour la mise en oeuvre des procédés selon l'invention décrits précédemment, il peut être nécessaire d'introduire des groupes protecteurs des fonctions amino, hydroxy, carboxy afin d'éviter des réactions secondaires. Les fonctions amino peuvent par exemple être bloquées sous forme de carbamates de tert-butyle ou de méthyle puis régénérées au moyen d'iodotriméthylsilane ou de carbamates de benzyle puis régénérées par hydrogénation après avoir mis en oeuvre le procédé selon l'invention. Les fonctions hydroxy peuvent par exemple être bloquées sous forme de benzoate puis régénérées par hydrolyse en milieu alcalin après avoir mis en oeuvre le procédé selon l'invention.

Les énantiomères des composés de formule (I) contenant au moins un site asymétrique peuvent être obtenus par dédoublement des racémiques par exemple par chromatographie sur colonne chirale ou par synthèse à partir des précurseurs chiraux.

Comme phase chirale, on utilise de préférence une phase dont le sélecteur chiral, qui est, de préférence, la dinitro-3,5 benzoyl-D-phénylglycine, est éloigné de la silice par un bras aminoalcanoyle contenant 3 à 14 atomes de carbone fixé sur les fonctions amines d'une silice aminopropyle et dont les fonctions silanols libres sont bloquées par des radicaux trialcoysilyles.

Cette phase chirale, qui constitue un autre objet de la présente invention, peut être définie par la structure suivante ;

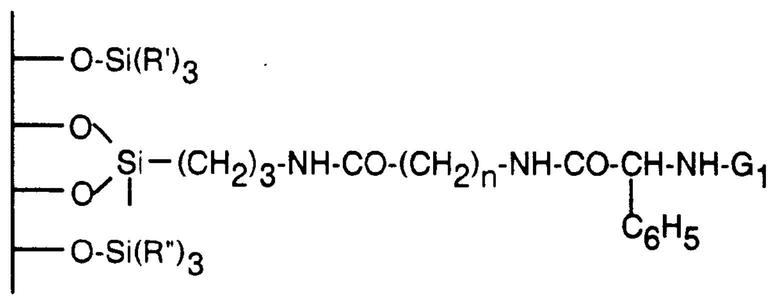 (XXX)

dans laquelle les symboles R′, identiques ou différents, et R″, identiques ou différents, représentent des radicaux alcoyles contenant 1 à 10 atomes de carbone, $G_1$ représente un groupement électro-attracteur et n représente un nombre entier compris entre 3 et 13 inclusivement.

De préférence, l'un des symboles R′ représente un radical alcoyle contenant 7 à 10 atomes de carbone et les deux autres représentent un radical alcoyle contenant 1 à 2 atomes de carbone et de préférence un radical méthyle, les symboles R″ sont identiques et représentent un radical méthyle ou éthyle, $G_1$ représente un radical benzoyle éventuellement substitué, de préférence, par un ou plusieur radicaux nitro tels que le radical dinitro-3,5 benzoyle et n est égal à 10.

La nouvelle phase chirale selon l'invention peut être préparée par action sur une silice aminopropyle de l'anhydride d'un acide aminoalcanoïque contenant 3 à 14 atomes de carbone dont la fonction amine est protégée par un groupement protecteur tel que le radical tert.butoxycarbonyle, suivie du blocage d'une partie des fonctions silanols par des radicaux $Si(R')_3$ tels que définis précédemment, puis, après élimination du groupement protecteur de la fonction amine, de l'amidification au moyen de D-phénylglycine dont la fonction amine est protégée par un groupement électroattracteur $G_1$ tel que défini précédemment, et enfin du blocage des fonctions silanols résiduelles par des radicaux $Si(R'')_3$ tels que définis précédemment.

Généralement, l'action de l'anhydride d'un acide aminoalcanoïque protégé sur la silice aminopropyle est

effectuée en opérant dans un solvant organique anhydre tel que le diméthylformamide à une température voisine de 20° C.

Le blocage des fonctions silanols par des groupements $-Si(R_3)$ tel que définis précédemment est effectuée par action d'un halogénotrialkylsilane sur la silice aminopropyle greffée par des restes aminoalcanoylés en opérant dans un solvant organique tel que le chlorure de méthylène en présence d'un agent basique tel que la pyridine.

L'élimination des groupements protecteur des restes aminoalcanoylés s'effectue généralement, lorsque le groupement protecteur est un radical tert.butoxycarbonyle, par action de l'acide trifluoroacétique dans un solvant organique tel que le chlorure de méthylène.

L'amidification au moyen de la D-phénylglycine dont la fonction amine est protégée est effectuée en présence d'un agent de condensation tel que la N-éthoxycarbonyl éthoxy-2 dihydro-1,2 quinoléine en opérant dans un solvant organique anhydre tel que le diméthylformamide.

Le blocage des fonctions silanols résiduelles par des radicaux - $Si(R')_3$ tels que définis précédemment est généralement effectuée au moyen de trialkylsilylimidazole en opérant dans un solvant organique tel que le chlorure de méthylène.

Les composés de formule (I) peuvent être purifiés par les méthodes connues habituelles, par exemple par cristallisation, chromatographie ou extractions.

Les composés de formule (I) peuvent être éventuellement transformés en sels d'addition avec un acide minéral ou organique par action d'un tel acide au sein d'un solvant organique tel qu'un alcool, une cétone, un éther ou un solvant chloré.

Les composés de formule (I) comportant un reste carboxy, sulfo ou alk-$SO_3H$ peuvent également être transformés en sels métalliques ou en sels d'addition avec les bases azotées selon des méthodes connues en soi. Ces sels peuvent être obtenus par action d'une base métallique (alcaline ou alcalinoterreuse par exemple), de l'ammoniac, d'une amine ou d'un sel d'un acide organique sur un composé de formule (I), dans un solvant. Le sel formé est séparé par les méthodes habituelles.

Ces sels font également partie de l'invention.

Comme exemples de sels pharmaceutiquement acceptables, peuvent être cités les sels d'addition avec les acides minéraux ou organiques (tels que acétate, propionate, succinate, benzoate, fumarate, maléate, oxalate, méthanesulfonate, iséthionate, théophyllinacétate, salicylate, méthylène-bis-β-oxynaphtoate, chlorhydrate, sulfate, nitrate et phosphate), les sels avec les métaux alcalins (sodium, potassium, lithium) ou avec les métaux alcalinoterreux (calcium, magnésium), le sel d'ammonium, les sels de bases azotées (éthanolamine, triméthylamine, méthylamine, benzylamine, N-benzyl-β-phénéthylamine, choline, arginine, leucine, lysine, N-méthyl glucamine).

Les composés de formule (I) présentent des propriétés pharmacologiques intéressantes. Ces composés possèdent une forte affinité pour les récepteurs de la cholécystokinine (CCK) et de la gastrine et sont donc utiles dans le traitement et la prévention des désordres liés à la CCK et à la gastrine au niveau du système nerveux et de l'appareil gastrointestinal.

C'est ainsi que ces composés peuvent être utilisés pour le traitement ou la prévention des psychoses, des troubles anxieux, de la maladie de Parkinson, de la diskinésie tardive, du syndrôme du colon irritable, de la pancréatite aiguë, des ulcères, des désordres de la motilité intestinale, de certaines tumeurs sensibles à la CCK et comme régulateur de l'appétit.

Ces composés ont également un effet de potentialisation sur l'activité analgésique des médicaments narcotiques et non narcotiques. En outre, ils peuvent avoir un effet analgésique propre.

Par ailleurs, les composés ayant une forte affinité pour les récepteurs CCK modifient les capacités de mémorisation. En conséquence, ces composés peuvent être efficaces dans les troubles de la mémoire.

L'affinité des composés de formule (I) pour les récepteurs CCK a été déterminée selon une technique inspirée de celle de A. SAITO et coll . (J. Neuro. Chem., 37, 483-490 (1981)) au niveau du cortex cérébral et au niveau du pancréas.

Dans ces tests, la $CI_{50}$ des composés de formule (I) est généralement inférieure ou égale à 1000 nM.

Par ailleurs, il est connu que les produits qui reconnaissent les récepteurs centraux de la CCK ont une spécificité similaire pour les récepteurs de la gastrine dans le tractus gastrointestinal (BOCK et coll., J. Med. Chem., 32, 16-23 (1989); REYFELD et coll., Am. J. Physiol., 240, G255-266 (1981); BEINFELD et coll., Neuropetides, 3,411-427 (1983).

Les composés de formule (I) présentent une toxicité faible. Leur $DL_{50}$ est généralement supérieure à 40 mg/kg par voie sous cutanée chez la souris.

D'un intérêt particulier sont les composés de formule (I) pour lesquels R représente un radical méthylène, un atome de soufre ou un radical SO, $R_1$ représente un radical phényle éventuellement substitué, $R_2$ représente un radical phényle ou alcoxycarbonyle, $R_4$ et $R_5$ représente un atome d'hydrogène, $R_3$ représente un radical

phénylamino dont le noyau phényle est substitué par un radical carboxy, -alk-COOH, -S-alk-COOH, hydroxyalkyle, alk'-COOH ou alk$SO_3^-$, hydroxyiminoalkyle. Plus particulièrement intéressants sont les produits de formule (I) dans laquelle $R_1$ et $R_2$ sont en position cis l'un par rapport à l'autre.

D'un intéret particulier sont les composés suivants :

- {[(hydroxy-1 éthyl-(RS))-3 phényl)-3 uréido]-2 acétyl}-1 phényl-5 prolinate de tert-butyle-(2RS,5SR),
- acide {{[tert-butoxycarbonyl-2 phényl-5 pyrrolidinyl-1 (2S,5R))-2 oxo-2 éthyl]-3 uréido}-3 phényl}-2 propionique (forme B)
- acide {{[(tert-butoxycarbonyl-2 phényl-5 pyrrolidinyl-1)-2 oxo-2 éthyl]-3 uréido}-3 phénylthio} acétique-(2RS,5SR),
- acide {[(tert-butoxycarbonyl-4 (fluoro-2 thiazolidinyl-3)-2 oxo-2 éthyl]-3 uréido}-3 phénylacétique-(2R,4R),
- acide {{[(tert-butoxycarbonyl-2 (fluoro-2 phényl)-2 thiazolidinyl-3-(2R,4R))-2 oxo-2 éthyl]-3 uréido}-3 phényl}-2 propionique (forme B),
- {{[(tert-butoxycarbonyl-4 phényl-2 thiazolidinyl-3-(2R,4R)))-2 oxo-2 éthyl]-3 uréido}-3 phényl}-1 éthanesulfonate de potassium-(RS), mélange des formes A et B,
- {{[(tert-butoxycarbonyl-2 phényl-5 pyrrolidinyl-1-(2S,5R))-2 oxo-2 éthyl]-3 uréido}-3 phényl}-1 éthanesulfonate de potassium-(RS),
- {{[(tert-butoxycarbonyl-2 phényl-5 pyrrolidinyl-1)-2 oxo-2 éthyl]-3 uréïdo}-3 phényl-1 méthanesulfonate de potassium-(2S,5R).
- acide {[tert-butoxycarbonyl-2 phényl-5 pyrrolidinyl-1)-2 oxo-2 éthyl]-3 uréido}-3 benzoïque-(2S,5R),
- acide {{[tert-butoxycarbonyl-2 (fluoro-2 phényl)-5 pyrrolidinyl-1]-2 oxo-2 éthyl}-3 uréido}-3 benzoique-(2RS,5SR),
- acide {[(diphényl-2,5 pyrrolidinyl-1)-2 oxo-2 éthyl]-3 uréido}-3 benzoïque-(cis),
- acide {{[(hydroxy-2 phényl)-2 phényl-5 pyrrolidinyl-1]-2 oxo-2 uréido}-3 phénylacétique-(2RS,5SR),
- acide {[(tert-butoxycarbonyl-4 phényl-2 thiazolidinyl-3)-2 oxo-2 éthyl]-3 uréido}-3 phénylacétique-(2R,4R),
- acide {[(tert-butoxycarbonyl-4 phényl-2 thiazolidinyl-3)-2 oxo-2 éthyl]-3 uréido}-3 benzoïque-(2R,4R),
- acide {{[(tert-butoxycarbonyl-4 (fluoro-2 phényl)-2 oxyde-1 thiazolidinyl-3-(1RS,2R,4R))-2 oxo-2 éthyl}-3 uréido}-3 phényl}-2 propionique (forme A),
- acide {[(tert-butoxycarbonyl-4 (difluoro-2,3 phényl)-2 thiazolidinyl-3)-2 oxo-2 éthyl]-3 uréido}-3 phénylacétique-(2R,4R),
- {{[(hydroxyimino-1 éthyl)-3 phényl-(E)]-3 uréido}-2 acétyl}-1 phényl-5 prolinate de tert-butyle-(2RS,5SR).

Les exemples suivants illustrent l'invention sans la limiter.

## Exemple 1

A une suspension de 3,1 g de phényl-5 prolinate de tert-butyle-(2RS,5SR) et de 2,6 g d'acide [(méthyl-3 phényl)-3 uréido]-2 acétique dans 100 $cm^3$ de dichloro-1,2 éthane anhydre chauffée à reflux, on ajoute lentement 0,9 $cm^3$ de chlorure de sulfinyle. Le mélange réactionnel est encore chauffé à reflux pendant 15 minutes puis refroidi à 50°C et neutralisé à pH 7-8 par addition d'une solution aqueuse à 10% d'hydrogénocarbonate de sodium. La phase organique est lavée par 3 fois 50 $cm^3$ d'eau, séchée sur sulfate de magnésium, filtrée et concentrée à sec sous pression réduite. Le produit brut obtenu est purifié par chromatographie sur silice [éluant : dichlorométhane-méthanol (98/2 en volumes)]. Les fractions contenant le produit attendu sont réunies et concentrées à sec sous pression réduite à une température voisine de 45°C. On obtient, après recristallisation dans l'acétonitrile, 1 g de {[(méthyl-3 phényl)-3 uréido]-2 acétyl}-1 phényl-5 prolinate de tert-butyle-(2RS,5SR) fondant à 156°C.

A - L'acide [(méthyl-3 phényl)-3 uréido]-2 acétique peut être préparé de la manière suivante : à une solution de 30 g de glycine et de 53 g d'hydrogénocarbonate de sodium dans 600 $cm^3$ d'eau, on ajoute en 15 minutes 53,2 g d'isocyanate de méthyl-3 phényle. Le mélange réactionnel est agité pendant 4 heures à une température voisine de 25°C puis lavé par 200 $cm^3$ d'acétate d'éthyle et acidifié à pH 1 avec 200 $cm^3$ d'une solution aqueuse d'acide chlorhydrique 4N. Le produit insoluble est séparé par filtration, lavé avec trois fois 80 $cm^3$ d'eau et séché à l'air. On obtient ainsi 72 g d'acide [(méthyl-3 phényl)-3 uréido]-2 acétique fondant à 208°C.

B - Le phényl-5 prolinate de tert-butyle-(2RS,5SR) peut être préparé de la manière suivante : une suspension de 45 g de chlorhydrate de phényl-5 proline-(2RS,5SR) dans 500 $cm^3$ de chloroforme anhydre est agitée et refroidie à une température voisine de 5°C. On ajoute goutte à goutte 5,5 $cm^3$ d'acide sulfurique concentré, puis on sature le mélange réactionnel par de l'isobutène pendant 2 heures sous agitation en maintenant la température à 5°C. Après retour à une température voisine de 20°C, l'agitation est poursuivie

pendant 20 heures. Le mélange réactionnel est ensuite amené à pH 8 avec une solution aqueuse de soude 4N. La phase organique est décantée, lavée par 3 fois 100 $cm^3$ d'eau, séchée sur sulfate de magnésium et concentrée à sec sous pression réduite à une température voisine de 40°C. On obtient ainsi 40 g de phényl-5 prolinate de tert-butyle-(2RS,5SR), sous forme d'une huile orangée, utilisée telle quelle dans les synthèses ultérieures.

Le chlorhydrate de phényl-5 proline-(2RS,5SR) peut être préparé selon la méthode décrite par H. GERSHON et A.SCALA, J. Org. Chem.,26, 2347-50 (1961)

**Exemple 2**

A une solution de 2 g d'(amino-2 acétyl)-1 phényl-5 prolinate de tert-butyle-(2RS,5SR) dans 20 $cm^3$ de tétrahydrofuranne anhydre, on ajoute 0,9 $cm^3$ d'isocyanate de méthoxy-3 phényle. Le mélange réactionnel est agité pendant 20 heures à une température voisine de 25°C. Après évaporation du solvant sous pression réduite à une température voisine de 45°C, le produit brut obtenu est purifié par chromatographie sur silice [éluant : dichlorométhane-méthanol (95/5 en volumes)]. Les fractions contenant le produit attendu sont réunies, puis concentrées à sec sous pression réduite. On obtient, après recristallisation dans l'acétonitrile, 1,9 g de {[(méthoxy-3 phényl)-3 uréido]-2 acétyl}-1 phényl-5 prolinate de tert-butyle-(2RS,5SR) fondant à 174°C.

A - L'(amino-2 acétyl)-1 phényl-5 prolinate de tert-butyle-(2RS,5SR) peut être préparé de la manière suivante : à une solution de 16 g de (tert-butoxycarbonylamino-2 acétyl)-1 phényl-5 prolinate de tert-butyle-(2RS,5SR) dans 150 $cm^3$ de chloroforme, on ajoute goutte à goutte 5,6 $cm^3$ d'iodotriméthylsilane à une température voisine de 25°C. Le mélange réactionnel est agité pendant 20 heures à cette température puis concentré à sec sous pression réduite à une température voisine de 45°C. Le produit brut obtenu est purifié par chromatographie sur silice [éluant : dichlorométhane-méthanol (90/10 en volumes)]. Les fractions contenant le produit attendu sont réunies puis concentrées à sec sous pression réduite. On obtient ainsi 10 g d'(amino-2 acétyl)-1 phényl-5 prolinate de tert-butyle-(2RS,5SR) sous forme d'un produit blanc amorphe, utilisé tel quel dans les synthèses ultérieures.

**RMN du proton** (250 MHz, DMSO $D_6$, d en ppm), 2 rotamères à température ambiante, 1,5 (s, 9H, $C(CH_3)_3$), 1,8 à 2,4 (bm, 4H, $CH_2$-$CH_2$); 2,7, 3,25, 3,45 et 3,6 (bd, 2H, AB, $CH_2CO_2$); 4,3 (bt, 1H, CHN); 5,05 (bm, 1H, CHN); 7,2 à 7,8 (m, 5H, aromatiques).

B - Le (tert-butoxycarbonylamino-2 acétyl)-1 phényl-5 prolinate de tert-butyle-(2RS,5SR) peut être préparé de la manière suivante : à une solution de 11,5 g de phényl-5 prolinate de tert-butyle-(2RS,5SR) et de 8,2 g d'acide tert-butoxycarbonylamino-2 acétique dans 150 $cm^3$ d'acétonitrile anhydre maintenue à une température voisine de 0°C, on ajoute en 30 minutes une solution de 9,6 g de N,N'-dicyclohexylcarbodiimide dans 50 $cm^3$ d'acétonitrile anhydre. Le mélange réactionnel est agité pendant 16 heures à une température voisine de 25°C, puis le produit insoluble est séparé par filtration et lavé par trois fois 30 $cm^3$ de dichlorométhane. Le filtrat est concentré à sec sous pression réduite à une température voisine de 45°C. On obtient, après recristallisation dans le pentane, 16 g de (tert-butoxycarbonylamino-2 acétyl)-1 phényl-5 prolinate de tert-butyle-(2RS,5SR) fondant à 112°C.

**Exemple 3**

A une solution de 1,8 g de N,N'-diimidazole-carbonyle dans 50 $cm^3$ de dichloro-1,2 éthane anhydre, on ajoute lentement une solution de 3,1 g d'(amino-2 acétyl)-1 phényl-5 prolinate de tert-butyle-(2RS,5SR) dans 50 $cm^3$ de dichloro-1,2 éthane anhydre. Le mélange réactionnel est agité pendant 1 heure à une température voisine de 25°C puis on ajoute 1,4 g d'(amino-3 phényl)-1 éthanol-(RS). Le milieu réactionnel est alors chauffé à reflux, sous agitation, pendant 4 heures. Après refroidissement le mélange est lavé par 3 fois 50 $cm^3$ d'eau; la phase organique est séchée sur sulfate de magnésium et le solvant est évaporé à sec sous pression réduite à une température de 45°C. Le résidu huileux obtenu est purifié par chromatographie sur silice [éluant : dichlorométhane-méthanol (95/5 en volumes)] et les fractions contenant le produit attendu sont réunies puis concentrées à sec sous pression réduite. On obtient, après recristallisation dans l'acétonitrile, 1,8 g d' {[(hydroxy-1 éthyl-(RS))-3 phényl)-3 uréido]-2 acétyl}-1 phényl-5 prolinate de tert-butyle-(2RS,5SR) fondant à 160°C.

**Exemple 4**

En opérant d'une manière analogue à celle décrite à l'exemple 3, mais à partir de 1,8 g de N,N'-diimidazole-carbonyle, de 3,1 g d'(amino-2 acétyl)-1 phényl-5 prolinate de tert-butyle-(2RS,5SR) en solution dans 100 $cm^3$ de dichloro-1,2 éthane anhydre et de 1,3 $cm^3$ de méthylthio-3 aniline, on obtient, après recristallisation dans

l'acétonitrile, 1,8 g de {[(méthylthio-3 phényl)-3 uréido]-2 acétyl}- 1 phényl-5 prolinate de tert-butyle-(2RS,5SR) fondant à 163°C.

**Exemple 5**

En opérant d'une manière analogue à celle décrite à l'exemple 3, mais à partir de 1,8 g de N,N'-diimidazole-carbonyle, de 3,1 g d'(amino-2 acétyl)-1 phényl-5 prolinate de tert-butyle-(2RS,5SR) en solution dans 100 $cm^3$ de dichloro-1,2 éthane anhydre et de 1,25 g d'amino-3 phénylméthanol on obtient, après recristallisation dans l'acétonitrile, 1,8 g d' {[(hydroxyméthyl-3 phényl)-3 uréido]-2 acétyl}-1 phényl-5 prolinate de tert-butyle-(2RS,5SR) fondant à 168°C.

**Exemple 6**

En opérant d'une manière analogue à celle décrite à l'exemple 3, mais à partir de 1,8 g de N,N'-diimidazole-carbonyle, de 3,1 g d'(amino-2 acétyl)-1 phényl-5 prolinate de tert-butyle-(2RS,5SR) en solution dans 100 $cm^3$ de dichloro-1,2 éthane anhydre et de 1,5 g d'amino-3 acétophénone, on obtient, après recristallisation dans un mélange cyclohexane-méthanol (9/1 en volumes), 1,1 g d'{[(acétyl-3 phényl)-3 uréido]-2 acétyl}-1 phényl-5 prolinate de tert-butyle-(2RS,5SR) fondant à 122°C.

**Exemple 7**

A une solution de 3 g d'{[(acétyl-3 phényl)-3 uréido]-2 acétyl}-1 phényl-5 prolinate de tert-butyle-(2RS,5SR) dans 12 $cm^3$ de méthanol et 6 $cm^3$ de pyridine, on ajoute 0,5 g de chlorhydrate d'hydroxylamine en solution dans 6 $cm^3$ d'eau . Le mélange réactionnel est chauffé à reflux pendant 2 heures. Après évaporation des solvants sous pression réduite à une température voisine de 45°C, le résidu est repris par 100 $cm^3$ d'acétate d'éthyle et la phase organique est lavée par 3 fois 50 $cm^3$ d'eau, séchée sur sulfate de magnésium, filtrée et concentrée à sec sous pression réduite. Le produit brut obtenu est purifié par chromatographie sur silice [éluant : dichlorométhane-méthanol (95/5 en volumes)]. Les fractions contenant le produit attendu sont réunies et concentrées à sec sous pression réduite à une température voisine de 40°C. On obtient, après recristallisation dans l'acétonitrile, 1,1 g d'{{[(hydroxyimino-1 éthyl)-3 phényl]-3 uréido}-2 acétyl}-1 phényl-5 prolinate de tert-butyle-(E)-(2RS,5SR) fondant à 118°C.

**Exemple 8**

En opérant d'une manière analogue à celle décrite à l'exemple 3, mais à partir de 12,5 g de N,N'-diimidazole-carbonyle, de 21,3 g d'(amino-2 acétyl)-1 phényl-5 prolinate de tert-butyle-(2RS,5SR) en solution dans 400 $cm^3$ de dichloro-1,2 éthane anhydre et de 10,5 $cm^3$ d'amino-3 benzoate d'éthyle, on obtient 24,8 g d'{[(éthoxycarbonyl-3 phényl)-3 uréido]-2 acétyl}-1 phényl-5 prolinate de tert-butyle-(2RS,5SR) sous forme de meringue blanche [RMN du proton (250 MHz, DMSO $D_6$, $\delta$ en ppm,), 2 rotamères à température ambiante, coalescence des raies à 120°C, description générale valable pour les autres produits de la série : 1,35 (t, 3H, $CH_3$ de l'éthyle); 1,50 (bs, 9H, $(CH_3)_3$); 1,90 à 2,5 (m, 4H, H en 3 et 4 sur la pyrrolidine); 3,9 et 3,5 (ABX, 2H, $CH_2N$); 4,3 (q, 2H, $CH_2O$); 4,5 (vbdd, 1H, H en 2 sur la pyrrolidine); 5,15 (dd, 1H, H en 5 sur la pyrrolidine); 6,2 (bdd, 1H, NH); 7,2 à 7,6 (m, 8H, aromatiques); 8 (bs, 1H, H en 2 sur le phényl de l'urée); 8,7 (bs, 1H, NH). Spectre infra-rouge (KBr), bandes caractéristiques en $cm^{-1}$ : 3375, 3150, 3090, 3065, 3030, 2980, 2930, 2870, 1720, 1635, 1610, 1595, 1555, 1490, 1450, 1430, 1390, 1365, 1300, 1285, 1235, 1180, 1150, 1105, 1030, 860, 840, 755, 700, 685].

**Exemple 9**

A une solution de 8 g d'{[(éthoxycarbonyl-3 phényl)-3 uréido]-2 acétyl}-1 phényl-5 prolinate de tert-butyle-(2RS,5SR) dans 120 $cm^3$ de méthanol, on ajoute 0,9 g d'hydroxyde de potassium en solution dans 60 $cm^3$ d'eau distillée. Le mélange réactionnel est agité pendant 3 heures à une température voisine de 25°C, puis concentré à 50 $cm^3$ sous pression réduite. La solution obtenue est diluée avec 30 $cm^3$ d'eau, lavée par 2 fois 50 $cm^3$ d'acétate d'éthyle , acidifiée à pH 2 avec une solution aqueuse 4N d'acide chlorhydrique et extraite par 3 fois 100 $cm^3$ de dichlorométhane. Les phases organiques sont réunies, lavées par 2 fois 50 $cm^3$ d'eau, séchées sur sulfate de magnésium et concentrées à sec sous pression réduite à une température voisine de 40°C. Le produit brut obtenu est purifié par chromatographie sur silice [éluant : dichlorométhane-méthanol (90/10 en volumes)]. Les fractions contenant le produit attendu sont réunies et concentrées à sec sous pression réduite. On

obtient, après recristallisation dans l'acétate d'éthyle, 4,5 g d'acide {[(tert-butoxycarbonyl-2 phényl-5 pyrrolidinyl-1)-2 oxo-2 éthyl]-3 uréido}-3 benzoïque-(2RS,5SR) fondant à 236°C.

**Exemple 10**

En opérant d'une manière analogue à celle décrite à l'exemple 3, mais à partir de 1,8 g de N,N'-diimidazolecarbonyle, de 3,1 g d'(amino-2 acétyl)-1 phényl-5 prolinate de tert-butyle-(2RS,5SR) en solution dans 100 $cm^3$ de dichloro-1,2 éthane anhydre et de 1,4 g d'(amino-3 phényl)-2 éthanol, on obtient, après recristallisation dans l'acétonitrile, 1,5 g d' {{[(hydroxy-2 éthyl)-3 phényl]-3 uréido}-2 acétyl}-1 phényl-5 prolinate de tert-butyle-(2RS,5SR) fondant à 162°C.

A - L'(amino-3 phényl)-2 éthanol peut être préparé de la manière suivante : à une solution de 15 g de (nitro-3 phényl)-2 éthanol dans 250 $cm^3$ d'éthanol, on ajoute 0,75 g de palladium à 5% sur charbon. La suspension est agitée pendant 2 heures à une température voisine de 25°C sous atmosphère d'hydrogène (130 kPa). Le catalyseur est séparé par filtration et le filtrat est concentré à sec sous pression réduite à 45°C. On obtient ainsi 12 g d'(amino-3 phényl)-2 éthanol sous forme d'une huile orangée, utilisée telle quelle dans les synthèses ultérieures.

**Exemple 11**

En opérant d'une manière analogue à celle décrite à l'exemple 3, mais à partir de 3,6 g de N,N'-diimidazolecarbonyle, de 6,2 g d'(amino-2 acétyl)-1 phényl-5 prolinate de tert-butyle-(2RS,5SR) en solution dans 150 $cm^3$ de dichloro-1,2 éthane anhydre et de 3,9 g d'amino-3 cinnamate d'éthyle-(E), on obtient 4,8 g de {[(tert-butoxycarbonyl-2 phényl-5 pyrrolidinyl-1-(2RS,5SR))-2 oxo-2 éthyl]-3 uréido}-3 cinnamate d'éthyle-(E) [RMN du proton (250 MHz, DMSO $D_6$, $\delta$ en ppm, J en Hz), 2 rotamères à température ambiante, coalescence des raies à 120°C: 1,3 (t, 3H, $CH_3$); 1,5 (bs, 9H, $(CH_3)_3$); 4,25 (q, 2H, $CH_2O$); 6,4 (d, 1H, J=15, CH= trans); 7,10 à 7,70 (m, 10H, aromatiques et CH= trans). Spectre infra-rouge (KBr), bandes caractéristiques en $cm^{-1}$ : 3375, 3150, 3070, 3035, 2980, 2940, 2880, 1740, 1700, 1640, 1610, 1590, 1560, 1495, 1490, 1455, 1430, 1395, 1370, 1310, 1270, 1220, 1180, 1160, 1040, 990, 860, 840, 790, 760, 705, 685].

L'amino-3 cinnamate d'éthyle-(E) peut être préparé selon la méthode décrite dans la demande de brevet NL 7416449 (C.A. 84, 58882q).

**Exemple 12**

En opérant d'une manière analogue à celle décrite à l'exemple 9, mais à partir de 3,7 g de {[(tert-butoxycarbonyl-2 phényl-5 pyrrolidinyl-1-(2RS,5SR))-2 oxo-2 éthyl]-3 uréido}-3 cinnamate d'éthyle-(E) en solution dans 60 $cm^3$ de méthanol et de 0,4 g d'hydroxyde de potassium dissous dans 20 $cm^3$ d'eau et après traitement, on obtient 1 g d'acide {[(tert-butoxycarbonyl-2 phényl-5 pyrrolidinyl-1-(2RS,5SR))-2 oxo-2 éthyl]-3 uréido}-3 cinnamique-(E) [RMN du proton (250 MHz, DMSO $D_6$, $\delta$ en ppm, J en Hz), 2 rotamères à température ambiante, description du rotamère majoritaire, description générale valable pour les autres produits de la série : 1,5 (bs, 9H, $(CH_3)_3$); 1,9 et 2,2 (2m, 4H, H en 3 et 4 sur la pyrrolidine); 3,2 et 3,9 (ABX, 2H, $CH_2N$); 4,35 (dd, 1H, H en 2 sur la pyrrolidine); 5,20 (dd, 1H, H en 5 sur la pyrrolidine); 6,30 (dd, 1H, NH); 6,4 (bd, 1H, J=15, CH= trans); 7,1 à 7,7 (m, 10H, aromatiques et CH= trans); 8,7 (s, 1H, NH). Spectre infra-rouge (KBr), bandes caractéristiques en $cm^{-1}$ : 3380, 3075, 3030, 3700 à 2250 avec max à 2475, 2980, 2935, 2875, 1735, 1705, 1695, 1640, 1610, 1590, 1560, 1495, 1450, 1430, 1395, 1370, 1315, 1250, 1225, 1155, 985, 910, 890, 860, 840, 790, 760, 705, 685].

**Exemple 13**

En opérant d'une manière analogue à celle décrite à l'exemple 3, mais à partir de 3,6 g de N,N'-diimidazolecarbonyle, de 6,2 g d'(amino-2 acétyl)-1 phényl-5 prolinate de tert-butyle-(2RS,5SR) en solution dans 125 $cm^3$ de dichloro-1,2 éthane anhydre et de 3,9 g d'(amino-3 phényl)-3 propionate d'éthyle, on obtient, après cristallisation dans un mélange pentane-isopropanol (60/40 en volumes), 5,3 g de {[(tert-butoxycarbonyl-2 phényl-5 pyrrolidinyl-1)-2 oxo-2 éthyl]-3 uréido}-3 phényl-3 propionate d'éthyle-(2RS,5SR) fondant à 96°C.

L' (amino-3 phényl)-3 propionate d'éthyle peut être préparé d'une manière analogue à celle décrite à l'exemple 10 §A, mais à partir de 16,8 g de nitro-3 cinnamate d'éthyle-(E) en solution dans 500 $cm^3$ d'éthanol et de 0,9 g de palladium à 5% sur charbon. On obtient ainsi 14,2 g d'(amino-3 phényl)-3 propionate d'éthyle sous forme d'une huile utilisée telle quelle dans les synthèses ultérieures.

Le nitro-3 cinnamate d'éthyle-(E) peut être préparé de la manière suivante : à une solution de 31 g d'acide

nitro-3 cinnamique-(E) dans 300 $cm^3$ d'éthanol on ajoute 5 $cm^3$ d'acide sulfurique pur. Le mélange réactionnel est agité à reflux pendant 3 heures. Après refroidissement et addition de 50 $cm^3$ d'eau, la solution est concentrée à environ 60 $cm^3$ sous pression réduite à 40°C. On ajoute 250 $cm^3$ d'acétate d'éthyle puis la phase organique est lavée successivement par 2 fois 100 $cm^3$ d'une solution aqueuse de soude 2N, 2 fois 100 $cm^3$ d'eau, puis séchée sur sulfate de magnésium et concentrée à sec sous pression réduite à 40°C. On obtient ainsi, 32 g de nitro-3 cinnamate d'éthyle-(E) fondant à 70°C.

L'acide nitro-3 cinnamique-(E) peut être préparé de la manière suivante : un mélange de 30,2 g de nitro-3 benzaldéhyde, de 20,8 g d'acide malonique, de 15,8 g de pyridine et de 0,15 $cm^3$ de pipéridine est chauffé à reflux pendant 1 heure. Après refroidissement, on ajoute 50 $cm^3$ d'eau et le produit insoluble est séparé par filtration, lavé par 3 fois 50 $cm^3$ d'eau et séché à l'air. On obtient ainsi 31 g d'acide nitro-3 cinnamique-(E) fondant à 205°C.

**Exemple 14**

En opérant d'une manière analogue à celle décrite à l'exemple 9, mais à partir de 3,9 g de {[(tert-butoxycarbonyl-2 phényl-5 pyrrolidinyl-1)-2 oxo-2 éthyl]-3 uréido}-3 phényl-3 propionate d'éthyle-(2RS,5SR) en solution dans 60 $cm^3$ de méthanol et de 0,45 g d'hydroxyde de potassium dissous dans 20 $cm^3$ d'eau et après traitement, on obtient 2,1 g d'acide {[(tert-butoxycarbonyl-2 phényl-5 pyrrolidinyl-1)-2 oxo-2 éthyl]-3 uréido}-3 phényl-3 propionique-(2RS,5SR) [RMN du proton (250 MHz, DMSO $D_6$, $\delta$ en ppm), 2 rotamères à température ambiante, coalescence des raies à 120°C; 1,5 (bs, 9H, $(CH_3)_3$); 2,5 (t, 2H, $CH_2$); 2,8 (t, 2H, $CH_2$); 6,8 à 7,60 (m, 9H, aromatiques). Spectre infra-rouge (KBr), bandes caractéristiques en $cm^{-1}$ : 3380, 3700 à 2250 avec max à 2625, 3160, 3060, 3030, 2980, 2930, 2880, 1735, 1635, 1610, 1595, 1560, 1495, 1450, 1440, 1395, 1370, 1310, 1225, 1155, 905, 890, 865, 840, 790, 760, 705].

**Exemple 15**

En opérant d'une manière analogue à celle décrite à l'exemple 3, mais à partir de 2,9 g de N,N'-carbonyldiimidazole, de 5 g d'(amino-2 acétyl)-1 phényl-5 prolinate de tert-butyle-(2RS,5SR) en solution dans 100 $cm^3$ de dichloro-1,2 éthane anhydre et de 3,2 g d'amino-3 phénoxypropionate d'éthyle, on obtient 4,9 g de {[(tert-butoxycarbonyl-2 phényl-5 pyrrolidinyl-1)-2 oxo-2 éthyl]-3 uréido]-3 phénoxyacétate d'éthyle-(2RS,5SR) [RMN du proton (200 MHz, DMSO $D_6$, $\delta$ en ppm), 2 rotamères à température ambiante, description du rotamère majoritaire : 1,15 (bt, 3H, $CH_3$ de l'éthyle); 1,50 (bs, 9H, $(CH_3)_3$); 4,2 (q, 2H, $CH_2$ de l'éthyle); 4,5 (bs, 2H, $OCH_2CO$); 6,4 (bd, 1H, H en 6 sur le phényl de l'urée); 6,8 à 7,75 (m, 8H, aromatiques). Spectre infra-rouge (KBr), bandes caractéristiques en $cm^{-1}$ : 3375, 3150, 3090, 3060, 3030, 2980, 2930, 2875, 1758, 1735, 1700, 1638, 1600, 1550, 1495, 1450, 1430, 1390, 1365, 1295, 1220, 1190, 1155, 1085, 1030, 860, 840, 760, 700, 690].

L'amino-3 phénoxyacétate d'éthyle peut être préparé comme décrit à l'exemple 10 §A, mais à partir de 18 g de nitro-3 phénoxyacétate d'éthyle en solution dans 250 $cm^3$ d'éthanol et de 0,2 g de palladium à 5% sur charbon. On obtient ainsi 15 g d'amino-3 phénoxyacétate d'éthyle sous forme d'une huile utilisée telle quelle dans les synthèses ultérieures.

Le nitro-3 phénoxyacétate d'éthyle peut être préparé de la manière suivante : à une solution de 13,9 g de nitro-3 phénol dans 125 $cm^3$ de diméthylformamide anhydre, on ajoute, en 20 minutes, 4,8 g d'une suspension huileuse (50% en poids) d'hydrure de sodium. Le mélange obtenu est agité à une température voisine de 25°C pendant 30 minutes, puis on ajoute, en 10 minutes, 10,8 $cm^3$ de chloracétate d'éthyle. Le mélange réactionnel est agité pendant 20 heures à une température voisine de 20°C, puis versé dans 400 $cm^3$ d'eau et extrait par 3 fois 200 $cm^3$ d'acétate d'éthyle. Les phases organiques réunies sont séchées sur sulfate de magnésium et concentrées à sec sous pression réduite à une température de 35°C. On obtient 18 g de nitro-3 phénoxyacétate d'éthyle sous forme d'une huile orangée utilisée telle quelle dans les synthèses ultérieures.

**Exemple 16**

En opérant d'une manière analogue à celle décrite à l'exemple 9, mais à partir de 3,7 g de {[(tert-butoxycarbonyl-2 phényl-5 pyrrolidinyl-1)-2 oxo-2 éthyl]-3 uréido}-3 phénoxyacétate d'éthyle-(2RS,5SR) en solution dans 80 $cm^3$ de méthanol et de 0,4 g d'hydroxyde de potassium dissous dans 40 $cm^3$ d'eau. Après traitement et recristallisation dans l'acétate d'isopropyle, on obtient 1,4 g d'acide {[(tert-butoxycarbonyl-2 phényl-5 pyrrolidinyl-1)-2 oxo-2 éthyl]-3 uréido}-3 phénoxyacétique-(2RS,5SR) fondant à 192°C.

**Exemple 17**

En opérant d'une manière analogue à celle décrite à l'exemple 3, mais à partir de 3,6 g de N,N'-diimidazole-carbonyle, de 6,2 g d'(amino-2 acétyl)-1 phényl-5 prolinate de tert-butyle-(2RS,5SR) en solution dans 150 $cm^3$ de dichloro-1,2 éthane anhydre et de 4,2 g d'(amino-3 phénylthio)acétate d'éthyle, on obtient 4,9 g de {{[(tert-butoxycarbonyl-2 phényl-5 pyrrolidinyl-1)-2 oxo-2 éthyl]-3 uréido}-3 phénylthio}acétate d'éthyle-(2RS,5SR) [RMN du proton (300 MHz, DMSO $D_6$, $\delta$ en ppm), 2 rotamères à température ambiante, description du rotamère majoritaire : 1,2 (t, 3H, $CH_3$ de l'éthyle); 1,5 (bs, 9H, $(CH_3)_3$); 3,8 (bs, 2H, $CH_2S$); 4,2 (q, 2H, $CH_2O$ de l'éthyle); 6,9 à 7,7 (m, 9H, aromatiques). Spectre infra-rouge (KBr), bandes caractéristiques en $cm^{-1}$ : 3365, 3130, 3085, 3065, 3030, 2980, 2930, 2875, 1735, 1700, 1635, 1585, 1545, 1480, 1498, 1425, 1450, 1395, 1365, 1305, 1295, 1275, 1220, 1150, 1030, 885, 865, 840, 780, 760, 700, 690].

L' (amino-3 phénylthio)acétate d'éthyle peut être préparé de la manière suivante : à une solution de 12,5 g d'amino-3 thiophénol dans 200 $cm^3$ d'éthanol, on ajoute en 5 minutes 16,7 g de bromoacétate d'éthyle. Le mélange est agité à une température voisine de 20°C pendant 3 heures, puis concentré à sec sous pression réduite à 40°C. Le produit obtenu est dissous dans 100 $cm^3$ d'acétate d'éthyle et lavé par 100 $cm^3$ d'une solution aqueuse de soude 1 N. La phase organique est séparée, lavée par 2 fois 50 $cm^3$ d'eau, séchée sur sulfate de magnésium et concentrée à sec sous pression réduite. Le produit obtenu est purifié par chromatographie sur silice [éluant : acétate d'éthyle-cyclohexane (70/30 en volumes)]. Les fractions contenant le produit attendu sont réunies et concentrées à sec sous pression réduite à 40°C. On obtient ainsi 13 g d'(amino-3 phénylthio)acétate d'éthyle sous forme d'une huile utilisée telle quelle dans les synthèses ultérieures.

**Exemple 18**

En opérant d'une manière analogue à celle décrite à l'exemple 9, mais à partir de 4 g de {{[(tert-butoxycarbonyl-2 phényl-5 pyrrolidinyl-1)-2 oxo-2 éthyl]-3 uréido}-3 phénylthio}acétate d'éthyle-(2RS,5SR) en solution dans 80 $cm^3$ de méthanol et de 0,45 g d'hydroxyde de potassium dissous dans 40 $cm^3$ d'eau et après traitement et cristallisation dans un mélange oxyde d'isopropyle-acétate d'isopropyle (50/50 en volumes), on obtient 2 g d'acide {{[(tert-butoxycarbonyl-2 phényl-5 pyrrolidinyl-1)-2 oxo-2 éthyl]-3 uréido}-3 phénylthio}acétique-(2RS,5SR) fondant à 136°C.

**Exemple 19**

En opérant d'une manière analogue à celle décrite à l'exemple 3, mais à partir de 3,6 g de N,N'-diimidazole-carbonyle, de 6,2 g d'(amino-2 acétyl)-1 phényl-5 prolinate de tert-butyle-(2RS,5SR) en solution dans 150 $cm^3$ de dichloro-1,2 éthane anhydre et de 3,7 g d'amino-5 salicylate d'éthyle, on obtient 3,1 g de {[(tert-butoxycarbonyl-2 phényl-5 pyrrolidinyl-1)-2 oxo-2 éthyl]-3 uréido}-5 salicylate d'éthyle-(2RS,5SR) fondant à 150°C.

L'amino-5 salicylate d' éthyle peut être préparé d'une manière analogue à celle décrite à l'exemple 10 §A, mais à partir de 10 g de nitro-5 salicylate d'éthyle en solution dans 200 $cm^3$ d'éthanol et de 0,5 g de palladium à 5% sur charbon. On obtient ainsi 8,5 g d'amino-5 salicylate d'éthyle sous forme d'une huile orangée utilisée telle quelle dans les synthèses ultérieures.

Le nitro-5 salicylate d'éthyle peut être préparé de la manière suivante : à une solution de 10 g d'acide nitro-5 salicylique dans 250 $cm^3$ d'éthanol on ajoute 3 $cm^3$ d'acide sulfurique concentré. Le mélange réactionnel est agité à reflux pendant 70 heures. Après refroidissement et addition de 50 $cm^3$ d'eau, la solution est concentrée à environ 60 $cm^3$ sous pression réduite à 40°C. On ajoute 250 $cm^3$ d'acétate d'éthyle puis la phase organique est lavée par 2 fois 100 $cm^3$ d'eau distillée, séchée sur sulfate de magnésium et concentrée à sec sous pression réduite. Le produit brut obtenu est purifié par chromatographie sur silice [éluant : dichlorométhane]. Les fractions contenant le produit attendu sont réunies et concentrées à sec sous pression réduite à 40°C. On obtient ainsi 10 g de nitro-5 salicylate d'éthyle fondant à 97°C.

**Exemple 20**

En opérant d'une manière analogue à celle décrite à l'exemple 9, mais à partir de 4,7 g de {[(tert-butoxycarbonyl-2 phényl-5 pyrrolidinyl-1)-2 oxo-2 éthyl]-3 uréido}-5 salicylate d'éthyle-(2RS,5SR) en solution dans 80 $cm^3$ de méthanol et de 1,04 g d'hydroxyde de potassium dissous dans 40 $cm^3$ d'eau et après traitement, on obtient 2,3 g d'acide {[(tert-butoxycarbonyl-2 phényl-5 pyrrolidinyl-1)-2 oxo-2 éthyl]-3 uréido}-5 salicylique-(2RS,5SR) fondant à 190°C.

**Exemple 21**

En opérant d'une manière analogue à celle décrite à l'exemple 2 mais à partir de 6,2 g d'(amino-2 acétyl)-1 phényl-5 prolinate de tert-butyle-(2RS,5SR) en solution dans 150 $cm^3$ de tétrahydrofuranne anhydre et de 4,2 g d'isocyanato-3 phénylacétate de méthyle, on obtient 6 g de {[(tert-butoxycarbonyl-2 phényl-5 pyrrolidinyl-1)-2 oxo-2 éthyl]-3 uréido}-3 phénylacétate de méthyle-(2RS,5SR) [RMN du proton (200 MHz, DMSO $D_6$, $\delta$ en ppm), 2 rotamères à température ambiante, coalescence des raies à 120°C : 1,5 (s, 9H, $(CH_3)_3$); 3,6 (s, 2H, $CH_2CO$); 3,65 (s, 3H, $OCH_3$); 6,8 à 7,7 (m, 9H, aromatiques). Spectre infra-rouge (KBr), bandes caractéristiques en $cm^{-1}$ : 3365, 3155, 3110, 3090, 3060, 3030, 2975, 2950, 2930, 2875, 1738, 1700, 1650, 1610, 1595, 1560, 1495, 1435, 1395, 1365, 1315, 1250, 1220, 1155, 1015, 905, 890, 860, 845, 780, 760, 700].

L'isocyanato-3 phénylacétate de méthyle est préparé de la manière suivante : à une suspension de 1 g de charbon et 6 $cm^3$ de diphosgène dans 70 $cm^3$ de toluène, on ajoute, à une température voisine de -20°C et sous argon, 8,25 g d'amino-3 phénylacétate de méthyle en solution dans 100 $cm^3$ de toluène. Le mélange réactionnel est agité et maintenu à -20°C pendant 15 minutes, puis, après retour à une température voisine de 20°C, chauffé à reflux pendant 2 heures et 30 minutes. Le mélange est alors dégazé par barbottage d'argon pendant 30 minutes, filtré sur célite, rincé avec 50 $cm^3$ de dichlorométhane et concentré sous pression réduite à une température voisine de 50°C. On obtient ainsi 9,30 g d'isocyanato-3 phénylacétate de méthyle sous forme d'un liquide jaune conservé sous argon et utilisé tel quel dans les synthèses ultérieures.

L'amino-3 phénylacétate de méthyle peut être préparé d'une manière analogue à celle décrite à l'exemple 10 §A, mais à partir de 37,1 g de nitro-3 phénylacétate de méthyle en solution dans 550 $cm^3$ de méthanol et de 2 g de palladium sur charbon. On obtient ainsi 28,2 g d'amino-3 phénylacétate de méthyle sous forme d'un liquide jaune foncé utilisé tel quel dans les synthèses ultérieures.

Le nitro-3 phénylacétate de méthyle peut être préparé selon la méthode décrite par SEGERS et A. BRUYLANTS, Bul. Soc. Chim. Belg., 64, 87, (1955).

**Exemple 22**

En opérant d'une manière analogue à celle décrite à l'exemple 9, mais à partir de 4,9 g de {[(tert-butoxycarbonyl-2 phényl-5 pyrrolidinyl-1)-2 oxo-2 éthyl]-3 uréido}-3 phénylacétate de méthyle-(2RS,5SR) en solution dans 80 $cm^3$ de méthanol et de 0,56 g d'hydroxyde de potassium dissous dans 40 $cm^3$ d'eau et après traitement, on obtient 1 g d'acide (tert-butoxycarbonyl-2 phényl-5 pyrrolidinyl-1)-2 oxo-2 éthyl]-3 uréido}-3 phénylacétique-(2RS,5SR) [RMN du proton (200 MHz, DMSO $D_6$, $\delta$ en ppm), 2 rotamères à température ambiante, coalescence des raies à 120°C : 1,5 (bs, 9H, $(CH_3)_3$); 3,5 (s, 2H, $CH_2CO$); 6,8 à 7,7 (m, 9H, aromatiques). Spectre infra-rouge (KBr), bandes caractéristiques en $cm^{-1}$ : 3380, 3700 à 2250 avec max à 2625, 3155, 3110, 3090, 3060, 3030, 2975, 2930, 2880, 1735, 1635, 1610, 1595, 1560, 1495, 1450, 1395, 1365, 1310, 1225, 1155, 905, 890, 860, 840, 780, 760, 705].

**Exemple 23**

En opérant d'une manière analogue à celle décrite à l'exemple 1, mais à partir de 2,2 g de phényl-5 prolinate d'éthyle-(2RS,5SR), de 2,1 g d'acide [(méthyl-3 phényl)-3 uréido]-2 acétique en suspension dans 50 $cm^3$ de dichloro-1,2 éthane anhydre et de 0,72 $cm^3$ de chlorure de sulfinyle, on obtient, après recristallisation dans l'acétonitrile, 1,2 g de {[(méthyl-3 phényl)-3 uréido]-2 acétyl}-1 phényl-5 prolinate d'éthyle-(2RS,5SR) fondant à 115°C.

Le phényl-5 prolinate d'éthyle-(2RS,5SR) peut être préparé selon la méthode décrite par F. LEONARD, brevet GB 997097, [C.A., 62P, 9109e (1965)].

**Exemple 24**

En opérant d'une manière analogue à celle décrite à l'exemple 3, mais à partir de 4,1 g de N,N'-diimidazole-carbonyle, de 7 g d'(amino-2 acétyl)-1 phényl-5 prolinate d'éthyle-(2RS,5SR) en solution dans 135 $cm^3$ de dichloro-1,2 éthane anhydre et de 4,1 g d'amino-3 benzoate d'éthyle, on obtient 1,5 g d'{[(éthoxycarbonyl-3 phényl)-3 uréido]-2 acétyl}-1 phényl-5 prolinate d'éthyle-(2RS,5SR) fondant à 136°C.

L'(amino-2 acétyl)-1 phényl-5 prolinate d'éthyle-(2RS,5SR) peut être préparé de manière analogue à celle décrite à l'exemple 2 §A, mais à partir de 11,1 g de (tert-butoxycarbonylamino-2 acétyl)-1 phényl-5 prolinate d'éthyle-(2RS,5SR) et de 4,3 $cm^3$ d'iodotriméthylsilane en solution dans 150 $cm^3$ de chloroforme anhydre. On obtient ainsi 7 g d'(amino-2 acétyl)-1 phényl-5 prolinate d'éthyle-(2RS,5SR), utilisé tel quel dans les synthèses ultérieures.

Le (tert-butoxycarbonylamino-2 acétyl)-1 phényl-5 prolinate d'éthyle-(2RS,5SR) peut être préparé comme décrit à l'exemple 2 §B, mais à partir d'une solution contenant 7 g de phényl-5 prolinate d'éthyle-(2RS,5SR), 5,6 g d'acide tert-butoxycarbonylamino-2 acétique et 6,6 g de N,N'-dicyclohexylcarbodiimide dans 65 $cm^3$ d'acétonitrile anhydre. On obtient ainsi 11,1 g de (tert-butoxycarbonylamino-2 acétyl)-1 phényl-5 prolinate d'éthyle-(2RS,5SR) sous forme d'une huile orangée utilisée telle quelle dans les synthèses ultérieures.

**Exemple 25**

En opérant d'une manière analogue à celle décrite à l'exemple 1, mais à partir de 2 g de phényl-5 prolinate de cyclopropylméthyle-(2RS,5SR), de 1,7 g d'acide [(méthyl-3 phényl)-3 uréido]-2 acétique en suspension dans 50 $cm^3$ de dichloro-1,2 éthane anhydre et de 0,6 $cm^3$ de chlorure de sulfinyle, on obtient, après recristallisation dans l'acétonitrile, 1,1 g de {[(méthyl-3 phényl)-3 uréido]-2 acétyl}-1 phényl-5 prolinate de cyclopropylméthyle-(2RS,5SR) fondant à 130°C.

A - Le phényl-5 prolinate de cyclopropylméthyle-(2RS,5SR) peut être préparé de la manière suivante : à une solution de 3,5 g de (tert-butoxycarbonyl)-1 phényl-5 prolinate de cyclopropylméthyle-(2RS,5SR) dans 50 $cm^3$ de chloroforme anhydre, on ajoute goutte à goutte 1,5 $cm^3$ d'iodotriméthylsilane. Le mélange réactionnel est agité pendant 20 heures à une température voisine de 25°C puis concentré à sec sous pression réduite à une température de 45°C. Le produit brut obtenu est purifié par chromatographie sur silice [éluant : dichlorométhane-méthanol (97/3 en volumes)]. Les fractions contenant le produit attendu sont réunies puis concentrées à sec sous pression réduite. On obtient ainsi 2 g de phényl-5 prolinate de cyclopropylméthyle-(2RS,5SR) sous forme d'une huile orangée utilisée telle quelle dans les synthèses ultérieures.

B - Le (tert-butoxycarbonyl)-1 phényl-5 prolinate de cyclopropylméthyle-(2RS,5SR) peut être préparé de la manière suivante : à une solution de 7 g de (tert-butoxycarbonyl)-1 phényl-5 proline-(2RS,5SR)) et de 4,6 g de chlorure de paratoluènesulfonyle dans 40 $cm^3$ de pyridine anhydre, on ajoute goutte à goutte, sous agitation, 1,8 g de cyclopropylméthanol à une température voisine de 0°C. Après retour à une température de 20°C, l'agitation est poursuivie pendant 20 heures, puis le mélange est versé dans 100 $cm^3$ d'eau et le produit extrait par 3 fois 100 $cm^3$ d'acétate d'éthyle. Les phases organiques réunies sont lavées par 2 fois 100 $cm^3$ d'eau, 2 fois 100 $cm^3$ d'une solution aqueuse d'acide chlorhydrique N, 2 fois 100 $cm^3$ d'une solution aqueuse de soude N puis 3 fois 100 $cm^3$ d'eau, séchées sur sulfate de magnésium et concentrées à sec sous pression réduite à une température voisine de 45°C. Le produit brut obtenu est purifié par chromatographie sur silice [éluant : dichlorométhane-méthanol (97/3 en volumes)]. Les fractions contenant le produit attendu sont réunies et concentrées à sec sous pression réduite à une température voisine de 45°C. On obtient ainsi 3,6 g de (tert-butoxycarbonyl)-1 phényl-5 prolinate de cyclopropylméthyle-(2RS,5SR) sous forme d'une huile orangée utilisée telle quelle dans les synthèses ultérieures.

C - La (tert-butoxycarbonyl)-1 phényl-5 proline-(2RS,5SR) peut être préparée de la manière suivante : à une solution de 22,8 g de chlorhydrate de phényl-5 proline-(2RS,5SR) et de 22 g de carbonate de sodium dans 160 $cm^3$ d'eau, on ajoute goutte à goutte, sous agitation, 21,8 g de dicarbonate de ditert-butyle en solution dans 120 $cm^3$ de dioxanne. Le mélange réactionnel est agité pendant 20 heures à une température voisine de 20°C, puis le précipité formé est éliminé par filtration. Le filtrat est lavé par 2 fois 100 $cm^3$ d'acétate d'éthyle et acidifié à pH 1 par une solution aqueuse d'acide chlorhydrique 4N. La phase aqueuse acide est extraite par 3 fois 150 $cm^3$ de dichlorométhane. Les extraits organiques réunis sont lavés par 2 fois 50 $cm^3$ d'eau , séchés sur sulfate de magnésium et concentrés à sec sous pression réduite à une température voisine de 40°C. Après recristallisation dans l'acétonitrile, on obtient 24 g de (tert-butoxycarbonyl)-1 phényl-5 proline-(2RS,5SR) fondant à 170°C.

**Exemple 26**

En opérant d'une manière analogue à celle décrite à l'exemple 1, mais à partir de 3 g de phényl-5 prolinate d'isopropyle-(2RS,5SR), 2,7 g d'acide [(méthyl-3 phényl)-3 uréido]-2 acétique en suspension dans 75 $cm^3$ de dichloro-1,2 éthane anhydre et de 1 $cm^3$ de chlorure de sulfinyle, on obtient, après purification, 1,2 g de {[(méthyl-3 phényl)-3 uréido]-2 acétyl}-1 phényl-5 prolinate d'isopropyle-(2RS,5SR) [RMN du proton (200 MHz, $CDCl_3$, δ en ppm), 2 rotamères à température ambiante, description du rotamère majoritaire : 1,1 (d, 6H, $(CH_3)_2$); 1,7 à 2,5 (m, 4H, H en 3 et 4 sur la pyrrolidine); 2,15 (s, 3H, $CH_3$); 3 et 4,1 (2bd, 2H, $CH_2N$); 4,4 (bt, 1H, H en 2 sur la pyrrolidine); 4,9 (m, 2H, CH de l'isopropyl et H en 5 sur la pyrrolidine); 6,6 à 7,5 (m, 9H, aromatiques). Spectre infra-rouge (KBr), bandes caractéristiques en $cm^{-1}$ : 3365, 3150, 3060, 3030, 2980, 2935, 2875, 1738, 1700, 1645, 1615, 1595, 1560, 1495, 1450, 1430, 1375, 1305, 1295, 1280, 1210, 1190, 1145, 1120, 915, 890, 860, 780, 755, 705].

Le phényl-5 prolinate d'isopropyle-(2RS,5SR) peut être préparé de manière analogue à celle décrite dans

l'exemple 25 §A, mais à partir de 5 g de (tert-butoxycarbonyl)-1 phényl-5 prolinate d'isopropyle-(2RS,5SR) et de 2,4 $cm^3$ de iodotriméthylsilane en solution dans 50 $cm^3$ de chloroforme anhydre. On obtient ainsi 3 g de phényl-5 prolinate d'isopropyle-(2RS,5SR) sous forme d'une huile jaune utilisée telle quelle dans les synthèses ultérieures.

Le (tert-butoxycarbonyl)-1 phényl-5 prolinate d'isopropyle-(2RS,5SR) peut être préparé comme décrit à l'exemple 25 §B, mais à partir de 5,85 g de (tert-butoxycarbonyl)-1 phényl-5 proline-(2RS,5SR), de 3,85 g de chlorure de paratoluènesulfonyle et de 1,6 g de propanol-2 dans 30 $cm^3$ de pyridine anhydre. Après traitement, on obtient 5 g de (tert-butoxycarbonyl)-1 phényl-5 prolinate d'isopropyle-(2RS,5SR) sous forme d'une huile jaune utilisée telle quelle dans les synthèses ultérieures.

**Exemple 27**

Les énantiomères du {[(méthyl-3 phényl)-3 uréido]-2 acétyl}-1 phényl-5 prolinate de tert-butyle-(2RS,5SR) ont été séparés par chromatographie liquide haute performance sur phase chirale type PIRCKLE en utilisant 400 g de (D)-N-3,5-dinitrobenzoyle phénylglycine, greffée sur silice aminopropyle comme phase stationnaire chargée dans une colonne de 200 mm de longueur et de 80 mm de diamètre avec comme phase mobile, un mélange hexane-propanol-2-chlorure de méthylène (85/7,5/7,5). A partir de 1 g de racémique on obtient :

- 0,48 g de {[(méthyl-3 phényl}-3 uréido]-2 acétyl}-1 phényl-5 prolinate de tert-butyle-(2S,5R) fondant à 79°C, $[\alpha]_D^{20}$= +33,4° ±0,9° (c=1 ; méthanol) [RMN du proton (300 MHz, DMSO $D_6$, δ en ppm), 2 rotamères à température ambiante, description du rotamère majoritaire : 1,5 (s, 3H, $(CH_3)_3$); 1,9 et 2,2 (2m, 4H, H en 3 et 4 sur la pyrrolidine); 2,2 (s, 3H, $CH_3$); 3,2 et 3,9 (ABX, 2H,$CH_2N$); 4,35 (dd, 1H, H en 2 sur la pyrrolidine); 5,20 (dd, 1H, H en 5 sur la pyrrolidine); 6,30 (dd, 1H, NH); 6,70 (bd, 1H, H en 4 sur le phényl de l'urée); 7,1 à 7,7 (m, 8H, aromatiques); 8,7 (s, 1H, NH)].
- 0,48 g de {[(méthyl-3 phényl)-3 uréido]-2 acétyl}-1 phényl-5 prolinate de tert-butyle-(2R,5S) fondant à 79°C, $[\alpha]_D^{20}$ = -31,6° ±0,8° (c=1 ; méthanol).

Le support peut être préparé de la manière suivante:

Dans un tricol de six litres, on met en suspension 600 g de silice aminopropyle (100 Å - 10 µm - $NH_2$ ; Macherey-Nagel) dans 2 l de diméthylformamide. On ajoute 95 g d'anhydride de l'acide N-tert-butoxycarbonylamino-11 undécanoïque et on agite le mélange réactionnel pendant 18 heures à une température voisine de 20° C. La silice est séparée par filtration et lavée successivement par deux fois 1500 $cm^3$ de dichlorométhane puis deux fois 1500 $cm^3$ de diméthylformamide. La silice ainsi lavée est remise en suspension dans 2 l de diméthylformamide et on ajoute 95 g d'anhydride de l'acide N-tert-butoxycarbonylamino-11 undécanoique puis on agite le mélange réactionnel pendant 18 heures à une température voisine de 20° C. La silice est séparée par filtration, lavée successivement par deux fois 600 $cm^3$ de dichlorométhane, deux fois 600 $cm^3$ de tetrahydrofuranne, deux fois 600 $cm^3$ de méthanol et deux fois 600 $cm^3$ d'oxyde de diéthyle puis séchée sous pression réduite à une température voisine de 20° C. On obtient ainsi 610 g de silice désignée par l'appellation "BOC-$C_{11}$-$C_3$-silice" sous forme de poudre blanche dont la structure est confirmée par le spectre infra-rouge et dont l'analyse élémentaire (trouvée) est : C %= 8,8 ; H % = 1,7 ; N % = 1,2.

Dans un tricol de six litres, on met en suspension 607 g de silice "BOC-$C_{11}$-$C_3$-silice" dans 21 de dichlorométhane et 69 $cm^3$ de pyridine. On ajoute goutte à goutte 530 $cm^3$ de diméthyloctylchlorosilane et on agite le mélange réactionnel pendant 16 heures à une température voisine de 20° C. Le solide obtenu est séparé par filtration et lavé successivement par deux fois 1 l de dichlorométhane, deux fois 1 l de méthanol, deux fois 1 l de tetrahydrofuranne, deux fois 1 l de dichlorométhane et deux fois 1 l d'oxyde de diéthyle puis séché sous pression réduite à une température voisine de 20° C. On obtient ainsi 712 g de silice désignée par l'appellation "BOC-$C_{11}$-$C_3$-silice-O-$Si(CH_3)_2(CH_2)_7CH_3$" sous forme de poudre blanche dont la structure est confirmée par le spectre infra-rouge et dont l'analyse élémentaire (trouvée) est : C %= 12,1 ; H % = 2,4; N % = 1,0.

Dans un tricol de six litres, on met en suspension 711 g de silice "BOC-$C_{11}$-$C_3$-silice-O-$Si(CH_3)_2(CH_2)_7CH_3$" dans 2200 $cm^3$d'une solution à 6% en volumes d'acide trifluoroacétique dans le dichlorométhane. On agite le mélange réactionnel pendant 5 heures à une température voisine de 20° C. La silice est séparée par filtration et lavée successivement par deux fois 1 l de dichlorométhane, deux fois 1 l d'un mélange dichlorométhane/diisopropyléthylamine (70/30 en volumes), 1 l de dichlorométhane, deux fois 1 l de tetrahydrofuranne, deux fois 1 l de méthanol et deux fois 1 l d'oxyde de diéthyle puis séchée sous pression réduite à une température voisine de 50°C. La silice ainsi lavée et séchée est remise en suspension dans 2l d'une solution à 6% en volumes d'acide trifluoroacétique dans le dichlorométhane. On agite le mélange réactionnel pendant 16 heures à une température voisine de 20° C. La silice est séparée par filtration et lavée successivement par deux fois 1,5 l de dichlorométhane, deux fois 1 l d'un mélange dichlorométhane/diisopropyléthylamine (70/30 en volumes), 1,5 l de dichlorométhane, deux fois 2 l de tetrahydrofuranne, deux fois 2 l de méthanol et deux fois 2 l d'oxyde de diéthyle puis séchée sous pression réduite à une température voisine de 50° C. On obtient ainsi 607 g de

silice désignée par l'appellation "$C_{11}$-$C_3$-silice-O-Si$(CH_3)_2(CH_2)_7CH_3$" sous forme de poudre blanche dont la structure est confirmée par le spectre infra-rouge et dont l'analyse élémentaire (trouvée) est : C %= 8,8 ; H % = 1,7; N%=1,3.

Dans un tricol de quatre litres, on met en suspension 400 g de silice "$C_{11}$-$C_3$-silice-O-Si$(CH_3)_2(CH_2)_7CH_3$" dans 1800 $cm^3$ de diméthylformamide. On ajoute 42 g de dinitro-3,5 benzoyl-D-phénylglycine et 30 g d'éthoxy-2 éthoxycarbonyl-1 dihydro-1,2 quinoléine et on agite le mélange réactionnel pendant 16 heures à une température voisine de 20° C. La silice est séparée par filtration et lavée successivement par deux fois 1 l de dichlorométhane, deux fois 1 l de tetrahydrofuranne deux fois 1 l de méthanol et deux fois 1 l d'oxyde de diéthyle. La silice ainsi lavée est remise en suspension dans 2 l de diméthylformamide et on ajoute 30 g d'éthoxy-2 éthoxycarbonyl-1 dihydro-1,2 quinoléine et 42 g de dinitro-3,5 benzoyl-D-phénylglycine puis on agite le mélange réactionnel pendant 5 heures à une température voisine de 20° C. La silice est séparée par filtration, lavée successivement par deux fois 1 l de diméthylformamide, deux fois 1 l de dichlorométhane, deux fois 1 l de tetrahydrofuranne deux fois 1 l de méthanol et deux fois 1 l d'oxyde de diéthyle puis séchée sous pression réduite à une température voisine de 140° C. On obtient ainsi 434 g de silice désignée par l'appellation "DNB-D-Phg-$C_{11}$-$C_3$-silice-OSi$(CH_3)_2(CH_2)_7CH_3$" sous forme de poudre blanche dont la structure est confirmée par le spectre infra-rouge et dont l'analyse élémentaire (trouvée) est : C %= 12,3 ; H % = 1,8 ; N % = 2,1.

Dans un tricol de quatre litres, on met en suspension 434 g de silice "DNB-D-Phg-$C_{11}$-$C_3$-silice-OSi$(CH_3)_2(CH_2)_7CH_3$" dans 1,3 l de dichlorométhane et on ajoute 100 $cm^3$ de diméthyloctylméthoxysilane puis on agite le mélange réactionnel pendant 54 heures à une température voisine de 20° C. La silice est séparée par filtration, lavée successivement par deux fois 1 l de dichlorométhane, deux fois 1 l de méthanol, deux fois 1 l de tetrahydrofuranne et deux fois 1 l de dichlorométhane puis séchée sous pression réduite à une température voisine de 140° C. On obtient ainsi 425 g de silice désignée par l'appellation "DNB-D-Phg-$C_{11}$-$C_3$-silice -OSi$(CH_3)_2(CH_2)_7CH_3$réoctylée" sous forme de poudre blanche dont la structure est confirmée par le spectre infra-rouge et dont l'analyse élémentaire (trouvée) est : C %= 12,7; H % = 1,9; N % = 2,0.

Dans un tricol de quatre litres, on met en suspension 425 g de silice "DNB-D-Phg-$C_{11}$-$C_3$-silice-OSi$(CH_3)_2(CH_2)_7CH_3$réoctylée" dans 1,3 l de dichlorométhane. On ajoute goutte à goutte 545 $cm^3$ de triméthylsilylimidazole et on agite le mélange réactionnel pendant 15 heures à une température voisine de 20° C. Le solide obtenu est séparé par filtration et lavé successivement par deux fois 1 l de tetrahydrofuranne, deux fois 1 l de méthanol, deux fois 1 l d'acétone et deux fois 1 l de dichlorométhane puis séché sous pression réduite à une température voisine de 20° C. On obtient ainsi 431 g de silice désignée par l'appellation "DNB-D-Phg-$C_{11}$-$C_3$-silice-[O-Si$(CH_3)_2(CH_2)_7CH_3$]-[O-Si$(CH_3)_3$]" sous forme de poudre blanche dont la structure est confirmée par le spectre infra-rouge et dont l'analyse élémentaire (trouvée) est : C %= 13,7 ; H % = 2,2 ; N % = 2,0.

L'anhydride de l'acide N-tert-butoxycarbonylamino-11 undécanoïque peut être préparé de la manière suivante :
à une solution de 30,1 g d'acide N-tert-butoxycarbonylamino-11 undécanoïque dans 480 $cm^3$d'acétate d'éthyle, maintenue à une température voisine de 5° C, on ajoute en 10 minutes une solution de 10,63 g de N,N'-dicyclohexylcarbodiimide dans 120 $cm^3$ d'acétate d'éthyle. Le mélange réactionnel est agité pendant 1 heure à une température voisine de 5° C puis pendant 16 heures à une température voisine de 20° C. Le précipité formé est séparé par filtration et lavé par 30 $cm^3$ d'acétate d'éthyle. Le filtrat est concentré sous pression réduite à 30° C. Le solide obtenu est séché sous vide à une température voisine de 30° C. On obtient ainsi 31 g d'anhydride de l'acide N-tert-butoxycarbonylamino-11 undécanoïque fondant à 58°C.

L'acide N-tert-butoxycarbonylamino-11 undécanoïque peut être préparé selon la méthode décrite par J.T. SPARROW J. Org. Chem., 41, 1350 (1976).

**Exemple 28**

A une solution de 0,08 g d'(amino-2 acétyl)-1 phényl-5 prolinate de tert-butyle-(2S,5R) dans 10 $cm^3$ de tétrahydrofuranne anhydre, on ajoute 0,05 $cm^3$ d'isocyanate de méthyl-3 phényle. Le mélange réactionnel est agité pendant 3 heures à une température voisine de 25°C, puis le solvant est évaporé sous pression réduite à 45°C. Le produit brut obtenu est purifié par chromatographie sur silice [éluant : dichlorométhane-méthanol (98/2 en volumes)]. Les fractions contenant le produit attendu sont réunies et concentrées à sec sous pression réduite. On obtient ainsi 0,04 g de {[(méthyl-3 phényl)-3 uréido]-2 acétyl}-1 phényl-5 prolinate de tert-butyle-(2S,5R) dont les donnnées analytiques sont en accord avec celles de l'énantiomère dextrogyre préparé par chromatographie sur phase stationnaire chirale du produit racémique, ($[\alpha]_D^{20}$ = +35,6° ±0,8° (C = 0,11 %; MeOH)).

L'(amino-2 acétyl)-1 phényl-5 prolinate de tert-butyle-(2S,5R) peut être préparé de la manière suivante : à une solution de 0,08 g d'acide (tert-butoxycarbonylamino-2 acétyl)-1 phényl-5 pyrrolidinecarboxylique-2-(2S,5R) dans 20 $cm^3$ de chloroforme refroidie à 5°C, on ajoute 0,1 $cm^3$ d'acide sulfurique concentré. Le milieu

réactionnel est saturé par de l'isobutène pendant 2 heures sous agitation en maintenant la température à 5°C. Après retour à une température voisine de 20°C, l'agitation est poursuivie pendant 20 heures. La solution est amenée à pH 8 par addition d'une solution aqueuse de bicarbonate de sodium à 10 % et la phase aqueuse est séparée puis extraite par 3 fois 30 $cm^3$ de chloroforme. Les phases organiques réunies sont lavées par 2 fois 10 $cm^3$ d'eau, séchées sur sulfate de magnésium et concentrées à sec sous pression réduite à 40°C. On obtient ainsi 0,08 g d'(amino-2 acétyl)-1 phényl-5 prolinate de tert-butyle-(2S,5R), sous forme d'une huile jaune orangée utilisée telle quelle dans les synthèses ultérieures.

L'acide (tert-butoxycarbonylamino-2 acétyl)-1 phényl-5 pyrrolidine-carboxylique-2-(2S,5R) peut être préparé de la manière suivante : à une solution de 0,16 g de (tert-butoxycarbonylamino-2 acétyl)-1 phényl-5 prolinate de méthyle-(2S,5R) dans 15 $cm^3$ d'un mélange dioxanne-eau (70/30 en volumes) refroidi à 5°C, on additionne 0,5 $cm^3$ d'une solution aqueuse de soude 1N. Après retour à une température voisine de 20°C, l'agitation est poursuivie pendant 12 heures puis on additionne à nouveau, 0,5 $cm^3$ d'une solution aqueuse de soude 1N. Après 3 heures sous agitation, à une température voisine de 20°C, le milieu réactionnel est lavé par 2 fois 10 cm3 d'éther puis amené à un pH voisin de 2 par addition de 20 $cm^3$ d'une solution aqueuse d'acide chlorhydrique 0,1 N. Le mélange réactionnel est extrait par 3 fois 40 $cm^3$ d'acétate d'éthyle. Les phases organiques réunies sont séchées sur sulfate de magnésium et concentrées à sec sous pression réduite à 35°C. On obtient ainsi 0,09 g d'acide (tert-butoxycarbonylamino-2 acétyl)-1 phényl-5 pyrrolidinecarboxylique-2-(2S,5R) sous forme d'une résine incolore utilisée telle quelle dans les synthèses ultérieures.

Le (tert-butoxycarbonylamino-2 acétyl)-1 phényl-5 prolinate de méthyle-(2S,5R) peut être préparé de la manière suivante : à une solution de 0,1 g de phényl-5 prolinate de méthyle-(2S,5R) et de 0,1 g d'acide tert-butoxycarbonylamino-2 acétique dans 10 $cm^3$ d'acétonitrile anhydre maintenue à une température voisine de 0°C, on ajoute en une fois 0,12 g de N,N'-dicyclohexylcarbodiimide. Le mélange réactionnel est agité pendant 16 heures à une température voisine de 25°C, puis le produit insoluble est séparé par filtration et lavé par trois fois 5 $cm^3$ de dichlorométhane. Le filtrat est concentré à sec sous pression réduite à 40°C. Le résidu huileux obtenu est purifié par chromatographie sur silice [éluant : acétate d'éthyle-cyclohexane (30/70 en volumes)]. Les fractions contenant le produit attendu sont réunies et concentrées à sec sous pression réduite à 40°C. On obtient ainsi 0,16 g de (tert-butoxycarbonylamino-2 acétyl)-1 phényl-5 prolinate de méthyle-(2S,5R) sous forme d'une huile incolore utilisée telle quelle dans les synthèses ultérieures.

Le phényl-5 prolinate de méthyle-(2S,5R) peut être préparé de la manière suivante : à une solution de 0,6 g de méthoxycarbonyl-1 phényl-5 prolinate de méthyle-(2S,5RS) dans 15 $cm^3$ de chloroforme, on ajoute goutte à goutte 0,45 $cm^3$ d'iodotriméthylsilane à une température voisine de 25°C. Le mélange est chauffé à reflux pendant 18 heures. Après refroidissement à une température voisine de 25°C on ajoute 20 $cm^3$ d'eau. La phase aqueuse est séparée puis extraite par 2 fois 20 $cm^3$ de chloroforme. Les phases organiques sont réunies, lavées par 2 fois 10 $cm^3$ d'eau, séchées sur sulfate de magnésium et concentrées à sec sous pression réduite à 40°C. Le mélange des deux diastéréoisomères obtenu est purifié par chromatographie sur silice [éluant : acétate d'éthyle-cyclohexane (30/70 en volumes)]. Les fractions contenant le produit attendu sont réunies et concentrées à sec sous pression réduite à 40°C. On obtient ainsi 0,12 g de phényl-5 prolinate de méthyle-(2S,5R) sous forme d'une huile utilisée telle quelle dans les synthèses ultérieures.

Le méthoxycarbonyl-1 phényl-5 prolinate de méthyle-(2S,5RS) peut être préparé de la manière suivante : à une solution de 5,1 g de méthoxy-5 méthoxycarbonyl-1 prolinate de méthyle-(2S,5RS) dans 400 $cm^3$ de benzène refroidie à une température voisine de 5°C on additionne 3,1 g de trichlorure d'aluminium. Le milieu réactionnel est laissé 12 heures sous agitation à une température voisine de 25°C, puis on additionne 50 $cm^3$ d'une solution aqueuse saturée de bicarbonate de sodium. Les hydroxydes d'aluminium sont séparés par filtration et lavés par 2 fois 20 $cm^3$ de chlorure de méthylène. Les phases organiques réunies sont lavées par 2 fois 50 $cm^3$ d'eau, séchées sur sulfate de magnésium et concentrées à sec sous pression réduite à 40°C. Le résidu huileux obtenu est purifié par chromatographie sur silice [éluant : acétate d'éthyle-cyclohexane (20/80 en volumes)]. Les fractions contenant le produit attendu sont réunies et concentrées à sec sous pression réduite à 40°C. On obtient ainsi 0,6 g de méthoxycarbonyl-1 phényl-5 prolinate de méthyle-(2S,5RS) sous forme d'une huile jaune pâle, mélange (40/60 en poids) des isomères (2S,5R) et (2S,5S) utilisée telle quelle dans les synthèses ultérieures.

Le méthoxy-5 méthoxycarbonyl-1 prolinate de méthyle-(2S,5RS) peut être préparé selon la méthode décrite par T. SHONO et coll, J. Am. Chem. Soc., 104, 6697 (1982).

**Exemple 29**

Les énantiomères de l'acide {[(tert-butoxycarbonyl-2 phényl-5 pyrrolidinyl-1)-2 oxo-2 éthyl]-3 uréido}-3 benzoïque-(2RS,5SR) ont été séparés par chromatographie liquide haute performance sur phase chirale type PIRCKLE, en utilisant 400 g de (D)-N-3,5-dinitrobenzoyle phénylglycine, greffée sur silice aminopropyle ,

comme phase stationnaire chargée dans une colonne de 220 mm de longueur et de 70 mm de diamètre avec comme phase mobile, un mélange heptane-propanol-2-acide trifluoroacétique (80/20/0,05 en volumes). A partir de 3,5 g de racémique on obtient:

- 0,72 g d'acide {((tert-butoxycarbonyl-2 phényl-5 pyrrolidinyl-1)-2 oxo-2 éthyl]-3 uréido}-3 benzoïque-(2S,5R) fondant à 204°C, $[\alpha]_D^{20}$= +32,3° ±0,7° (c=1,08 ; méthanol)
- 0,54 g d'acide {[(tert-butoxycarbonyl-2 phényl-5 pyrrolidinyl-1)-2 oxo-2 éthyl]-3 uréido}-3 benzoïque-(2R,5S) fondant à 240°C, $[\alpha]_D^{20}$ = -33,1° ±0,8° (c=1,03 ; méthanol).

**Exemple 30**

En opérant d'une manière analogue à celle décrite à l'exemple 2, mais à partir de 2 g d'(amino-2 acétyl)-1 diphényl-2,5 pyrrolidine-(cis) et de 0,9 $cm^3$ d'isocyanate de méthyl-3 phényle en solution dans 25 $cm^3$ de tétrahydrofuranne anhydre, on obtient, après recristallisation dans le propanol-2, 1,4 g de [(diphényl-2,5 pyrrolidinyl-1)-2 oxo-2 éthyl]-1 (méthyl-3 phényl)-3 urée-(2RS,5SR) fondant à 168°C.

L'(amino-2 acétyl)-1 diphényl-2,5 pyrrolidine-(cis) peut être préparée d'une manière analogue à celle décrite à l'exemple 2 §A, mais à partir de 6,6 g de (tert-butoxycarbonylamino-2 acétyl)-1 diphényl-2,5 pyrrolidine-(cis) et de 2,5 $cm^3$ d'iodotriméthylsilane en solution dans 70 $cm^3$ de chloroforme anhydre. On obtient ainsi 4,8 g d'(amino-2 acétyl)-1 diphényl-2,5 pyrrolidine-(cis) sous forme d'une huile orangée, utilisée telle quelle dans les synthèses ultérieures.

**RMN du proton** (300 MHz, DMSO $D_6$, d en ppm), 2 rotamères à température ambiante, 1,8 à 2,4 (bm, 4H, $CH_2$-$CH_2$); 2,7 et 3,3 (bd, 2H, AB, $CH_2CO_2$); 5,0 (bm, 1H, CHN); 5,2 (bm, 1H, CHN); 7,2 à 7,5 (m, 10H, aromatiques).

**Masse** (impact électronique, 70eV, m/z), 280 ($M^+$), 263 ($M^+$-$NH_2$), 222 ($M^+$-$COCH_2NH_2$), 91 ($C_6H_5CH_2^+$).

Le (tert-butoxycarbonylamino-2 acétyl)-1 diphényl-2,5 pyrrolidine-(cis) peut être préparé comme décrit à l'exemple 2 §B, mais à partir d'une solution de 6,6 g de diphényl-2,5 pyrrolidine (mélange des isomères cis-trans), de 5,2 g d'acide (tert-butoxycarbonylamino)-2 acétique et de 6,1 g de N,N′-dicyclohexylcarbodiimide dans 50 $cm^3$ d'acétonitrile anhydre. On obtient, après séparation par chromatographie sur silice [éluant : dichlorométhane-méthanol (98/2 en volumes)], 3,6 g de (tert-butoxycarbonylamino-2 acétyl)-1 diphényl-2,5 pyrrolidine-(cis) sous forme d'une huile orangée utilisée telle quelle dans les synthèses ultérieures.

La diphényl-2,5 pyrrolidine (mélange des isomères cis-trans) peut être préparée selon la méthode décrite par C.G. OVERBERGER, M. VALENTINE et J-P. ANSELME, J. Amer. Chem. Soc., 91, 687-94 (1969).

**Exemple 31**

En opérant d'une manière analogue à celle décrite à l'exemple 9, mais à partir de 3 g de {[(diphényl-2,5 pyrrolidinyl-1)-2 oxo-2 éthyl]-3 uréido}-3 benzoate d'éthyle-(cis) en solution dans 50 $cm^3$ de méthanol et de 0,7 g d'hydroxyde de potassium dissous dans 20 $cm^3$ d'eau et après traitement et recristallisation dans l'éthanol, on obtient 2 g d'acide {[(diphényl-2,5 pyrrolidinyl-1)-2 oxo-2 éthyl]-3 uréido}-3 benzoïque-(cis) fondant à 255°C.

Le {[(diphényl-2,5 pyrrolidinyl-1)-2 oxo-2 éthyl]-3 uréido}-3 benzoate d'éthyle-(cis) peut être préparé d'une manière analogue à celle décrite à l'exemple 3, mais à partir de 3,4 g d'(amino-2 acétyl)-1 diphényl-2,5 pyrrolidine-(cis), de 2,2 g de N,N′-diimidazole-carbonyle en solution dans 75 $cm^3$ de dichloro-1,2 éthane anhydre et de 2 g d'amino-3 benzoate d'éthyle. On obtient, après purification, 3 g de {[(diphényl-2,5 pyrrolidinyl-1)-2 oxo-2 éthyl]-3 uréido}-3 benzoate d'éthyle-(cis) sous forme d'une meringue blanche utilisée telle quelle dans les synthèses ultérieures.

**Exemple 32**

En opérant d'une manière analogue à celle décrite à l'exemple 2, mais à partir de 1,8 g d'(amino-2 acétyl)-1 phényl-6 pipéridinecarboxylate-2 de tert-butyle-(2RS,5SR) en solution dans 30 $cm^3$ de tétrahydrofuranne anhydre, on ajoute 0,7 $cm^3$ d'isocyanate de méthyl-3 phényle. On obtient, après recristallisation dans l'oxyde de diisopropyle, 0,7 g de {[(méthyl-3 phényl)-3 uréido]-2 acétyl}-1 phényl-6 pipéridinecarboxylate-2 de tert-butyle-(2RS,5SR) fondant à 123°C.

L'(amino-2 acétyl)-1 phényl-6 pipéridinecarboxylate-2 de tert-butyle-(2RS,5SR) peut être préparé d'une manière analogue à celle décrite à l'exemple 2 §A, mais à partir de 5,9 g de (tert-butoxycarbonylamino-2 acétyl)-1 phényl-6 pipéridine carboxylate-2 de tert-butyle-(2RS,5SR) et de 2 $cm^3$ d'iodotriméthylsilane en solution dans 50 $cm^3$ de chloroforme anhydre. On obtient ainsi 1,8 g d'(amino-2 acétyl)-1 phényl-6 pipéridino-2 carboxylate de tert-butyle-(2RS,5SR) sous forme d'une huile orangée utilisée telle quelle dans les synthèses ultérieures.

**Masse** (impact électronique, 70eV, m/z), 318 ($M^+$), 159 ($M^+$-$COCH_2NH_2$ et -$CO_2tBu$).

Le (tert-butoxycarbonylamino-2 acétyl)-1 phényl-6 pipéridinecarboxylate-2 de tert-butyle-(2RS,5SR) peut être préparé comme décrit à l'exemple 2 §B, mais à partir d'une solution de 5,1 g de phényl-6 pipéridinecarboxylate-2 de tert-butyle-(2RS,5SR), de 3,4 g d'acide (tert-butoxycarbonylamino)-2 acétique et de 4 g de N,N'-dicyclohexylcarbodiimide en solution dans 90 $cm^3$ d'acétonitrile anhydre. On obtient 5,9 g de (tert-butoxycarbonylamino-2 acétyl)-1 phényl-6 pipéridinecarboxylate-2 de tert-butyle-(2RS,5SR) sous forme d'une huile orangée utilisée telle quelle dans les synthèses ultérieures.

Le phényl-6 pipéridinecarboxylate-2 de tert-butyle-(2RS,5SR) peut être préparé d'une manière analogue à celle décrite à l'exemple 1 §B, mais à partir de 14 g de chlorhydrate de l'acide phényl-6 pipéridinecarboxylique -2-(2RS,5SR), en suspension dans un mélange de 5 $cm^3$ d'acide sulfurique concentré dans 250 $cm^3$ de chloroforme anhydre. Le mélange est saturé par de l'isobutène. Après traitement, on obtient 5,2 g de phényl-6 pipéridinecarboxylate-2 de tert-butyle-(2RS,5SR) fondant à 68°C.

Le chlorhydrate de l'acide phényl-6 pipéridinecarboxylique-2-(2RS,5SR) peut être préparé de la manière suivante : 14 g de chlorhydrate de l'acide phényl-6 tétrahydro-2,3,4,5 pyridinecarboxylique-2, 0,7 g d'oxyde de platine, en suspension dans 200 $cm^3$ d'éthanol sont agités pendant 3 heures à une température voisine de 20°C sous atmosphère d'hydrogène (130 kPa). Le catalyseur est éliminé par filtration et le filtrat est concentré à sec sous pression réduite à 45°C. Après recristallisation dans l'acétonitrile, on obtient 14 g de chlorhydrate d'acide phényl-6 pipéridinecarboxylique-2-(2RS,5SR) fondant à 184°C.

Le chlorhydrate de l'acide phényl-6 tétrahydro-2,3,4,5 pyridinecarboxylique-2 peut être préparé de la manière suivante : à une solution d'éthylate de sodium, préparée à partir de 4,1 g de sodium dans 180 $cm^3$ d'éthanol anhydre, on ajoute 38,3 g d'acétamidomalonate d'éthyle. Après 1 heure d'agitation à une température voisine de 20°C, on verse 40 g de (chloro-3 propyl)-2 phényl-2 dioxolane-1,3 et 2,1 g d'iodure de potassium. Le mélange réactionnel est chauffé à reflux pendant 24 heures puis concentré à sec sous pression réduite à une température voisine de 45°C. Le résidu pâteux est repris par 400 $cm^3$ de dichlorométhane, lavé par 3 fois 100 $cm^3$ d'eau, séché sur sulfate de magnésium et concentré à sec sous pression réduite. Le produit brut est purifié par chromatographie sur silice [éluant : dichlorométhane]. Les fractions contenant le produit attendu sont réunies et concentrées à sec sous pression réduite. On obtient ainsi 25 g d'huile orangée que l'on met en suspension dans 102 $cm^3$ d'eau et 102 $cm^3$ d'acide chlorhydrique 12N. Le mélange réactionnel est chauffé à reflux, sous agitation, pendant 5 heures. Après refroidissement la solution acide est lavée par 3 fois 100 $cm^3$ d'oxyde de diéthyle, puis concentrée à sec sous pression réduite à une température voisine de 50°C. On obtient, après recristallisation dans l'acétonitrile, 14 g de chlorhydrate de l'acide phényl-6 tétrahydro-2,3,4,5 pyridinecarboxylique-2 fondant à 146°C.

Le (chloro-3 propyl)-2 phényl-2 dioxolane-1,3 peut être préparé selon la méthode décrite par M.T. WILLS, J.E. WILLS, L. Von DOLLEN, B.L. BUTLER et J. PORTER, J. Org. Chem., 45 (12), 2495 (1980).

**Exemple 33**

A une suspension de 0,5 g de (furyl-2)-5 prolinate de tert-butyle-(2S,5R) et de 0,48 g d'acide [(méthyl-3 phényl)-3 uréido]-2 acétique dans 50 $cm^3$ d'un mélange anhydre acétonitrile-dichloro-1,2 éthane (80/20 en volumes) maintenue à une température voisine de 0°C, on ajoute en 10 minutes une solution de 0,48 g de N,N'-dicyclohexylcarbodiimide dans 10 $cm^3$ d'acétonitrile anhydre. Le mélange réactionnel est agité pendant 16 heures à une température voisine de 25°C, puis le produit insoluble est séparé par filtration et lavé par trois fois 5 $cm^3$ de dichlorométhane. Le filtrat est concentré à sec sous pression réduite à 40°C. Le résidu huileux obtenu est purifié par chromatographie sur silice [éluant : acétate d'éthyle-cyclohexane (40/60 en volumes)]. Les fractions contenant le produit attendu sont réunies puis concentrées à sec sous pression réduite à 40°C. On obtient ainsi, après recristallisation dans un mélange acétate d'éthyle-cyclohexane (50/50 en volumes), 0,15 g de (furyl-2)-5 {[(méthyl-3 phényl)-3 uréido]-2 acétyl}-1 prolinate de tert-butyle -(2S,5R) fondant à 144°C ($[\alpha]_D^{20}$= +4,8° ±0,8° (C = 0,51%; MeOH)) [RMN du proton (250 MHz, DMSO $D_6$, δ en ppm), 2 rotamères à température ambiante, coalescence à 120°C, description à 120°C: 1,50 (bs, 9H, $(CH_3)_3$); 2,10 (bm, 2H, $CH_2$); 2,30 (bm, 2H, $CH_2$); 2,30 (s, 3H, $CH_3$); 3,8 et 4,0 (ABX, 2H, $CH_2N$); 4,45 (bt, 1H, NCHCOO); 5,20 (bdd, 1H, NCHC); 6,15 (bt, 1H, NH); 6,40 (bdd, 1H, $C_4H_4O$, en position 4); 6,50 (bd, 1H, $C_4H_4O$ en position 3); 6,70 (bd, 1H, N-$C_6H_4$-C en position 4); 7,15 à 7,25 (m, 3H aromatiques); 7,50 (bs, 1H, $C_4H_4O$ en position 5); 8,40 (bs, 1H, NH).Spectre infra-rouge (KBr), bandes caractéristiques en $cm^{-1}$: 3365, 2975, 2930, 2850, 1735, 1640, 1615, 1595, 1560, 1490, 1390, 1365, 1150, 780, 745, 695.

Le (furyl-2)-5 prolinate de tert-butyle-(2S,5R) peut être préparé de la manière suivante : à une solution de 3,8 g de tert-butoxycarbonyl-1 (furyl-2)-5 prolinate de tert-butyle-(2S,5RS) dans 50 $cm^3$ de chloroforme, on ajoute goutte à goutte 1,77 $cm^3$ d'iodotriméthylsilane à une température voisine de 25°C. L'agitation est poursuivie pendant 30 minutes, puis on ajoute 20 $cm^3$ d'eau. La phase aqueuse est séparée puis extraite par 2 fois 20 $cm^3$ de chloroforme. Les phases organiques sont réunies, lavées successivement par 30 $cm^3$ d'une solution aqueuse saturée d'hydrogénocarbonate de sodium et par 2 fois 30 $cm^3$ d'eau, puis séchées sur sulfate de magnésium et concentrées à sec sous pression réduite à 40°C. Le mélange des deux diastéréoisomères obtenu est séparé par chromatographie sur silice [éluant : acétate d'éthyle-cyclohexane (20/80 en volumes)]. Les fractions contenant le produit attendu sont réunies et concentrées à sec sous pression réduite à 40°C. On obtient ainsi 0,50 g de (furyl-2)-5 prolinate de tert-butyle-(2S,5R) sous forme d'une huile jaune-orangée utilisée telle quelle dans les synthèses ultérieures.

Le tert-butoxycarbonyl-1 (furyl-2)-5 prolinate de tert-butyle-(2S,5RS) peut être préparé de la manière suivante : à une solution de 9,6 g de tert-butoxycarbonyl-1 méthoxy-5 prolinate de tert-butyle-(2S,5RS) dans 100 $cm^3$ de furanne, on ajoute 0,42 g d'acide paratoluènesulfonique. Le mélange réactionnel est agité pendant 24 heures à une température voisine de 25°C, puis on ajoute 1 g d'hydrogénocarbonate de sodium. Le milieu hétérogène est concentré à sec sous pression réduite à une température voisine de 30°C. Le résidu obtenu est repris dans 250 $cm^3$ d'acétate d'éthyle et lavé par 2 fois 50 $cm^3$ d'eau. La phase organique est séchée sur sulfate de magnésium et concentrée à sec sous pression réduite à 30°C. Le résidu huileux obtenu est purifié par chromatographie sur silice [éluant : acétate d'éthyle-cyclohexane (5/95 en volumes)]. Les fractions contenant le produit attendu sont réunies et concentrées à sec sous pression réduite à 35°C. On obtient ainsi 3,8 g de tert-butoxycarbonyl-1 (furyl-2)-5 prolinate de tert-butyle-(2S,5RS) fondant à 70°C, mélange (30/70 en poids) des isomères (2S,5R) et (2S,5S) utilisé tel quel dans les synthèses ultérieures.

Le tert-butoxycarbonyl-1 méthoxy-5 prolinate de tert-butyle-(2S,5RS) peut être préparé selon la méthode décrite par T. SHONO et Coll, J. Am. Chem. Soc., 104, 6697 (1982) mais à partir du tert-butoxycarbonyl-1 prolinate de tert-butyle-(2S).

Le tert-butoxycarbonyl-1 prolinate de tert-butyle-(2S) peut être préparé selon la méthode décrite par S. YOSHIFUJI et Coll., Chem. Pharm. Bull., 34, 3873 (1986).

**Exemple 34**

A une solution de 2,0 g d'(amino-2 acétyl)-3 phényl-2 thiazolidinecarboxylate-4 de tert-butyle-(2R,4R) dans 25 $cm^3$ de tétrahydrofuranne anhydre, on ajoute 0,8 $cm^3$ d'isocyanate de méthyl-3 phényle. Le mélange réactionnel est agité pendant 12 heures à une température voisine de 25°C, puis dilué avec 100 $cm^3$ d'acétate d'éthyle. La phase organique est lavée par 2 fois 40 $cm^3$ d'eau, séchée sur sulfate de magnésium et concentrée à sec sous pression réduite à 40°C. Le produit brut obtenu est purifié par chromatographie sur silice [éluant : chlorure de méthylène-méthanol (98/2 en volumes )]. Les fractions contenant le produit attendu sont réunies et concentrées à sec sous pression réduite à 40°C. On obtient ainsi, après battage dans l'heptane 0,5 g de {[(méthyl-3 phényl)-3 uréido]-2 acétyl}-3 phényl-2 thiazolidinecarboxylate-4 de tert-butyle-(2R,4R) sous forme d'une poudre blanche amorphe ($[\alpha]_D^{20}$ = +70,0° ±1,3° (C = 1,02%; $CHCl_3$)) [RMN du proton (200 MHz, $CDCl_3$, d en ppm), 2 rotamères à température ambiante, description pour le rotamère majoritaire à 25°C: 1,45 (bs, 9H, $(CH_3)_3$); 2,2 (s, 3H, $CH_3$); 3,2 ($A_2X$, 2H, $CH_2S$); 3,2 et 4,3 (ABX, 2H, $CH_2N$); 4,9 (t, 1H, CHN); 6,1 (bdd, 1H, NH); 6,2 (s, 1H, SCHN); 6,8 à 7,7 (m, 9H aromatiques). Spectre infra-rouge (KBr), bandes caractéristiques en $cm^{-1}$: 3375, 2975, 2930, 1735, 1650, 1610, 1595, 1560, 1490, 1455, 1370, 1150, 780, 730, 695].

A - L'(amino-2 acétyl)-3 phényl-2 thiazolidinecarboxylate-4 de tert-butyle-(2R,4R) peut être préparé de la manière suivante : à une solution de 2,5 g de (tert-butoxycarbonylamino-2 acétyl)-3 phényl-2 thiazolidinecarboxylate-4 de tert-butyle-(2R,4R) dans 15 $cm^3$ de chloroforme, on ajoute goutte à goutte 0,9 $cm^3$ d'iodotriméthylsilane à une température voisine de 25°C. Le mélange réactionnel est agité pendant 1 heure à une température voisine de 25°C, puis on ajoute 15 $cm^3$ d'eau. La phase aqueuse est séparée puis extraite par 2 fois 10 $cm^3$ de chloroforme. Les phases organiques sont réunies, lavées successivement par 20 $cm^3$ d'eau, 20 $cm^3$ d'une solution aqueuse saturée d'hydrogénocarbonate de sodium et 20 $cm^3$ d'eau puis séchées sur sulfate de magnésium et concentrées à sec sous pression réduite à 40°C. On obtient ainsi 2 g d'(amino-2 acétyl)-3 phényl-2 thiazolidinecarboxylate-4 de tert-butyle-(2R,4R) sous forme d'une huile jaune pâle utilisée telle quelle dans les synthèses ultérieures.

**RMN du proton** (200 MHz, DMSO $D_6$, d en ppm), 2 rotamères à température ambiante, 1,5 (s, 9H, $C(CH_3)_3$), 2,9 à 4,2 (vbm, 4H, 2 $CH_2$); 4,8 et 5,4 (2m, 1H, NCHCO); 6,2 et 6,55 (2s 1H, NCHS); 7,2 à 7,8 (m, 5H, aromatiques); 8,3 (vbs, 1H, $NH_3^+$).

B - Le (tert-butoxycarbonylamino-2 acétyl)-3 phényl-2 thiazolidinecarboxylate-4 de tert-butyle-(2R,4R) peut être préparé de la manière suivante : à une solution de 37,4 g de phényl-2 thiazolidinecarboxylate-4 de tert-butyle-(2RS,4R) et de 24,7 g d'acide tert-butoxycarbonylamino-2 acétique dans 150 $cm^3$ d'acétonitrile anhydre maintenue à une température voisine de 0°C, on ajoute en 30 minutes une solution de 29,1 g de N,N'-dicyclohexylcarbodiimide dans 80 $cm^3$ d'acétonitrile anhydre. Le mélange réactionnel est agité

pendant 16 heures à une température voisine de 25°C, puis le produit insoluble est séparé par filtration et lavé par trois fois 20 $cm^3$ d'acétonitrile. Le filtrat est concentré à sec sous pression réduite à 45°C. Le produit brut est purifié par chromatographie sur silice [éluant : acétate d'éthyle-cyclohexane (30/70 en volumes)]. Les fractions contenant le produit attendu sont réunies et concentrées à sec sous pression réduite. On obtient ainsi 39,6 g de (tert-butoxycarbonylamino-2 acétyl)-3 phényl-2 thiazolidinecarboxylate-4 de tert-butyle-(2R,4R) sous forme d'une huile jaune épaisse utilisée telle quelle dans les synthèses ultérieures, ($[\alpha]_D^{20}$ = + 60,3° ±1,1° (C =0,98%; $CHCl_3$)).

C - Le phényl-2 thiazolidinecarboxylate-4 de tert-butyle-(2RS,4R) peut être préparé de la manière suivante : à une suspension de 53,7 g d'acide phényl-2 thiazolidinecarboxylique-4-(2RS,4R) dans 590 $cm^3$ de chloroforme refroidie à une température voisine de 5°C, on ajoute goutte à goutte 15 $cm^3$ d'acide sulfurique concentré. Le milieu réactionnel est saturé par de l'isobutène pendant 5 heures en agitant et en maintenant la température à 5°C. Après retour à une température voisine de 20°C, l'agitation est poursuivie pendant 20 heures. La solution est amenée à pH 8 par addition d'une solution aqueuse saturée d'hydrogénocarbonate de sodium puis la phase aqueuse est séparée et extraite par 2 fois 300 $cm^3$ de chloroforme. Les phases organiques réunies sont lavées par 2 fois 300 $cm^3$ d'eau, séchées sur sulfate de magnésium et concentrées à sec sous pression réduite à 40°C. Le résidu huileux obtenu est purifié par chromatographie sur silice [éluant : acétate d'éthyle-cyclohexane (20/80 en volumes)]. Les fractions contenant le produit attendu sont réunies puis concentrées à sec sous pression réduite. On obtient ainsi 42,8 g de phényl-2 thiazolidinecarboxylate-4 de tert-butyle-(2RS,4R) sous forme d'une huile jaune pâle, mélange (40/60 en poids) des isomères (2R,4R) et (2S,4R), utilisée telle quelle dans les synthèses ultérieures.

D - L'acide phényl-2 thiazolidinecarboxylique-4-(2RS,4R) peut être préparé de la manière suivante : à une suspension de 2,4 g de L-cystéine dans 35 $cm^3$ d'éthanol, on ajoute goutte à goutte, à une température voisine de 50°C, 2,3 $cm^3$ de benzaldéhyde. Le milieu réactionnel est chauffé à reflux pendant 2 heures. Après refroidissement à une température voisine de 25°C, le produit insoluble est séparé par filtration et lavé successivement par 20 $cm^3$ d'éthanol et par 20 $cm^3$ d'oxyde de diéthyle. On obtient ainsi 3,7 g d'acide phényl-2 thiazolidinecarboxylique-4-(2RS,4R) fondant à 190°C, utilisé tel quel dans les synthèses ultérieures.

**Exemple 35**

On opère d'une manière analogue à celle décrite à l'exemple 34 mais à partir de 0,3 g d'(amino-2 acétyl)-3 phényl-2 thiazolidinecarboxylate-4 de tert-butyle-(2S,4S) et de 0,12 $cm^3$ d'isocyanate de méthyl-3 phényle. On obtient ainsi, après battage dans le cyclohexane, 0,16 g de {[(méthyl-3 phényl)-3 uréido]-2 acétyl}-3 phényl-2 thiazolidinecarboxylate-4 de tert-butyle-(2S,4S) sous forme d'une poudre amorphe jaune pâle ($[\alpha]_D^{20}$ = -62,0° ±1,1° (C = 1,02%; $CHCl_3$)). [RMN du proton (250 MHz, DMSO $D_6$, δ en ppm), 2 rotamères à température ambiante, coalescence des raies à 120°C, description générale valable pour les autres produits de la famille des thiazolidines (à 120°C) : 1,50 (bs, 9H, $(CH_3)_3$); 2,3 (s, 3H, $CH_3$); 3,25 et 3,5 (ABX, 2H, $CH_2S$); 3,7 et 4,05 (ABX, 2H, $CH_2N$); 5,0 (bdd, 1H, CHN); 6,2 (bds, 1H, NH); 6,4 (bs, 1H, SCHN); 6,7 (bd, 1H, N-$C_6H_4$-C en position 4 ou 6); 7,05 à 7,2 (m, 3H, N-$C_6H_4$-C en position 2, 4 et 5); 7,35 (m, 3H, $C_6H_5$); 7,65 (bd, 2H, $C_6H_5$); 8,35 (bs, 1H, NH). Spectre infra-rouge (KBr), bandes caractéristiques en $cm^{-1}$: 3380, 2990, 2940, 2860, 1745, 1650, 1615, 1600, 1560, 1495, 1460, 1375, 1155, 785, 735, 700].

L'(amino-2 acétyl)-3 phényl-2 thiazolidinecarboxylate-4 de tert-butyle-(2S,4S) peut être préparé comme à l'exemple 34 §A, mais à partir de 0,53 g de (tert-butoxycarbonylamino-2 acétyl)-3 phényl-2 thiazolidinecarboxylate-4 de tert-butyle-(2S,4S) et de 0,18 $cm^3$ d'iodotriméthylsilane. On obtient ainsi 0,31 g d'(amino-2 acétyl)-3 phényl-2 thiazolidinecarboxylate-4 de tert-butyle-(2S,4S) sous forme d'une huile jaune pâle utilisée telle quelle dans les synthèse ultérieures.

Le (tert-butoxycarbonylamino-2 acétyl)-3 phényl-2 thiazolidinecarboxylate-4 de tert-butyle-(2S,4S) peut être préparé comme décrit à l'exemple 34 §B, mais à partir de 0,75 g de phényl-2 thiazolidinecarboxylate-4 de tert-butyle-(2RS,4S), de 0,53 g d'acide tert-butoxycarbonylamino-2 acétique et de 0,62 g de N,N'-dicyclohexylcarbodiimide. On obtient ainsi 0,56 g de (tert-butoxycarbonylamino-2 acétyl)-3 phényl-2 thiazolidinecarboxylate-4 de tert-butyle-(2S,4S) sous forme d'une poudre amorphe beige utilisée telle quelle dans les synthèses ultérieures. ($[\alpha]_D^{20}$= -52,4° ±1,1 ° (C =1,01 %; $CHCl_3$)).

Le phényl-2 thiazolidinecarboxylate-4 de tert-butyle-(2RS,4S) peut être préparé comme à l'exemple 34 §C, mais à partir de 4,5 g d'acide phényl-2 thiazolidinecarboxylique-4-(2RS,4S), de 1,3 $cm^3$ d'acide sulfurique concentré et d'un excès d'isobutène. On obtient ainsi 1,5 g de phényl-2 thiazolidinecarboxylate-4 de tert-butyle-(2RS,4S) sous forme d'une huile jaune pâle, mélange (50/50 en poids) des isomères (2R,4S) et (2S,4S), utilisée telle quelle dans les synthèses ultérieures.

L'acide phényl-2 thiazolidinecarboxylique-4-(2RS,4S) peut être préparé comme à l'exemple 34 §D mais à

partir de 3,0 g de D-cystéine et de 2,85 $cm^3$ de benzaldéhyde. On obtient ainsi 4,5 g d'acide phényl-2 thiazolidinecarboxylique-4-(2RS,4S) fondant à 190°C, utilisé tel quel dans les synthèses ultérieures.

**Exemple 36**

A une solution de 1,1 g de N,N'-diimidazole-carbonyle dans 30 $cm^3$ de chloroforme, on ajoute une solution de 0,97 g d'amino-3 benzoate d'éthyle dans 10 $cm^3$ de chloroforme. Le mélange réactionnel est agité pendant 6 heures à une température voisine de 25°C puis on ajoute 1,3 g d'(amino-2 acétyl)-3 phényl-2 thiazolidinecarboxylate-4 de tert-butyle-(2R,4R) en solution dans 10 $cm^3$ de chloroforme. Le mélange réactionnel est agité pendant 24 heures à une température voisine de 25°C, puis lavé successivement par 50 $cm^3$ d'eau, 40 $cm^3$ d'une solution aqueuse d'acide chlorhydrique 0,5 N et 50 $cm^3$ d'eau. La phase organique est séparée, séchée sur sulfate de magnésium et concentrée à sec sous pression réduite à 40°C. Le résidu huileux obtenu est purifié par chromatographie sur silice [éluant : acétate d'éthyle-cyclohexane (35/65 en volumes)] et les fractions contenant le produit attendu sont réunies et concentrées à sec sous pression réduite à 40°C. On obtient ainsi, après battage dans l'heptane, 1,0 g d'{[(éthoxycarbonyl-3 phényl)-3 uréido]-2 acétyl}-3 phényl-2 thiazolidinecarboxylate-4 de tert-butyle-(2R,4R) sous forme d'une poudre blanche amorphe fondant à 85°C ($[\alpha]_D^{20}$= + 61,5° ±1,2° (C = 0,97%; $CHCl_3$)) [RMN du proton (200 MHz, DMSO $D_6$, δ en ppm), 2 rotamères à température ambiante, coalescence des raies à 120°C, déplacements chimiques caractéristiques à 120°C : 1,3 (s, 3H, $CH_3$); 4,3 (q, 2H, $CH_2O$); 7,3 à 7,7 (m, 8H aromatiques); 8,0 (bs, 1H, N-$C_6H_4$-C en position 2). Spectre infra-rouge (KBr), bandes caractéristiques en $cm^{-1}$: 3375, 2980, 2930, 1720, 1655, 1610, 1600, 1560, 1490, 1370, 1240, 1155, 760, 735, 700, 690].

**Exemple 37**

A une solution de 0,6 g de N,N'-diimidazole-carbonyle dans 15 $cm^3$ de chloroforme, on ajoute une solution de 1,0 g d'(amino-2 acétyl)-3 phényl-2 thiazolidinecarboxylate-4 de tert-butyle-(2R,4R) dans 10 $cm^3$ de chloroforme. Le mélange réactionnel est agité pendant 2 heures à une température voisine de 25°C puis on ajoute 0,44 g d'(amino-3 phényl)-2 éthanol en solution dans 5 $cm^3$ de chloroforme. Le mélange réactionnel est agité pendant 12 heures à une température voisine de 25°C, puis concentré à sec sous pression réduite à 40°C. Le résidu huileux obtenu est purifié par chromatographie sur silice [éluant : acétate d'éthyle] et les fractions contenant le produit attendu sont réunies et concentrées à sec sous pression réduite à 40°C. On obtient ainsi, après recristallisation dans l'acétonitrile, 0,18 g d'{[(hydroxy-2 éthyl)-3 phényl)-3 uréido]-2 acétyl}-3 phényl-2 thiazolidinecarboxylate-4 de tert-butyle-(2R,4R) sous forme de cristaux blancs fondant à 164°C ($[\alpha]_D^{20}$= + 59,6° ±1,8° (C = 0,50%; DMF)) [RMN du proton (300 MHz, DMSO $D_6$, δ en ppm), 2 rotamères à température ambiante, coalescence des raies à 120°C, déplacements chimiques caractéristiques à 120°C : 2,7 et 3,60 (2t, 4H, $CH_2CH_2O$); 6,8 (bd, 1H, N-$C_6H_4$-C en position 4 ou 6); 7,05 à 7,20 (m, 3H, N-$C_6H_4$-C en position 2,5 et 6); 7,3 (m, 3H, $C_6H_5$); 7,6 (d, 2H, $C_6H_5$). Spectre infra-rouge (KBr), bandes caractéristiques en $cm^{-1}$: 3320, 2975, 2930, 2880, 2850, 1740, 1660, 1610, 1590, 1560, 1510, 1480, 1450, 1365, 1150, 1060, 790, 730, 695].

**Exemple 38**

A une solution de 1,3 g de N,N'-diimidazole-carbonyle dans 15 $cm^3$ de tétrahydrofuranne anhydre, on ajoute en 2 heures une solution de 2,6 g d'(amino-2 acétyl)-3 phényl-2 thiazolidinecarboxylate-4 de tert-butyle-(2R,4R) dans 15 $cm^3$ de tétrahydrofuranne anhydre. Le mélange réactionnel est agité à une température voisine de 25°C pendant 12 heures puis concentré à sec sous pression réduite à 40°C. Le résidu huileux obtenu est purifié par chromatographie sur silice [éluant : acétate d'éthyle]. Une solution de 2,8 g d'[(imidazolyl-1 carboxamido)-2 acétyl]-3 phényl-2 thiazolidinecarboxylate-4 de tert-butyle-(2R,4R) ainsi obtenu et de 2,7 g d'amino-3 phénylacétate de méthyle dans 100 $cm^3$ de toluène est chauffée à reflux pendant 4 heures. Après refroidissement à une température voisine de 25°C, le mélange réactionnel est lavé successivement par 50 $cm^3$ d'eau, 50 $cm^3$ d'une solution aqueuse d'acide chlorhydrique 1N et 2 fois 50 $cm^3$ d'eau. La phase organique est séparée, séchée sur sulfate de magnésium et concentrée à sec sous pression réduite à 40°C. Le résidu huileux obtenu est purifié par chromatographie sur silice [éluant : acétate d'éthyle-cyclohexane (50/50 en volumes)]. Les fractions contenant le produit attendu sont réunies puis concentrées à sec sous pression réduite à 30°C. On obtient ainsi, après battage dans l'heptane, 1,9 g de {[(tert-butoxycarbonyl-4 phényl-2 thiazolidinyl-3)-2 oxo-2 éthyl]-3 uréido}-3 phénylacétate de méthyle-(2R,4R) sous forme d'une poudre beige pâle fondant à 69°C ($[\alpha]_D^{20}$ = + 59,1° ±1,2° (C = 0,55%; $CHCl_3$)) [RMN du proton (200 MHz, DMSO $D_6$, δ en ppm), 2 rotamères à température ambiante, coalescence des raies à 120°C, déplacements chimiques caractéristiques à 120°C : 3,7 (2s, 5H, $CH_2CO_2CH_3$); 6,8 (d, 1H, N-$C_6H_4$-C en position 4 ou 6); 7,1 à 7,7 (m, 8H aromatiques). Spectre

infra-rouge (KBr), bandes caractéristiques en $cm^{-1}$: 3370, 2980, 2950, 2930, 1740, 1650, 1610, 1595, 1560, 1495, 1370, 1240, 1155, 780, 735, 700].

**Exemple 39**

A une solution de 1,2 g de {[(tert-butoxycarbonyl-4 phényl-2 thiazolidinyl-3)-2 oxo-2 éthyl]-3 uréido}-3 phénylacétate de méthyle-(2R,4R) dans 25 $cm^3$ d'un mélange eau-tétrahydrofuranne (30/70 en volumes) on ajoute, à une température voisine de 5°C, 0,1 g d'hydroxyde de lithium. Le mélange réactionnel est agité pendant 12 heures à une température voisine de 25°C, puis concentré à 5 $cm^3$ sous pression réduite à 40°C. La solution obtenue est diluée avec 50 $cm^3$ d'eau, lavée par 2 fois 30 $cm^3$ d'éther éthylique, puis acidifiée à pH 2 avec 2,8 $cm^3$ d'une solution aqueuse d'acide chlorhydrique 1N. Le produit insoluble est séparé par filtration, lavé par 3 fois 10 $cm^3$ d'eau puis séché à l'air. On obtient ainsi 0,7 g d'acide {[(tert-butoxycarbonyl-4 phényl-2 thiazolidinyl-3)-2 oxo-2 éthyl]-3 uréido}-3 phénylacétique-(2R,4R) fondant à 140°C ($[\alpha]_D^{20}$= + 62,6° ±1,6° (C = 0,64%; DMF)) [RMN du proton (200 MHz, DMSO $D_6$, $\delta$ en ppm), 2 rotamères à température ambiante, coalescence des raies à 120°C, déplacements chimiques caractéristiques à 120°C : 3,60 (bs, 2H, $CH_2CO_2H$); 6,8 (bd, 1H, N-$C_6H_4$-C en position 6); 7,15 à 7,80 (m, 8H aromatiques). Spectre infra-rouge (KBr), bandes caractéristiques en $cm^{-1}$: 3385, 2985, 2945, 2625, 1735, 1650, 1615, 1600, 1560, 1500, 1375, 1240, 1155, 785, 735, 705].

**Exemple 40**

On opère d'une manière analogue à celle décrite à l'exemple 33, mais à partir de 3,5 g de (fluoro-2 phényl)-2 thiazolidinecarboxylate-4 de tert-butyle-(2RS,4R), de 2,3 g d'acide [(méthyl-3 phényl)-3 uréido]-2 acétique et de 2,3 g de N,N'-dicyclohexylcarbodiimide. On obtient ainsi, après battage dans l'heptane, 1,05 g de {[(méthyl-3 phényl)-3 uréido]-2 acétyl}-3 (fluoro-2 phényl)-2 thiazolidinecarboxylate-4 de tert-butyle-(2R,4R) sous forme d'un solide blanc fondant à 107°C ($[\alpha]_D^{20}$= +61,8° ±1,5° (C =0,66%; DMF)) [RMN du proton (200 MHz, DMSO $D_6$, $\delta$ en ppm), 2 rotamères à température ambiante, coalescence des raies à 120°C, déplacements chimiques caractéristiques à 120°C : 2,2 (bs, 3H, $CH_3$); 6,7 (bd, 1H, N-$C_6H_4$-C en position 4 ou 6); 7,1 à 7,40 (m, 6H aromatiques); 7,9 (bm, 1H, $C_6H_4F$ en position 3). Spectre infra-rouge (KBr), bandes caractéristiques en $cm^{-1}$: 3370, 2975, 2925, 1735, 1645, 1615, 1590, 1560, 1490, 1460, 1370, 1150, 775, 760, 690].

Le (fluoro-2 phényl)-2 thiazolidinecarboxylate-4 de tert-butyle-(2RS,4R) peut être préparé comme décrit à l'exemple 34 §C, mais à partir de 5,7 g d'acide (fluoro-2 phényl)-2 thiazolidinecarboxylique-4-(2RS,4R) en solution dans 60 $cm^3$ de chloroforme, de 1,5 $cm^3$ d'acide sulfurique concentré et d'un excès d'isobutène. On obtient ainsi 5,8 g de (fluoro-2 phényl)-2 thiazolidinecarboxylate-4 de tert-butyle-(2RS,4R) sous forme d'une huile jaune, mélange (50/50 en poids) des isomères (2R,4R) et (2S,4R), utilisée telle quelle dans les synthèses ultérieures.

L'acide (fluoro-2 phényl)-2 thiazolidinecarboxylique-4-(2RS,4R) peut être préparé comme décrit à l'exemple 34 §D mais à partir de 21,2 g de L-cystéine et de 19,2 $cm^3$ de fluoro-2 benzaldéhyde. On obtient ainsi 28,2 g d'acide (fluoro-2 phényl)-2 thiazolidinecarboxylique-4-(2RS,4R) fondant à 147°C, utilisé tel quel dans les synthèses ultérieures.

**Exemple 41**

A une solution de 4,2 g de {{[tert-butoxycarbonyl-4 (méthoxy-3 phényl)-2 thiazolidinyl-3]-2 oxo-2 éthyl}-3 uréido}-3 benzoate de triméthylsilyl-2 éthyle-(2R,4R) dans 80 $cm^3$ de tétrahydrofuranne on ajoute, à une température voisine de 25°C, 13,7 $cm^3$ d'une solution 1M de fluorure de tétrabutylammonium dans le tétrahydrofuranne. Le mélange réactionnel est agité pendant 12 heures à une température voisine de 25°C, puis acidifié avec 15 $cm^3$ d'une solution aqueuse d'acide sulfurique 1N. Après extraction par 3 fois 50 $cm^3$ d'acétate d'éthyle, les phases organiques réunies sont lavées par 60 $cm^3$ d'eau puis extraites par 2 fois 20 $cm^3$ d'une solution aqueuse de soude 1N. Les phases aqueuses sont séparées, lavées par 2 fois 40 $cm^3$ d'oxyde de diéthyle, acidifiées à pH 4 par une solution aqueuse d'acide sulfurique 4N et extraites par 3 fois 50 $cm^3$ d'oxyde de diéthyle. Les phases organiques sont réunies, séchées sur sulfate de magnésium et concentrées à sec sous pression réduite à 30°C. Le solide obtenu est dissous dans 10 $cm^3$ d'une solution aqueuse de soude 0,5 N. La solution obtenue est filtrée et acidifiée par 5 $cm^3$ d'une solution aqueuse d'acide chlorhydrique 1N. Le produit insoluble est séparé par filtration, lavé par 2 fois 5 $cm^3$ d'eau et séché à l'air. On obtient ainsi 1,48 g d'acide {{[tert-butoxycarbonyl-4 (méthoxy-3 phényl)-2 thiazolidinyl-3]-2 oxo-2 éthyl}-3 uréido}-3 benzoïque-(2R,4R) fondant à 125°C ($[\alpha]_D^{20}$= +74° ±2° (C = 0,32%; DMF)) [RMN du proton (200 MHz, DMSO $D_6$, $\delta$ en ppm), 2 rotamères à température ambiante, coalescence des raies à 120°C, déplacements chimiques caractéristiques à 120°C : 3,8 (s, 3H, $OCH_3$); 6,85 (bd, 1H, O-$C_6H_4$-C en position 4); 7,2 à 7,6 (m, 6H aromatiques); 8,0 (bs, 1H, N-$C_6H_4$-

en position 2). Spectre infra-rouge (KBr), bandes caractéristiques en $cm^{-1}$: 3380, 2975, 2930, 2830, 2600, 1715, 1690, 1655, 1600, 1555, 1490, 1370, 1230, 1150, 1050, 755, 690, 680].

A - Le {{[tert-butoxycarbonyl-4 (méthoxy-3 phényl)-2 thiazolidinyl-3]-2 oxo-2 éthyl}-3 uréido}-3 benzoate de triméthylsilyl-2 éthyle-(2R,4R) peut être préparé de la manière suivante : à une solution de 3,5 g de (méthoxy-3 phényl)-2 thiazolidinecarboxylate-4 de tert-butyle-(2RS,4R) et de 4,0 g d'acide {[(triméthylsilyl-2 éthoxycarbonyl)-3 phényl]-3 uréido}-2 acétique dans 20 $cm^3$ de tétrahydrofuranne anhydre refroidie à une température voisine de 5°C, on ajoute en 10 minutes une solution de 2,45 g de N,N'-dicyclohexylcarbodiimide dans 10 $cm^3$ de tétrahydrofuranne anhydre. Le mélange réactionnel est agité pendant 16 heures à une température voisine de 25°C. Le produit insoluble est séparé par filtration et lavé par 2 fois 10 $cm^3$ de tétrahydrofuranne. Le filtrat est concentré à sec sous pression réduite à 40°C. Le résidu huileux obtenu est purifié par chromatographie sur silice [éluant : acétate d'éthyle-cyclohexane (30/70 en volumes)]. Les fractions contenant le produit attendu sont réunies et concentrées à sec sous pression réduite à 40°C. On obtient ainsi 4,85 g de {{[tert-butoxycarbonyl-4 (méthoxy-3 phényl)-2 thiazolidinyl-3]-2 oxo-2 éthyl}-3 uréido}-3 benzoate de triméthylsilyl-2 éthyle-(2R,4R) sous forme d'une poudre beige amorphe utilisée telle quelle dans les synthèses ultérieures, ($[\alpha]_D^{20}$= + 67,2° ±1,3° (C = 0,52%; $CHCl_3$)).

B - Le (méthoxy-3 phényl)-2 thiazolidinecarboxylate-4 de tert-butyle-(2RS,4R) peut être préparé d'une manière analogue à celle décrite à l'exemple 34 §C mais à partir de 14,0 g d'acide (méthoxy-3 phényl)-2 thiazolidinecarboxylique-4-(2RS,4R), de 3,0 $cm^3$ d'acide sulfurique concentré et d'un excès d'isobutène. Le produit brut obtenu est purifié par chromatographie sur silice [éluant : acétate d'éthyle-cyclohexane (30/70 en volumes)]. Les fractions contenant le produit attendu sont réunies et concentrées à sec sous pression réduite à 40°C. On obtient ainsi 11,0 g de (méthoxy-3 phényl)-2 thiazolidinecarboxylate-4 de tert-butyle-(2RS,4R) sous forme d'une huile jaune, mélange (40/60 en poids) des isomères (2R,4R) et (2S,4R) utilisée telle quelle dans les synthèses ultérieures.

C - L'acide (méthoxy-3 phényl)-2 thiazolidinecarboxylique-4-(2RS,4R) peut être préparé comme décrit à l'exemple 34 §D mais à partir de 21,2 g de L-cystéine et de 23 $cm^3$ de méthoxy-3 benzaldéhyde. On obtient ainsi 38,0 g d'acide (méthoxy-3 phényl)-2 thiazolidinecarboxylique-4-(2RS,4R) fondant à 171°C, utilisé tel quel dans les synthèses ultérieures.

D -L'acide {[(triméthylsilyl-2 éthoxycarbonyl)-3 phényl]-3 uréido}-2 acétique peut être préparé de la manière suivante : à une solution de 24,7 g d'(éthoxycarbonylméthyl-3 uréido)-3 benzoate de triméthylsilyl-2 éthyle dans 500 $cm^3$ de tétrahydrofuranne on ajoute une solution de 4,45 g de potasse dans 160 $cm^3$ d'eau. Le mélange réactionnel est agité pendant 12 heures à une température voisine de 25°C, puis concentré à 150 $cm^3$ sous pression réduite à 40°C. La solution obtenue est diluée avec 200 $cm^3$ d'eau, lavée par 2 fois 50 $cm^3$ d'acétate d'éthyle, acidifiée avec 68 $cm^3$ d'une solution aqueuse d'acide chlorhydrique 1N et extraite par 3 fois 100 cm3 d'oxyde de diéthyle. Les phases organiques sont réunies, séchées sur sulfate de magnésium et concentrées à sec sous pression réduite. On obtient, après cristallisation dans l'oxyde de diisopropyle, 17,2 g d'acide {[(triméthylsilyl-2 éthoxycarbonyl)-3 phényl]-3 uréido}-2 acétique fondant à 173°C.

E - L'(éthoxycarbonylméthyl-3 uréido)-3 benzoate de triméthylsilyl-2 éthyle peut être préparé de la manière suivante : une solution de 29,5 g d'(imidazolyl-1 carboxamido)-2 acétate d'éthyle et de 36,2 g d'amino-3 benzoate de triméthylsilyl-2 éthyle dans 1000 $cm^3$ de toluène est agitée à reflux pendant 3 heures. Après refroidissement à une température voisine de 25°C, le mélange réactionnel est lavé par 3 fois 50 $cm^3$ d'eau, séché sur sulfate de magnésium et concentré à sec sous pression réduite à 40°C. Le résidu huileux obtenu est purifié par chromatographie sur silice [éluant : acétate d'éthyle-cyclohexane (40/60 en volumes)]. Les fractions contenant le produit attendu sont réunies et concentrées à sec sous pression réduite à 40°C. On obtient ainsi 24,7 g d'(éthoxycarbonylméthyl-3 uréido)-3 benzoate de triméthylsilyl-2 éthyle sous forme d'une huile jaune épaisse utilisée telle quelle dans les synthèses ultérieures.

F - L'(imidazolyl-1 carboxamido)-2 acétate d'éthyle peut être préparé de la manière suivante : à une solution de 50,0 g de N,N'-diimidazole-carbonyle et de 43 $cm^3$ de triéthylamine dans 800 $cm^3$ de tétrahydrofuranne anhydre, on ajoute en 2 heures, 42,3 g de chlorhydrate d'amino-2 acétate d'éthyle. Le mélange est agité pendant 24 heures à une température voisine de 25°C puis le produit insoluble est séparé par filtration et lavé par 2 fois 50 $cm^3$ de tétrahydrofuranne. Le filtrat est concentré à sec sous pression réduite à 40°C. Le résidu huileux obtenu est purifié par chromatographie sur silice [éluant : acétate d'éthyle]. Les fractions contenant le produit attendu sont réunies et concentrées à sec sous pression réduite. On obtient ainsi 36,7 g d'(imidazolyl-1 carboxamido)-2 acétate d'éthyle fondant à 103°C.

G - L'amino-3 benzoate de triméthylsilyl-2 éthyle peut être préparé de la manière suivante : à une solution de 42,7 g de nitro-3 benzoate de triméthylsilyl-2 éthyle dans 600 $cm^3$ d'éthanol, on ajoute 2,1 g de palladium sur noir à 5 %. La suspension est agitée pendant 2 heures à une température voisine de 25°C sous atmosphère d'hydrogène (100 kPa). Le catalyseur est ensuite séparé par filtration et le filtrat est concentré

à sec sous pression réduite à 40°C. On obtient ainsi 36,2 g d'amino-3 benzoate de triméthylsilyl-2 éthyle sous forme d'une huile utilisée telle quelle dans les synthèses ultérieures.

H - Le nitro-3 benzoate de triméthylsilyl-2 éthyle peut être préparé de la manière suivante : à une solution de 66,0 g de chlorure de nitro-3 benzoyle dans 1000 $cm^3$ de dichloro-1,2 éthane refroidie à 5°C, on ajoute une solution de 41,8 g de triméthylsilyl-2 éthanol dans 200 $cm^3$ de dichloro-1,2 éthane et 51 $cm^3$ de triéthylamine. Le mélange réactionnel est agité pendant 12 heures à une température voisine de 25°C puis le produit insoluble est séparé par filtration et lavé par 2 fois 50 $cm^3$ de dichloro-1,2 éthane. Le filtrat est concentré à sec sous pression réduite à 40°C. Le résidu huileux obtenu est purifié par chromatographie sur silice [éluant : acétate d'éthyle-cyclohexane (30/70 en volumes)]. Les fractions contenant le produit attendu sont réunies et concentrées à sec sous pression réduite à 40°C. On obtient ainsi 76,9 g de nitro-3 benzoate de triméthylsilyl-2 éthyle sous forme d'une huile utilisée telle quel le dans les synthèses ultérieures.

**Exemple 42**

On opère d'une manière analogue à celle décrite à l'exemple 41, mais à partir de 2,3 g de {{[tert-butoxycarbonyl-4 (fluoro-2 phényl)-2 thiazolidinyl-3]-2 oxo-2 éthyl}-3 uréido}-3 benzoate de triméthylsilyl-2 éthyle-(2R,4R) et de 7,6 $cm^3$ d'une solution 1M de fluorure de tétrabutylammonium. Le produit brut est purifié par chromatographie sur silice [éluant : acétate d'éthyle-méthanol (90/10 en volumes)]. Les fractions contenant le produit attendu sont réunies et concentrées à sec sous pression réduite à 40°C. On obtient ainsi, après battage dans l'oxyde de diisopropyle, 0,4 g d'acide {{[tert-butoxycarbonyl-4 (fluoro-2 phényl)-2 thiazolidinyl-3]-2 oxo-1 éthyl}-2 uréido}-3 benzoïque-(2R,4R) sous forme d'un solide amorphe ($[\alpha]_D^{20}$= +68,3° ±1,8° (C = 0,57%; $CHCl_3$)) [RmN du proton (200 MHz, DMSO $D_6$, δ en ppm), 2 rotamères à température ambiante, coalescence des raies à 120°C, déplacements chimiques caractéristiques à 120°C : 7,2 à 7,6 (m, 6H aromatiques); 7,9 (bt, 1H, F-$C_6H_4$- en position 3, $J_{HF}$= 9 Hz); 8,0 (bs, 1H, N-$C_6H_4$- en position 2). Spectre infra-rouge (KBr), bandes caractéristiques en $cm^{-1}$: 3380, 2980, 2930, 2600, 1735, 1690, 1655, 1610, 1590, 1555, 1490, 1455, 1370, 1230, 1150, 760, 680].

Le {{[tert-butoxycarbonyl-4 (fluoro-2 phényl)-2 thiazolidinyl-3]-2 oxo-2 éthyl}-3 uréido}-3 benzoate de triméthylsilyl-2 éthyle-(2R,4R) peut être préparé d'une manière analogue à celle décrite à l'exemple 41 §A mais à partir de 3,35 g de (fluoro-2 phényl)-2 thiazolidinecarboxylate-4 de tert-butyle-(2RS,4R), de 4,0 g d'acide {[(triméthylsilyl-2 éthoxycarbonyl)-3 phényl]-3 uréido}-2 acétique et de 2,45 g de N,N'-dicyclohexylcarbodiimide. Le produit brut est purifié par chromatographie sur silice [éluant : acétate d'éthyle-cyclohexane (30/70 en volumes)]. Les fractions contenant le produit attendu sont réunies et concentrées à sec sous pression réduite à 40°C. On obtient ainsi 2,4 g de {{[tert-butoxycarbonyl-4 (fluoro-2 phényl)-2 thiazolidinyl-3]-2 oxo-2 éthyl}-3 uréido}-3 benzoate de triméthylsilyl-2 éthyle-(2R,4R) sous forme d'un solide beige amorphe utilisé tel quel dans les synthèses ultérieures, ($[\alpha]_D^{20}$ = + 50,2° ±1,6° (C = 0,38%; $CHCl_3$)).

**Exemple 43**

On opère d'une manière analogue à celle décrite à l'exemple 41, mais à partir de 1,6 g de {{[tert-butoxycarbonyl-4 (fluoro-3 phényl)-2 thiazolidinyl-3]-2 oxo-2 éthyl}-3 uréido}-3 benzoate de triméthylsilyl-2 éthyle-(2R,4R) et de 5,3 $cm^3$ d'une solution 1M de fluorure de tétrabutylammonium. Le produit brut est purifié par chromatographie sur silice [éluant : acétate d'éthyle-méthanol (90/10 en volumes)]. Les fractions contenant le produit attendu sont réunies et concentrées à sec sous pression réduite à 40°C. On obtient ainsi, après battage dans l'oxyde de diisopropyle, 0,22 g d'acide {{[tert-butoxycarbonyl-4 (fluoro-3 phényl)-2 thiazolidinyl-3]-2 oxo-2 éthyl}-3 uréïdo}-3 benzoïque-(2R,4R) sous forme d'un solide amorphe ($[\alpha]_D^{20}$= + 79,0° ±2,0° (C = 0,43%; $CHCl_3$)) [RMN du proton (250 MHz, DMSO $D_6$, δ en ppm), 2 rotamères à température ambiante, déplacements chimiques caractéristiques à 25°C: 1,5 (bs, 9H, $(CH_3)_3$); 7,1 à 7,6 (m, 7H aromatiques); 8,0 (bs, 1H, N-$C_6H_4$- en position 2). Spectre infra-rouge (KBr), bandes caractéristiques en $cm^{-1}$: 3380, 2975, 2930, 2600, 1715, 1695, 1650, 1610, 1590, 1560, 1490, 1450, 1370, 1240, 1150, 780, 755, 685].

Le {{[tert-butoxycarbonyl-4 (fluoro-3 phényl)-2 thiazolidinyl-3]-2 oxo-2 éthyl}-3 uréido}-3 benzoate de triméthylsilyl-2 éthyle-(2R,4R) peut être préparé d'une manière analogue à celle décrite à l'exemple 41 §A mais à partir de 1,68 g de (fluoro-3 phényl)-2 thiazolidinecarboxylate-4 de tert-butyle-(2RS,4R), de 2,0 g d'acide {[(triméthylsilyl-2 éthoxycarbonyl)-3 phényl]-3 uréido}-2 acétique et de 1,23 g de N,N'-dicyclohexylcarbodiimide. Le produit brut est purifié par chromatographie sur silice [éluant : acétate d'éthyle-cyclohexane (30/70 en volumes)]. Les fractions contenant le produit attendu sont réunies et concentrées à sec sous pression réduite à 40°C. On obtient ainsi 1,6 g de {{[tert-butoxycarbonyl-4 (fluoro-3 phényl)-2 thiazolidinyl-3]-2 oxo-2 éthyl}-3 uréido}-3 benzoate de triméthylsilyl-2 éthyle-(2R,4R) sous forme d'un solide beige amorphe utilisé tel quel dans

les synthèses ultérieures, ($[\alpha]_D^{20}$= + 45,7° ±1,5° (C = 0,53%; $CHCl_3$)).

Le (fluoro-3 phényl)-2 thiazolidinecarboxylate-4 de tert-butyle-(2RS,4R) peut être préparé d'une manière analogue à celle décrite à l'exemple 34 §C mais à partir de 11,4 g d'acide (fluoro-3 phényl)-2 thiazolidinecarboxylique-4-(2RS,4R) en solution dans 200 cm³ de chloroforme, de 3,0 cm³ d'acide sulfurique concentré et d'un excès d'isobutène. Le produit brut obtenu est purifié par chromatographie sur silice [éluant : acétate d'éthyle-cyclohexane (30/70 en volumes)]. Les fractions contenant le produit attendu sont réunies et concentrées à sec sous pression réduite à 40°C. On obtient ainsi 10,3 g de (fluoro-3 phényl)-2 thiazolidinecarboxylate-4 de tert-butyle-(2RS,4R) sous forme d'une huile jaune, mélange (30/70 en poids) des isomères (2R,4R) et (2S,4R), utilisée telle quelle dans les synthèses ultérieures.

L'acide (fluoro-3 phényl)-2 thiazolidinecarboxylique-4-(2RS,4R) peut être préparé comme décrit à l'exemple 34 §D mais à partir de 21,2 g de L-cystéine et de 20 cm³ de fluoro-3 benzaldéhyde. On obtient ainsi 30,2 g d'acide (fluoro-3 phényl)-2 thiazolidinecarboxylique-4-(2RS,4R) fondant à 178°C, utilisé tel quel dans les synthèses ultérieures.

**Exemple 44**

On opère d'une manière analogue à celle décrite à l'exemple 41, mais à partir de 2,0 g de {{[tert-butoxycarbonyl-4 (fluoro-4 phényl)-2 thiazolidinyl-3]-2 oxo-2 éthyl}-3 uréido}-3 benzoate de triméthylsilyl-2 éthyle-(2R,4R) et de 6,6 cm³ d'une solution 1M de fluorure de tétrabutylammonium. Le produit brut est purifié par chromatographie sur silice [éluant : acétate d'éthyle]. Les fractions contenant le produit attendu sont réunies et concentrées à sec sous pression réduite à 40°C. On obtient ainsi, après battage dans l'oxyde de diisopropyle, 0,74 g d'acide {{[tert-butoxycarbonyl-4 (fluoro-4 phényl)-2 thiazolidinyl-3]-2 oxo-1 éthyl}-2 uréido}-3 benzoïque-(2R,4R) fondant à 154°C (($[\alpha]_D^{20}$= +49,7° ±1,3° (C = 0,46%; DMF)) [RMN du proton (250 MHz, DMSO $D_6$, $\delta$ en ppm), 2 rotamères à température ambiante, déplacements chimiques caractéristiques à 25°C: 1,5 (bs, 9H, $(CH_3)_3$); 7,1 à 7,8 (m, 7H aromatiques); 8,0 (bs, 1H, N-$C_6H_4$- en position 2). Spectre infra-rouge (KBr), bandes caractéristiques en $cm^{-1}$: 3380, 2985, 2945, 2625, 1695, 1650, 1610, 1560, 1510, 1490, 1375, 1235, 1155, 800, 760, 685].

Le {{[tert-butoxycarbonyl-4 (fluoro-4 phényl)-2 thiazolidinyl-3]-2 oxo-2 éthyl}-3 uréido}-3 benzoate de triméthylsilyl-2 éthyle-(2R,4R) peut être préparé d'une manière analogue à celle décrite à l'exemple 41 §A mais à partir de : 1,68 g de (fluoro-4 phényl)-2 thiazolidinecarboxylate-4 de tert-butyle-(2RS,4R), de 2,0 g d'acide {[(triméthylsilyl-2 éthoxycarbonyl)-3 phényl]-3 uréido}-2 acétique et de 1,23 g de N,N'-dicyclohexylcarbodiimide. Le produit brut est purifié par chromatographie sur silice [éluant : acétate d'éthyle-cyclohexane (30/70 en volumes)]. Les fractions contenant le produit attendu sont réunies et concentrées à sec sous pression réduite à 40°C. On obtient ainsi 2,0 g de {{[tert-butoxycarbonyl-4 (fluoro-4 phényl)-2 thiazolidinyl-3]-2 oxo-2 éthyl}-3 uréido}-3 benzoate de triméthylsilyl-2 éthyle-(2R,4R) sous forme d'un solide beige amorphe utilisé tel quel dans les synthèses ultérieures, ($[\alpha]_D^{20}$ = +46,6° ±1° (C = 0,50%; $CHCl_3$)).

Le (fluoro-4 phényl)-2 thiazolidinecarboxylate-4 de tert-butyle-(2RS,4R) peut être préparé d'une manière analogue à celle décrite à l'exemple 34 §C mais à partir de 11,4 g d'acide (fluoro-4 phényl)-2 thiazolidine-carboxylique-4-(2RS,4R), de 3,0 cm³ d'acide sulfurique concentré et d'un excès d'isobutène. Le produit brut obtenu est purifié par chromatographie sur gel de silice [éluant : acétate d'éthyle-cyclohexane (30/70 en volumes)]. Les fractions contenant le produit attendu sont réunies et concentrées à sec sous pression réduite à 40°C. On obtient ainsi 10,0 g de (fluoro-4 phényl)-2 thiazolidinecarboxylate-4 de tert-butyle-(2RS,4R) sous forme d'une huile jaune, mélange (40/60 en poids) des isomères (2R,4R) et (2S,4R), utilisée telle quelle dans les synthèses ultérieures.

L'acide (fluoro-4 phényl)-2 thiazolidinecarboxylique-4-(2RS,4R) peut être préparé comme décrit à l'exemple 34 §D mais à partir de 21,2 g de L-cystéine et de 20 cm³ de fluoro-4 benzaldéhyde. On obtient ainsi 34,3 g d'acide (fluoro-4 phényl)-2 thiazolidinecarboxylique-4-(2RS,4R) fondant à 186°C, utilisé tel quel dans les synthèses ultérieures.

**Exemple 45**

On opère d'une manière analogue à celle décrite à l'exemple 41, mais à partir de 2,0 g de {{[tert-butoxycarbonyl-4 (chloro-2 phényl)-2 thiazolidinyl-3]-2 oxo-2 éthyl}-3 uréido}-3 benzoate de triméthylsilyl-2 éthyle-(2R,4R) et de 6,5 cm³ d'une solution 1M de fluorure de tétrabutylammonium. Le produit brut est purifié par chromatographie sur silice [éluant : acétate d'éthyle-méthanol (90/10 en volumes)]. Les fractions contenant le produit attendu sont réunies et concentrées à sec sous pression réduite à 40°C. On obtient ainsi, après battage dans l'oxyde de diisopropyle, 0,65 g d'acide {{[tert-butoxycarbonyl-4 (chloro-2 phényl)-2 thiazolidinyl-3]-2 oxo-2 éthyl}-3 uréido}-3 benzoïque-(2R,4R) sous forme d'un solide amorphe ($[\alpha]_D^{20}$= + 88° ± 2° (C = 0,39%;

$CHCl_3$)) [RMN du proton (200 MHz, DMSO $D_6$, $\delta$ en ppm), 2 rotamères à température ambiante, coalescence des raies à 120°C, déplacements chimiques caractéristiques à 120°C : 7,3 à 7,6 (m, 7H aromatiques); 7,95 (bs, 1H, N-$C_6H_4$- en position 2). Spectre infra-rouge (KBr), bandes caractéristiques en $cm^{-1}$: 3390, 2985, 2940, 2600, 1735, 1660, 1610, 1595, 1560, 1375, 1240, 1155, 755, 685].

Le {{[tert-butoxycarbonyl-4 (chloro-2 phényl)-2 thiazolidinyl-3]-2 oxo-2 éthyl}-3 uréido}-3 benzoate de triméthylsilyl-2 éthyle-(2R,4R) peut être préparé d'une manière analogue à celle décrite à l'exemple 41 §A mais à partir de 1,6 g de (chloro-2 phényl)-2 thiazolidinecarboxylate-4 de tert-butyle-(2RS,4R), de 2,0 g d'acide {[(triméthylsilyl-2 éthoxycarbonyl)-3 phényl]-3 uréido}-2 acétique et de 1,23 g de N,N'-dicyclohexylcarbodiimide. Le produit brut est purifié par chromatographie sur silice [éluant : acétate d'éthyle-cyclohexane (30/70 en volumes)]. Les fractions contenant le produit attendu sont réunies et concentrées à sec sous pression réduite à 40°C. On obtient ainsi 2,0 g de {{[tert-butoxycarbonyl-4 (chloro-2 phényl)-2 thiazolidinyl-3]-2 oxo-2 éthyl}-3 uréïdo}-3 benzoate de triméthylsilyl-2 éthyle-(2R,4R) sous forme d'un solide beige amorphe utilisé tel quel dans les synthèses ultérieures, ($[\alpha]_D^{20}$ = + 87,0° ±2,0° (C = 0,46%; $CHCl_3$)).

Le (chloro-2 phényl)-2 thiazolidinecarboxylate-4 de tert-butyle-(2RS,4R) peut être préparé d'une manière analogue à celle décrite à l'exemple 34 §C mais à partir de 12,2 g d'acide (chloro-2 phényl)-2 thiazolidinecarboxylique-4-(2RS,4R), de 3,0 $cm^3$ d'acide sulfurique concentré et d'un excès d'isobutène. Le produit brut obtenu est purifié par chromatographie sur silice [éluant : acétate d'éthyle-cyclohexane (30/70 en volumes)]. Les fractions contenant le produit attendu sont réunies et concentrées à sec sous pression réduite à 40°C. On obtient ainsi 11,0 g de (chloro-2 phényl)-2 thiazolidinecarboxylate-4 de tert-butyle-(2RS,4R) sous forme d'une huile jaune, mélange (40/60 en poids) des isomères (2R,4R) et (2S,4R), utilisée telle quelle dans les synthèses ultérieures.

L'acide (chloro-2 phényl)-2 thiazolidinecarboxylique-4-(2RS,4R) peut être préparé comme à l'exemple 34 §D mais à partir de 21,2 g de L-cystéine et de 25,8 g de chloro-2 benzaldéhyde. On obtient ainsi 21,9 g d'acide (chloro-2 phényl)-2 thiazolidinecarboxylique-4-(2RS,4R) fondant à 144°C, utilisé tel quel dans les synthèses ultérieures.

**Exemple 46**

On opère d'une manière analogue à celle décrite à l'exemple 41, mais à partir de 4,0 g de {{[tert-butoxycarbonyl-4 (hydroxy-3 phényl)-2 thiazolidinyl-3]-2 oxo-2 éthyl}-3 uréido}-3 benzoate de triméthylsilyl-2 éthyle-(2R,4R) et de 11,8 $cm^3$ d'une solution 1M de fluorure de tétrabutylammonium. Le produit brut est purifié par chromatographie sur silice [éluant : acétate d'éthyle]. Les fractions contenant le produit attendu sont réunies et concentrées à sec sous pression réduite à 40°C. Le résidu solide est dissous dans 8,0 $cm^3$ d'une solution aqueuse de soude 0,5 N. La solution est filtrée et acidifiée par 4,1 $cm^3$ d'une solution aqueuse d'acide chlorhydrique 1N. Le produit insoluble est séparé par filtration, lavé par 2 fois 5 $cm^3$ d'eau et séché à l'air. On obtient ainsi 0,65 g d'acide {{[tert-butoxycarbonyl-4 (hydroxy-3 phényl)-2 thiazolidinyl-3]-2 oxo-2 éthyl}-3 uréido}-3 benzoïque-(2R,4R) fondant à 150°C. $(\alpha]_D^{20}$= + 62° ±2° (C = 0,28%; DMF)) [RMN du proton (200 MHz, DMSO $D_6$, $\delta$ en ppm), 2 rotamères à température ambiante, coalescence des raies à 120°C, déplacements chimiques caractéristiques à 120°C : 6,8 (bd, 1 H, O-$C_6H_4$-C en position 4); 7,0 à 7,6 (m, 6H aromatiques); 8,0 (bs, 1H, N-$C_6H_4$-en position 2). Spectre infra-rouge (KBr), bandes caractéristiques en $cm^{-1}$: 3380, 2975, 2930, 2625, 1700, 1650, 1590, 1560, 1490, 1370, 1235, 1150, 785, 760, 690, 680].

Le {{[tert-butoxycarbonyl-4 (hydroxy-3 phényl)-2 thiazolidinyl-3]-2 oxo-2 éthyl}-3 uréido}-3 benzoate de triméthylsilyl-2 éthyle-(2R,4R) peut être préparé d'une manière analogue à celle décrite à l'exemple 41 §A mais à partir de 1,66 g d'(hydroxy-3 phényl)-2 thiazolidinecarboxylate-4 de tert-butyle-(2RS,4R), de 2,0 g d'acide {[(triméthylsilyl-2 éthoxycarbonyl)-3 phényl]-3 uréido}-2 acétique et de 1,33 g de N,N'-dicyclohexylcarbodiimide. On obtient ainsi 4,0 g de {{[tert-butoxycarbonyl-4 (hydroxy-3 phényl)-2 thiazolidinyl-3]-2 oxo-2 éthyl}-3 uréido}-3 benzoate de triméthylsilyl-2 éthyle-(2R,4R) sous forme d'une poudre jaune amorphe utilisée telle quelle dans les synthèses ultérieures.

L'(hydroxy-3 phényl)-2 thiazolidinecarboxylate-4 de tert-butyle-(2RS,4R) peut être préparé d'une manière analogue à celle décrite à l'exemple 34 §C mais à partir de 11,3 g d'acide (hydroxy-3 phényl)-2 thiazolidinecarboxylique-4-(2RS,4R) en solution dans 200 $cm^3$ de chloroforme, de 3,0 $cm^3$ d'acide sulfurique concentré et d'un excès d'isobutène. Le produit brut obtenu est purifié par chromatographie sur silice [éluant : acétate d'éthyle-cyclohexane (30/70 en volumes)]. Les fractions contenant le produit attendu sont réunies et concentrées à sec sous pression réduite à 40°C. On obtient ainsi 4,5 g d'(hydroxy-3 phényl)-2 thiazolidinecarboxylate-4 de tert-butyle-(2RS,4R) sous forme d'une huile jaune, mélange (50/50 en poids) des isomères (2R,4R) et (2S,4R), utilisée telle quelle dans les synthèses ultérieures.

L'acide (hydroxy-3 phényl)-2 thiazolidinecarboxylique-4-(2RS,4R) peut être préparé comme à l'exemple 34 §D mais à partir de 21,2 g de L-cystéine et de 22,8 g d'hydroxy-3 benzaldéhyde. On obtient ainsi 37,5 g

d'acide (hydroxy-3 phényl)-2 thiazolidinecarboxylique-4-(2RS,4R) fondant à 207°C, utilisé tel quel dans les synthèses ultérieures.

**Exemple 47**

On opère d'une manière analogue à celle décrite à l'exemple 41, mais à partir de 1,3 g de {[(tert-butoxycarbonyl-4 phényl-2 thiazolidinyl-3)-2 oxo-2 éthyl]-3 uréido}-3 benzoate de triméthylsilyl-2 éthyle-(2R,4R) et de 4,5 $cm^3$ d'une solution molaire de fluorure de tétrabutylammonium. Le produit brut est purifié par chromatographie sur silice [éluant : acétate d'éthyle-méthanol (95/5 en volumes)]. Les fractions contenant le produit attendu sont réunies et concentrées à sec sous pression réduite à 40°C. Le résidu solide est dissous dans 4,0 $cm^3$ d'une solution aqueuse de soude 0,5 N. La solution est filtrée, et acidifiée par 2,1 $cm^3$ d'une solution aqueuse d'acide chlorhydrique 1N. Le produit insoluble est séparé par filtration, lavé par 2 fois 5 $cm^3$ d'eau et séché à l'air. On obtient ainsi 0,33 g d'acide {[(tert-butoxycarbonyl-4 phényl-2 thiazolidinyl-3)-2 oxo-2 éthyl]-3 uréïdo}-3 benzoïque-(2R,4R} fondant à 134°C. ($[\alpha]_D^{20}$ = + 85° ±3° (C = 0,40%; $CHCl_3$)) [RMN du proton (250 MHz, DMSO $D_6$, $\delta$ en ppm), 2 rotamères à température ambiante, coalescence des raies à 120°C, déplacements chimiques caractéristiques à 120°C: 7,3 à 7,7 (m, 8H aromatiques); 8,0 (bs, 1H, N-$C_6H_4$-C en position 2). Spectre infra-rouge (KBr), bandes caractéristiques en $cm^{-1}$: 3380,2980, 2930, 2600, 1690, 1650, 1610, 1590, 1555, 1490, 1370, 1235, 1150, 760, 730, 700, 680].

Le {[(tert-butoxycarbonyl-4 phényl-2 thiazolidinyl-3)-1 oxo-2 éthyl]-3 uréido}-3 benzoate de triméthylsilyl-2 éthyle-(2R,4R) peut être préparé d'une manière analogue à celle décrite à l'exemple 38 mais à partir de 1,7 g d'[(imidazolyl-1 carboxamido)-2 acétyl]-3 phényl-2 thiazolidinecarboxylate-4 de tert-butyle-(2R,4R) et de 1,2 g d'amino-3 benzoate de triméthylsilyl-2 éthyle. Le produit brut est purifié par chromatographie sur silice [éluant : acétate d'éthyle-cyclohexane (30/70 en volumes)]. Les fractions contenant le produit attendu sont réunies et concentrées à sec sous pression réduite à 40°C. On obtient ainsi 1,3 g de {[(tert-butoxycarbonyl-4 phényl-2 thiazolidinyl-3)-2 oxo-2 éthyl]-3 uréido}-3 benzoate de triméthylsilyl-2 éthyle-(2R,4R) sous forme d'une huile orange utilisée telle quelle dans les synthèses ultérieures.

**Exemple 48**

On opère d'une manière analogue à celle décrite à l'exemple 41, mais à partir de 4,0 g de {{[tert-butoxycarbonyl-4 (diméthylamino-4 phényl)-2 thiazolidinyl-3]-2 oxo-2 éthyl}-3 uréido}-3 benzoate de triméthylsilyl-2 éthyle-(2R,4R) et de 4,2 $cm^3$ d'une solution 1M de fluorure de tétrabutylammonium. Le produit brut est purifié par chromatographie sur silice [éluant : acétate d'éthyle-méthanol (90/10 en volumes)]. Les fractions contenant le produit attendu sont réunies et concentrées à sec sous pression réduite à 40°C. On obtient ainsi, après battage dans l'oxyde de diisopropyle, 0,35 g d'acide {{[tert-butoxycarbonyl-4 (diméthylamino-4 phényl)-2 thiazolidinyl-3]-2 oxo-2 éthyl}-3 uréïdo}-3 benzoïque-(2R,4R) sous forme d'un solide amorphe ($[\alpha]_D^{20}$= + 138° ±3° (C = 0,37%; $CHCl_3$)) [RMN du proton (200 MHz, DMSO $D_6$, $\delta$ en ppm), 2 rotamères à température ambiante, coalescence des raies à 120°C, déplacements chimiques caractéristiques à 120°C: 6,7, (bd, 2H, $(Me)_2N$-$C_6H_4$ en position 3 et 5); 7,3 à 7,6 (m, 5H aromatiques); 8,0 (bs, 1H, N-$C_6H_4$- en position 2). Spectre infra-rouge (KBr), bandes caractéristiques en $cm^{-1}$: 3380, 2975, 2930, 2805, 2600, 1715, 1690, 1650, 1610, 1555, 1520, 1485, 1365, 1230, 1150, 815, 760, 685].

A - Le {{[tert-butoxycarbonyl-4 (diméthylamino-4 phényl)-2 thiazolidinyl-3]-2 oxo-2 éthyl}-3 uréido}-3 benzoate de triméthylsilyl-2 éthyle-(2R,4R) peut être préparé d'une manière analogue à celle décrite à l'exemple 41 §A mais à partir de 1,66 g de (diméthylamino-4 phényl)-2 thiazolidinecarboxylate-4 de tert-butyle-(2RS,4R), de 2,0 g d'acide {[(triméthylsilyl-2 éthoxycarbonyl)-3 phényl]-3 uréido}-2 acétique et de 1,23 g de N,N'-dicyclohexylcarbodiimide. Le produit brut est purifié par chromatographie sur silice [éluant : acétate d'éthyle-cyclohexane (30/70 en volumes)]. Les fractions contenant le produit attendu sont réunies et concentrées à sec sous pression réduite à 40°C. On obtient ainsi 1,3 g de {{[tert-butoxycarbonyl-4 (diméthylamino-4 phényl)-2 thiazolidinyl-3]-2 oxo-2 éthyl}-3 uréido}-3 benzoate de triméthylsilyl-2 éthyle-(2R,4R) sous forme d'un solide beige amorphe utilisé tel quel dans les synthèses ultérieures, ($[\alpha]_D^{20}$= + 93° ±2° (C = 0,49%; $CHCl_3$)).

B - Le (diméthylamino-4 phényl)-2 thiazolidinecarboxylate-4 de tert-butyle-(2RS,4R) peut être préparé de la manière suivante : à une solution de 8,1 g de tert-butoxycarbonyl-3 (diméthylamino-4 phényl)-2 thiazolidinecarboxylate-4 de tert-butyle-(2RS,4R) dans 100 $cm^3$ de chloroforme, on introduit goutte à goutte 2,95 $cm^3$ d'iodotriméthylsilane à une température voisine de 25°C. Le mélange réactionnel est agité pendant 18 heures à cette température, puis on ajoute 50 $cm^3$ d'eau. La phase organique est séparée, lavée par 2 fois 30 $cm^3$ d'une solution aqueuse saturée d'hydrogénocarbonate de sodium et 2 fois 30 $cm^3$ d'eau puis extraite par 3 fois 30 $cm^3$ d'une solution aqueuse d'acide chlorhydrique 1N. Les phases aqueuses sont

réunies, lavées par 2 fois 20 $cm^3$ de dichlorométhane, amenées à pH 7 par une solution aqueuse de soude 4N et extraites par 3 fois 30 $cm^3$ de dichlorométhane. Les phases organiques réunies sont séchées sur sulfate de magnésium et concentrées à sec sous pression réduite à 40°C. Le résidu huileux obtenu est purifié par chromatographie sur silice [éluant : oxyde de diisopropyle]. Les fractions contenant le produit attendu sont réunies et concentrées à sec sous pression réduite. On obtient ainsi 2,3 g de (diméthylamino-4 phényl)-2 thiazolidinecarboxylate-4 de tert-butyle-(2RS,4R) fondant à 142°C, mélange (50/50 en poids) des isomères (2R,4R) et (2S,4R) utilisé tel quel dans les synthèses ultérieures.

C - Le tert-butoxycarbonyl-3 (diméthylamino-4 phényl)-2 thiazolidinecarboxylate-4 de tert-butyle-(2RS,4R) peut être préparé de la manière suivante : à une solution de 14,8 g d'acide tert-butoxycarbonyl-3 (diméthylamino-4 phényl)-2 thiazolidinecarboxylique-4-(2RS,4R) et de 16,0 g de chlorure de paratoluènesulfonyle dans 70 $cm^3$ de pyridine anhydre, refroidie à une température voisine de 0°C, on ajoute 3,1 g de tert-butanol. Après retour à une température de 20°C, l'agitation est poursuivie pendant 20 heures, puis le mélange est versé dans 200 $cm^3$ d'eau et extrait par 3 fois 100 $cm^3$ d'acétate d'éthyle. Les phases organiques réunies sont lavées successivement par 2 fois 100 $cm^3$ d'eau, 2 fois 100 $cm^3$ d'une solution aqueuse de soude 1N et 2 fois 100 $cm^3$ d'eau, puis séchées sur sulfate de magnésium et concentrées à sec sous pression réduite à 40°C. Le produit brut obtenu est purifié par chromatographie sur silice [éluant : oxyde de diisopropyle]. Les fractions contenant le produit attendu sont réunies et concentrées à sec sous pression réduite à une température voisine de 45°C. On obtient ainsi 8,1 g de tert-butoxycarbonyl-3 (diméthylamino-4 phényl)-2 thiazolidinecarboxylate-4 de tert-butyle-(2RS,4R) sous forme d'une huile orange utilisée telle quelle dans les synthèses ultérieures.

D - L'acide tert-butoxycarbonyl-3 (diméthylamino-4 phényl)-2 thiazolidinecarboxylique-4-(2RS,4R) peut être préparé de la manière suivante : à une solution de 12,6 g d'acide (diméthylamino-4 phényl)-2 thiazolidinecarboxylique-4-(2RS,4R), de 5,25 g de carbonate de sodium dans 100 $cm^3$ d'eau, on ajoute goutte à goutte, sous agitation, 10,9 g de dicarbonate de ditert-butyle en solution dans 50 $cm^3$ de dioxanne. Le mélange réactionnel est agité pendant 20 heures à une température voisine de 20°C, puis le précipité formé est séparé par filtration. Le filtrat est lavé par 2 fois 100 $cm^3$ d'acétate d'éthyle, acidifié à pH 4 par une solution aqueuse d'acide chlorhydrique 4N puis extrait par 3 fois 100 $cm^3$ d'acétate d'éthyle. Les phases organiques réunies sont lavées par 2 fois 50 $cm^3$ d'eau, séchées sur sulfate de magnésium et concentrées à sec sous pression réduite à 40°C. On obtient ainsi, après cristallisation dans l'oxyde de diisopropyle, 14,8 g d'acide tert-butoxycarbonyl-3 (diméthylamino-4 phényl)-2 thiazolidinecarboxylique-4-(2RS,4R) fondant à 175°C.

L'acide (diméthylamino-4 phényl)-2 thiazolidinecarboxylique-4-(2RS,4R) peut être préparé comme à l'exemple 34 §D mais à partir de 21,2 g de L-cystéine et de 27,5 g de diméthylamino-4 benzaldéhyde. On obtient ainsi 41,5 g d'acide (diméthylamino-4 phényl)-2 thiazolidinecarboxylique-4-(2RS,4R) fondant à 184°C, utilisé tel quel dans les synthèses ultérieures.

**Exemple 49**

On opère d'une manière analogue à celle décrite à l'exemple 41, mais à partir de 0,95 g de {{[tert-butoxycarbonyl-4 (pyridyl-3)-2 thiazolidinyl-3]-2 oxo-2 éthyl}-3 uréido}-3 benzoate de triméthylsilyl-2 éthyle-(2R,4R) et de 3,2 $cm^3$ d'une solution 1M de fluorure de tétrabutylammonium. Le produit brut est purifié par chromatographie sur silice [éluant : acétate d'éthyle]. Les fractions contenant le produit attendu sont réunies et concentrées à sec sous pression réduite à 40°C. On obtient ainsi, après battage dans l'oxyde de diisopropyle, 0,36 g d'acide {{[tert-butoxycarbonyl-4 (pyridyl-3)-2 thiazolidinyl-3]-2 oxo-2 éthyl}-3 uréido}-3 benzoïque-(2R,4R) fondant à 147°C ($[\alpha]_D^{20}$= +51,1° ±1,7° (C 0,49%; DMF)) [RMN du proton (200 MHz, DMSO $D_6$, $\delta$ en ppm), 2 rotamères à température ambiante, coalescence des raies à 120°C, déplacements chimiques caractéristiques à 120°C : 7,3 à 7,6 (m, 5H aromatiques); 8,0 (bs, 1H, N-$C_6H_4$-C en position 2); 8,5 (bs, 1H, H en position 2 du pyridyl); 8,8 (bs, 1H, H en position 6 du pyridyl). Spectre infra-rouge (KBr), bandes caractéristiques en $cm^{-1}$: 3385, 2975, 2930, 2600, 2500, 1730, 1660, 1610, 1590, 1560, 1485, 1370, 1240, 1150, 760, 710, 685].

Le {{[tert-butoxycarbonyl-4 (pyridyl-3)-2 thiazolidinyl-3]-2 oxo-2 éthyl}-3 uréido}-3 benzoate de triméthylsilyl-2 éthyle-(2R,4R) peut être préparé d'une manière analogue à celle décrite à l'exemple 41 §A mais à partir de 2,0 g de (pyridyl-3)-2 thiazolidinecarboxylate-4 de tert-butyle-(2RS,4R), de 2,5 g d'acide {[(triméthylsilyl-2 éthoxycarbonyl)-3 phényl]-3 uréido}-2 acétique et de 1,8 g de N,N'-dicyclohexylcarbodiimide. Le produit brut est purifié par chromatographie sur silice [éluant : acétate d'éthyle-cyclohexane (40/60 en volumes)]. Les fractions contenant le produit attendu sont réunies et concentrées à sec sous pression réduite à 40°C. On obtient ainsi 1,0 g de {{[tert-butoxycarbonyl-4 (pyridyl-3)-2 thiazolidinyl-3]-2 oxo-2 éthyl}-3 uréido}-3 benzoate de triméthylsilyl-2 éthyle-(2R,4R) sous forme d'une huile jaune utilisée telle quelle dans les synthèses ultérieures.

Le (pyridyl-3)-2 thiazolidinecarboxylate-4 de tert-butyle-(2RS,4R) peut être préparé d'une manière analogue à celle décrite à l'exemple 48 §B mais à partir de 3,4g de tert-butoxycarbonyl-3 (pyridyl-3)-2 thiazolidinecarboxylate-4 de tert-butyle-(2RS,4R) et de 1,7 $cm^3$ d'iodotriméthylsilane. On obtient ainsi 2,1 g de (pyridyl-3)-2 thiazolidinecarboxylate-4 de tert-butyle-(2RS,4R) sous forme d'une huile jaune utilisée telle quelle dans les synthèses ultérieures.

Le tert-butoxycarbonyl-3 (pyridyl-3)-2 thiazolidinecarboxylate-4 de tert-butyle-(2RS,4R) peut être préparé d'une manière analogue à celle décrite à l'exemple 48 §C mais à partir de 9,0 g d'acide tert-butoxycarbonyl-3 (pyridyl-3)-2 thiazolidinecarboxylique-4-(2RS,4R), de 5,7 g de chlorure de paratoluènesulfonyle et de 2,2 g de tert-butanol. Le produit brut obtenu est purifié par chromatographie sur silice [éluant acétate d'éthyle-cyclohexane (50/50 en volumes)]. Les fractions contenant le produit attendu sont réunies et concentrées à sec sous pression réduite à 40°C. On obtient ainsi 1,25 g de tert-butoxycarbonyl-3 (pyridyl-3)-2 thiazolidinecarboxylate-4 de tert-butyle-(2RS,4R) sous forme d'une huile orange utilisée telle quelle dans les synthèses ultérieures.

L'acide tert-butoxycarbonyl-3 (pyridyl-3)-2 thiazolidinecarboxylique-4-(2RS,4R) peut être préparé d'une manière analogue à celle décrite à l'exemple 48 §D mais à partir de 26,5 g d'acide (pyridyl-3)-2 thiazolidinecarboxylique-4-(2RS,4R), de 130 $cm^3$ d'une solution de soude 1N et de 28,4 g de dicarbonate de ditert-butyle. On obtient ainsi 31,6 g d'acide tert-butoxycarbonyl-3 (pyridyl-3)-2 thiazolidinecarboxylique-4-(2RS,4R) sous forme d'une poudre amorphe jaune utilisée telle quelle dans les synthèses ultérieures.

L'acide (pyridyl-3)-2 thiazolidinecarboxylique-4-(2RS,4R) peut être préparé comme à l'exemple 34 §D mais à partir de 25,0 g de L-cystéine et de 22 $cm^3$ de nicotinaldéhyde. On obtient ainsi 26,6 g d'acide (pyridyl-3)-2 thiazolidinecarboxylique-4-(2RS,4R) fondant à 149°C, utilisé tel quel dans les synthèses ultérieures.

**Exemple 50**

On opère d'une manière analogue à celle décrite à l'exemple 39 mais à partir de 2,5 g de {{[(tert-butoxycarbonyl-4 phényl-2 thiazolidinyl-3)-2 oxo-2 éthyl]-3 uréido}-3 phényl}-3 propionate d'éthyle-(2R,4R) et de 0,2 g d'hydroxyde de lithium. Le produit brut est purifié par chromatographie sur silice [éluant : acétate d'éthyle-méthanol (90/10 en volumes). Les fractions contenant le produit attendu sont réunies et concentrées à sec sous pression réduite à 40°C. On obtient ainsi 0,25 g d'acide {{[(tert-butoxycarbonyl-4 phényl-2 thiazolidinyl-3)-2 oxo-2 éthyl]-3 uréido}-3 phényl}-3 propionique-(2R,4R) sous forme de solide beige amorphe ($[\alpha]_D^{20}$= + 58,4° ±1,6° (C = 0,61 %; $CHCl_3$)) [RMN du proton (200 MHz, DMSO $D_6$, δ en ppm), 2 rotamères à température ambiante, déplacements chimiques caractéristiques à 25°C: 1,5 (bs, 9H, $(CH_3)_3$); 2,5 (bt, 2H, $CH_2$); 2,8 (bt, 2H, $CH_2$); 6,8 (bd, 1H, N-$C_6H_4$-C en position 4); 7,1 à 7,5 (m, 7H aromatiques); 7,7 (bd, 2H aromatiques). Spectre infra-rouge (KBr), bandes caractéristiques en $cm^{-1}$: 3380, 2975, 2930, 2620, 1730, 1645, 1610, 1590, 1555, 1490, 1370, 1235, 1150, 785, 730, 695].

Le {{[(tert-butoxycarbonyl-4 phényl-2 thiazolidinyl-3)-2 oxo-2 éthyl]-3 uréido}-3 phényl}-3 propionate d'éthyle-(2R,4R) peut être préparé d'une manière analogue à celle décrite à l'exemple 38 mais à partir de 3,75 g d'[(imidazolyl-1 carboxamido)-2 acétyl]-3 phényl-2 thiazolidinecarboxylate-4, de tert-butyle-(2R,4R) et de 3,5 g d'(amino-3 phényl)-3 propionate d'éthyle. Le résidu huileux obtenu est purifié par chromatographie sur silice [éluant : acétate d'éthyle-cyclohexane (30/70 en volumes)] et les fractions contenant le produit attendu sont réunies puis concentrées à sec sous pression réduite à 40°C. On obtient ainsi 2,5 g de {{[(tert-butoxycarbonyl-4 phényl-2 thiazolidinyl-3)-2 oxo-2 éthyl]-3 uréido}-3 phényl}-3 propionate d'éthyle-(2R,4R) sous forme d'une huile utilisée telle quelle dans les synthèses ultérieures.

**Exemple 51**

On opère d'une manière analogue à celle décrite à l'exemple 39 mais à partir de 2,4 g de {[(tert-butoxycarbonyl-4 phényl-2 thiazolidinyl-3)-2 oxo-2 éthyl]-3 uréido}-3 phénoxyacétate d'éthyle-(2R,4R) et de 0,2 g d'hydroxyde de lithium. Le produit brut est purifié par chromatographie sur silice [éluant : acétate d'éthyle]. Les fractions contenant le produit attendu sont réunies et concentrées à sec sous pression réduite à 40°C. On obtient ainsi, après battage dans l'oxyde de diisopropyle, 1,3 g d'acide {[(tert-butoxycarbonyl-4 phényl-2 thiazolidinyl-3)-2 oxo-2 éthyl]-3 uréido}-3 phénoxyacétique-(2R,4R) sous forme de solide amorphe ($[\alpha]_D^{20}$= + 63,7° ±1,2° (C = 0,96%; $CHCl_3$)) [RMN du proton (200 MHz, DMSO $D_6$, δ en ppm), 2 rotamères à température ambiante, coalescence des raies à 120°C, déplacements chimiques caractéristiques à 120°C: 4,5 (bs, 2H, $OCH_2CO_2$); 6,5 (ddd, 1H, N-$C_6H_4$-C en position 4); 6,9 (d, 1H aromatique); 7,1 à 7,7 (m, 7H aromatiques). Spectre infra-rouge (KBr), bandes caractéristiques en $cm^{-1}$: 3380, 2975, 2930, 2630, 2550, 1735, 1640, 1600, 1555, 1490, 1370, 1240, 1150, 770, 730, 695].

Le {[(tert-butoxycarbonyl-4 phényl-2 thiazolidinyl-3)-2 oxo-2 éthyl]-3 uréido}-3 phénoxyacétate d'éthyle-(2R,4R) peut être préparé d'une manière analogue à celle décrite à l'exemple 38 mais à partir de 4,4 g d'[(imidazolyl-1 carboxamido)-2 acétyl]-3 phényl-2 thiazolidinecarboxylate-4 de tert-butyle-(2R,4R) et de 4,1 g d'amino-3 phénoxyacétate d'éthyle. Le résidu huileux obtenu est purifié par chromatographie sur silice [éluant : acétate d'éthyle-cyclohexane (35/65 en volumes)]. Les fractions contenant le produit attendu sont réunies et concentrées à sec sous pression réduite à 40°C. On obtient ainsi 2,5 g de {{[(tert-butoxycarbonyl-4 phényl-2 thiazolidinyl-3)-2 oxo-2 éthyl]-3 uréido}-3 phénoxyacétate d'éthyle-(2R,4R) sous forme d'une poudre beige amorphe utilisée telle quelle dans les synthèses ultérieures.

**Exemple 52**

A une solution de 2,5 g d'{[(acétyl-3 phényl)-3 uréido]-2 acétyl}-3 phényl-2 thiazolidinecarboxylate-4 de tert-butyle-(2R,4R) dans 10 $cm^3$ de méthanol et 5 $cm^3$ de pyridine, on ajoute 0,4 g de chlorhydrate d'hydroxylamine en solution dans 5 $cm^3$ d'eau. Le mélange réactionnel est agité à reflux pendant 2 heures. Après évaporation des solvants sous pression réduite à 45°C, le résidu est repris par 100 $cm^3$ d'acétate d'éthyle. La phase organique est lavée par 3 fois 50 $cm^3$ d'eau, séchée sur sulfate de magnésium et concentrée à sec sous pression réduite. Le produit brut obtenu est purifié par chromatographie sur silice [éluant : acétate d'éthyle-cyclohexane (50/50 en volumes)]. Les fractions contenant le produit attendu sont réunies et concentrées à sec sous pression réduite à 40°C. On obtient, après battage dans l'oxyde de diisopropyle, 0,5 g d'{{[(hydroxyimino-1 éthyl)-3 phényl-(E)]-3 uréido}-2 acétyl}-3 phényl-2 thiazolidinecarboxylate-4 de tert-butyle-(2R,4R) sous forme d'un solide amorphe ($[\alpha]_D^{20}$= + 54° ±1,5° (C = 0,61%;$CHCl_3$)) [RMN du proton (300 MHz, DMSO $D_6$, δ en ppm), 2 rotamères à température ambiante, coalescence des raies à 120°C, déplacements chimiques caractéristiques à 120°C: 2,1 (s, 3H, $CH_3$); 7,2 à 7,6 (m, 8H aromatiques); 7,7 (bs, 1H, N-$C_6H_4$-C en position 2); 10,8 (bs, 1H, NOH). Un effet NOE a été observé entre le méthyle à 2,1 ppm et l'oxime NOH à 10,8 ppm. Spectre infrarouge (KBr), bandes caractéristiques en $cm^{-1}$: 3350, 2975, 2930, 1740, 1650, 1610, 1590, 1560, 1370, 1240, 1150, 1005, 790, 730, 695].

L'{[(acétyl-3 phényl)-3 uréido]-2 acétyl}-3 phényl-2 thiazolidinecarboxylate-4 de tert-butyle-(2R,4R) peut être préparé d'une manière analogue à celle décrite à l'exemple 38 mais à partir de 3,75 g d'[(imidazolyl-1 carboxamido)-2 acétyl]-3 phényl-2 thiazolidinecarboxylate-4 de tert-butyle-(2R,4R) et de 1,46 g d'amino-3 acétophénone. Le résidu huileux obtenu est purifié par chromatographie sur silice [éluant : acétate d'éthyle-cyclohexane (50/50 en volumes)]. Les fractions contenant le produit attendu sont réunies et concentrées à sec sous pression réduite à 40°C. On obtient ainsi 2,5 g d'{[(acétyl-3 phényl)-3 uréido]-2 acétyl}-3 phényl-2 thiazolidinecarboxylate-4 de tert-butyle-(2R,4R) sous forme d'une poudre jaune amorphe utilisée telle quelle dans les synthèses ultérieures.

**Exemple 53**

On opère d'une manière analogue à celle décrite à l'exemple 38 mais à partir de 2,3 g d'[(imidazolyl-1 carboxamido)-2 acétyl]-3 phényl-2 thiazolidinecarboxylate-4 de tert-butyle-(2R,4R) et de 4,7 g d'amino-3 benzylsulfonate de tétra-n-butyl ammonium. Le produit brut obtenu est dissous dans 20 $cm^3$ d'acétone et on ajoute une solution de 1,86 g de nonafluorobutanesulfonate de potassium dans 20 $cm^3$ d'acétone. Le milieu réactionnel est agité 18 heures à une température voisine de 25°C, puis on ajoute 80 $cm^3$ d'oxyde de diisopropyle. Le produit insoluble est séparé par filtration, lavé par 2 fois 3 $cm^3$ d'un mélange d'acétone et d'oxyde de diisopropyle (30-70 en volumes). On obtient ainsi, après battage dans l'acétonitrile, 0,75 g de {[(tert-butoxycarbonyl-4 phényl-2 thiazolidinyl-3)-2 oxo-2 éthyl]-3 uréido}-3 benzylsulfonate de potassium-(2R,4R) fondant à 185°C ($[\alpha]_D^{20}$= + 37,3° ±1,4° (C= 0,38%; $CHCl_3$)) [RMN du proton (300 MHz, DMSO $D_6$, δ en ppm), 2 rotamères à température ambiante, coalescence des raies à 120°C, déplacements chimiques caractéristiques à 120°C : 3,7 (AB,2H, $CH_2SO_3$); 6,9 (bd, 1H, N-$C_6H_4$-C en 6); 7,1 à 7,6 (m, 8H aromatiques). Spectre infra-rouge (KBr), bandes caractéristiques en $cm^{-1}$: 3400, 2980, 2945, 1740, 1660, 1615, 1600, 1560, 1500, 1375, 1220, 1200, 1160, 1050, 800, 735, 700].

L'amino-3 benzylsulfonate de tétra-n-butyl ammonium peut être préparé d'une manière analogue à celle décrite à l'exemple 41 §G mais à partir de 11,6 g de nitro-3 benzylsulfonate de tétra-n-butyl ammonium et de 0,3 g de palladium sur noir à 5%. On obtient ainsi 10,5 g d'amino-3 benzylsulfonate de tétra-n-butyl ammonium sous forme d'une huile utilisée telle quelle dans les synthèses ultérieures.

Le nitro-3 benzylsulfonate de tétra-n-butyl ammonium peut être préparé de la manière suivante : à 800 $cm^3$ d'une solution aqueuse de dihydrogénophosphate de potassium 0,5 M, on ajoute 6,9 g de nitro-3 benzylsulfonate de sodium, puis 9,9 g d'hydrogénosulfonate de tétra-n-butyl ammonium. Le mélange est extrait par 500 $cm^3$ chlorure de méthylène. La phase organique est séchée sur sulfate de magnésium et concentrée à sec sous pression réduite à 4-0°C. On obtient ainsi, 13 g de nitro-3 benzylsulfonate de tétra-n-butyl ammonium sous forme d'une huile utilisée telle quelle dans les synthèses ultérieures.

Le nitro-3 benzylsulfonate de sodium peut être préparé selon la méthode décrite par PURGOTTI et MONTI, Gazz. Chim. Ital., 30, II, 247.

**Exemple 54**

On opère d'une manière analogue à celle décrite à l'exemple 39 mais à partir de 1,5 g de {{[(tert-butoxycarbonyl-4 phényl-2 thiazolidinyl-3-(2R,4R))-2 oxo-2 éthyl]-3 uréido}-3 phényl}-2 propionate de méthyle-(RS) et de 0,11 g d'hydroxyde de lithium. Le produit brut est purifié par chromatographie sur silice [éluant : acétate d'éthyle]. Les fractions contenant le produit attendu sont réunies et concentrées à sec sous pression réduite à 40°C. On obtient ainsi, après battage dans l'oxyde de diisopropyle, 0,25 g d'acide {{[(tert-butoxycarbonyl-4 phényl-2 thiazolidinyl-3-(2R,4R))-2 oxo-2 éthyl]-3 uréido}-3 phényl}-2 propionique-(RS) sous forme d'un solide amorphe [RMN du proton (200 MHz, DMSO $D_6$, δ en ppm), 2 rotamères à température ambiante, coalescence des raies à 120°C, déplacements chimiques caractéristiques à 120°C: 1,4 (d, 3H, $CH_3$); 3,5 (bm, 1H, Ph-CH-$CO_2$); 6,9 (bd, 1H, N-$C_6H_4$-C en position 4); 7,1 à 7,4 (m, 6H aromatiques); 7,7 (bd, 2H aromatiques). Spectre infra-rouge (KBr), bandes caractéristiques en $cm^{-1}$: 3385, 2975, 2935, 1735, 1650, 1610, 1560, 1495, 1455, 1370, 1240, 1155, 785, 730, 700].

Le {{[(tert-butoxycarbonyl-4 phényl-2 thiazolidinyl-3-(2R,4R))-2 oxo-2 éthyl]-3 uréido}-3 phényl}-2 propionate de méthyle-(RS) peut être préparé d'une manière analogue à celle décrite à l'exemple 38 mais à partir de 3,75 g d'[(imidazolyl-1 carboxamido)-2 acétyl]-3 phényl-2 thiazolidinecarboxylate-4 de tert-butyle-(2R,4R) et de 3,22 g d'(amino-3 phényl)-2 propionate de méthyle-(RS). Le résidu huileux obtenu est purifié par chromatographie sur gel de silice [éluant : acétate d'éthyle] et les fractions contenant le produit attendu sont réunies et concentrées à sec sous pression réduite à 40°C. On obtient ainsi 3,1 g de {{[(tert-butoxycarbonyl-4 phényl-2 thiazolidinyl-3-(2R,4R))-2 oxo-2 éthyl]-3 uréido}-3 phényl}-2 propionate de méthyle-(RS) sous forme d'une poudre beige amorphe utilisée telle quelle dans les synthèses ultérieures.

L'(amino-3 phényl)-2 propionate de méthyle-(RS) peut être préparé d'une manière analogue à celle décrite à l'exemple 41 §G mais à partir de 4 g de (nitro-3 phényl)-3 propionate de méthyle-(RS) et de 0,3 g de palladium sur noir à 5%. On obtient ainsi, 3,3 g d'(amino-3 phényl)-2 propionate de méthyle-(RS) sous forme d'une huile utilisée telle quelle dans les synthèses ultérieures.

Le (nitro-3 phényl)-3 propionate de méthyle-(RS) peut être préparé de la manière suivante : on fait barboter pendant 3 heures de l'acide chlorhydrique gazeux dans une solution de 5 g de (nitro-3 phényl)-2 propionitrile-(RS) dans 40 $cm^3$ de méthanol. Le mélange obtenu est agité à reflux pendant 30 minutes, et le produit insoluble séparé par filtration. Le filtrat est concentré à sec sous pression réduite à 40°C. Le produit brut est purifié par chromatographie sur silice [éluant : éther de pétrole-acétate d'éthyle (80/20 en volumes)]. Les fractions contenant le produit attendu sont réunies et concentrées à sec sous pression réduite à 40°C. On obtient ainsi 4 g de (nitro-3 phényl)-3 propionate de méthyle-(RS) sous forme d'une huile utilisée telle quelle dans les synthèses ultérieures.

Le (nitro-3 phényl)-2 propionitrile-(RS) peut être préparé selon la méthode décrite par E. Felder et Coll., J. Med. Chem., 13, 559 (1970).

**Exemple 55**

En opérant d'une manière analogue à celle décrite à l'exemple 41 §A mais à partir de 1,7 g de (fluoro-4 phényl)-2 thiazolidine-4 carboxylate de tert-butyle-(2R,4R) en solution dans 20 $cm^3$ d'acétonitrile anhydre, de 1,24 g de N,N'-dicyclohexylcarbodiimide et de 1,3 g d'acide (indolyl-2 carboxamido)-2 acétique dans un mélange de 40 $cm^3$ d'acétonitrile anhydre et de 40 $cm^3$ de dichlorométhane, on obtient, après cristallisation dans l'oxyde de diisopropyle, 1,7 g de (fluoro-4 phényl)-2 [(indolyl-2 carboxamido)-2 acétyl]-3 thiazolidine-4 carboxylate de tert-butyle-(2R,4R) fondant à 176°C.

L'acide (indolyl-2 carboxamido)-2 acétique peut être obtenu selon la méthode décrite par J.R. JOHNSON et coll., J. Am. Chem. Soc.,69 2370 (1947).

**Exemple 56**

En opérant d'une manière analogue à celle décrite à l'exemple 1 mais à partir de 1,2 g de N-méthyl N-phényl phényl-5 prolinamide-(2RS, 5SR), de 0,94 g d'acide [(méthyl-3 phényl)-3 uréido]-2 acétique en suspension dans 25 $cm^3$ de dichloro-1,2 éthane et de 0,33 $cm^3$ de chlorure de sulfinyle, on obtient, après recristallisation dans l'acétonitrile, 1 g de N-méthyl [(méthyl-3 phényl)-3 uréidoacétyl]-1 N-phényl phényl-5 prolinamide-(2RS, 5SR) fondant à 210°C.

Le N-méthyl N-phényl phényl-5 prolinamide-(2RS, 5SR) peut être préparé d'une manière analogue à celle

décrite à l'exmple 25 §A, mais à partir de 2,2 g de (tert-butoxycarbonyl)-1 N-méthyl N-phényl phényl-5 prolinamide-(2RS, 5SR) dans 25 $cm^3$ de chloroforme anhydre et de 0,85 $cm^3$ d'iodotriméthylsilane. On obtient 1,2 g de N-méthyl N-phényl phényl-5 prolinamide-(2RS, 5SR) sous forme d'une huile orangée utilisée telle quelle dans les synthèses ultérieures.

A Le (tert-butoxycarbonyl)-1 N-méthyl N-phényl phényl-5 prolinamide-(2RS, 5SR) peut être préparé de la manière suivante: à une suspension de 2,9 g de (tert-butoxycarbonyl)-1 phényl-5 proline-(2RS, 5SR) et de 1,1 $cm^3$ de N-méthyl aniline dans 25 $cm^3$ d'acétonitrile anhydre, on ajoute goutte à goutte à une température voisine de 0°C une solution de 2,1 g de N,N'-dicyclohexylcarbodiimide dans 15 $cm^3$ d'acétonitrile anhydre. Le milieu réactionnel est agité pendant 30 minutes à une température voisine de 0°C puis pendant 20 heures à une température voisine de 20°C. Le produit insoluble est ensuite séparé par filtration et lavé par 2 fois 20 $cm^3$ de dichlorométhane. Le filtrat est concentré à sec, sans pression réduite à une température voisine de de 40°C. Le produit brut obtenu est purifié par chromatographie sur silice [éluant: dichlorométhane-métanol (99/1 en volumes)]. Les fractions contenant le produit attendu sont réunies puis concentrées à sec sous pression réduite. On obtient ainsi après cristallisation dans le pentane, 2,2 g de (tert-butoxycarbonyl)-1 N-méthyl N-phényl phényl-5 prolinamide-(2RS, 5SR) fondant à 132°C.

**Exemple 57**

En opérant d'une manière analogue à celle décrite à l'exmple 1 mais à partir de 3 g de phényl-5 (tétrahydro-1,2,3,4 quinolyl-1) carbonyl-2 pyrrolidine (2RS, 5SR), de 2 g d'acide [(méthyl-3 phényl)-3 uréido]-2 acétique en suspension dans 100 $cm^3$ de dichloro-1,2 éthane et de 0,7 $cm^3$ de chlorure de sulfinyle, on obtient, après recristallisation dans l'acétonitrile 1,5 g de {[(méthyl-3 phényl)-3 uréido]-2 acétyl}-1 phényl-5 (tétrahydro-1,2,3,4 quinolyl-1) carbonyl-2 pyrrolidine-(2RS, 5SR) fondant à 158°C.

La phényl-5 (tétrahydro-1,2,3,4 quinolyl-1) carbonyl-2 pyrrolidine-(2RS, 5SR) peut être préparée d'une manière analogue à celle décrite à l'exmple 25 §A mais à partir de 4,1 g de (tert-butoxycarbonyl)-1 phényl-5 (tétrahydro-1,2,3,4 quinolyl-1) carbonyl-2 pyrrolidine-(2RS, 5SR) dans 50 $cm^3$ de chloroforme anhydre et de 1,4 $cm^3$ d'iodotriméthylsilane. On obtient 3 g de phényl-5 (tétrahydro-1,2,3,4 quinolyl-1) carbonyl-2 pyrrolidine-(2RS, 5SR) sous forme d'une huile orangée utilisée telle quelle dans les synthèses ultérieures.

La (tert-butoxycarbonyl)-1 phényl-5 (tétrahydro-1,2,3,4 quinolyl-1) carbonyl-2 pyrrolidine-(2RS, 5SR) peut être préparée d'une manière analogue à celle décrite à l'exemple 56 §A mais à partir de 8,7 g de (tert-butoxycarbonyl)-1 phényl-5 proline-(2RS, 5SR), de 3,8 $cm^3$ de tétrahydro-1,2,3,4 quinoléine dans 75 $cm^3$ d'acétonitrile anhydre et de 6,2 g de N,N'-dicyclohexylcarbodiimide dans 45 $cm^3$ d'acétonitrile anhydre.On obtient, après cristallisation dans l'hexane, 4,1 g de (tert-butoxycarbonyl)-1 phényl-5 (tétrahydro-1,2,3,4 quinolyl-1) carbonyl-2 pyrrolidine-(2RS, 5SR) fondant à 132°C.

**Exemple 58**

En opérant d'une manière analogue à celle décrite à l'exemple 1 mais à partir de 1,8 g de (diméthyl-3,3 pipéridino) carbonyl-2 phényl-5 pyrrolidine-(2RS, 5SR), de 1,3 g d'acide [(méthyl-3 phényl)-3 uréido]-2 acétique dans 75 $cm^3$ de dichloro-1,2 éthane et de 0,5 $cm^3$ de chlorure de sulfinyle, on obtient, après recristallisation dans l'acétonitrile 0,7 g de (diméthyl-3,3 pipéridino) carbonyl-2{(méthyl-3 phényl)-3 uréido]-2 acétyl}-1 phényl-5 pyrrolidine-(2RS, 5SR) fondant à 150°C.

La (diméthyl-3,3 pipéridino)carbonyl-2 phényl-5 pyrrolidine-(2RS, 5SR) peut être préparée d'une manière analogue à celle décrite à l'exemple 25 §A mais à partir de 5,0 g de (tert-butoxycarbonyl)-1 (diméthyl-3,3 pipéridino) carbonyl-2 phényl-5 pyrrolidine-(2RS, 5SR) dans 50 $cm^3$ de chloroforme anhydre et de 1,9 $cm^3$ d'iodotriméthylsilane. On obtient par cristallisation dans l'oxyde de diisopropyle, 1,8 g de (diméthyl-3,3 pipéridino)carbonyl-2 phényl-5 pyrrolidine-(2RS, 5SR) fondant à 163°C.

La (tert-butoxycarbonyl)-1 (diméthyl-3,3 pipéridino) carbonyl-2 phényl-5 pyrrolidine-(2RS, 5SR) peut être préparée d'une manière analogue à celle décrite à l'exemple 56 §A mais à partir de 5,8 g de (tert-butoxycarbonyl)-1 phényl-5 proline-(2RS, 5SR), de 2,6 $cm^3$ de diméthyl-3,3 pipéridine dans 50 $cm^3$ d'acétonitrile anhydre et de 4,1 g de N,N'-dicyclohexylcarbodiimide dans 30 $cm^3$ d'acétonitrile anhydre. On obtient, après cristallisation dans l'hexane, 4 g de (tert-butoxycarbonyl)-1 (diméthyl-3,3 pipéridino) carbonyl-2 phényl-5 pyrrolidine-(2 RS, 5SR) fondant à 110°C.

**Exemple 59**

En opérant d'une manière analogue à celle décrite à l'exemple 2 mais à partir de 1,9 g d'(amino-2 acétyl)-1 phényl-5 N-tert-butyl-prolinamide-(2RS, 5SR) en solution dans 20 $cm^3$ de tétrahydrofuranne anhydre et de

0,82 $cm^3$ d'isocyanate de méthyl-3 phényle, on obtient, après recristallisation dans l'acétonitrile, 0,7 g de N-tert-butyl [(méthyl-3 phényl)-3 uréidoacétyl]-1 phényl-5 prolinamide-(2RS, 5SR) fondant à 169°C.

L'(amino-2 acétyl)-1 phényl-5 N-tert-butyl-prolinamide-(2RS, 5SR) peut être préparé d'une manière analogue à celle décrite à l'exemple 2 §A , mais à partir de 2,7 g de (tert-butoxycarbonylamino-2 acétyl)-1 N-tert-butyl phényl-5 prolinamide-(2RS, 5SR) en solution dans 50 $cm^3$ de chloroforme anhydre et de 0,95 $cm^3$ d'iodotriméthylsilane. On obtient 1,9 g d'(amino-2 acétyl)-1 phényl-5 N-tert-butyl-prolinamide-(2RS, 5SR) sous forme d'une poudre amorphe utilisée telle quelle dans les synthèses ultérieures.

**RMN du proton** (250 MHz, DMSO $D_6$, d en ppm), 2 rotamères à température ambiante, 1,35 (s, 9H, $C(CH_3)_3$), 1,8 à 2,4 (bm, 4H, $CH_2$-$CH_2$); 2,7, 3,25 et 3,8 (d, 2H, AB, $CH_2CO_2$); 4,45 (bm, 1H, CHN); 5,1 (bm, 1H, CHN); 7,2 à 8 (m, 6H, aromatiques et NH).

Le (tert-butoxycarbonylamino-2 acétyl)-1 N-tert-butyl phényl-5 prolinamide peut être préparé d'une manière analogue à celle décrite à l'exemple 2 §B mais à partir 1,8 g de N-tert-butyl phényl-5 prolinamide-(2RS, 5SR), de 1,28g d'acide tert-butoxycarbonylamino-2 acétique dans 50 $cm^3$ d'acétonitrile anhydre et de 1,5 g de N,N'-dicyclohexylcarbodiimide dans 25 $cm^3$ d'acétonitrile anhydre. On obtient, après cristallisation dans l'éther de pétrole, 2,7 g de (tert-butoxy-carbonylamino-2 acétyl)-1 N-tert-butyl phényl-5 prolinamide fondant à 117°C.

Le N-tert-butyl phényl-5 prolinamide-(2RS, 5SR) peut être préparé d'une manière analogue à celle décrite à l'exemple 25 §A mais à partir de 3,5 g de (tert-butoxycarbonyl)-1 N-tert-butyl phényl-5 prolinamide-(2RS, 5SR) dans 50 $cm^3$ de chloroforme anhydre et de 2,1 $cm^3$ d'iodotriméthylsilane. On obtient 1,8 g de N-tert-butyl phényl-5 prolinamide-(2RS, 5SR) sous forme d'une huile jaune utilisée telle quelle dans les synthèses ultérieures.

Le tert-butoxycarbonyl-1 N-tert-butyl phényl-5 prolinamide-(2RS,5SR) peut être obtenu de la manière suivante: à une solution de 2,9 g de tert-butoxycarbonyl-1 phényl-5 proline-(2RS,5SR) dans 50 $cm^3$ de dichloro-1,2 éthane on ajoute, à une température voisine de 20°C, 1,8 g de N,N'-diimidazole-carbonyle. Le milieu réactionnel est agité pendant 2 heures à une température voisine de 20°C puis on ajoute 1$cm^3$ de tert-butylamine et chauffe à reflux pendant pendant 5 heures en agitant. Après refroidissement, le mélange réactionnel est dilué par 100 $cm^3$ de dichlorométhane et lavé successivement par 2 fois 50 $cm^3$ d'une solution aqueuse normale d'acide chlorhydrique puis par 3 fois 50 $cm^3$ d'eau. La phase organique est séchée sur sulfate de magnésium puis évaporée à sec sans pression réduite. Le résidu obtenu est purifié par chromatographie sur silice [éluant: dichlorométhane-méthanol (97,5/2,5 en volumes)] et les fractions contenant le produit attendu sont réunies et concentrées à sec sans pression réduite. On obtient ainsi 3 g de tert-butoxycarbonyl-1 N-tert-butyl phényl prolinamide-(2RS, 5SR) sous forme d'une huile jaune utilisée telle quelle dans les synthèses ultérieures.

**Exemple 60**

En opérant d'une manière analogue à celle décrite à l'exemple 9, mais à partir de 10,6 g de {[(diphényl-2,5 pyrrolidinyl-1)-2 oxo-2 éthyl]-3 uréido}-3 phénylacétate de méthyle-(cis) en solution dans 150 $cm^3$ de méthanol et de 1,7 g d'hydroxyde de potassium dissous dans 30 $cm^3$ d'eau et après traitement et recristallisation dans l'éthanol, on obtient 1,2 g d'acide {[(diphényl-2,5 pyrrolidinyl-1)-2 oxo-2 éthyl]-3 uréido}-3 phénylacétique-(cis) fondant à 152°C.

Le {[(diphényl-2,5 pyrrolidinyl-1)-2 oxo-2 éthyl]-3 uréido}-3 phénylacétate de méthyle-(cis) peut être préparé d'une manière analogue à celle décrite à l'exemple 41 §A, mais à partir de 4,45 g de diphényl-2,5 pyrrolidine-(cis), de 5,32 g d'acide [(méthoxycarbonylméthyl-3 phényl)-3 uréido]-2 acétique et de 4,2 g de N,N'-dicyclohexylcarbodiimide dans 40 $cm^3$ d'acétonitrile. Après traitement, on obtient 10,6 g de {[(diphényl-2,5 pyrrolidinyl-1)-2 oxo-2 éthyl]-3 uréido}-3 phénylacétate de méthyle-(cis) sous forme d'une huile utilisée telle quelle dans les synthèses ultérieures.

L'acide [(méthoxycarbonylméthyl-3 phényl)-3 uréido]-2 acétique peut être préparé d'une manière analogue à celle décrite à l'exemple 1 §A, mais à partir de 9,42 g de glycine, de 34,69 g de carbonate de potassium dans 220 $cm^3$ d'eau et de 24 g d'isocyanato-3 phénylacétate de méthyle dissous dans 170 $cm^3$ de dioxanne-1,4. Après traitement et recristallisation dans l'acétate d'éthyle, on obtient 46,85 g d'acide [(méthoxycarbonylméthyl-3 phényl)-3 uréido]-2 acétique fondant à 136°C.

**Exemple 61**

En opérant d'une manière analogue à celle décrite à l'exemple 9, mais à partir de 4,8 g de {{[(diphényl-2,5 pyrrolidinyl-1)-2 oxo-2 éthyl]-3 uréido}-3 phényl}-3 propionate d'éthyle-(cis) en solution dans 25 $cm^3$ de méthanol et de 0,25 g d'hydroxyde de potassium dissous dans 5 $cm^3$ d'eau et après traitement, on obtient 0,9 g d'acide {{[(diphényl-2,5 pyrrolidinyl-1)-2 oxo-2 éthyl]-3 uréido}-3 phényl}-3 propionique-(cis) fondant à 170°C.

Le {{[(diphényl-2,5 pyrrolidinyl-1)-2 oxo-2 éthyl]-3 uréido}-3 phényl}-3 propionate d'éthyle-(cis) peut être préparé d'une manière analogue à celle décrite à l'exemple 41 §A, mais à partir de 0,68 g de diphényl-2,5 pyrrolidine-(cis), de 0,9 g d'acide {[(éthoxycarbonyl-2 éthyl)-3 phényl]-3 uréido}-2 acétique et de 0,63 g de N,N'-dicyclohexylcarbodiimide dans 6 $cm^3$ d'acétonitrile. Après traitement, on obtient 1,8 g de {{[(diphényl-2,5 pyrrolidinyl-1)-2 oxo-2 éthyl]-3 uréido}-3 phényl}-3 propionate d'éthyle-(cis) sous forme d'une huile utilisée telle quelle dans les synthèses ultérieures.

L'acide {[(éthoxycarbonyl-2 éthyl)-3 phényl]-3 uréido}-2 acétique peut être préparé d'une manière analogue à celle décrite à l'exemple 1 §A, mais à partir de 0,44 g de glycine, de 1,63 g de carbonate de potassium dans 10 $cm^3$ d'eau et de 1,3 g d'(isocyanato-3 phényl)-3 propionate d'éthyle en solution dans 8 $cm^3$ de dioxanne-1,4. Après traitement, on obtient 0,9 g d'acide {[(éthoxycarbony1-2 éthyl)-3 phényl]-3 uréido}-2 acétique sous forme de solide amorphe utilisé tel quel dans les synthèses ultérieures.

L'(isocyanato-3 phényl)-3 propionate d'éthyle peut être préparé de la manière suivante : à une suspension de 0,45g de charbon dans 3 g d'(amino-3 phényl)-3 propionate d'éthyle en solution dans 60 $cm^3$ de toluène, on ajoute en 30 minutes à une température voisine de -20°C, une solution de 2,36 g de carbonate de bis trichlorométhyle dans 20 $cm^3$ de toluène. Le mélange réactionnel est chauffé à une température voisine de 110°C pendant 2 heures et 30 minutes, refroidi à une température voisine de 20°C, filtré sur célite et concentré sous pression réduite. On obtient ainsi 3,5 g d'(isocyanato-3 phényl)-3 propionate d'éthyle sous forme d'une huile utilisée telle quelle dans les synthèses ultérieures.

**Exemple 62**

En opérant d'une manière analogue à celle décrite à l'exemple 9, mais à partir de 1,2 g de {{[(méthoxy-2 phényl)-2 phényl-5 pyrrolidinyl-1]-2 oxo-2 éthyl}-3 uréido}-3 phénylacétate de méthyle-(2RS,5SR) en solution dans 20 $cm^3$ de méthanol et de 0,19 g d'hydroxyde de potassium en solution dans 4 $cm^3$ d'eau et après traitement et cristallisation dans l'oxyde de diéthyle, on obtient 0,6 g d'acide {{[(méthoxy-2 phényl)-2 phényl-5 pyrrolidinyl-1]-2 oxo-2 éthyl}-3 uréido}-3 phénylacétique-(2RS,5SR) fondant à 140°C.

Le {{[(méthoxy-2 phényl)-2 phényl-5 pyrrolidinyl-1]-2 oxo-2 éthyl}-3 uréido}-3 phénylacétate de méthyle-(cis) peut être préparé d'une manière analogue à celle décrite à l'exemple 41 §A, mais à partir de 0,57 g de (méthoxy-2 phényl)-2 phényl-5 pyrrolidine-(2RS,5SR), de 0,6 g d'acide [(méthoxycarbonylméthyl-3 phényl)-3 uréido]-2 acétique et de 0,46 g de N,N'-dicyclohexylcarbodiimide dans 4,5 $cm^3$ d'acétonitrile. Après traitement, on obtient 1,2 g de {{[(méthoxy-2 phényl)-2 phényl-5 pyrrolidinyl-1]-2 oxo-2 éthyl}-3 uréido}-3 phénylacétate de méthyle-(2RS,5SR) sous forme d'une huile utilisée telle quelle dans les synthèses ultérieures.

A - La (méthoxy-2 phényl)-2 phényl-5 pyrrolidine-(2RS,5SR) peut être préparée de la manière suivante : à une solution de 2,25 g de N-benzyl o-méthoxybenzaldimine dans 20 $cm^3$ de tétrahydrofuranne, on ajoute goutte à goutte, sous agitation, 12,8 $cm^3$ d'une solution 1,6 M de butyl lithium dans l'hexane à une température voisine de -78°C. Après retour à une température voisine de 20°C, l'agitation est poursuivie pendant 40 heures, puis le mélange réactionnel est versé dans 100 $cm^3$ d'une solution aqueuse saturée en chlorure d'ammonium et le produit extrait par 50 $cm^3$ puis deux fois 25 $cm^3$ d'oxyde de diisopropyle. Les phases organiques réunies sont séchées sur sulfate de magnésium et concentrées à sec sous pression réduite à une température voisine de 35°C. Le résidu est dissous dans 25 $cm^3$ d'une solution aqueuse normale d'acide chlorhydrique et la solution est chauffée à reflux pendant 1 minute. Après refroidissement à une température voisine de 20°C, la solution est lavée par trois fois 25 $cm^3$ d'oxyde de diisopropyle, puis le pH est ajusté à 9 à l'aide d'une solution aqueuse N d'hydroxyde de sodium et le produit extrait par trois fois 25 $cm^3$ d'oxyde de diisopropyle. Les extraits organiques sont réunis, lavés par deux fois 25 $cm^3$ d'eau, séchés sur sulfate de magnésium et concentrés à sec sous pression réduite à une température voisine de 35°C. Le produit obtenu est purifié par chromatographie sur silice [éluant : éther de pétrole-oxyde de diisopropyle (50-50 en volumes)]. Les fractions contenant le produit attendu sont réunies et concentrées à sec sous pression réduite à une température voisine de 45°C. On obtient ainsi 0,9 g de (méthoxy-2 phényl)-2 phényl-5 pyrrolidine-(2RS,5SR) sous forme d'un solide amorphe utilisé tel quel dans les synthèses ultérieures.

B - La N-benzyl o-méthoxybenzaldimine peut être préparée de la manière suivante : une solution de 60 $cm^3$ d'orthométhoxybenzaldéhyde, de 55 $cm^3$ de benzylamine et de 50 mg d'acide paratoluène sulfonique dans 150 $cm^3$ de toluène est chauffée pendant trois heures au reflux en recueillant l'eau formée à l'aide d'un appareil de Dean-Stark. Après retour à une température voisine de 20°C, la phase organique est lavée par 100 $cm^3$ d'une solution aqueuse saturée en hydrogénocarbonate de sodium puis 100 $cm^3$ d'eau, séchée sur sulfate de magnésium et concentrée sous pression réduite. Après distillation, on obtient 57,3 g de N-benzyl o-méthoxybenzaldimine distillant entre 155 et 159°C sous 130 Pa.

**Exemple 63**

En opérant d'une manière analogue à celle décrite à l'exemple 9, mais à partir de 0,46 g d'{{[(hydroxy-3 phényl)-2 phényl-5 pyrrolidinyl-1]-2 oxo-2 éthyl}-3 uréido}-3 phénylacétate de méthyle-(2RS,5SR) en solution dans 10 $cm^3$ de méthanol et de 0,1 g d'hydroxyde de potassium en solution dans 2 $cm^3$ d'eau et après traitement et recristallisation dans l'éthanol, on obtient 0,2 g d'acide {{[(hydroxy-3 phényl}-2 phényl-5 pyrrolidinyl-1]-2 oxo-2 éthyl}-3 uréido}-3 phénylacétique-(2RS,5SR) fondant à 166°C.

L'{{[(hydroxy-3 phényl)-2 phényl-5 pyrrolidinyl-1]-2 oxo-2 éthyl}-3 uréido}-3 phénylacétate de méthyle-(2RS,5SR) peut être préparé d'une manière analogue à celle décrite à l'exemple 41 §A, mais à partir de 0,48 g d'(hydroxy-3 phényl)-2 phényl-5 pyrrolidine-(2RS,5SR), de 0,54 g d'acide [(méthoxycarbonylméthyl-3 phényl)-3 uréido]-2 acétique et de 0,42 g de N,N'-dicyclohexylcarbodiimide dans 5 $cm^3$ d'acétonitrile. Après traitement, on obtient 0,73 g d'{{[(hydroxy-3 phényl)-2 phényl-5 pyrrolidinyl-1]-2 oxo-2 éthyl}-3 uréido}-3 phénylacétate de méthyle-(2RS,5SR) sous forme d'une meringue utilisée telle quelle dans les synthèses ultérieures.

L'(hydroxy-3 phényl)-2 phényl-5 pyrrolidine-(2RS,5SR) peut être préparée d'une manière analogue à celle décrite à l'exemple 62 §A mais à partir de 4,22 g de N-benzyl m-hydroxybenzaldimine en solution dans 40 $cm^3$ de tétrahydrofuranne et de 33,6 $cm^3$ d'une solution 2,5M de butyl lithium dans l'hexane. Après traitement, on obtient 3,3 g d'(hydroxy-3 phényl)-2 phényl-5 pyrrolidine-(2RS,5SR) sous forme d'une huile utilisée telle quelle dans les synthèses ultérieures.

La N-benzyl m-hydroxybenzaldimine peut être préparée d'une manière analogue à celle décrite à l'exemple 62 §A mais à partir de 61,1 g d'hydroxy-3 benzaldéhyde et de 55 $cm^3$ de benzylamine dans 450 $cm^3$ de toluène. Après traitement, on obtient 100,3 g de N-benzyl m-hydroxybenzaldimine fondant à 151°C.

**Exemple 64**

En opérant d'une manière analogue à celle décrite à l'exemple 9, mais à partir de 4 g de {[(diphényl-2,5 pyrrolidinyl-1)-2 oxo-2 éthyl]-3 uréido}-4 phénylacétate de benzyle-(cis) en solution dans 65 $cm^3$ de méthanol et de 0,61 g d'hydroxyde de potassium en solution dans 12 $cm^3$ d'eau et après traitement on obtient 1,8 g d'acide {[(diphényl-2,5 pyrrolidinyl-1)-2 oxo-2 éthyl]-3 uréido}-4 phénylacétique-(cis)fondant à 221°C, [RMN du proton (200 MHz, DMSO $D_6$, d en ppm,), 1,4 à 2,4 (m, 4H, $CH_2$-$CH_2$); 3,3 (s, 2H, $CH_2CO_2$); 3,2 et 3,8 (ABX, 2H, $CH_2$NH); 5,1 (m, 2H, 2 CHN); 6,1 (t, 1H échangeable, NH); 6,9 (d, 2H, aromatiques en para); 7,1 à 7,4 (m, 12H, aromatiques); 8,6 (s, 1H échangeable, NH)], [Spectre infra-rouge (KBr), bandes caractéristiques en $cm^{-1}$ 3375, 3095, 3070, 3035, 2980, 2880, 1735, 1700, 1630, 1610, 1515, 1450, 1560, 805, 760, 705].

Le {[(diphényl-2,5 pyrrolidinyl-1)-2 oxo-2 éthyl]-3 uréido}-4 phénylacétate de benzyle-(cis) peut être préparé d'une manière analogue à celle décrite à l'exemple 41 §A, mais à partir de 1,7 g de diphényl-2,5 pyrrolidine-(cis), de 2,6 g d'acide [(benzyloxycarbonylméthyl-4 phényl)-3 uréido]-2 acétique et de 1,6 g de N,N'-dicyclohexylcarbodiimide dans 15 $cm^3$ d'acétonitrile. Après traitement, on obtient 4 g de {[(diphényl-2,5 pyrrolidinyl-1)-2 oxo-2 éthyl]-3 uréido}-4 phénylacétate de benzyle-(cis) sous forme d'une meringue utilisée telle quelle dans les synthèses ultérieures.

L'acide [(benzyloxycarbonylméthyl-4 phényl)-3 uréido]-2 acétique peut être préparé d'une manière analogue à celle décrite à l'exemple 1 §A, mais à partir de 3,68 g de glycine, de 4,1 g d'hydrogénocarbonate de sodium dans 150 $cm^3$ d'eau et de 13 g d'isocyanato-4 phénylacétate de benzyle en solution dans 60 $cm^3$ de dioxanne-1,4. Après traitement, on obtient 5,2 g d'acide [(benzyloxycarbonylméthyl-4 phényl)-3 uréido]-2 acétique fondant à 192°C.

A - L'isocyanato-4 phénylacétate de benzyle peut être préparé de la manière suivante : à une suspension de 1 g de charbon dans un mélange de 5,9 $cm^3$ de chloroformiate de trichlorométhyle et de 50 $cm^3$ de toluène, on ajoute en 15 minutes à une température voisine de -25°C, une solution de 11,8 g d'amino-4 phénylacétate de benzyle dans 150 $cm^3$ de toluène. Le mélange réactionnel est agité à une température voisine de 25°C pendant deux heures, puis chauffé à une température voisine de 110°C pendant 2 heures. Après refroidissement à une température voisine de 25°C, le milieu réactionnel est dégazé par barbottage d'azote, filtré sur papier filtre et concentré sous pression réduite à une température voisine de 52°C. On obtient ainsi 15,4 g d'isocyanato-4 phénylacétate de benzyle sous forme d'une huile utilisée telle quelle dans les synthèses ultérieures. L'amino-4 phénylacétate de benzyle peut être préparé selon la méthode décrite par E. ZURABYAN et coll., Izv. Akad. Nauk. SSSR, Ser. Khim., (11) 2036 (1964).

**Exemple 65**

En opérant d'une manière analogue à celle décrite à l'exemple 9, mais à partir de 3,5 g de {{[(méthyl-2 phényl)-2 phényl-5 pyrrolidinyl-1]-2 oxo-2 éthyl}-3 uréido}-3 phénylacétate de méthyle-(2RS,5SR) en solution

dans 60 $cm^3$ de méthanol et de 0,56 g d'hydroxyde de potassium en solution dans 11 $cm^3$ d'eau et après traitement et recristallisation dans l'acétate d'éthyle, on obtient 2,2 g d'acide {{[(méthyl-2 phényl)-2 phényl-5 pyrrolidinyl-1]-2 oxo-2 éthyl}-3 uréido}-3 phénylacétique-(2RS,5SR) fondant à 133°C.

Le {{[(méthyl-2 phényl)-2 phényl-5 pyrrolidinyl-1]-2 oxo-2 éthyl}-3 uréido}-3 phénylacétate de méthyle-(2RS,5SR) peut être préparé d'une manière analogue à celle décrite à l'exemple 41 §A, mais à partir de 1,6 g de (méthyl-2 phényl)-2 phényl-5 pyrrolidine-(2RS,5SR), de 1,8 g d'acide [(méthoxycarbonylméthyl-3 phényl)-3 uréido]-2 acétique et de 1,4 g de N,N'-dicyclohexylcarbodiimide dans 15 $cm^3$ d'acétonitrile. Après traitement, on obtient 3,5 g de {{[(méthyl-2 phényl)-2 phényl-5 pyrrolidinyl-1]-2 oxo-2 éthyl}-3 uréido}-3 phénylacétate de méthyle-(2RS,5SR) sous forme d'une meringue utilisée telle quelle dans les synthèses ultérieures.

La (méthyl-2 phényl)-2 phényl-5 pyrrolidine-(2RS,5SR) peut être préparée d'une manière analogue à celle décrite à l'exemple 62 §A mais à partir de 4,18 g de N-benzyl o-méthylbenzaldimine en solution dans 40 $cm^3$ de tétrahydrofuranne et de 25,6 $cm^3$ d'une solution 2,5 M de butyl lithium dans l'hexane. Après traitement, on obtient 1,7 g de (méthyl-2 phényl)-2 phényl-5 pyrrolidine-(2RS,5SR) sous forme d'une huile utilisée telle quelle dans les synthèses ultérieures.

La N-benzyl o-méthylbenzaldimine peut être préparée selon la méthode décrite par A. PADVA et coll., J. Amer. Chem. Soc., 91 2653 (1969).

**Exemple 66**

En opérant d'une manière analogue à celle décrite à l'exemple 9, mais à partir de 1,8 g d'{{[(hydroxy-3 phényl)-2 (méthoxy-3 phényl)-5 pyrrolidinyl-1]-2 oxo-2 éthyl}-3 uréido}-3 benzoate d'éthyle-(2RS,5SR) en solution dans 30 $cm^3$ de méthanol et de 0,29 g d'hydroxyde de potassium en solution dans 6 $cm^3$ d'eau et après traitement et cristallisation dans l'oxyde de diéthyle, on obtient 1 g d'acide {{[(hydroxy-3 phényl)-2 (méthoxy-3 phényl)-5 pyrrolidinyl-1]-2 oxo-2 éthyl}-3 uréido}-3 benzoïque-(2R*,5S*) fondant à 172°C.

L'{{[(hydroxy-3 phényl)-2 (méthoxy-3 phényl)-5 pyrrolidinyl-1]-2 oxo-2 éthyl}-3 uréido}-3 benzoate d'éthyle-(2RS,5SR) peut être préparé d'une manière analogue à celle décrite à l'exemple 41 §A, mais à partir de 1,3 g d'(hydroxy-3 phényl)-2 (méthoxy-3 phényl)-5 pyrrolidine-(2RS,5SR), de 1,3 g d'acide [(éthoxycarbonyl-3 phényl)-3 uréido]-2 acétique et de 1 g de N,N'-dicyclohexylcarbodiimide dans 11 $cm^3$ d'acétonitrile. Après traitement, on obtient 1,8 g d'{{[(hydroxy-3 phényl)-2 (méthoxy-3 phényl)-5 pyrrolidinyl-1]-2 oxo-2 éthyl}-3 uréido}-3 benzoate d'éthyle-(2RS,5SR) sous forme d'une meringue utilisée telle quelle dans les synthèses ultérieures.

L'(hydroxy-3 phényl)-2 (méthoxy-3 phényl)-5 pyrrolidine-(2RS,5SR) peut être préparée d'une manière analogue à celle décrite à l'exemple 62 §A mais à partir de 7,2 g de N-[(hydroxy-3 phényl)méthylène] (méthoxy-3 phényl)méthylamine dans 60 $cm^3$ de tétrahydrofuranne et de 50,4 $cm^3$ d'une solution 2,5 M de butyl lithium dans l'hexane. Après traitement, on obtient 4,5 g d'(hydroxy-3 phényl)-2 (méthoxy-3 phényl)-5 pyrrolidine-(2RS,5SR) sous forme d'une huile utilisée telle quelle dans les synthèses ultérieures.La N-[(hydroxy-3 phényl)méthylène] (méthoxy-3 phényl)méthylamine peut être préparée d'une manière analogue à celle décrite à l'exemple 62 §B mais à partir de 13,2 g d'hydroxy-3 benzaldéhyde et de 13,7 g de m-méthoxybenzylamine dans 90 $cm^3$ de toluène. Après traitement, on obtient 20,7 g de N-[(hydroxy-3 phényl)méthylène] (méthoxy-3 phényl)méthylamine fondant à 103°C.

L'acide [(éthoxycarbonyl-3 phényl)-3 uréido]-2 acétique peut être préparé comme décrit à l'exemple 1 §A, mais à partir de 9,85 g de glycine, de 11 g d'hydrogénocarbonate de sodium en solution dans 150 $cm^3$ d'eau et de 25 g d'isocyanato-3 benzoate d'éthyle. Après traitement, on obtient 16 g d'acide [(éthoxycarbonyl-3 phényl)-3 uréido]-2 acétique fondant à 174°C.

**Exemple 67**

En opérant d'une manière analogue à celle décrite à l'exemple 9, mais à partir de 1,8 g d'{{[(hydroxy-3 phényl)-2 phényl-5 pyrrolidinyl-1]-2 oxo-2 éthyl}-3 uréido}-4 phénylacétate de benzyle-(2RS,5SR) en solution dans 30 $cm^3$ de méthanol et de 0,27 g d'hydroxyde de potassium en solution dans 6 $cm^3$ d'eau et après traitement et cristallisation dans l'oxyde de diéthyle, on obtient 0,5 g d'acide {{[(hydroxy-3 phényl)-2 phényl-5 pyrrolidinyl-1]-2 oxo-2 éthyl}-3 uréido}-4 phénylacétique-(2RS,5SR) fondant à 260°C.

L'{{[(hydroxy-3 phényl)-2 phényl-5 pyrrolidinyl-1]-2 oxo-2 éthyl}-3 uréido}-4 phénylacétate de benzyle-(2RS,5SR) peut être préparé d'une manière analogue à celle décrite à l'exemple 41 §A, mais à partir de 1,4 g d'(hydroxy-3 phényl)-2 phényl-5 pyrrolidine-(2RS,5SR), de 2,0 g d'acide [(méthoxycarbonylméthyl-3 phényl)-3 uréido]-2 acétique et de 1,2 g de N,N'-dicyclohexylcarbodiimide dans 11 $cm^3$ d'acétonitrile. Après traitement, on obtient 1.8 g d'{{[(hydroxy-3 phényl)-2 phényl-5 pyrrolidinyl-1]-2 oxo-2 éthyl}-3 uréido}-4 phénylacétate de benzyle-(2RS,5SR) sous forme d'une meringue utilisée telle quelle dans les synthèses ultérieures.

**Exemple 68**

En opérant d'une manière analogue à celle décrite à l'exemple 9, mais à partir de 1,3 g de {{[(diphényl-2,5 pyrrolidinyl-1)-2 oxo-2 éthyl]-3 uréido}-4 phénylthio}acétate de tert-butyle-(cis) en solution dans 20 $cm^3$ de méthanol et de 0,17 g d'hydroxyde de potassium en solution dans 4 $cm^3$ d'eau et après traitement, on obtient 0,5 g d'acide {{[(diphényl-2,5 pyrrolidinyl-1)-2 oxo-2 éthyl]-3 uréido}-4 phénylthio}acétique-(cis) fondant vers 200°C, [RMN du proton (200 MHz, DMSO $D_6$, d en ppm,), 1,8 à 2,4 (m, 4H, $CH_2$-$CH_2$); 3,35 (s, 2H, $CH_2S$); 3,3 et 3,85 (ABX, 2H, $CH_2N$): 5,1 (m, 2H, 2 CHN); 6,5 (t, 1H échangeable, NH); 7,1 à 7,4 (m, 14H, aromatiques); 9 (s, 1H échangeable, NH)], [Spectre infra-rouge (KBr), bandes caractéristiques en $cm^{-1}$ 3400, 3090, 3060, 3030, 2975, 2875, 1640, 1595, 1540, 1495, 1450, 1400, 825, 760, 700].

Le {{[(diphényl-2,5 pyrrolidinyl-1)-2 oxo-2 éthyl]-3 uréido}-4 phénylthio}acétate de tert-butyle-(cis) peut être préparé d'une manière analogue à celle décrite à l'exemple 41 §A, mais à partir de 0,46 g de diphényl-2,5 pyrrolidine, de 0,7 g d'acide [(tert-butoxycarbonylméthylthio-4 phényl)-3 uréido]-2 acétique et de 0,42 g de N,N'-dicyclohexylcarbodiimide dans 5 $cm^3$ d'acétonitrile. Après traitement, on obtient 1,3 g de {{[(diphényl-2,5 pyrrolidinyl-1)-2 oxo-2 éthyl]-3 uréido}-4 phénylthio}acétate de tert-butyle-(cis) sous forme d'une meringue utilisée telle quelle dans les synthèses ultérieures.

L'acide [(tert-butoxycarbonylméthylthio-4 phényl)-3 uréido]-2 acétique peut être préparé d'une manière analogue à celle décrite à l'exemple 1 §A, mais à partir de 0,65 g de glycine, de 2,4 g de carbonate de potassium dans 15 $cm^3$ d'eau et de 2,3 g d'(isocyanato-4 phénylthio)acétate de tert-butyle en solution dans 12 $cm^3$ de dioxanne-1,4. Après traitement, on obtient 1,7 g d'acide [(tert-butoxycarbonylméthylthio-4 phényl)-3 uréido]-2 acétique sous forme d'une meringue utilisée telle quelle dans les synthèses ultérieures.

L'(isocyanato-4 phénylthio)acétate de tert-butyle peut être préparé d'une manière analogue à celle décrite à l'exemple 64 §A mais à partir de 3,1 g d'(amino-4 phénylthio)acétate de tert-butyle dans 117 $cm^3$ de toluène, de 0,26 g de charbon et de 1,56 $cm^3$ de chloroformiate de trichlorométhyle dans 30 $cm^3$ de toluène. Après traitement, on obtient 2,3 g d'(isocyanato-4 phénylthio)acétate de tert-butyle sous forme d'une huile utilisée telle quelle dans les synthèses ultérieures.

L'(amino-4 phénylthio)acétate de tert-butyle peut être préparé d'une manière analogue à celle décrite à l'exemple 17 pour la préparation de l'(amino-3 phénylthio)acétate d'éthyle, mais à partir de 5 g d'amino-4 thiophénol et de 6,4 $cm^3$ de bromoacétate de tert-butyle dans 80 $cm^3$ d'éthanol. On obtient ainsi 7,45 g d'(amino-4 phénylthio)acétate de tert-butyle sous forme d'une huile utilisée telle quelle dans les synthèses ultérieures.

**Exemple 69**

En opérant d'une manière analogue à celle décrite à l'exemple 9, mais à partir de 0,8 g d' {{[(hydroxy-2 phényl)-2 phényl-5 pyrrolidinyl-1]-2 oxo-2 éthyl}-3 uréido}-3 benzoate d'éthyle-(2RS,5SR} en solution dans 15 $cm^3$ de méthanol et de 0,27 g d'hydroxyde de potassium en solution dans 3 $cm^3$ d'eau et après traitement et cristallisation dans l'oxyde de diisopropyle, on obtient 0,21 g d'acide {{[(hydroxy-2 phényl)-2 phényl-5 pyrrolidinyl-1]-2 oxo-2 éthyl}-3 uréido}-3 benzoïque-(2RS,5SR) fondant vers 160°C, [RMN du proton (200 MHz, DMSO $D_6$, d en ppm,), 1,8 à 2,5 (2m, 4H, $CH_2$-$CH_2$); 3,3 et 4 (ABX, 2H, $CH_2N$); 5,2 (m, 2H, 2 CHN); 6,7 à 7,4 (m, 13H, aromatiques); 6,8 (bt) 9(s) 9,8(bs) 12,8(vbs) (4H échangeables, 1 OH, 1 $CO_2H$ et 2 NH)], [Spectre infra-rouge (KBr), bandes caractéristiques en $cm^{-1}$ 3400, 3070, 2980, 1695, 1630, 1560, 1490, 1460, 760, 700, 685].

L'{{[(hydroxy-2 phényl)-2 phényl-5 pyrrolidinyl-1]-2 oxo-2 éthyl}-3 uréido}-3 benzoate d'éthyle-(2RS,5SR) peut être préparé d'une manière analogue à celle décrite à l'exemple 41 §A, mais à partir de 0,5 g d'(hydroxy-2 phényl)-2 phényl-5 pyrrolidine-(2RS,5SR), de 0,56 g d'acide [(éthoxycarbonyl-3 phényl)-3 uréido]-2 acétique et de 0,43 g de N,N'-dicyclohexylcarbodiimide dans 7,5 $cm^3$ d'acétonitrile. Après traitement, on obtient 0,85 g d'{{[(hydroxy-2 phényl)-2 phényl-5 pyrrolidinyl-1]-2 oxo-2 éthyl}-3 uréido}-3 benzoate d'éthyle-(2RS,5SR) sous forme d'une meringue utilisée telle quelle dans les synthèses ultérieures.

L'(hydroxy-2 phényl)-2 phényl-5 pyrrolidine-(2RS,5SR) peut être préparée de la manière suivante : à une solution de 8,6 $cm^3$ de diisopropylamine dans 60 $cm^3$ de tétrahydrofuranne, on ajoute en dix minutes, à une température voisine de -78°C, 24 $cm^3$ d'une solution 2,5 M de butyl lithium. Le milieu réactionnel est ensuite agité à une température voisine de 20°C pendant quinze minutes puis refroidi à une température voisine de -70°C. On ajoute alors en cinq minutes, à une température voisine de -70°C, une solution de 4,2 g de N-benzyl o-hydroxybenzaldimine dans 5 $cm^3$ de tétrahydrofuranne. La solution est agitée pendant dix minutes à une température voisine de -70°C puis on laisse la température remonter vers 20°C. Le milieu réactionnel est ensuite saturé par barbottage d'éthylène pendant 40 heures, puis versé dans 150 $cm^3$ d'une solution aqueuse saturée en chlorure d'ammonium et le produit extrait par 100 $cm^3$ puis deux fois 50 $cm^3$ d'oxyde de diéthyle. Les phases organiques réunies sont séchées sur sulfate de magnésium et concentrées à sec sous pression réduite à une

température voisine de 35°C. Le résidu est dissous dans 150 cm3 d'une solution aqueuse normale d'acide chlorhydrique et la solution est chauffée à reflux pendant 1 minute. Après refroidissement à une température voisine de 20°C, la solution est lavée par trois fois 100 $cm^3$ d'oxyde de diéthyle, puis le pH est ajusté à 11 à l'aide d'une solution aqueuse 4N d'hydroxyde de sodium et le produit extrait par trois fois 75 $cm^3$ d'oxyde de diéthyle. Les extraits organiques sont réunis, lavés par deux fois 50 $cm^3$ d'eau, séchés sur sulfate de magnésium et concentrés à sec sous pression réduite à une température voisine de 35°C. Le produit obtenu est purifié par chromatographie sur silice [éluant : dichlorométhane puis dichlorométhane-méthanol (95-5 en volumes)]. Les fractions contenant le produit attendu sont réunies et concentrées à sec sous pression réduite à une température voisine de 45°C. On obtient ainsi 0,5 g d'(hydroxy-2 phényl)-2 phényl-5 pyrrolidine-(2RS,5SR) sous forme d'une huile utilisée telle quelle dans les synthèses ultérieures.

La N-benzyl o-hydroxybenzaldimine peut être préparée d'une manière analogue à celle décrite à l'exemple 62 §B mais à partir de 21,2 $cm^3$ d'hydroxy-2 benzaldéhyde et de 22 $cm^3$ de benzylamine dans 180 $cm^3$ de toluène. Après traitement, on obtient 38,2 g de N-benzyl o-hydroxybenzaldimine sous forme d'un solide fondant en-dessous de 50°C.

**Exemple 70**

En opérant d'une manière analogue à celle décrite à l'exemple 9, mais à partir de 1,4 g de {{[(hydroxy-2 phényl)-2 phényl-5 pyrrolidinyl-1]-2 oxo-2 éthyl}-3 uréido}-3 phénylacétate de méthyle-(2RS,5SR) en solution dans 30 $cm^3$ de méthanol et de 0,49 g d'hydroxyde de potassium en solution dans 6 $cm^3$ d'eau et après traitement et cristallisation dans l'oxyde de diisopropyle, on obtient 0,45 g d'acide {{[(hydroxy-2 phényl)-2 phényl-5 pyrrolidinyl-1]-2 oxo-2 éthyl}-3 uréido}-3 phénylacétique-(2RS,5SR) fondant vers 165°C.

L'{{[(hydroxy-2 phényl)-2 phényl-5 pyrrolidinyl-1]-2 oxo-2 éthyl}-3 uréido}-3 phénylacétate de méthyle-(2RS,5SR) peut être préparé d'une manière analogue à celle décrite à l'exemple 41 §A, mais à partir de 1,8 g d'(hydroxy-3 phényl)-2 phényl-5 pyrrolidine-(2RS,5SR), de 2 g d'acide [(méthoxycarbonylméthyl-3 phényl)-3 uréido]-2 acétique et de 1,55 g de N,N'-dicyclohexylcarbodiimide dans 30 $cm^3$ d'acétonitrile. Après traitement, on obtient 1,4 g d'{{[(hydroxy-2 phényl)-2 phényl-5 pyrrolidinyl-1]-2 oxo-2 éthyl}-3 uréido}-3 phénylacétate de méthyle-(2RS,5SR) sous forme d'une meringue utilisée telle quelle dans les synthèses ultérieures.

**Exemple 71**

On opère d'une manière analogue à celle décrite à l'exemple 41, mais à partir de 0,2 g de {{[tert-butoxycarbonyl-2 (indolyl-3)-5 pyrrolidinyl-1]-2 oxo-2 éthyl}-3 uréido}-3 benzoate de triméthylsilyl-2 éthyle-(2S,5R) et de 1,0 $cm^3$ d'une solution 1M de fluorure de tétrabutylammonium. Le produit brut est dissous dans 25 $cm^3$ d'acétate d'éthyle et extrait par 2 fois 5 $cm^3$ d'une solution aqueuse d'hydroxyde de sodium 0,1N. Les phases aqueuses sont amenées à pH 2 par addition d'une solution aqueuse d'acide chlorhydrique 1N. Le produit précipité est séparé par filtration, lavé par 2 fois 5 $cm^3$ d'eau distillée et séché à l'air. On obtient ainsi 0,12g d'acide {{[tert-butoxycarbonyl-2 (indolyl-3)-5 pyrrolidinyl-1]-2 oxo-2 éthyl}-3 uréido}-3 benzoïque-(2S,5R) sous forme d'un solide fondant à 160°C, $[\alpha]_D^{20}$= +10,7° ±1,1° (C = 0,43%; $CH_3OH$), [RMN du proton (300 MHz, DMSO $D_6$, d en ppm, J en Hz):1,5 (s, 9H,-$C(CH_3)_3$); 1,8 à 2,5 (m, 4H, -$CH_2$-$CH_2$-); 3,6 et 4,1 (2dd, J= 17,5 et 5,1, 2H , N-CO-$CH_2$-N); 4,26 (dd, J= 9 et 8, 1H, N-CH-COO-); 5,52 (d, J = 8,5, 1H, N-CH-Ar); 6,38 (t, J= 5,1 H, -NH-CO-); 7,0 à 7,7 (m, 7H, aromatiques); 7,68 (s, 1H, N-CH=); 8 (bs, 1H, en position 2 sur NH-Ph-COO); 9,03 (massif, 1H, Ph-NH-CO-); 11,05 (massif, 1H, -NH-),un effet NOE a été observé entre N-CH= de l'indole et le proton en position 5 de la pyrrolidine], [Spectre infra-rouge (KBr), bandes caractéristiques en $cm^{-1}$: 3380, 2980, 2935, 2700 à 2250 (bande large), 1720 à 1695 (bande large), 1635, 1595, 1560, 1490, 1460, 1370, 1155, 745, 685].

Le {{[tert-butoxycarbonyl-2 (indolyl-3)-5 pyrrolidinyl-1]-2 oxo-2 éthyl}-3 uréido}-3 benzoate de triméthylsilyl-2 éthyle-(2S,5R) peut être préparé d'une manière analogue à celle décrite à l'exemple 41 §A mais à partir de 0,5 g d'(indolyl-3)-5 pyrrolidinecarboxylate-2 de tert-butyle-(2S,5RS), de 0,59 g d'acide {[(triméthylsilyl-2 éthoxycarbonyl)-3 phényl]-3 uréido}-2 acétique et de 0,36 g de N,N'-dicyclohexylcarbodiimide. Après une première chromatographie sur silice [éluant acétate d'éthyle-cyclohexane (40/60 en volumes)], les fractions contenant le mélange des diastéréoisomères sont concentrées à sec sous pression réduite à 40°C et le résidu obtenu est purifié par une deuxième chromatographie sur silice [éluant acétate d'éthyle-cyclohexane (avec gradient d'acétate d'éthyle). Les fractions contenant chacun des deux diastéréoisomères sont réunies et concentrées à sec sous pression réduite à 40°C. On obtient ainsi 0,20 g de {{[tert-butoxycarbonyl-4 (indolyl-3)-5 pyrrolidinyl-1]-2 oxo-2 éthyl}-3 uréido}-3 benzoate de triméthylsilyl-2 éthyle-(2S,5R) (premier produit d'élution) et 0,26 g de {{[tert-butoxycarbonyl-4 (indolyl-3)-5 pyrrolidinyl-1]-2 oxo-2 éthyl}-3 uréido}-3 benzoate de triméthylsilyl-2 éthyle-(2S,5S) (deuxième produit d'élution).

L'(indolyl-3)-5 pyrrolidinecarboxylate-2 de tert-butyle-(2S,5RS) peut être préparé d'une manière analogue à celle décrite à l'exemple 48 §B mais à partir de 0,7 g de tert-butoxycarbonyl-1 (indolyl-3)-5 pyrrolidinecarboxylate-2 de tert-butyle-(2S,5R) et de 0,25 $cm^3$ d'iodotriméthylsilane. On obtient ainsi 0,50 g d'(indolyl-3)-5 pyrrolidinecarboxylate-5 de tert-butyle-(2S,5RS) sous forme d'une huile utilisée telle quelle dans les synthèses ultérieures.

Le tert-butoxycarbonyl-1 (indolyl-3)-5 pyrrolidinecarboxylate-2 de tert-butyle-(2S,5R) peut être préparé de la manière suivante : à une solution de 10,0 g de tert-butoxycarbonyl-1 méthoxy-5-pyrrolidinecarboxylate-2 de tert-butyle-(2S,5RS) et de 0,6 g d'acide paratoluènesulfonique dans 100 $cm^3$ de chlorure de méthylène anhydre, refroidie à une température voisine de 5°C, on ajoute une solution de 3,5 g d'indole dans 30 $cm^3$ de chlorure de méthylène. En fin d'addition, le milieu réactionnel est amené à une température voisine de 25°C, agité 2 heures à cette température puis hydrolysé par addition de 40 $cm^3$ d'une solution aqueuse saturée d'hydrogénocarbonate de sodium. La phase organique est décantée et la phase aqueuse est extraite par 2 fois 50 $cm^3$ de chlorure de méthylène. Les phases organiques sont rassemblées, séchées sur sulfate de magnésium et concentrées à sec sous pression réduite à 30°C. Le résidu obtenu est purifié par chromatographie sur silice [éluant : acétate d'éthyle-cyclohexane (10/90 en volumes)]. Les fractions contenant le produit attendu sont réunies et concentrées à sec sous pression réduite à 35°C. On obtient ainsi, après battage dans l'oxyde de diisopropyle 1,2 g de tert-butoxycarbonyl-1 (indolyl-3)-5 pyrrolidinecarboxylate-2 de tert-butyle-(2S,5R) (premier produit d'élution) fondant à 214°C et 0,8 g de tert-butoxycarbonyl-1 (indolyl-3)-5 pyrrolidinecarboxylate-2 de tert-butyle-(2S,5S) (deuxième produit d'élution) fondant à 194°C.

**Exemple 72**

On opère d'une manière analogue à celle décrite à l'exemple 41, mais à partir de 0,25 g de {{[tert-butoxycarbonyl-2 (indolyl-3)-5 pyrrolidinyl-1]-2 oxo-2 éthyl}-3 uréido}-3 benzoate de triméthylsilyl-2 éthyle-(2S,5S) et de 1,5 $cm^3$ d'une solution 1M de fluorure de tétrabutylammonium. Le produit brut est dissous dans 25 $cm^3$ d'acétate d'éthyle et extrait par 2 fois 10 $cm^3$ d'une solution aqueuse d'hydroxyde de sodium 0,1N. Les phases aqueuses sont amenées à pH 2 par addition d'une solution aqueuse d'acide chlorhydrique 1N. Le produit précipité est séparé par filtration, lavé par 2 fois 5 $cm^3$ d'eau distillée et séché à l'air. On obtient ainsi 0,09 g d'acide {{[tert-butoxycarbonyl-2 (indolyl-3)-5 pyrrolidinyl-1]-2 oxo-2 éthyl}-3 uréido}-3 benzoïque-(2S,5S) sous forme d'un produit amorphe, $[\alpha]_D^{20}$= -42,9° ±1,5° (C = 0,503%; $CH_3OH$), [RMN du proton (300 MHz, DMSO $D_6$, d en ppm, J en Hz): 1,44 (s, 9H,-$C(CH_3)_3$); 1,7 à 2,4 (m, 4H, -$CH_2$-$CH_2$-); 3,59 et 3,99 (2dd, J= 18 et 6, 2H , N-CO-$CH_2$-N); 4,55 (d, J= 9 Hz, 1H, N-CH-COO-); 5,53 (d, J = 8, 1H, N-CH-Ar); 6,3 (t, J= 6,1 Hz,1H, -NH-CO-); 6,9 à 7,6 (m, 7H, aromatiques); 7,15 (bs, 1H, N-CH=); 7,94 (s large, 1H, en position 2 sur NH-Ph-COO); 8,95 (massif, 1H, Ph-NH-CO-); 11,02 (massif, 1H, -NH-), 12,75 (massif, 1H, COOH). Deux effets NOE ont été observés entre N-CH= de l'indole et les protons en position 2 et 5 de la pyrrolidine], [Spectre infra-rouge (KBr), bandes caractéristiques en $cm^{-1}$: 3385, 2975, 2930, 2700 à 2250 (bande large), 1730, 1690, 1640, 1560, 1490, 1460, 1395, 1370, 1155, 740, 685].

**Exemple 73**

On opère d'une manière analogue à celle décrite à l'exemple 38 mais à partir de 4,84 g d'[(imidazolyl-1 carboxamido)-2 acétyl]-3 phényl-2 thiazolidinecarboxylate-4 de tert-butyle-(2R,4R) et de 8,85 g d'(amino-3 phényl)-1 éthanesulfonate de tétra-n-butyl ammonium-(RS). Au produit brut obtenu, sous forme de sel de tétra-n-butyl-ammonium, dissous dans 20 $cm^3$ d'acétone on ajoute une solution de 2,5 g de nonafluorobutanesulfonate de potassium dans 15 $cm^3$ d'acétone. Après 2 heures d'agitation à une température voisine de 25°C on ajoute 220 $cm^3$ d'oxyde de diisopropyle. Le produit insoluble est séparé par filtration, lavé par 2 fois 3 $cm^3$ d'un mélange d'acétone et d'oxyde de diisopropyle (30-70 en volumes) et purifié par chromatographie sur silice [éluant : acétate d'éthyle-méthanol (85/15 en volumes)]. Les fractions contenant le produit attendu sont réunies et concentrées à sec sous pression réduite à 40°C. On obtient ainsi, après battage dans l'acétonitrile, 0,40 g de {{[(tert-butoxycarbonyl-4 phényl-2 thiazolidinyl-3-(2R,4R)-2 oxo-2 éthyl]-3 uréido}-3 phényl}-1 éthanesulfonate de potassium-(RS) (mélange des formes A et B) fondant à 240°C sous forme d'un solide ($[\alpha]_D^{20}$= + 50,1° ±1,5° (C= 0,57%; $CH_3OH$), [RMN du proton (200 MHz, DMSO $D_6$ + quelques gouttes de $CD_3COOD$, d en ppm, J en Hz), 2 rotamères à température ambiante, coalescence des raies à 120°C, déplacements chimiques caractéristiques à 120°C : 1,51 (m, 12H,-$C(CH_3)_3$ et $CH_3$); 3,28 et 3,45 (2dd, J= 12,5 et 6, 2H, S-$CH_2$-); 3,7 (m,2H, -CH-et 1H de N-$COCH_2$-N); 4,05 (d, J = 17, 1H, l'autre H de N-$COCH_2$-N); 5,0 (t, J = 6, 1H, N-CH-COO); 6,4 (s, 1H, S-CH-N); 6,97 (d large, J = 8, 1H, en position 4 sur CO-NH-Ph); 7,08 (t, J = 8, 1H, en position 5 sur CO-NH-Ph); 7,2 à 7,5 (m, 5H, aromatiques); 7,64 (bd, J = 8, 2H, aromatiques)], [Spectre infra-rouge (KBr), bandes caractéristiques en $cm^{-1}$: 3370, 2970, 2920, 2870, 1725, 1655, 1610, 1590, 1555, 1490, 1450, 1365, 1205,

1150, 1025, 725, 695].

L'(amino-3 phényl)-1 éthanesulfonate de tétra-n-butyl ammonium-(RS) peut être préparé d'une manière analogue à celle décrite à l'exemple 41 §G mais à partir de 22,4 g de (nitro-3 phényl)-1 éthanesulfonate de tétra-n-butyl ammonium-(RS) et de 0,8 g de palladium sur noir à 5%. On obtient ainsi 20,7 g d'(amino-3 phényl)-1 éthanesulfonate de tétra-n-butyl ammonium sous forme d'une huile utilisée telle quelle dans les synthèses ultérieures.

Le (nitro-3 phényl)-1 éthanesulfonate de tétra-n-butyl ammonium-(RS) peut être préparé de la manière suivante : à une solution de 9,45 g de sulfite de sodium dans 125 $cm^3$ d'eau distillée, on ajoute 11,5 g de (bromo-1 éthyl)-3 nitrobenzène-(5). Le milieu réactionnel est chauffé pendant 3 heures à une température voisine de 70°C, refroidi à une température voisine de 25°C, puis transvasé dans 1450 $cm^3$ d'une solution aqueuse de dihydrogénophosphate de potassium 0,5 M. On ajoute ensuite 17,8 g d'hydrogénosulfate de tétra-n-butyl ammonium et le mélange est extrait par 300 $cm^3$ de chlorure de méthylène. La phase organique est lavée successivement par 3 fois 300 $cm^3$ d'eau et 300 $cm^3$ d'une solution aqueuse saturée de chlorure de sodium, séchée sur sulfate de magnésium et concentrée à sec sous pression réduite à 40°C. On obtient ainsi, 22,4g de (nitro-3 phényl)-1 éthanesulfonate de tétra-n-butyl ammonium-(RS) sous forme d'une huile utilisée telle quelle dans les synthèses ultérieures.

Le (bromo-1 éthyl)-3 nitrobenzène-(RS) peut être préparé selon la méthode décrite par E. FELDER et coll., J. Med. Chem., 13, 559 (1970).

**Exemple 74**

On opère d'une manière analogue à celle décrite à l'exemple 38 mais à partir de 2,42 g d'[(imidazolyl-1 carboxamido)-2 acétyl]-3 phényl-2 thiazolidinecarboxylate-4 de tert-butyle-(2R,4R) et de 1,4 g d'(amino-3 phényl)-1 éthanol. Le produit brut est purifié par chromatographie sur silice [éluant : acétate d'éthyle-cyclohexane (75/25 en volumes)]. Les fractions contenant le produit attendu sont réunies et concentrées à sec sous pression réduite. On obtient ainsi, après recristallisation dans l'acétonitrile, 0,62 g d'{{[(hydroxy-1 éthyl)-3 phényl-(RS)]-3 uréido}-2 acétyl}-3 phényl-2 thiazolidinecarboxylate-4 de tert-butyle-(2R,4R) (mélange des formes A et B) fondant à 160°C, ($[\alpha]_D^{20}$ = + 60,7° ±1,3° (C = 0,51 %; DMF), [RMN du proton (200 MHz, plus quelques gouttes de $CD_3COOD$, d en ppm, J en Hz), 2 rotamères à température ambiante, coalescence des raies à 120°C, déplacements chimiques caractéristiques à 120°C : 1,35 (d, J = 6,5, 3H,-$CH_3$); 1,5 (s, 9H,-$C(CH_3)_3$); 3,29 et 3,45 (2dd, J= 12,5 et 6,2H, S-$CH_2$-); 3,72 (bd, 1H, de N-$COCH_2$-N); 4,06 (d, J = 17, 1H, l'autre H de N-$COCH_2$-N); 4,68 (q, J= 6,5, 1H, -CH-O-); 5,0 (t, J = 6, 1H, N-CH-COO); 6,4 (s,1H, S-CH-N); 6,93 (d large, J = 8, 1H, en position 4 sur CO-NH-Ph); 7,14 (t, J = 8, 1H, en position 5 sur CO-NH-Ph); 7,22 (dd, J = 3 et 2,5, 1H, en position 2 sur CO-NH-Ph); 7,2 à 7,5 (m, 4H, aromatiques); 7,64 (bd, J = 8, 2H, aromatiques)], [Spectre infra-rouge (KBr), bandes caractéristiques en $cm^{-1}$: 3350, 2975, 2930, 1740, 1660, 1615, 1590, 1560, 1480, 1445, 1365, 1155, 1060, 730, 705, 700].

**Exemple 75**

On opère d'une manière analogue à celle décrite à l'exemple 34 mais à partir de 2,0 g d'(amino-2 acétyl)-3 phényl-2 thiazolidinecarboxylate-4 de tert-butyle-(2R,4R) et de 1,6 g d'isocyanate de chloro-4 trifluorométhyl-3 phényle. Le produit brut obtenu est purifié par chromatographie sur silice [éluant : acétate d'éthyle-cyclohexane (30/70 en volumes )]. Les fractions contenant le produit attendu sont réunies et concentrées à sec sous pression réduite à 40°C. On obtient ainsi, après battage dans 20 $cm^3$ d'hexane, 0,7 g de {[(chloro-4 trifluorométhyl-3 phényl)-3 uréido]-2 acétyl}-3 phényl-2 thiazolidinecarboxylate-4 de tert-butyle-(2R,4R) sous forme d'une poudre amorphe, ($[\alpha]_D^{20}$ = + 58,2° ±1,3° (C = 0,49%; $CH_3OH$), [RMN du proton (250 MHz, DMSO $D_6$, d en ppm, J en Hz), 2 rotamères à température ambiante, coalescence des raies à 130°C, déplacements chimiques caractéristiques à 130°C: 1,53 (s, 9H,-$C(CH_3)_3$); 3,29 et 3,47 (2dd, J= 12,5 et 6,5, 2H, S-$CH_2$-); 3,73 (massif, 1H, de N-$COCH_2$-N); 4,10 (dd, J = 17,5 et 4, 1H, l'autre H de N-$COCH_2$-N); 5,0 (massif, 1H, N-CH-COO); 6,33 (massif, 1H, -NHCO-); 6,4 (s,1H, S-CH-N); 7,2 à 7,8 (m,7H, aromatiques); 7,97 (bs, 1H, en position 2 sur CO-NH-Ph); 8,98 (massif, 1H, -CO-NH-Ph)], [Spectre infra-rouge (KBr), bandes caractéristiques en $cm^{-1}$: 3375, 2980, 2930, 2875, 1740, 1640, 1595, 1550, 1485, 1455, 1370, 1325, 1175, 1150, 895, 730, 695].

**Exemple 76**

On opère d'une manière analogue à celle décrite à l'exemple 41, mais à partir de 1,37 g de {{[tert-butoxycarbonyl-4 (méthyl-2 phényl)-2 thiazolidinyl-3]-2 oxo-2 éthyl}-3 uréido}-3 benzoate de triméthylsilyl-2 éthyle-(2R,4R) et de 4,6 $cm^3$ d'une solution de fluorure de tétrabutylammonium 1M. Le produit brut est dissous

dans 25 $cm^3$ d'une solution saturée d'hydrogénocarbonate de sodium et lavé par 25 $cm^3$ d'acétate d'éthyle. La phase aqueuse est amenée à pH 2 par addition d'une solution aqueuse d'acide chlorhydrique 4N. Le produit précipité est séparé par filtration, lavé à l'eau distillée jusqu'à neutralité des eaux de lavage et séché à l'air. On obtient ainsi 0,89 g d'acide {{[tert-butoxycarbonyl-4 (méthyl-2 phényl)-2 thiazolidinyl-3]-2 oxo-1 éthyl}-2 uréido}-3 benzoïque-(2R,4R) sous forme d'un solide fondant à 152°C, ($[\alpha]_D^{20}$= + 70,3° ±1,9° (C = 0,53%; DMF), [RMN du proton (200 MHz, DMSO $D_6$ plus quelques gouttes de $CD_3COOD$, d en ppm, J en Hz), 2 rotamères à température ambiante, coalescence des raies à 110°C, déplacements chimiques caractéristiques à 110°C : 1,56 (s, 9H,-$C(CH_3)_3$); 2,42 (s, 3H, Ar-$CH_3$); 3,28 et 3,48 (2dd, J= 12 et 6,5, 2H, S-$CH_2$-); 3,65 (massif, 1H, de N-$COCH_2$-N); 4,05 (d, J = 17,5, 1H, l'autre H de N-$COCH_2$-N); 4,98 (t, J = 6,5, 1H, N-CH-COO); 6,24 (massif, 1H, -NH-CO-); 6,46 (s,1H, S-CH-N); 7,10 à 7,65 (m,5H, aromatiques); 7,33 (t, J = 8, 1H, en position 5 sur CO-NH-Ph); 7,94 (massif,1H, en position 6 sur S-CH-Ph-); 8,0 (dd, J = 2 et 3, 1H, en position 2 sur CO-NH-Ph); 8,67 (massif, 1H, -CO-NH-Ph)], [Spectre infra-rouge (KBr), bandes caractéristiques en $cm^{-1}$: 3380, 2980, 2935, 2700 à 2250 (bande large), 1715 à 1695 (bande large), 1655, 1610, 1595, 1560, 1490, 1370, 1155, 760, 745, 685].

Le {{[tert-butoxycarbonyl-4 (méthyl-2 phényl)-2 thiazolidinyl-3]-2 oxo-2 éthyl}-3 uréido}-3 benzoate de triméthylsilyl-2 éthyle-(2R,4R) peut être préparé d'une manière analogue à celle décrite à l'exemple 41 §A mais à partir de 2,1 g de (méthyl-2 phényl)-2 thiazolidinecarboxylate-4 de tert-butyle-(2RS,4R), de 2,5 g d'acide {[(triméthylsilyl-2 éthoxycarbonyl)-3 phényl]-3 uréido}-2 acétique et de 1,6 g de N,N'-dicyclohexylcarbodiimide. Le produit brut est purifié par chromatographie sur silice [éluant : acétate d'éthyle-cyclohexane (40/60 en volumes)]. Les fractions contenant le produit attendu sont réunies et concentrées à sec sous pression réduite à 40°C. On obtient ainsi 1,7 g de {{[tert-butoxycarbonyl-4 (méthyl-2 phényl)-2 thiazolidinyl-3]-2 oxo-1 éthyl}-2 uréido}-3 benzoate de triméthylsilyl-2 éthyle-(2R,4R) sous forme d'un solide amorphe utilisé tel quel dans les synthèses ultérieures.

Le (méthyl-2 phényl)-2 thiazolidinecarboxylate-4 de tert-butyle-(2RS,4R) peut être préparé d'une manière analogue à celle décrite à l'exemple 34 §C mais à partir de 10,0 g d'acide (méthyl-2 phényl)-2 thiazolidinecarboxylique-4-(2RS,4R) en solution dans 130 $cm^3$ de chloroforme, de 3,0 $cm^3$ d'acide sulfurique concentré et d'un excès d'isobutène. Le produit brut obtenu est purifié par chromatographie sur silice [éluant : acétate d'éthyle-cyclohexane (10/90 en volumes)]. Les fractions contenant le produit attendu sont réunies et concentrées à sec sous pression réduite à 40°C. On obtient ainsi 3,8 g de (méthyl-2 phényl)-2 thiazolidinecarboxylate-4 de tert-butyle-(2RS,4R) sous forme d'une huile, mélange des isomères (2R,4R) et (2S,4R), utilisée telle quelle dans les synthèses ultérieures.

L'acide (méthyl-2 phényl)-2 thiazolidinecarboxylique-4-(2RS,4R) peut être préparé comme à l'exemple 34 §D mais à partir de 12,5 g de L-cystéine et de 12,0 $cm^3$ de méthyl-2 benzaldéhyde. On obtient ainsi 18,4 g d'acide (méthyl-2 phényl)-2 thiazolidinecarboxylique-4-(2RS,4R) fondant à 167°C, utilisé tel quel dans les synthèses ultérieures.

**Exemple 77**

A une solution de 0,95 g de {{[tert-butoxycarbonyl-4 (fluoro-2 phényl)-2 thiazolidinyl-3]-2 oxo-2 éthyl}-3 uréido}-3 phénylacétate de méthyle-(2R,4R) dans 6 $cm^3$ d'un mélange eau-méthanol (30/70 en volumes) on ajoute, à une température voisine de 25°C, 0,12 g d'hydroxyde de potassium. Le mélange réactionnel est agité pendant 3 heures à une température voisine de 25°C, puis concentré de moitié environ sous pression réduite. La solution obtenue est diluée avec 40 $cm^3$ d'eau, lavée par 2 fois 30 $cm^3$ d'éther éthylique, puis est amenée à pH 2 par addition de 2,1 $cm^3$ d'une solution aqueuse d'acide sulfurique 1N. Le produit insoluble est séparé par filtration et purifié par chromatographie sur silice [éluant : acétate d'éthyle-méthanol (90/10 en volumes)]. Les fractions contenant le produit attendu sont réunies et concentrées à sec sous pression réduite à 40°C. Le produit amorphe ainsi obtenu (0,26 g) est dissous dans une solution aqueuse d'hydroxyde de sodium 1N. Cette solution est filtrée puis acidifiée par addition d'une solution aqueuse d'acide sulfurique 1N. Le produit ainsi précipité est séparé par filtration, lavé par 3 fois 10 $cm^3$ d'eau puis séché à l'air. On obtient ainsi 0,26 g d'acide {{[tert-butoxycarbonyl-4 (fluoro-2 phényl)-2 thiazolidinyl-3]-2 oxo-2 éthyl}-3 uréido}-3 phénylacétique-(2R,4R) fondant à 110°C, ($[\alpha]_D^{20}$= + 51,1° ±1,6° (C = 0,528%; DMF), [RMN du proton (200 MHz, DMSO D6 plus quelques gouttes de $CD_3COOD$, d en ppm, J en Hz), 2 rotamères à température ambiante, coalescence des raies à 120°C, déplacements chimiques caractéristiques à 120°C : 1,55 (s, 9H,-$C(CH_3)_3$); 3,34 et 3,52 (2dd, J= 12,5 et 6,5, 2H, S-$CH_2$-); 3,51 (s, 2H, $ArCH_2COO$-); 3,8 et 4,07 (2d, J = 17,5, 2H, N-$COCH_2$-N); 5,02 (t, J = 6,5, 1H, N-CH-COO); 6,55 (s,1H, S-CH-N); 6,85 (bd, J = 8, 1H, en position 4 sur CO-NH-Ph); 7,05 à 7,4 (m,6H, aromatiques); 7,93 (bt, J = 8,5, 1H, en position 6 sur S-CH-Ph-)], [Spectre infra-rouge (KBr), bandes caractéristiques en $cm^{-1}$: 3380, 2980, 2930, 2700 à 2250 (bande large), 1735, 1650, 1615, 1595, 1560, 1490, 1460, 1370, 1230, 1155, 760, 705].

Le {{[tert-butoxycarbonyl-4 (fluoro-2 phényl)-2 thiazolidinyl-3]-2 oxo-2 éthyl}-3 uréido}-3 phénylacétate de méthyle-(2R,4R) peut être préparé d'une manière analogue à celle décrite à l'exemple 41 §A mais à partir de 1,46 g de (fluoro-2 phényl)-2 thiazolidinecarboxylate-4 de tert-butyle-(2RS,4R), de 1,37 g d'acide {[(méthoxycarbonylméthyl)-3 phényl]-3 uréido}-2 acétique et de 1,15 g de N,N'-dicyclohexylcarbodiimide. Le produit brut est purifié par chromatographie sur silice [éluant : acétate d'éthyle-cyclohexane (50/50 en volumes)]. Les fractions contenant le produit attendu sont réunies et concentrées à sec sous pression réduite à 40°C. On obtient ainsi 0,95 g de {{[tert-butoxycarbonyl-4 (fluoro-2 phényl)-2 thiazolidinyl-3]-2 oxo-2 éthyl}-3 uréido}-3 phénylacétate de méthyle-(2R,4R) sous forme d'un solide amorphe utilisé tel quel dans les synthèses ultérieures.

**Exemple 78**

On opère d'une manière analogue à celle décrite à l'exemple 77, mais à partir de 0,76 g de {{[tert-butoxycarbonyl-4 (méthoxy-2 phényl)-2 thiazolidinyl-3]-2 oxo-2 éthyl}-3 uréido}-3 phénylacétate de méthyle-(2R,4R) dans 6 $cm^3$ d'un mélange eau-méthanol (30/70 en volumes) et de 0,09 g d'hydroxyde de potassium. Le produit brut (0,45g) est dissous dans 7,5 $cm^3$ d'une solution aqueuse d'hydroxyde de sodium 0,1 N. La solution ainsi obtenue est lavée par 2 fois 10 $cm^3$ d'éther diéthylique, amenée à pH 2 par addition d'une solution aqueuse d'acide sulfurique 1N puis extraite par 2 fois 20 cm3 d'acétate d'éthyle. Les phases organiques sont réunies, séchées sur sulfate de magnésium et concentrées à sec sous pression réduite à 40°C. On obtient ainsi, après battage dans l'oxyde de diisopropyle, 0,25 g d'acide {{[tert-butoxycarbonyl-4 (méthoxy-2 phényl)-2 thiazolidinyl-3]-2 oxo-2 éthyl}-3 uréido}-3 phénylacétique-(2R,4R) sous forme d'un produit amorphe, ($[\alpha]_D^{20}$= + 6,4° ±1,0° (C = 0,466%; $CHCl_3$), [RMN du proton (200 MHz, DMSO $D_6$ plus quelques gouttes de $CD_3COOD$, d en ppm, J en Hz), 2 rotamères à température ambiante, coalescence des raies à 120°C, déplacements chimiques caractéristiques à 120°C : 1,55 (s, 9H,-C($CH_3$)$_3$); 3,22 et 3,45 (2dd, J= 12,5 et 7, 2H, S-$CH_2$-); 3,51 (s, 2H, Ar$CH_2$COO-); 3,65 (bd, 1H, de N-CO$CH_2$-N); 3,93 (s, 3H, -O$CH_3$); 4,05 (d, J = 17,5, 1H, l'autre H de N-CO$CH_2$-N); 4,91 (t, J = 7, 1H, N-CH-COO); 6,5 (s,1H, S-CH-N); 6,85 (bd, J = 8, 1H, en position 4 sur CO-NH-Ph); 6,9 à 7,4 (m,6H, aromatiques); 7,9 (bd, J = 8, 1H, en position 6 sur S-CH-Ph-)], [Spectre infra-rouge (KBr), bandes caractéristiques en $cm^{-1}$: 3390, 2975, 2930, 2840, 2700 à 2250 (bande large), 1735, 1645, 1610, 1600, 1595, 1560, 1495, 1370, 1245, 1155, 1025, 760, 705].

Le {{[tert-butoxycarbonyl-4 (méthoxy-2 phényl)-2 thiazolidinyl-3]-2 oxo-2 éthyl)-3 uréido}-3 phénylacétate de méthyle-(2R,4R) peut être préparé d'une manière analogue à celle décrite à l'exemple 41 §A mais à partir de 1,9 g de (méthoxy-2 phényl)-2 thiazolidinecarboxylate-4 de tert-butyle-(2RS,4R), de 1,73 g d'acide {[(méthoxycarbonylméthyl-3 phényl]-3 uréido}-2 acétique et de 1,4 g de N,N'-dicyclohexylcarbodiimide. Le produit brut est purifié par chromatographie sur silice [éluant : acétate d'éthyle-cyclohexane (50/50 en volumes)]. Les fractions contenant le produit attendu sont réunies et concentrées à sec sous pression réduite à 40°C. On obtient ainsi 1,5 g de {{[tert-butoxycarbonyl-4 (méthoxy-2 phényl)-2 thiazolidinyl-3]-2 oxo-2 éthyl}-3 uréido}-3 phénylacétate de méthyle-(2R,4R) sous forme d'un solide blanc amorphe utilisé tel quel dans les synthèses ultérieures.

Le (méthoxy-2 phényl)-2 thiazolidinecarboxylate-4 de tert-butyle-(2RS,4R) peut être préparé d'une manière analogue à celle décrite à l'exemple 34 §C mais à partir de 3,5 g d'acide (méthoxy-2 phényl)-2 thiazolidinecarboxylique-4-(2RS,4R) en solution dans 50 $cm^3$ de chloroforme, de 1,0 $cm^3$ d'acide sulfurique concentré et d'un excès d'isobutène. Le produit brut obtenu est purifié par chromatographie sur silice [éluant : acétate d'éthyle-cyclohexane (30/70 en volumes)]. Les fractions contenant le produit attendu sont réunies et concentrées à sec sous pression réduite à 40°C. On obtient ainsi 2,0 g de (méthoxy-2 phényl)-2 thiazolidinecarboxylate-4 de tert-butyle-(2RS,4R) sous forme d'une huile, mélange des isomères (2R,4R) et (2S,4R), utilisée telle quelle dans les synthèses ultérieures.

L'acide (méthoxy-2 phényl)-2 thiazolidinecarboxylique-4-(2RS,4R) peut être préparé comme à l'exemple 34 §D mais à partir de 7,26 g de L-cystéine et de 8,9 g de méthoxy-2 benzaldéhyde. On obtient ainsi 3,7 g d'acide (méthoxy-2 phényl)-2 thiazolidinecarboxylique-4-(2RS,4R) fondant à 170°C, utilisé tel quel dans les synthèses ultérieures.

**Exemple 79**

On opère d'une manière analogue à celle décrite à l'exemple 77, mais à partir de 0,70 g de {{[tert-butoxycarbonyl-4 (fluoro-2 phényl)-2 thiazolidinyl-3-(2R,4R)]-2 oxo-2 éthyl}-3 uréido}-3 mandélate de méthyle-(RS) dans 7 $cm^3$ d'un mélange eau-méthanol (30/70 en volumes) et de 0,08 g d'hydroxyde de potassium. Le produit brut (0,45g) est dissous dans 8,5 $cm^3$ d'une solution aqueuse d'hydroxyde de sodium 0,1N. La solution ainsi obtenue est lavée par 2 fois 25 $cm^3$ d'acétate d'éthyle, filtrée et acidifiée à pH 2 par addition d'une solution aqueuse d'acide chlorhydrique 1N. Le produit précipité est séparé par filtration, lavé par 2 fois 10 $cm^3$ d'eau

et séché à l'air. On obtient ainsi, 0,15 g d'acide {{[tert-butoxycarbonyl-4 (fluoro-2 phényl)-2 thiazolidinyl-3-(2R,4R)]-2 oxo-2 éthyl}-3 uréido}-3 mandélique-(RS) sous forme d'un produit blanc fondant à 135°C, [RMN du proton (200 MHz, DMSO $D_6$ plus quelques gouttes de $CD_3COOD$, d en ppm, J en Hz), 2 rotamères à température ambiante, coalescence des raies à 110°C, déplacements chimiques caractéristiques à 100°C : 1,55 (s, 9H,-$C(CH_3)_3$); 3,32 et 3,53 (2dd, J= 12,5 et 6,5, 2H, S-$CH_2$-); 3,8 (bd, 1H, de N-$COCH_2$-N); 4,08 (d, J = 17,5, 1H, l'autre H de N-$COCH_2$-N); 5 (m et s, 2H en totalité respectivement N-CH-COO et Ar-CH-); 6,2 (massif, 1H, -NHCO-); 6,54 (s,1H, S-CH-N); 7,02 (bd, J = 7,5, 1H, en position 4 sur CO-NH-Ph); 7,1 à 7,5 (m, 6H, aromatiques); 7,94 (dd, J = 7,5 et 9, 1H, en position 6 sur S-CH-Ph-); 8,5 (massif, -CONH-Ar)], [Spectre infra-rouge (KBr), bandes caractéristiques en $cm^{-1}$: 3390, 2980, 2930, 2840, 2700 à 2250 (bande large), 1735, 1650, 1610, 1560, 1490, 1455, 1370, 1230, 1150, 1060, 790, 760, 700].

Le {{[tert-butoxycarbonyl-4 (fluoro-2 phényl)-2 thiazolidinyl-3-(2R,4R)]-2 oxo-2 éthyl}-3 uréido}-3 mandélate de méthyle-(2R,4R) peut être préparé d'une manière analogue à celle décrite à l'exemple 38 mais à partir de 3,2 g d'[(imidazolyl-1 carboxamido)-2 acétyl]-3 (fluoro-2 phényl)-2 thiazolidinecarboxylate-4 de tert-butyle-(2R,4R) et de 2,7 g d'amino-3 mandélate de méthyle-(RS). Le produit brut est purifié par deux chromatographies successives sur silice [éluant : chlorure de méthylène-méthanol (95/5 en volumes) pour la première chromatographie; éluant : acétate d'éthyle pour la deuxième chromatographie]. Les fractions contenant le produit attendu sont réunies et concentrées à sec sous pression réduite. On obtient ainsi 1,6 g de {{[tert-butoxycarbonyl-4 (fluoro-2 phényl)-2 thiazolidinyl-3-(2R,4R)]-2 oxo-2 éthyl}-3 uréido}-3 mandélate de méthyle-(RS) sous forme de meringue, utilisée telle quelle dans les synthèses ultérieures.

L'[(imidazolyl-1 carboxamido)-2 acétyl]-3 (fluoro-2 phényl)-2 thiazolidinecarboxylate-4 de tert-butyle-(2R,4R) peut être préparé de la manière suivante : à une solution de 7 g d'(amino-2 acétyl)-3 (fluoro-2 phényl)-2 thiazolidinecarboxylate-4 de tert-butyle-(2R,4R) dans 100 $cm^3$ de tétrahydrofuranne anhydre, on additionne lentement, à une température voisine de 25°C, une solution de 4,8 g de N,N'-diimidazole-carbonyle dans 50 $cm^3$ de tétrahydrofuranne anhydre. Le milieu réactionnel est agité 12 heures à cette température puis est concentré à sec sous pression réduite à 40°C. Le résidu obtenu est dissous dans 200 $cm^3$ d'acétate d'éthyle et lavé par 2 fois 50 $cm^3$ d'eau. La phase organique est séchée sur sulfate de magnésium et évaporée à sec sous pression réduite à 40°C. On obtient ainsi 8,7 g d'[(imidazolyl-1 carboxamido)-2 acétyl]-3 (fluoro-2 phényl)-2 thiazolidinecarboxylate-4 de tert-butyle-(2R,4R) sous forme d'une huile utilisée telle quelle dans les synthèses ultérieures.

L'(amino-2 acétyl)-3 (fluoro-2 phényl)-2 thiazolidinecarboxylate-4 de tert-butyle-(2R,4R) peut être préparé d'une manière analogue à celle décrite à l'exemple 34 §A, mais à partir de 15,0 g de (tert-butoxycarbonylamino-2 acétyl)-3 (fluoro-2 phényl)-2 thiazolidinecarboxylate-4 de tert-butyle-(2R,4R) et de 5,34 $cm^3$ d'iodotriméthylsilane. On obtient ainsi 10 g d'(amino-2 acétyl)-3 (fluoro-2 phényl)-2 thiazolidinecarboxylate-4 de tert-butyle-(2R,4R) sous forme d'une huile utilisée telle quelle dans les synthèses ultérieures.

Le (tert-butoxycarbonylamino-2 acétyl)-3 (fluoro-2 phényl)-2 thiazolidinecarboxylate-4 de tert-butyle-(2R,4R) peut être préparé d'une manière analogue à celle décrite à l'exemple 34 §B mais à partir de 25,0 g de (fluoro-2 phényl)-2 thiazolidinecarboxylate-4 de tert-butyle-(2RS,4R), de 15,5 g d'acide tert-butoxycarbonylamino-2 acétique et de 18,2 g de N,N'-dicyclohexylcarbodiimide. Le produit brut est purifié par chromatographie sur silice [éluant : acétate d'éthyle-cyclohexane (30/70 en volumes)]. Les fractions contenant le produit attendu sont réunies et concentrées à sec sous pression réduite. On obtient ainsi 25,0 g de (tert-butoxycarbonylamino-2 acétyl)-3 (fluoro-2 phényl)-2 thiazolidinecarboxylate-4 de tert-butyle-(2R,4R) sous forme d'une huile utilisée telle quelle dans les synthèses ultérieures.

L'amino-3 mandélate de méthyle-(RS) peut être préparé de la manière suivante : à une solution de 15 g de nitro-3 mandélate de méthyle-(RS) dans 150 $cm^3$ d'éthanol, on ajoute 0,5 g de palladium sur charbon à 5%. La suspension est agitée pendant 2 heures à une température voisine de 25°C sous atmosphère d'hydrogène (100kPa). Le catalyseur est ensuite séparé par filtration et le filtrat est concentré à sec sous pression réduite à 40°C. On obtient ainsi 13,1 g d'amino-3 mandélate de méthyle-(RS) sous forme d'une huile utilisée telle quelle dans les synthèses ultérieures.

Le nitro-3 mandélate de méthyle-(RS) peut être préparé selon la méthode décrite par L.S. FOSDICK et J.C. CALANDRA, J. Am. Chem. Soc., 63, 1101 (1941).

**Exemple 80**

On opère d'une manière analogue à celle décrite à l'exemple 38 mais à partir de 2,17 g d'[(imidazolyl-1 carboxamido)-2 acétyl]-3 (fluoro-2 phényl)-2 thiazolidinecarboxylate-4 de tert-butyle-(2R,4R) et de 1,37 g d'(amino-3 phényl)-2 éthanol. Le produit brut est purifié par deux chromatographies successives sur silice [éluant : chlorure de méthylène-méthanol (95/5 en volumes) pour la première chromatographie; éluant : acétate d'éthyle pour la deuxième chromatographie]. Les fractions contenant le produit attendu sont réunies et concentrées à sec sous pression réduite. On obtient ainsi, 0,5 g d'{{[(hydroxy-2 éthyl)-3 phényl]-3 uréido}-2 acétyl}-3 (fluoro-2 phényl)-2 thiazolidinecarboxylate-4 de tert-butyle-(2R,4R) sous forme d'un produit amorphe, ($[\alpha]_D^{20}$ = + 69,0° ±2,0° (C = 0,48%; $CHCl_3$), [RMN du proton (200 MHz, DMSO $D_6$ plus quelques gouttes de $CD_3COOD$, d en ppm, J en Hz), 2 rotamères à température ambiante, coalescence des raies à 110°C, déplacements chimiques caractéristiques à 110°C : 1,55 (s, 9H,-$C(CH_3)_3$); 2,72 (t, J = 7, 2H, $ArCH_2$-); 3,31 et 3,52 (2dd, J = 12 et 6, 2H, S-$CH_2$); 3,67 (t, J = 7, 2H, -$CH_2O$-); 3,8 (bd, 1H, de N-$COCH_2$-N); 4,1 (d, J = 17, 1H, l'autre H de N-$COCH_2$-N); 5,01 (t, J = 6, 1H, N-CH-COO); 6,2 (massif, 1H, -NHCO-); 6,54 (s, 1H, S-CH-N); 6,81 (bd, J = 7,5, 1H, en position 4 sur CO-NH-Ph-); 7,05 à 7,45 (m,6H, aromatiques); 7,93 (bt, J = 8,5, 1H, en position 6 sur S-CH-Ph); 8,36 (massif, 1H, ArNHCO-)],[Spectre infra-rouge (KBr), bandes caractéristiques en $cm^{-1}$: 3380, 2975, 2930, 2875, 1740, 1655, 1610, 1590, 1560, 1490, 1460, 1370, 1230, 1150, 1050, 760, 700].

L'(amino-3 phényl)-2 éthanol peut être préparé selon la méthode décrite par B. CARNMALM et coll., Acta Pharm. Suecica, 11, 33 (1974).

**Exemple 81**

On opère d'une manière analogue à celle décrite à l'exemple 41, mais à partir de 1,37 g de {{[tert-butoxycarbonyl-4 (chloro-2 fluoro-6 phényl)-2 thiazolidinyl-3]-2 oxo-2 éthyl}-3 uréido}-3 benzoate de triméthylsilyl-2 éthyle-(2R,4R) et de 4,3 $cm^3$ d'une solution de fluorure de tétrabutylammonium 1M. Le produit brut est dissous dans 10 $cm^3$ d'une solution aqueuse d'hydroxyde de sodium 0,5 N et lavé par 2 fois 20 $cm^3$ d'acétate d'éthyle. La phase aqueuse est amenée à pH 2 par addition d'une solution aqueuse d'acide sulfurique 1N. Le produit précipité est séparé par filtration, lavé par 2 fois 5 $cm^3$ d'eau distillée et séché à l'air. On obtient ainsi 0,12g d'acide {{[tert-butoxycarbonyl-4 (chloro-2 fluoro-6 phényl)-2 thiazolidinyl-3]-2 oxo-2 éthyl}-3 uréido}-3 benzoïque-(2R,4R) fondant à 148°C, ($[\alpha]_D^{20}$= + 3,1° ±0,8° (C = 0,518%; DMF), [RMN du proton (250 MHz, DMSO $D_6$, d en ppm, J en Hz), 2 rotamères à température ambiante, coalescence des raies à 120°C, déplacements chimiques caractéristiques à 120°C: 1,5 (s, 9H,-$C(CH_3)_3$); 3,48 (dd, J = 12,5 et 6,5, 1H, de S-$CH_2$); 3,62 (dd, J= 12,5 et 5, 1H, l'autre H de S-$CH_2$-); 3,96 (dd, J = 17,5 et 5, 1H, de N-$COCH_2$-N); 4,18 (dd, J = 17,5 et 5,5, 1H, l'autre H de N-$COCH_2$-N); 5,11 (dd, J = 6,5 et 5, N-CH-COO); 6,24 (massif, 1H, -NHCO-); 6,01 (s,1H, S-CH-N); 7,05 à 7,65 (m,6H, aromatiques); 7,98 (bs, 1H, en position 2 sur CO-NH-Ph-); 8,66 (massif, 1H, Ar-NHCO-)], [Spectre infra-rouge (KBr), bandes caractéristiques en $cm^{-1}$: 3380, 2980, 2930, 2700 à 2250 (bande large), 1715, 1695, 1655, 1605, 1590, 1560, 1490, 1460, 1370, 1150, 785, 760, 680].

Le {{[tert-butoxycarbonyl-4 (chloro-2 fluoro-6 phényl)-2 thiazolidinyl-3]-2 oxo-2 éthyl}-3 uréido}-3 benzoate de triméthylsilyl-2 éthyle-(2R,4R) peut être préparé d'une manière analogue à celle décrite à l'exemple 34, mais à partir de 1,40 g d'(amino-2 acétyl)-3 (chloro-2 fluoro-6 phényl)-2 thiazolidinecarboxylate-4 de tert-butyle-(2R,4R) et de 1,4 g d'isocyanato-3 benzoate de triméthylsilyl-2 éthyle. Le produit brut obtenu est purifié par chromatographie sur silice [éluant : acétate d'éthyle-cyclohexane (30/70 en volumes)]. Les fractions contenant le produit attendu sont réunies et concentrées à sec sous pression réduite à 40°C. On obtient ainsi 1,44 g de {{[tert-butoxycarbonyl-4 (chloro-2 fluoro-6 phényl)-2 thiazolidinyl-3]-2 oxo-2 éthyl}-3 uréido}-3 benzoate de triméthylsilyl-2 éthyle-(2R,4R) sous forme d'une meringue utilisée telle quelle dans les synthèses ultérieures.

L'(amino-2 acétyl)-3 (chloro-2 fluoro-6 phényl)-2 thiazolidinecarboxylate-4 de tert-butyle-(2R,4R) peut être préparé d'une manière analogue à celle décrite à l'exemple 34 §A mais à partir de 1,86 g (tert-butoxycarbonylamino-2 acétyl)-3 (chloro-2 fluoro-6 phényl)-2 thiazolidinecarboxylate-4 de tert-butyle-(2R,4R) et de 0,67 $cm^3$ de iodotriméthylsilane. On obtient ainsi 1,4 g d'(amino-2 acétyl)-3 (chloro-2 fluoro-6 phényl)-2 thiazolidinecarboxylate-4 de tert-butyle-(2R,4R) sous forme d'une pâte utilisée telle quelle dans les synthèses ultérieures.

Le (tert-butoxycarbonylamino-2 acétyl)-3 (chloro-2 fluoro-6 phényl)-2 thiazolidinecarboxylate-4 de tert-butyle-(2R,4R) peut être préparé d'une manière analogue à celle décrite à l'exemple 34 §B mais à partir de 2,2 g de (chloro-2 fluoro-6 phényl)-2 thiazolidinecarboxylate-4 de tert-butyle-(2RS,4R), de 1,22 g d'acide tert-butoxycarbonylamino-2 acétique et de 1,45 g de N,N'-dicyclohexylcarbodiimide. Le produit brut est purifié par chromatographie sur silice [éluant : chlorure de méthylène-méthanol (98/2 en volumes)]. Les fractions contenant le produit attendu sont réunies et concentrées à sec sous pression réduite. On obtient ainsi 1,56 g de (tert-butoxycarbonylamino-2 acétyl)-3 (chloro-2 fluoro-6 phényl)-2 thiazolidinecarboxylate-4 de tert-butyle-(2R,4R) sous forme d'un solide utilisé tel quel dans les synthèses ultérieures.

Le (chloro-2 fluoro-6 phényl)-2 thiazolidinecarboxylate-4 de tert-butyle-(2RS,4R) peut être préparé d'une manière analogue à celle décrite à l'exemple 34 §C mais à partir de 6,1 g d'acide (chloro-2 fluoro-6 phényl)-2 thiazolidinecarboxylique-4-(2RS,4R) en solution dans 60 $cm^3$ de chloroforme, de 1,4 $cm^3$ d'acide sulfurique concentré et d'un excès d'isobutène. Le produit brut obtenu est purifié par chromatographie sur silice [éluant : acétate d'éthyle-cyclohexane (25/75 en volumes)]. Les fractions contenant le produit attendu sont réunies et concentrées à sec sous pression réduite à 40°C. On obtient ainsi 4,4 g de (chloro-2 fluoro-6 phényl)-2 thiazolidinecarboxylate-4 de tert-butyle-(2RS,4R) sous forme d'une huile, utilisée telle quelle dans les synthèses ultérieures.

L'acide (chloro-2 fluoro-6 phényl)-2 thiazolidinecarboxylique-4-(2RS,4R) peut être préparé comme à l'exemple 34 §D mais à partir de 11,4 g de L-cystéine et de 16,7 g de chloro-2 fluoro-6 benzaldéhyde. On obtient ainsi 12,3 g d'acide (chloro-2 fluoro-6 phényl)-2 thiazolidinecarboxylique-4-(2RS,4R) fondant à 148°C, utilisé tel quel dans les synthèses ultérieures.

L'isocyanato-3 benzoate de triméthylsilyl-2 éthyle peut être préparé comme à l'exemple 21 mais à partir de 2,37 g d'amino-3 benzoate de triméthylsilyl-2 éthyle, de 1,32 $cm^3$ de chloroformiate de trichlorométhyle et de 0,21 g de charbon. On obtient ainsi 2,6 g d'isocyanato-3 benzoate de triméthylsilyl-2 éthyle sous forme d'une huile utilisée telle quelle dans les synthèses ultérieures.

**Exemple 82**

On opère d'une manière analogue à celle décrite à l'exemple 77, mais à partir de 0,38 g de {{[tert-butoxycarbonyl-4 (fluoro-2 phényl)-2 thiazolidinyl-3-(2R,4R)]-1 oxo-1 propyl-2-(2S)}-3 uréido}-3 phénylacétate de méthyle dans 6 $cm^3$ d'un mélange eau-méthanol (30/70 en volumes) et de 0,05 g d'hydroxyde de potassium. Le produit brut est dissous dans 3,5 $cm^3$ d'une solution aqueuse d'hydroxyde de sodium 0,1N. La solution ainsi obtenue est lavée par 2 fois 10 $cm^3$ d'acétate d'éthyle, filtrée et amenée à pH 2 par addition d'une solution aqueuse d'acide sulfurique 1N. Le produit précipité est séparé par filtration, lavé par 2 fois 5 $cm^3$ d'eau et séché à l'air. On obtient ainsi, 0,13 g d'acide {{[tert-butoxycarbonyl-4 (fluoro-2 phényl)-2 thiazolidinyl-3-(2R,4R)]-1 oxo-1 propyl-2-(2S)}-3 uréido}-3 phénylacétique fondant à 110°C, $([\alpha]_D^{20}= + 103° \pm 4°$ (C = 0,244%; DMF), [RMN du proton (200 MHz, DMSO $D_6$, d en ppm, J en Hz), 2 rotamères à température ambiante, coalescence des raies à 120°C, déplacements chimiques caractéristiques à 120°C : 1,05 (d, J = 7, 3H, $-CH_3$); 1,55 (s, 9H,-$C(CH_3)_3$); 3,25 (dd, J = 12,5 et 7, 1H, de $S-CH_2$); 3,52 (dd, J= 12,5 et 6, 1H, l'autre H de $S-CH_2$-); 3,53 (s, 2H, $ArCH_2COO$); 4,4 (m, 1H, N-COCH-N); 4,86 (dd, J = 7 et 6, N-CH-COO); 6,3 (d, J = 8, 1H, -NHCO-); 6,83 (s,1H, S-CH-N); 6,89 (bd, J = 8, 1H, en position 4 sur CO-NH-Ph-); 7,10 à 7,5 (m,6H, aromatiques); 8,0 (bt, J = 8,5, 1H, en position 6 sur S-CH-Ph-); 8,29 (massif, 1H, ArNHCO-)], [Spectre infra-rouge (KBr), bandes caractéristiques en $cm^{-1}$: 3380, 2980, 2935, 2700 à 2250 (bande large), 1735, 1640, 1615, 1595, 1560, 1490, 1460, 1370, 1235, 1155, 760, 705].

Le {{[tert-butoxycarbonyl-4 (fluoro-2 phényl)-2 thiazolidinyl-3-(2R,4R)]-1 oxo-1 propyl-2-(2S)}-3 uréido}-3 phénylacétate de méthyle peut être préparé d'une manière analogue à celle décrite à l'exemple 34 mais à partir de 0,85 g d'(amino-2 propionyl-(2S))-3 (fluoro-2 phényl)-2 thiazolidinecarboxylate-4 de tert-butyle-(2R,4R) et de 0,53 g d'isocyanato-3 phénylacétate de méthyle. Le produit brut obtenu est purifié par chromatographie sur silice [éluant : acétate d'éthyle-cyclohexane (30/70 en volumes )]. Les fractions contenant le produit attendu sont réunies et concentrées à sec sous pression réduite à 40°C. On obtient ainsi 0,38 g de {{[tert-butoxycarbonyl-4 (fluoro-2 phényl)-2 thiazolidinyl-3-(2R,4R)]-1 oxo-1 propyl-2-(2S)}-3 uréido}-3 phénylacétate de méthyle sous forme d'une huile utilisée telle quelle dans les synthèses ultérieures.

L'(amino-2 propionyl-(2S))-3 (fluoro-2 phényl)-2 thiazolidinecarboxylate-4 de tert-butyle-(2R,4R) peut être préparé d'une manière analogue à celle décrite à l'exemple 34 §A mais à partir de 1,0 g (tert-butoxycarbonylamino-2 propionyl-(2S))-3 (fluoro-2 phényl)-2 thiazolidinecarboxylate-4 de tert-butyle-(2R,4R) et de 0,39 $cm^3$ d'iodotriméthylsilane. On obtient ainsi 0,85 g d'(amino-2 propionyl-(2S))-3 (fluoro-2 phényl)-2 thiazolidinecarboxylate-4 de tert-butyle- (2R,4R) sous forme d'une huile utilisée telle quelle dans les synthèses ultérieures.

Le (tert-butoxycarbonylamino-2 propionyl-(2S))-3 (fluoro-2 phényl)-2 thiazolidinecarboxylate-4 de tert-butyle-(2R,4R) peut être préparé d'une manière analogue à celle décrite à l'exemple 34 §B mais à partir de 2,0 g de (fluoro-2 phényl)-2 thiazolidinecarboxylate-4 de tert-butyle-(2RS,4R), de 1,36 g de N-tert-butoxycarbonyl-L-alanine et de 1,47 g de N,N'-dicyclohexylcarbodiimide. Le produit brut est purifié par chromatographie sur silice [éluant : acétate d'éthyle-cyclohexane (15/85 en volumes)]. Les fractions contenant le produit attendu sont réunies et concentrées à sec sous pression réduite. On obtient ainsi 1,0 g de (tert-butoxycarbonylamino-2 propionyl-(2S))-3 (fluoro-2 phényl)-2 thiazolidinecarboxylate-4 de tert-butyle-(2R,4R) sous forme d'une huile utilisée telle quelle dans les synthèses ultérieures.

**Exemple 83**

On opère d'une manière analogue à celle décrite à l'exemple 77, mais à partir de 0,48 g de {{[tert-butoxycarbonyl-4 (fluoro-2 phényl)-2 thiazolidinyl-3-(2R,4R)]-1 oxo-1 propyl-2-(2R)}-3 uréido}-3 phénylacétate de méthyle dans 7 $cm^3$ d'un mélange eau méthanol (30/70 en volumes) et de 0,06 g d'hydroxyde de potassium. Le produit obtenu après filtration sur silice [éluant acétate d'éthyle-méthanol (90/10 en volumes)] est dissous

dans 7,0 $cm^3$ d'une solution aqueuse d'hydroxyde de sodium 0,1N. La solution ainsi obtenue est filtrée et amenée à pH 2 par addition d'une solution aqueuse d'acide sulfurique 1N. Le produit précipité est séparé par filtration, lavé par 2 fois 5 $cm^3$ d'eau et séché à l'air. On obtient ainsi, 0,25 g d'acide {{[tert-butoxycarbonyl-4 (fluoro-2 phényl)-2 thiazolidinyl-3-(2R,4R)]-1 oxo-1 propyl-2}-3 uréido-(2R)}-3 phénylacétique fondant à 200°C, $[\alpha]_D^{20}$= + 83° ±2,0° (C = 0,48%; DMF), [RMN du proton (250 MHz, DMSO $D_6$, d en ppm, J en Hz), 2 rotamères à température ambiante, coalescence des raies à 120°C, déplacements chimiques caractéristiques à 120°C : 1,27 (d, J = 7,5, 3H, $-CH_3$); 1,54 (s, 9H,$-C(CH_3)_3$); 3,40 et 3,56 (2dd, J = 12,5 et 6,5, 2H, $S-CH_2-$); 3,50 (s, 2H, $-CH_2COO-$); 4,50 (m, 1H, N-COCH-N); 5,45 (massif, 1H, N-CH-COO); 6,3 (d, J = 8,5, 1H, -NHCO-); 6,50 (s,1H, S-CH-N); 6,9 (bd, J = 8, 1H, en position 4 sur CO-NH-Ph-); 7,0 à 7,45 (m,6H, aromatiques); 7,9 (bt, J = 8,5, 1H, en position 6 sur S-CH-Ph-); 8,3 (massif, 1H, ArNHCO-)], **[Spectre infra-rouge** (KBr), bandes caractéristiques en $cm^{-1}$: 3380, 2975, 2930, 2700 à 2250 (bande large), 1730, 1640, 1610, 1595, 1555, 1490, 1455, 1365, 1230, 1150, 755, 700].

Le {{[tert-butoxycarbonyl-4 (fluoro-2 phényl)-2 thiazolidinyl-3-(2R,4R)]-1 oxo-1 propyl-2-(2R)}-3 uréido}-3 phénylacétate de méthyle peut être préparé d'une manière analogue à celle décrite à l'exemple 34, mais à partir de 0,55 g d'(amino-2 propionyl-(2R))-3 (fluoro-2 phényl)-2 thiazolidinecarboxylate-4 de tert-butyle-(2R,4R) et de 0,37 g d'isocyanato-3 phénylacétate de méthyle. Le produit brut obtenu est purifié par chromatographie sur silice [éluant : acétate d'éthyle-cyclohexane (40/60 en volumes )]. Les fractions contenant le produit attendu sont réunies et concentrées à sec sous pression réduite à 40°C. On obtient ainsi 0,48 g de {{[tert-butoxycarbonyl-4 (fluoro-2 phényl)-2 thiazolidinyl-3-(2R,4R)]-1 oxo-1 propyl-2-(2R)}-3 uréido}-3 phénylacétate de méthyle sous forme d'un produit amorphe utilisé tel quel dans les synthèses ultérieures.

L'(amino-2 propionyl-(2R))-3 (fluoro-2 phényl)-2 thiazolidinecarboxylate-4 de tert-butyle-(2R,4R) peut être préparé d'une manière analogue à celle décrite à l'exemple 34 §A mais à partir de 0,8 g (tert-butoxycarbonylamino-2 propionyl-(2R))-3 (fluoro-2 phényl)-2 thiazolidinecarboxylate-4 de tert-butyle-(2R,4R) et de 0,30 $cm^3$ d'iodotriméthylsilane. On obtient ainsi 0,55 g d'(amino-2 propionyl-(2R))-3 (fluoro-2 phényl)-2 thiazolidinecarboxylate-4 de tert-butyle-(2R,4R) sous forme d'une huile utilisée telle quelle dans les synthèses ultérieures.

Le (tert-butoxycarbonylamino-2 propionyl- (2R))-3 (fluoro-2 phényl)-2 thiazolidinecarboxylate-4 de tert-butyle-(2R,4R) peut être préparé d'une manière analogue à celle décrite à l'exemple 34 §B mais à partir de 2,0 g de (fluoro-2 phényl)-2 thiazolidinecarboxylate-4 de tert-butyle-(2RS,4R), de 1,36 g de N-tert-butoxycarbonyl-D-alanine et de 1,47 g de N,N'-dicyclohexylcarbodiimide. Le produit brut est purifié par chromatographie sur silice [éluant : acétate d'éthyle-cyclohexane (15/85 en volumes)]. Les fractions contenant le produit attendu sont réunies et concentrées à sec sous pression réduite. On obtient ainsi 1,0 g de (tert-butoxycarbonylamino-2 propionyl-(2R))-3 (fluoro-2 phényl)-2 thiazolidinecarboxylate-4 de tert-butyle-(2R,4R) sous forme d'une huile utilisée telle quelle dans les synthèses ultérieures.

**Exemple 84**

A une solution de 0,35 g de {{{[tert-butoxycarbonyl-4 (fluoro-2 phényl)-2 thiazolidinyl-3-(2R,4R)]-2 oxo-2 éthyl}-3 uréido}-3 phényl}-2 propionate de benzyle (Forme B) dans 40 $cm^3$ d'acétate d'éthyle on ajoute 0,1 g de palladium sur charbon à 10%. La suspension est agitée pendant 48 h à une température voisine de 25° C sous atmosphère d'hydrogène (100 kPa). Le catalyseur est ensuite séparé par filtration et le filtrat est concentré à sec sous pression réduite à 40° C. Le résidu obtenu est dissous dans 10 $cm^3$ d'une solution aqueuse d'hydroxyde de sodium 0,1N et lavé par deux fois 10 $cm^3$d'oxyde de diéthyle. La phase aqueuse est amenée à pH 2 par addition d'une solution aqueuse d'acide sulfurique 1N. Le produit précipité est séparé par filtration, lavé par 2 fois 10 $cm^3$ d'eau et séché à l'air. On obtient ainsi, 0,12 g d'acide {{{[tert-butoxycarbonyl-4 (fluoro-2 phényl)-2 thiazolidinyl-3-(2R,4R)]-2 oxo-2 éthyl}-3 uréido}-3 phényl}-2 propionique . (forme B) fondant à 126 C.

$[\alpha]_D^{20}$= + 97 ±2 (C = 0,502%; DMF)

**RMN du proton** (200 MHz, DMSO $D_6$ plus quelques gouttes de $CD_3COOD$, d en ppm, J en Hz), 2 rotamères à température ambiante, coalescence des raies à 120 C, déplacements chimiques caractéristiques à 120 C : 1,4 (d, J = 7,5, 3H, $-CH_3$); 1,54 (s, 9H,$-C(CH_3)_3$); 3,32 (dd, J = 12 et 6,5, 1H, de $S-CH_2$); 3,52 (dd, J= 12 et 7, 1H,l'autre H de $S-CH_2-$); 3,62 (q, J = 7,5, 1H, Ar-CH-COO); 3,78 (bd, 1H, de $N-COCH_2-N$); 4,07 (d, J = 17, 1H, l'autre H de $N-COCH_2-N$); 5,0 (dd, J = 7 et 6,5, 1H, N-CH-COO); 6,17 (massif, 1H, -NHCO-); 6,53 (s,1H, S-CH-N); 6,86 (bd, J = 8, 1H, en position 4 sur CO-NH-Ph-); 7,10 à 7,5 (m, 6H, aromatiques); 7,92 (bt, J = 8,5, 1H, en position 6 sur S-CH-Ph-); 8,43 (massif, 1H, ArNHCO-).

**Spectre infra-rouge** (KBr), bandes caractéristiques en $cm^{-1}$: 3380, 2975, 2930, 2650 à 2250 (bande large), 1735, 1650, 1610, 1595, 1560, 1490, 1455, 1370, 1230, 1150, 760, 700.

A. Le {{{[tert-butoxycarbonyl-4 (fluoro-2 phényl)-2 thiazolidinyl-3-(2R,4R)]-2 oxo-2 éthyl}-3 uréido}-3 phényl}-2 propionate de benzyle (Forme B) peut être préparé d'une manière analogue à celle décrite à l'exemple **34** mais à partir de 1,02 g d'(amino-2 acétyl)-3 (fluoro-2 phényl)-2 thiazolidinecarboxylate-4 de tert-butyle-(2R,4R) et de 0,89 g d'(isocyanato-3 phényl)-2 propionate de benzyle (forme B). Le produit brut est purifié par chromatographie sur silice [éluant : acétate d'éthyle / cyclohexane (50/50 en volumes)].Les fractions contenant le produit attendu sont réunies et concentrées à sec sous pression réduite à 40° C. On obtient ainsi 1,2 g de {{{[tert-butoxycarbonyl-4 (fluoro-2 phényl)-2 thiazolidinyl-3-(2R,4R)]-2 oxo-2 éthyl}-3 uréido}-3 phényl}-2 propionate de benzyle (Forme B) sous forme d'une pâte jaune, utilisée telle quelle dans les synthèses ultérieures.

B. L'(isocyanato-3 phényl)-2 propionate de benzyle (forme B) peut être préparé comme à l'exemple **21** mais à partir de 2,85 g de (+)-(amino-3 phényl)-2 propionate de benzyle, de 1,48 $cm^3$ de chloroformiate de trichlorométhyle et de 0,24 g de charbon. On obtient ainsi 3,1 g d'(isocyanato-3 phényl)-2 propionate de benzyle (forme B) sous forme d'une huile orangée utilisée telle quelle dans les synthèses ultérieures.

C. L'(+)-(amino-3 phényl)-2 propionate de benzyle peut être préparé de la manière suivante: à un mélange de 8,0 g de (+)-(nitro-3 phényl)-2 propionate de benzyle dans 35 $cm^3$ de méthanol et 300 $cm^3$ d'eau, on ajoute 75 g de chlorure d'ammonium et 37,0 g de zinc en poudre. Le milieu réactionnel est chauffé à reflux pendant 1 h puis refroidi vers 0° C. Les sels insolubles sont séparés par filtration et le filtrat est extrait par 3 fois 200 $cm^3$ d'oxyde de diéthyle. Les phases organiques collectées sont lavées successivement par 100 $cm^3$ d'eau et par 100 $cm^3$ d'une solution aqueuse saturée de chlorure de sodium. On obtient ainsi, après séchage sur sulfate de magnésium et concentration à sec sous pression réduite à 40° C, 6,7 g de (+)-(amino-3 phényl)-2 propionate de benzyle sous forme d'une huile jaune utilisée telle quelle dans les synthèses ultérieures.

D. Le (+)-(nitro-3 phényl)-2 propionate de benzyle peut être préparé de la manière suivante: à un mélange contenant 9,75 g de (+)-acide (nitro-3 phényl)-2 propionique et 0,5 $cm^3$ de diméthylformamide dans 100 $cm^3$ de dichloro-1,2 éthane, on ajoute lentement 4,72 $cm^3$ de dichlorure d'oxalyle. Le milieu réactionnel est agité 3h à une température voisine de 25° C, puis on ajoute 5,4 g d'alcool benzylique. L'agitation est poursuivie 12 h à cette même température puis le mélange réactionnel est lavé successivement par 2 fois 200 $cm^3$ d'une solution aqueuse saturée d'hydrogénocarbonate de sodium, par 100 $cm^3$ d'eau et par 100 $cm^3$ d'une solution aqueuse saturée de chlorure de sodium. Les phases organiques collectées sont lavées successivement par 100 $cm^3$ d'eau et par 100 $cm^3$ d'une solution aqueuse saturée de chlorure de sodium. Les phases organiques collectées sont séchées sur sulfate de magnésium et concentrées à ses sous pression réduite à 40° C. Le résidu obtenu est purifié par chromatographie sur silice [éluant : acétate d'éthyle-cyclohexane (30/70 en volumes)]. Les fractions contenant le produit attendu sont réunies et concentrées à sec sous pression réduite à 40° C. On obtient ainsi 11,5 g de (+)-(nitro-3 phényl)-2 propioniate de benzyle sous forme d'une huile jaune utilisée telle quelle dans les synthèses ultérieures.

E. L'(+)-acide (nitro-3 phényl)-2 propionique peut être préparé de la manière suivante: 21,5 g de (nitro-3 phényl)-2 N-[hydroxy-2 phényl-1 éthyl-(R)]-propionamide (forme B) en solution dans un mélange de 450 $cm^3$ de dioxanne et de 450 $cm^3$ d'une solution aqueuse d'acide chlorhydriqe 4N sont chauffés à une température voisine de 80 C pendant 5 h, puis agités 12 h à une température voisine de 25° C. Le milieu réactionnel est concentré de moitié par évaporation sous pression réduite à 40° C, dilué par addition de 500 $cm^3$ d'eau et extrait par 2 fois 500 $cm^3$d'oxyde de diéthyle. Les phases organiques collectées sont lavées successivement par 3 fois 250 $cm^3$ d'eau et par 250 $cm^3$ d'une solution aqueuse saturée de chlorure de sodium. On obtient ainsi, après séchage sur sulfate de magnésium et concentration à sec sous pression réduite à 40° C, 14 g d'(+)-acide (nitro-3 phényl)-2 propionique (forme B) sous forme d'un solide crème utilisé tel quel dans les synthèses ultérieures.

F. Le (nitro-3 phényl)-2 N-[hydroxy-2 phényl-1 éthyl-(R)]-propionamide (forme B) peut être préparé de la manière suivante: à un mélange contenant 39,0 g d'acide (nitro-3 phényl)-2 propionique-(RS) et 0,5 $cm^3$ de diméthylformamide dans 400 $cm^3$ de dichloro-1,2 éthane, on ajoute lentement 17,2 $cm^3$ de dichlorure d'oxalyle. Le milieu réactionnel est agité 3h à une température voisine de 25° C puis concentré à sec sous pression réduite à 40° C. Le résidu obtenu est dissous dans 150 $cm^3$ de dichloro-1,2 éthane. La solution de chlorure d'acide ainsi obtenue est ajoutée à une solution de 27,4g de phényl-2 glycinol-(2R) en maintenant la température du milieu réactionnel en dessous de 10° C. En fin d'addition le mélange est agité 12 h à une température voisine de 25° C puis est lavé successivement par 1 fois 1000 $cm^3$d'eau, par 500 $cm^3$ d'une solution aqueuse d'acide chlorhydrique 1N, par 2 fois 500 $cm^3$ d'eau et par 1 fois 500 $cm^3$ d'une solution aqueuse saturée de chlorure de sodium. La phase organique collectée est séchée sur sulfate de magnésium et concentrée à sec sous pression réduite à 40° C. Les deux diastéréoisomères obtenus sont séparés par chromatographie sur silice [éluant : chlorure de méthylène-acétate d'éthyle (70/30 en volumes)]. Les fractions contenant chacun des deux diastéréoisomères sont réunies et concentrées à sec sous pression réduite à 40° C. On obtient ainsi 21,0 g de (nitro-3 phényl)-2 N-[hydroxy-2 phényl-1 éthyl-(R)]-propionamide (forme A) (premier produit d'élution fondant à 135° C et 19,0 g de (nitro-3 phényl)-2 N-[hydroxy-2 phényl-1 éthyl-(R)]-propionamide (forme B) (deuxième produit d'élution) fondant à 150° C.

**Exemple 85**

On opère d'une manière analogue à celle décrite à l'exemple 84, mais à partir de 3,8 g de {{{[tert-butoxycarbonyl-4 (fluoro-2 phényl)-2 thiazolidinyl-3-(2R,4R)]-2 oxo-2 éthyl}-3 uréido}-3 phényl}-2 propionate de benzyle (Forme A) et de 0,8 g de palladium sur charbon à 10%. On obtient ainsi, 1,4 g d'acide {{{[tert-butoxycarbonyl-4 (fluoro-2 phényl)-2 thiazolidinyl-3-(2R,4R)]-2 oxo-2 éthyl}-3 uréido}-3 phényl}-2 propionique (forme A) fondant à 145° C.

$[\alpha]_D^{20}$= + 20,0 ± 1,1 (C = 0,539 %; DMF)

**RMN du proton** (200 MHz, DMSO $D_6$, d en ppm, J en Hz), 2 rotamères à température ambiante, coalescence des raies à 120° C, déplacements chimiques caractéristiques à 120 C : 1,4 (d, J = 7, 3H, $-CH_3$); 1,53 (s, 9H,$-C(CH_3)_3$); 3,3 et 3,48 (2dd, J = 12,5 et 6, 2H, $S-CH_2$); 3,6 (q, J = 7, 1H, Ar-CH-COO); 3,77 et 4,05 (2dd, J = 17,5 et 5,5, 2H, $N-COCH_2-N$); 5,0 (t, J = 6, 1H, N-CH-COO); 6,17 (t, J = 5,5, 1H, -N HCO-); 6,53 (s, 1H, S-CH-N); 6,85 (bd, J = 7,5, 1H, en position 4 sur CO-NH-Ph-); 7,05 à 7,45 (m, 6H, aromatiques); 7,91 (dt, J = 8 et 1, 1H, en position 6 sur S-CH-Ph-); 8,43 (massif, 1H, ArNHCO-).

**Spectre infra-rouge** (KBr), bandes caractéristiques en $cm^{-1}$: 3380, 3100 à 3000, 2975, 2930, 2875, 2750 à 2350 (bande large), 1735, 1650, 1615, 1595, 1555, 1490, 1460, 1420, 1395, 1370, 1155, 760, 700.

Le {{{[tert-butoxycarbonyl-4 (fluoro-2 phényl)-2 thiazolidinyl-3-(2R,4R)]-2 oxo-2 éthyl}-3 uréido}-3 phényl}-2 propionate de benzyle (Forme A) peut être préparé d'une manière analogue à celle décrite à l'exemple **34** mais à partir de 4,0 g d'(amino-2 acétyl)-3 (fluoro-2 phényl)-2 thiazolidinecarboxylate-4 de tert-butyle-(2R,4R) et de 4,0 g d'(isocyanato-3 phényl)-2 propionate de benzyle (forme A). Le produit brut est purifié par chromatographie sur silice [éluant : acétate d'éthyle / cyclohexane (40/60 en volumes)]. Les fractions contenant le produit attendu sont réunies et concentrées à sec sous pression réduite à 40° C. On obtient ainsi 3,8 g de {{{[tert-butoxycarbonyl-4 (fluoro-2 phényl)-2 thiazolidinyl-3-(2R,4R)]-2 oxo-2 éthyl}-3 uréido}-3 phényl}-2 propionate de benzyle (Forme A) sous forme d'un produit amorphe, utilisé tel quel dans les synthèses ultérieures.

L'(isocyanato-3 phényl)-2 propionate de benzyle (forme A) peut être préparé comme à l'exemple **21** mais à partir de 4,0 g de (-)-(amino-3 phényl)-2 propionate de benzyle, de 2,1 $cm^3$ de chloroformiate de trichlorométhyle et de 0,33 g de charbon. On obtient ainsi 4,7 g d'(isocyanato-3 phényl)-2 propionate de benzyle (forme A) sous forme d'une huile orangée utilisée telle quelle dans les synthèses ultérieures.

L'(-)-(amino-3 phényl)-2 propionate de benzyle peut être préparé d'une manière analogue à celle décrite à l'exemple 84C, mais à partir de 5,3 g de (-)-(nitro-3 phényl)-2 propionate de benzyle, de 50 g de chlorure d'ammonium et de 24,8 g de zinc en poudre. On obtient ainsi 4,2 g de (-)-(amino-3 phényl)-2 propionate de benzyle sous forme d'une huile jaune utilisée telle quel le dans les synthèses ultérieures.

L'(-)-(nitro-3 phényl)-2 propionate de benzyle peut être préparé d'une manière analogue à celle décrite à l'exemple 84D, mais à partir de 4,45 g de (-)-acide (nitro-3 phényl)-2 propionique, de 0,3 $cm^3$ de diméthylformamide et de 2,15 $cm^3$ de dichlorure d'oxalyle. Le produit brut est purifié par chromatographie sur silice [éluant : acétate d'éthyle-cyclohexane (30/70 en volumes)]. Les fractions contenant le produit attendu sont réunies et concentrées à sec sous pression réduite à 40°C. On obtient ainsi 5,6 g de (-)-(nitro-3 phényl)-2 propioniate de benzyle sous forme d'une huile jaune utilisée telle quelle dans les synthèses ultérieures.

L'(-)-acide (nitro-3 phényl)-2 propionique peut être préparé d'une manière analogue à celle décrite à l'exemple 84E, mais à partir de 9,4 g de (nitro-3 phényl)-2 N-[hydroxy-2 phényl-1 éthyl-(R)]-propionamide (forme A) en solution dans un mélange de 200 $cm^3$ de dioxanne et de 200 $cm^3$ d'une solution aqueuse d'acide chlorhydriqe 4N. On obtient ainsi 5,85 g d'(-)-acide (nitro-3 phényl)-2 propionique sous forme d'un solide crème utilisé tel quel dans les synthèses ultérieures.

**Exemple 86**

On opère d'une manière analogue à celle décrite à l'exemple 77, mais à partir de 0,68 g de {{[tert-butoxycarbonyl-4 (difluoro-2,3 phényl)-2 thiazolidinyl-3]-2 oxo-2 éthyl}-3 uréido}-3 phénylacétate de méthyle-(2R,4R) dans 6 $cm^3$ d'un mélange eau-méthanol (30/70 en volumes) et de 0,08 g d'hydroxyde de potassium. Le produit brut (0,45g) est dissous dans 7,5 $cm^3$ d'une solution aqueuse d'hydroxyde de sodium 0,1N. La solution ainsi obtenue est lavée par 2 fois 10 $cm^3$ d'éther diéthylique, amenée à pH 2 par addition d'une solution aqueuse d'acide sulfurique 1N puis extraite par 2 fois 20 $cm^3$d'acétate d'éthyle. Les phases organiques sont réunies, séchées sur sulfate de magnésium et concentrées à sec sous pression réduite à 40° C. Le produit obtenu est purifié par chromatographie sur silice [éluant : chlorure de méthylène-méthanol (80/20 en volumes)]. Les fractions contenant le produit attendu sont réunies et concentrées à sec sous pression réduite à 40° C. On obtient ainsi 0,05 g d'acide {{[tert-butoxycarbonyl-4 (difluoro-2,3 phényl)-2 thiazolidinyl-3]-2 oxo-2 éthyl}-

3 uréido}-3 phénylacétique-(2R,4R) sous forme d'un produit amorphe.

$[\alpha]_D^{20}$= + 64 ± 2 (C = 0,557%; $CHCl_3$)

**RMN du proton** (200 MHz, DMSO $D_6$ plus quelques gouttes de $CD_3COOD$, d en ppm, J en Hz), 2 rotamères à température ambiante, coalescence des raies à 120° C, déplacements chimiques caractéristiques à 120° C: 1,52 (s, 9H,-$C(CH_3)_3$); 3,31 et 3,51 (2dd, J = 12,5 et 6, 2H, S-$CH_2$); 3,46 (s, 2H, Ar-$CH_2$-COO); 3,82 (bd, 1H, de N-$COCH_2$-N); 4,05 (d, J = 17, 1H, l'autre H de N-$COCH_2$-N); 5,0 (t, J = 6, 1H, N-CH-COO); 6,15 (massif, 1H, -NHCO-); 6,5 (s,1H, S-CH-N); 6,82 (bd, J = 7,5, 1H, en position 4 sur CO-NH-Ph-); 7,05 à 7,45 (m, 5H, aromatiques); 7,68 (bt, J = 8, 1H, en position 6 sur S-CH-Ph-); 8,7 (massif, 1H, ArNHCO-).

**Spectre infra-rouge** (KBr), bandes caractéristiques en $cm^{-1}$: 3380, 3100 à 3000, 2975, 2930, 2750 à 2350 (bande large), 1735, 1655, 1615, 1595, 1560, 1490, 1405, 1370, 1150, 775, 745.

Le {{[tert-butoxycarbonyl-4 (difluoro-2,3 phényl)-2 thiazolidinyl-3]-2 oxo-2 éthyl}-3 uréido}-3 phénylacétate de méthyle-(2R,4R) peut être préparé d'une manière analogue à celle décrite à l'exemple **41 A** mais à partir de 1,5 g de (difluoro-2,3 phényl)-2 thiazolidinecarboxylate-4 de tert-butyle-(2RS,4R), de 1,33 g d'acide {[(méthoxycarbonylméthyl-3 phényl]-3 uréido}-2 acétique et de 1,0 g de N,N′-dicyclohexylcarbodiimide. Le produit brut est purifié par chromatographie sur silice [éluant : acétate d'éthyle-cyclohexane (40/60 en volumes)]. Les fractions contenant le produit attendu sont réunies et concentrées à sec sous pression réduite à 40° C. On obtient ainsi 0,4 g de {{[tert-butoxycarbonyl-4 (difluoro-2,3 phényl)-2 thiazolidinyl-3]-2 oxo-2 éthyl}-3 uréido}-3 phénylacétate de méthyle-(2R,4R) sous forme d'une huile incolore utilisée telle quelle dans les synthèses ultérieures.

Le (difluoro-2,3 phényl)-2 thiazolidinecarboxylate-4 de tert-butyle-(2RS,4R) peut être préparé d'une manière analogue à celle décrite à l'exemple **34 C** mais à partir de 14,3 g d'acide (difluoro-2,3 phényl)-2 thiazolidinecarboxylique-4-(2RS,4R) en solution dans 50 $cm^3$ de chloroforme, de 3,5 $cm^3$ d'acide sulfurique concentré et d'un excès d'isobutène. Le produit brut obtenu est purifié par chromatographie sur silice [éluant : acétate d'éthyle-cyclohexane (10/90 en volumes)]. Les fractions contenant le produit attendu sont réunies et concentrées à sec sous pression réduite à 40° C. On obtient ainsi 14,0 g de (difluoro-2,3 phényl)-2 thiazolidinecarboxylate-4 de tert-butyle-(2RS,4R) sous forme d'une huile jaune épaisse, mélange des isomères (2R,4R) et (2S,4R), utilisée telle quelle dans les synthèses ultérieures.

L'acide (difluoro-2,3 phényl)-2 thiazolidinecarboxylique-4-(2RS,4R) peut être préparé comme à l'exemple **34 D** mais à partir de 8,5 g de L-cystéine et de 10,1 g de difluoro-2,3 benzaldéhyde. On obtient ainsi 3,7 g d'acide (difluoro-2,3 phényl)-2 thiazolidinecarboxylique-4-(2RS,4R) fondant à 120° C, utilisé tel quel dans les synthèses ultérieures.

**Exemple 87**

En opérant d'une manière analogue à celle décrite à l'exemple 41 §A, mais à partir de 1,2 g de diphényl-2,4 thiazolidine-(2RS,4R), de 1,04 g d'acide [(méthyl-3 phényl)-3 uréido]-2 acétique et de 1,03 g de N,N′-dicyclohexylcarbodiimide dans 15 $cm^3$ d'acétonitrile et après traitement, on obtient 0,98 g de {[(méthyl-3 phényl)-3 uréido]-2 acétyl}-1 diphényl-2,4 thiazolidine-(2RS,4R) sous forme de meringue.

**Masse** (ionisation chimique à l'ammoniac, 70eV, m/z), 432 ($M^+$).

**Spectre infra-rouge** (KBr), bandes caractéristiques en $cm^{-1}$ 3340, 3085, 3060, 3030, 2920, 1635, 1610, 1490, 1450, 1560, 780, 765, 695.

La diphényl-2,4 thiazolidine-(2RS,4R) peut être préparée comme à l'exemple 34 §D, mais à partir de 1,9 g du chlorhydrate d'amino-2 phényl-2 éthanethiol-(R), de 2,75 $cm^3$ de triéthylamine et de 1,15 $cm^3$ de benzaldéhyde dans 25 $cm^3$ d'éthanol. On obtient ainsi 1,15 g de diphényl-2,4 thiazolidine-(2RS,4R) fondant à 120°C.

L'amino-2 phényl-2 éthanethiol-(R) peut être préparé selon la méthode décrite dans la demande de brevet JP 57193447 (C.A. 98, 178924r).

**Exemple 88**

En opérant d'une manière analogue à celle décrite à l'exemple 9, mais à partir de 1,35 g de {{[(fluoro-2 phényl)-2 phényl-4 thiazolidinyl-3]-2 oxo-2 éthyl}-3 uréido}-3 phénylacétate de méthyle-(2RS,4R) en solution dans 25 $cm^3$ de méthanol et de 0,22 g d'hydroxyde de potassium dissous dans 5 $cm^3$ d'eau et après traitement, on obtient 0,45 g d'acide {{[(fluoro-2 phényl)-2 phényl-4 thiazolidinyl-3]-2 oxo-2 éthyl}-3 uréido}-3 phénylacétique-(2RS,4R) sous forme de meringue.

**Masse** (ionisation chimique à l'ammoniac, 70eV, m/z), 494 ($M^+$), 343.

**Spectre infra-rouge** (KBr), bandes caractéristiques en $cm^{-1}$ 3375, 3060, 3025, 2930, 2700 à 2250, 1710, 1640, 1610, 1485, 1455, 1560, 760, 700.

Le {{[(fluoro-2 phényl)-2 phényl-4 thiazolidinyl-3]-2 oxo-2 éthyl}-3 uréido}-3 phénylacétate de méthyle-(2RS,4R) peut être préparé comme décrit à l'exemple 41 §A, mais à partir de 1,3 g de (fluoro-2 phényl)-2 phényl-4 thiazolidine-(2RS,4R), de 1,33 g d'acide [(méthoxycarbonylméthyl-3 phényl)-3 uréido]-2 acétique et de 1,03 g de N,N'-dicyclohexylcarbodiimide dans 15 $cm^3$ d'acétonitrile. Après traitement, on obtient 1,5 g de {{[(fluoro-2 phényl)-2 phényl-4 thiazolidinyl-3]-2 oxo-2 éthyl}-3 uréido}-3 phénylacétate de méthyle-(2RS,4R) sous forme d'une huile utilisée telle quelle dans les synthèses ultérieures.

La (fluoro-2 phényl)-2 phényl-4 thiazolidine-(2RS,4R) peut être préparée comme à l'exemple 34 §D, mais à partir de 1,9 g du chlorhydrate de l'amino-2 phényl-2 éthanethiol-(R), de 2,75 $cm^3$ de triéthylamine et de 1,37 g de fluoro-2 benzaldéhyde dans 25 $cm^3$ d'éthanol. On obtient ainsi 1,5 g de (fluoro-2 phényl)-2 phényl-4 thiazolidine-(2RS,4R) sous forme d'une huile utilisée telle quelle dans les synthèses ultérieures.

**Exemple 89**

On opère d'une manière analogue à celle décrite à l'exemple 9, mais à partir de 0,9 g d'{[(éthoxycarbonyl-3 phényl)-3 uréido]-2 acétyl}-1 (méthyl-2 phényl)-5 prolinate de tert-butyle-(2RS,5SR) en solution dans 30 $cm^3$ de méthanol et de 0,1 g d'hydroxyde de potassium dissous dans 15 $cm^3$ d'eau. Après traitement et cristallisation dans un mélange oxyde de diisopropyle-acétate d'isopropyle (9-1 en volumes), on obtient 0,35 g d'acide {{[tert-butoxycarbonyl-2 (méthyl-2 phényl)-5 pyrrolidinyl-1]-2 oxo-2 éthyl}-3 uréido}-3 benzoïque-(2RS,5SR) fondant à 235°C.

L'{[(éthoxycarbonyl-3 phényl)-3 uréido]-2 acétyl}-1 (méthyl-2 phényl)-5 prolinate de tert-butyle-(2RS,5SR) peut être préparé comme décrit à l'exemple 41 §A, mais à partir de 1,3 g de (méthyl-2 phényl)-5 prolinate de tert-butyle-(2RS,5SR), de 1,32 g d'acide [(éthoxycarbonyl-3 phényl)-3 uréido]-2 acétique et de 1,03 g de N,N'-dicyclohexylcarbodiimide dans 50 $cm^3$ de tétrahydrofuranne. Après traitement, on obtient 1,9 g d'{[(éthoxycarbonyl-3 phényl)-3 uréido]-2 acétyl}-1 (méthyl-2 phényl)-5 prolinate de tert-butyle-(2RS,5SR) sous forme d'une meringue utilisée telle quelle dans les synthèses ultérieures.

Le (méthyl-2 phényl)-5 prolinate de tert-butyle-(2RS,5SR) peut être préparé de la manière suivante : une suspension de 3 g de magnésium dans une solution de 4 g d'un mélange des deux épimères en 4 du (méthyl-2 phényl)-5 phénylsulfonyl-4 prolinate de tert-butyle-(2RS,5RS) dans 200 $cm^3$ de méthanol est agitée pendant trois heures à une température voisine de 20°C. Le mélange réactionnel est ensuite versé dans 200 $cm^3$ d'une solution aqueuse normale d'acide chlorhydrique, puis extrait par trois fois 200 $cm^3$ de dichlorométhane. Les extraits organiques sont réunis, lavés par deux fois 100 $cm^3$ d'une solution aqueuse saturée en chlorure de sodium, séchés sur sulfate de magnésium et concentrés sous pression réduite. Le résidu est chromatographié sur silice [éluant : cyclohexane-acétate d'éthyle (70-30 en volumes)]. Les fractions contenant le produit attendu sont réunies et concentrées sous pression réduite. On obtient ainsi 0,6 g de (méthyl-2 phényl)-5 prolinate de tert-butyle-(2RS,5SR) sous forme d'une huile utilisée telle quelle dans les synthèses ultérieures.

A- Le (méthyl-2 phényl)-5 phénylsulfonyl-4 prolinate de tert-butyle-(2RS,5RS) peut être préparé de la manière suivante : à une suspension de 5 g d'acétate d'argent dans une solution de 3,4 g de phénylvinylsulfone et de 4,7 g de N-(orthométhylbenzylidène)-glycinate de tert-butyle dans 150 $cm^3$ d'acétonitrile, on ajoute goutte à goutte 2,8 $cm^3$ de triéthylamine à une température voisine de 20°C. Le mélange réactionnel est agité pendant deux heures à une température voisine de 20°C, puis versé dans 200 $cm^3$ d'une solution aqueuse saturée en chlorure d'ammonium. La phase aqueuse est filtrée et extraite par trois fois 100 $cm^3$ de dichlorométhane. Les extraits organiques sont réunis, lavés par 100 $cm^3$ d'une solution aqueuse saturée en chlorure de sodium, séchés sur sulfate de magnésium et concentrés sous pression réduite. On obtient ainsi, après cristallisation dans l'oxyde de diéthyle, 5 g de (méthyl-2 phényl)-5 phénylsulfonyl-4 prolinate de tert-butyle-(2RS,5RS), (mélange des deux épimères en 4), fondant à 180°C.

B- Le N-(orthométhylbenzylidène)-glycinate de tert-butyle peut être préparé de la manière suivante : à une suspension de 3 g de tamis moléculaire 4Å dans une solution de 3,35 g de chlorhydrate de glycinate de tert-butyle dans 2,4 $cm^3$ d'orthotolualdéhyde et 50 $cm^3$ de dichlorométhane, on ajoute goutte à goutte 2,8 $cm^3$ de triéthylamine à une température voisine de 20°C. Le mélange réactionnel est agité pendant vingt heures à une température voisine de 20°C, filtré et concentré sous pression réduite. Le résidu est repris dans 250 $cm^3$ d'oxyde de diéthyle, filtré et concentré sous pression réduite. On obtient ainsi 4,7 g de N-(orthométhylbenzylidène)-glycinate de tert-butyle sous forme d'une huile utilisée telle quelle dans les synthèses ultérieures.

**Exemple 90**

On opère d'une manière analogue à celle décrite à l'exemple 9, mais à partir de 1,1 g d'{[(éthoxycarbonyl-3 phényl)-3 uréido]-2 acétyl}-1 (fluoro-2 phényl)-5 prolinate de tert-butyle-(2RS,5SR) en solution dans 30 $cm^3$ de méthanol et de 0,12 g d'hydroxyde de potassium dissous dans 15 $cm^3$ d'eau. Après traitement et cristallisation dans l'oxyde de diisopropyle, on obtient 0,4 g d'acide {{[tert-butoxycarbonyl-2 (fluoro-2 phényl)-5 pyrrolidinyl-1]-2 oxo-2 éthyl}-3 uréido}-3 benzoique-(2RS,5SR) fondant à 180°C.

L'{[(éthoxycarbonyl-3 phényl)-3 uréido]-2 acétyl}-1 (fluoro-2 phényl)-5 prolinate de tert-butyle-(2RS,5SR) peut être préparé comme décrit à l'exemple 41 §A, mais à partir de 0,8 g de (fluoro-2 phényl)-5 prolinate de tert-butyle-(2RS,5SR), de 0,8 g d'acide [(éthoxycarbonyl-3 phényl)-3 uréido]-2 acétique et de 0,62 g de N,N'-dicyclohexycarbodiimide dans 30 $cm^3$ de tétrahydrofuranne. Après traitement, on obtient 1,5 g d'{[(éthoxycarbonyl-3 phényl)-3 uréido]-2 acétyl}-1 (fluoro-2 phényl)-5 prolinate de tert-butyle-(2RS,5SR) sous forme d'une meringue utilisée telle quelle dans les synthèses ultérieures.

A- Le (fluoro-2 phényl)-5 prolinate de tert-butyle-(2RS,5SR) peut être préparé de la manière suivante : une suspension de 5,7 g d'hydrogénophosphate disodique et de 7,7 g d'amalgame de sodium à 6% (dans le mercure) dans une solution de 4,06 g d'un mélange des deux épimères en 4 du (fluoro-2 phényl)-5 phénylsulfonyl-4 prolinate de tert-butyle-(2RS,5RS) dans 150 $cm^3$ de méthanol est agitée pendant vingt heures à une température voisine de 20°C. Le mélange réactionnel est ensuite versé dans 200 $cm^3$ d'eau et le mercure est séparé par décantation. La phase aqueuse est extraite par trois fois 100 $cm^3$ d'acétate d'éthyle. Les extraits organiques sont réunis, lavés par deux fois 100 $cm^3$ d'eau, séchés sur sulfate de magnésium et concentrés sous pression réduite. Le résidu est chromatographié sur silice [éluant : cyclohexane-acétate d'éthyle (70-30 en volumes)]. Les fractions contenant le produit attendu sont réunies et concentrées sous pression réduite. On obtient ainsi 0,4 g de (fluoro-2 phényl)-5 prolinate de tert-butyle-(2RS,5SR) sous forme d'une huile utilisée telle quelle dans les synthèses ultérieures.

Le (fluoro-2 phényl)-5 phénylsulfonyl-4 prolinate de tert-butyle-(2RS,5RS) peut être préparé comme décrit à l'exemple 89 §A mais à partir de 4,8 g de N-(orthofluorobenzylidène)-glycinate de tert-butyle, de 5 g d'acétate d'argent, de 3,4 g de phénylvinylsulfone et de 2,8 $cm^3$ de triéthylamine. Après traitement et cristallisation dans l'oxyde de diéthyle, on obtient 8 g de (fluoro-2 phényl)-5 phénylsulfonyl-4 prolinate de tert-butyle-(2RS,5RS), mélange des deux épimères en 4), fondant à 200°C.

Le N-(orthofluorobenzylidène)-glycinate de tert-butyle peut être préparé comme décrit à l'exemple 89 §B mais à partir de 2 $cm^3$ d'orthofluorobenzaldéhyde, de 3,35 g de chlorhydrate de glycinate de tert-butyle, de 2,8 $cm^3$ de triéthylamine et de 3 g de tamis moléculaire 4Å dans 50 $cm^3$ de dichlorométhane. Après traitement, on obtient 4,8 g de N-(orthofluorobenzylidène)-glycinate de tert-butyle sous forme d'une huile utilisée telle quelle dans les synthèses ultérieures.

**Exemple 91**

On opère d'une manière analogue à celle décrite à l'exemple 2, mais à partir de 1,12 g d'(amino-2 acétyl)-1 phényl-5 prolinate de tert-butyle-(2RS,5SR) et de 1,3 $cm^3$ d'isocyanate de parachlorophényle dans 50 $cm^3$ de tétrahydrofuranne. Après traitement, on obtient 1,2 g de {[(chloro-4 phényl)-3 uréido]-2 acétyl}-1 phényl-5 prolinate de tert-butyle-(2RS,5SR) sous forme d'un solide amorphe [RMN du proton (250 MHz, DMSO $D_6$, d en ppm,), 1,50 (s, 9H, $(CH_3)_3$); 1,85 (m, 2H, $CH_2$); 2,2 et 2,4 (2m, 2H, $CH_2$); 3,25 et 3,85 (ABX, 2H, $CH_2N$); 4,30 (dd, 1H, CHN); 5,20 (dd, 1H, CHN); 6,3 (t, 1H échangeable, NH); 7,2 à 7,6 (m, 9H, aromatiques); 9 (s, 1H échangeble, NH)], [Spectre infra-rouge (KBr), bandes caractéristiques en $cm^{-1}$ 3370, 3065, 3030, 2980, 2930, 287, 1735, 1595, 1490, 1450, 1545, 1365, 1150, 830, 760, 700].

**Exemple 92**

On opère d'une manière analogue à celle décrite à l'exemple 3, mais à partir de 3,05 g d'(amino-2 acétyl)-1 phényl-5 prolinate de tert-butyle-(2RS,5SR), de 1,78 g de N,N'-diimidazole-carbonyle et de 4,5 g d'(amino-3 phényl)-1 éthanesulfonate de tetrabutyl ammonium-(RS) dans 100 $cm^3$ de dichloro-1,2 éthane. Après traitement, on obtient 0,6 g de {{[(tert-butoxycarbonyl-2 phényl-5 pyrrolidinyl-1)-2 oxo-2 éthyl]-3 uréido}-3 phényl}-1 éthanesulfonate de potassium-(2RS,5SR, mélange des deux épimères en 1) sous forme d'un solide amorphe, [RMN du proton (200 MHz, DMSO $D_6$, d en ppm,),1,6 (s+d, 12H, $CCH_3$ et $(CH_3)_3$); 1,8 à 2,4 (m, 4H, 2 $CH_2$); 3,5 (m, 1H, $CHSO_3$); 3,1 et 3,8 (ABX, 2H, $CH_2N$); 4,2 (dd, 1H, CHN); 5,1dd, 1H, CHN); 6,2 (t, 1H échangeable, NH); 6,8 à 7,6 (m, 9H, aromatiques); 8,7 (bs, 1H échangeable, NH)], [Spectre infra-rouge (KBr), bandes caractéristiques en $cm^{-1}$ 3380, 3060, 3025, 2975, 2930, 2875, 1730, 1555, 1490, 1445, 1390, 1360, 1215, 1115, 1030, 790, 755, 700].

**Exemple 93**

On opère d'une manière analogue à celle décrite à l'exemple 41 §A, mais à partir de 4,95 g de phényl-5 prolinate de tert-butyle-(2RS,5SR), de 5,04 g d'acide [(méthoxycarbonyl-3 phényl)-3 uréido]-2 acétique et de

4,12 g de N,N'-dicyclohexylcarbodiimide dans 75 $cm^3$ de tétrahydrofuranne. Après traitement et cristallisation dans un mélange oxyde de diisopropyle-acétate d'isopropyle (90-10 en volumes), on obtient 1,2 g de [(méthoxycarbonyl-3 phényl)-3 uréido]-2 acétyl}-1 phényl-5 prolinate de tert-butyle-(2RS,5SR) fondant à 141 °C.

L'acide [(méthoxycarbonyl-3 phényl)-3 uréido]-2 acétique peut être préparé comme décrit à l'exemple 1 §A, mais à partir de 7,5 g de glycine, de 8,4 g d'hydrogénocarbonate de sodium dans 150 $cm^3$ d'eau et de 17,7 g d'isocyanato-3 benzoate de méthyle. Après traitement, on obtient 14,5 g d'acide [(méthoxycarbonyl-3 phényl)-3 uréido]-2 acétique fondant à 215°C.

**Exemple 94**

On opère d'une manière analogue à celle décrite à l'exemple 9, mais à partir de 2,6 g d'{[(éthoxycarbonyl-3 phényl)-3 uréido]-2 acétyl}-1 phényl-5 prolinate de tert-butyle-(2S,5R) en solution dans 40 $cm^3$ de méthanol et de 0,33 g d'hydroxyde de potassium en solution dans 20 $cm^3$ d'eau. Après traitement, on obtient 1,8 g d'acide {[(tert-butoxycarbonyl-2 phényl-5 pyrrolidinyl-1)-2 oxo-2 éthyl]-3 uréido}-3 benzoique-(2S,5R) dont les données analytiques sont en accord avec celles de l'énantiomère dextrogyre préparé par chromatographie sur phase stationnaire chirale du produit racémique.

L'{[(éthoxycarbonyl-3 phényl)-3 uréido]-2 acétyl}-1 phényl-5 prolinate de tert-butyle-(2S,5R) peut être obtenu de la manière suivante : en partant de 6,6 g d'{[(éthoxycarbonyl-3 phényl)-3 uréido]-2 acétyl}-1 phényl-5 prolinate de tert-butyle-(2RS,5SR), on sépare par chromatographie liquide à haute performance sur 400 g de support, dont la préparation est décrite ci-après, contenu dans une colonne de 26 cm de longueur et de 6 cm de diamètre avec comme phase mobile le mélange hexane-éthanol (70-30 en volumes) au débit de 70 $cm^3$/min en ordre d'élution successive :

- 2,9 g d'{[(éthoxycarbonyl-3 phényl)-3 uréido]-2 acétyl}-1 phényl-5 prolinate de tert-butyle-(2S,5R) dont le pouvoir rotatoire est $[\alpha]^{20}_D$ = +30 ± 0,8 (c = 0,922 ; méthanol)
- 2,8 g d'{[(éthoxycarbonyl-3 phényl)-3 uréido]-2 acétyl}-1 phényl-5 prolinate de tert-butyle-(2R,5S) dont le pouvoir rotatoire est $[\alpha]^{20}_D$ = -27 ± 0,6 (c = 1,213 ; méthanol)

**Exemple 95**

On opère d'une manière analogue à celle décrite à l'exemple 9, mais à partir de 3,5 g de {[(méthoxycarbonyl-3 phényl)-3 uréido]-2 acétyl}-1 phényl-5 prolinate de tert-butyle-(2S,5R) en solution dans 60 $cm^3$ de méthanol et de 0,45 g d'hydroxyde de potassium en solution dans 30 $cm^3$ d'eau. Après traitement, on obtient 2,0 g d'acide {[(tert-butoxycarbonyl-2 phényl-5 pyrrolidinyl-1)-2 oxo-2 éthyl}-3 uréido}-3 benzoïque-(2S,5R) dont les données analytiques sont en accord avec celles de l'énantiomère dextrogyre préparé par chromatographie sur phase stationnaire chirale du produit racémique.

Le {[(méthoxycarbonyl-3 phényl)-3 uréido]-2 acétyl}-1 phényl-5 prolinate de tert-butyle-(2S,5R) peut être préparé comme décrit à l'exemple 41 §A, mais à partir de 0,1 g de phényl-5 prolinate de tert-butyle-(2S,5R), de 0,1 g d'acide [(méthoxycarbonyl-3 phényl)-3 uréido]-2 acétique et de 0,08 g de N,N'-dicyclohexylcarbodiimide dans 5 $cm^3$ de tétrahydrofuranne. Après traitement, on obtient 0,1 g de {[(méthoxycarbonyl-3 phényl)-3 uréido]-2 acétyl}-1 phényl-5 prolinate de tert-butyle-(2S,5R) dont les données analytiques sont en accord avec celles de l'énantiomère dextrogyre préparé par chromatographie sur phase stationnaire chirale du produit racémique.

Le phényl-5 prolinate de tert-butyle-(2S,5R) peut être préparé de la manière suivante : à une émulsion de 0,83 g de phényl-5 $\Delta_5$ pyrrolinecarboxylate de tert-butyle-2-(S) dans un mélange de 0,5 $cm^3$ d'éthanol et de 1,5 $cm^3$ d'eau, on ajoute en cinq minutes, à une température voisine de 2°C, une solution de 0,14 g de borohydrure de sodium et de 0,07 g de carbonate de potassium dans 0,8 $cm^3$ d'eau. Le milieu réactionnel est agité pendant 20 heures à une température voisine de 20°C puis on ajoute une solution de 0,14 g de borohydrure de sodium et de 0,07 g de carbonate de potassium dans 0,8 $cm^3$ d'eau. Le milieu réactionnel est encore agité pendant 70 heures à une température voisine de 20°C puis dilué par 25 $cm^3$ d'eau et extrait par trois fois 20 $cm^3$ de dichlorométhane. Les extraits organiques sont réunis, lavés par 10 $cm^3$ d'eau, séchés sur sulfate de magnésium et concentrés à sec sous pression réduite à 50°C. Le résidu est purifié par chromatographie sur silice [éluant dichlorométhane]. Les fractions contenant le produit attendu sont réunies et concentrées sous pression réduite. On obtient ainsi 0,1 g de phényl-5 prolinate de tert-butyle-(2S,5R) sous forme d'une huile utilisée telle quelle dans les synthèses ultérieures.

La phényl-5 Δ-5 pyrrolinecarboxylate de tert-butyle-2-(S) peut être préparée de la manière suivante : à une solution de 1,8 g de tert-butoxycarbonylamino-2 oxo-5 phényl-5 pentanoate de tert-butyle-(S) dans 25 $cm^3$ de dichlorométhane, on ajoute, à une température voisine de 20°C, 2,3 $cm^3$ d'acide trifluoroacétique. Le mélange réactionnel est agité pendant six heures à une température voisine de 20°C, puis on ajoute 120 $cm^3$ d'une so-

lution aqueuse saturée en hydrogénocarbonate de sodium. La phase organique est séparée par décantation, lavée par 20 $cm^3$ d'eau distillée, séchée sur sulfate de magnésium et concentrée sous pression réduite. On obtient ainsi 0,9g de phényl-5 Δ-5 pyrrolinecarboxylate de tert-butyle-2-(S) sous forme d'une huile utilisée telle quelle dans les synthèses ultérieures.

Le tert-butoxycarbonylamino-2 oxo-5 phényl-5 pentanoate de tert-butyle-(S) peut être préparé de la manière suivante : à une suspension de 0,72 g de magnésium dans 20 $cm^3$ de tétrahydrofuranne, on ajoute en 35 minutes, à une température comprise entre 20 et 30°C, une solution de 2,8 $cm^3$ de bromobenzène dans 60 $cm^3$ de tétrahydrofuranne. Le milieu réactionnel est encore agité à une température voisine de 24°C pendant 145 minutes puis est additionné en 20 minutes à une solution de 5,7 g de tert-butoxycarbonyl-1 oxo-5 pyrrolidinecarboxylate de tert-butyle-2-(S) dans 80 $cm^3$ de tétrahydrofuranne maintenue à une température voisine de -75°C. Le milieu réactionnel est encore agité pendant trois heures à une température voisine de -78°C puis réchauffé à une température voisine de -15°C. On ajoute alors en 15 minutes, 100 $cm^3$ d'une solution aqueuse de chlorure d'ammonium à 10%. La phase aqueuse est séparée par décantation et extraite par trois fois 100 $cm^3$ d'oxyde de diéthyle. Les phases organiques sont réunies et lavées par deux fois 25 $cm^3$ d'eau, séchées sur sulfate de magnésium et concentrées sous pression réduite à une température voisine de 50°C. Le résidu est purifié par cristallisation dans 20 $cm^3$ de pentane. On obtient ainsi 2,5 g de tert-butoxycarbonylamino-2 oxo-5 phényl-5 pentanoate de tert-butyle-(S) fondant à 107°C. Ce produit peut aussi se présenter sous forme de tert-butoxycarbonyl-1 hydroxy-5 phényl-5 pyrrolidinecarboxylate de tert-butyle-2-(2S,5RS) fondant à 85°C.
Le tert-butoxycarbonyl-1 oxo-5 pyrrolidinecarboxylate de tert-butyle-2-(S) peut être obtenu selon la méthode décrite par J. ACKERMANN et M. MATTHES, Helv. Chim. Acta, 73, 122-132, (1990).
Le {[(méthoxycarbonyl-3 phényl)-3 uréido]-2 acétyl}-1 phényl-5 prolinate de tert-butyle-(2S,5R) peut aussi être obtenu de la manière suivante : en partant de 7,8 g de {[(méthoxycarbonyl-3 phényl)-3 uréido]-2 acétyl}-1 phényl-5 prolinate de tert-butyle-(2RS,5SR), on sépare par chromatographie liquide à haute performance sur 400 g de support, dont la préparation est décrite dans l'exemple précédent, contenu dans une colonne de 26 cm de longueur et de 6 cm de diamètre avec comme phase mobile le mélange hexane éthanol (70-30 en volumes) au débit de 70 $cm^3$/min en ordre d'élution successive :

- 3,7 g de {[(méthoxycarbonyl-3 phényl)-3 uréido]-2 acétyl}-1 phényl-5 prolinate de tert-butyle-(2S,5R) dont le pouvoir rotatoire est $[\alpha]^{20}_D$ = +31,4 ± 1,2 (c = 0,579 ; méthanol)
- 3,6 g de {[(méthoxycarbonyl-3 phényl)-3 uréido]-2 acétyl}-1 phényl-5 prolinate de tert-butyle-(2R,5S) dont le pouvoir rotatoire est $[\alpha]^{20}_D$ = -28,2 ± 0,8 (c = 0,949 ; méthanol)

**Exemple 96**

On opère d'une manière analogue à celle décrite à l'exemple 9, mais à partir de 2,6 g d'{[(éthoxycarbonyl-3 phényl)-3 uréido]-2 acétyl}-1 phényl-5 prolinate de tert-butyle-(2R,5S) en solution dans 60 $cm^3$ de méthanol et de 0,3 g d'hydroxyde de potassium en solution dans 40 $cm^3$ d'eau. Après traitement, on obtient 1,3 g d'acide {[(tert-butoxycarbonyl-2 phényl-5 pyrrolidinyl-1)-2 oxo-2 éthyl]-3 uréido}-3 benzoique-(2R,5S) dont les données analytiques sont en accord avec celles de l'énantiomère lévogyre préparé par chromatographie sur phase stationnaire chirale du produit racémique.

**Exemple 97**

On opère d'une manière analogue à celle décrite à l'exemple 9, mais à partir de 3,38 g de {[(méthoxycarbonyl-3 phényl)-3 uréido]-2 acétyl}-1 phényl-5 prolinate de tert-butyle-(2R,5S) en solution dans 60 $cm^3$ de méthanol et de 0,45 g d'hydroxyde de potassium en solution dans 20 $cm^3$ d'eau. Après traitement, on obtient 2,0 g d'acide {[(tert-butoxycarbonyl-2 phényl-5 pyrrolidinyl-1)-2 oxo-2 éthyl]-3 uréido}-3 benzoique-(2R,5S) dont les données analytiques sont en accord avec celles de l'énantiomère lévogyre préparé par chromatographie sur phase stationnaire chirale du produit racémique.

**Exemple 98**

On opère comme à l'exemple 9, mais à partir de 1,9 g de {[(tert-butoxycarbonyl-2 phényl-5 pyrrolidinyl-1)-2 oxo-2 éthyl]-3 uréido}-3 phénylacétate de méthyle-(2S,5R) en solution dans 60 $cm^3$ de méthanol et de 0,22 g d'hydroxyde de potassium dissous dans 30 $cm^3$ d'eau. Après traitement, on obtient 0,65 g d'acide {[(tert-butoxycarbonyl-2 phényl-5 pyrrolidinyl-1)-2 oxo-2 éthyl]-3 uréido}-3 phénylacétique-(2S,5R) fondant à 112°C, $[\alpha]_D^{20}$ = +30,6° ± 0,8° (c=1 ; méthanol).

Le {[(tert-butoxycarbonyl-2 phényl-5 pyrrolidinyl-1)-2 oxo-2 éthyl]-3 uréido}-3 phénylacétate de méthyle-(2S,5R) peut être préparé comme décrit à l'exemple 41 §A, mais à partir de 1,2 g de phényl-5 prolinate de tert-butyle-(2S,5R), de 1,29 g d'acide [(méthoxycarbonylméthyl-3 phényl)-3 uréido]-2 acétique et de 1 g de N,N'-dicyclohexylcarbodiimide dans 50 $cm^3$ d'acétonitrile. Après traitement, on obtient 1,9 g de {[(tert-butoxycarbonyl-2 phényl-5 pyrrolidinyl-1)-2 oxo-2 éthyl]-3 uréido}-3 phénylacétate de méthyle-(2S,5R) sous forme d'une meringue utilisée telle quelle dans les synthèses ultérieures.

Le phényl-5 prolinate de tert-butyle-(2S,5R) peut être préparé de la manière suivante : à une solution de 1,25 g de phényl-5 Δ-5 pyrrolinecarboxylate de tert-butyle-2-(S) dans 50 $cm^3$ d'éthanol, on ajoute 0,02 g d'oxyde de platine. La suspension est agitée pendant 3 heures à une température voisine de 20°C sous atmosphère d'hydrogène (130 kPa). Le catalyseur est séparé par filtration et le filtrat est concentré à sec sous pression réduite à 45°C. Le résidu est purifié par chromatographie sur silice [éluant dichlorométhane-méthanol (97,5-2,5 en volumes)]. Les fractions contenant le produit attendu sont réunies et concentrées sous pression réduite. On obtient ainsi 1,2 g de phényl-5 prolinate de tert-butyle-(2S,5R) sous forme d'une huile utilisée telle quelle dans les synthèses ultérieures.

Le phényl-5 Δ-5 pyrrolinecarboxylate de tert-butyle-2-(S) peut être préparée de la manière suivante : à une solution de 3,65 g de tert-butoxycarbonylamino-2 oxo-5 phényl-5 pentanoate de tert-butyle-(S) dans 50 $cm^3$ de dichlorométhane, on ajoute, à une température voisine de 20°C, 1,4 $cm^3$ de iodotriméthylsilane. Le mélange réactionnel est agité pendant 20 heures à une température voisine de 20°C, puis on ajoute 50 $cm^3$ d'une solution aqueuse saturée en hydrogénocarbonate de sodium. La phase organique est séparée par décantation, lavée par 20 $cm^3$ d'eau distillée, séchée sur sulfate de magnésium et concentrée sous pression réduite. On obtient ainsi 2,0 g de phényl-5 Δ-5 pyrrolinecarboxylate de tert-butyle-2-(S) sous forme d'une huile utilisée telle quelle dans les synthèses ultérieures.

**Exemple 99**

On opère d'une manière analogue à celle décrite à l'exemple 2, mais à partir de 1,93 g d'(amino-2 acétyl)-1 phényl-5 prolinate de tert-butyle-(2RS,5SR) et de 3,61 g d'isocyanato-3 benzoate de benzyle dans 50 $cm^3$ de tétrahydrofuranne. Après traitement, on obtient 1 g de {[(benzyloxycarbonyl-3 phényl)-3 uréido]-2 acétyl}-1 phényl-5 prolinate de tert-butyle-(2RS,5SR) fondant à 75°C.

L'isocyanato-3 benzoate de benzyle peut être préparé comme décrit à l'exemple 64 §A, mais à partir de 27 g du chlorhydrate de l'amino-3 benzoate de benzyle dans 200 $cm^3$ de toluène, de 14,4 $cm^3$ de triéthylamine, de 2 g de charbon et de 12,5 $cm^3$ de chloroformiate de trichlorométhyle dans 200 $cm^3$ de toluène. Après traitement, on obtient 27 g d'isocyanato-3 benzoate de benzyle sous forme d'une huile utilisée telle quelle dans les synthèses ultérieures.

L'amino-3 benzoate de benzyle peut être préparé selon la méthode décrite par H.A. SHONLE et coll., J. Amer. Chem. Soc., 43, 361 (1921)

**Exemple 100**

A une solution de 1,7 g de {[(benzyloxycarbonyl-3 phényl)-3 uréido]-2 acétyl}-1 phényl-5 prolinate d'éthyle-(2RS,5SR) dans 100 $cm^3$ d'éthanol, on ajoute 0,5 g de palladium à 5% sur charbon. La suspension est agitée pendant 20 heures à une température voisine de 25°C sous atmosphère d'hydrogène (130 kPa). Le catalyseur est séparé par filtration et le filtrat est concentré à sec sous pression réduite. Le résidu est recristallisé dans 80 $cm^3$ d'éthanol. On obtient ainsi 0,7 g d'acide {[(éthoxycarbonyl-2 phényl-5 pyrrolidinyl-1)-2 oxo-2 éthyl]-3 uréido}-3 benzoïque-(2RS,5SR) fondant à 240°C.

Le {[(benzyloxycarbonyl-3 phényl)-3 uréido]-2 acétyl}-1 phényl-5 prolinate d'éthyle-(2RS,5SR) peut être préparé comme décrit à l'exemple 41 §A, mais à partir de 2 g de phényl-5 prolinate d'éthyle-(2RS,5SR), de 2,99 g d'acide [(benzyloxycarbonyl-3 phényl)-3 uréido]-2 acétique et de 2,07 g de N,N'-dicyclohexylcarbodiimide dans 180 $cm^3$ de tétrahydrofuranne. Après traitement, on obtient 1,95 g de {[(benzyloxycarbonyl-3 phényl)-3 uréido]-2 acétyl}-1 phényl-5 prolinate d'éthyle-(2RS,5SR) sous forme d'une meringue utilisée telle quelle dans les synthèses ultérieures.

L'acide [(benzyloxycarbonyl-3 phényl)-3 uréido]-2 acétique peut être préparé comme décrit à l'exemple 1 §A, mais à partir de 3,97 g de glycine et de 14,62 g de carbonate de potassium en solution dans 90 $cm^3$ d'eau et de 13,4 g d'isocyanato-3 benzoate de benzyle en solution dans 70 $cm^3$ de dioxanne-1,4. Après traitement, on obtient 13 g d'acide [(benzyloxycarbonyl-3 phényl)-3 uréido]-2 acétique utilisé tel quel dans les synthèses ultérieures.

Le phényl-5 prolinate d'éthyle-(2RS,5SR) peut être obtenu de la manière suivante : à une solution de 5,5 g de phényl-5 proline-(2RS,5SR) dans 50 $cm^3$ d'éthanol, on ajoute goutte à goutte 0,5 $cm^3$ d'acide sulfurique concentré. Le milieu réactionnel est ensuite agité à une température voisine de 80°C pendant cinq heures puis refroidi à une température voisine de 20°C et concentré sous pression réduite. Le résidu est repris dans 50 $m^3$

d'eau, amené à un pH voisin de 9 par une solution aqueuse normale d'hydroxyde de sodium et extrait par trois fois 100 $cm^3$ d'acétate d'éthyle. Les extraits organiques réunis sont lavés par 50 $cm^3$ d'une solution aqueuse saturée en chlorure de sodium, séchés sur sulfate de magnésium et concentrés sous pression réduite. Le résidu est chromatographié sur silice [éluant cyclohexane-acétate d'éthyle (90-10 en volumes)]. Les fractions contenant le produit attendu sont réunies et concentrées sous pression réduite. On obtient ainsi 2,16 g de phényl-5 prolinate d'éthyle-(2RS,5SR) sous forme d'une huile utilisée telle quelle dans les synthèses ultérieures.

**Exemple 101**

On opère d'une manière analogue à celle décrite à l'exemple 100, mais à partir de 10,2 g de {[(benzyloxycarbonyl-3 phényl)-3 uréido]-2 acétyl}-1 phényl-5 prolinate de tert-butyle-(2RS,5SR) et de 1 g de palladium à 5% sur charbon dans 300 $cm^3$ d'éthanol. Après traitement, on obtient 7,1 g d'acide {[(tert-butoxycarbonyl-2 phényl-5 pyrrolidinyl-1)-2 oxo-2 éthyl]-3 uréido}-3 benzoïque-(2RS,5SR) fondant à 236°C.

**Exemple102**

On opère d'une manière analogue à celle décrite à l'exemple 100, mais à partir de 0,8 g de {[(benzyloxycarbonyl-3 phényl)-3 uréido]-2 acétyl}-1 (méthyl-3 phényl)-5 prolinate de tert-butyle-(2RS,5SR) en solution dans 50 $cm^3$ d'acétate d'éthyle et de 0,1 g de palladium à 5% sur charbon. Après traitement, on obtient 0,45 g d'acide {{[tert-butoxycarbonyl-2 (méthyl-3 phényl)-5 pyrrolidinyl-1]-2 oxo-2 éthyl}-3 uréido}-3 benzoïque-(2RS,5SR) fondant à 208°C.

Le {[(benzyloxycarbonyl-3 phényl)-3 uréido]-2 acétyl}-1 (méthyl-3 phényl)-5 prolinate de tert-butyle-(2RS,5SR) peut être préparé comme décrit à l'exemple 41 §A, mais à partir de 0,4 g de (méthyl-3 phényl)-5 prolinate de tert-butyle-(2RS,5SR), de 0,5 g d'acide [(benzyloxycarbonyl-3 phényl)-3 uréido]-2 acétique et de 0,31 g de N,N'-dicyclohexylcarbodiimide dans 20 $cm^3$ d'acétonitrile. Après traitement, on obtient 0,8 g de {[(benzyloxycarbonyl-3 phényl)-3 uréido]-2 acétyl}-1 (méthyl-3 phényl)-5 prolinate de tert-butyle-(2RS,5SR) sous forme d'une meringue utilisée telle quelle dans les synthèses ultérieures.

Le (méthyl-3 phényl)-5 prolinate de tert-butyle-(2RS,5SR) peut être préparé comme décrit à l'exemple 2 §A, mais à partir de 1g de tert-butoxycarbonyl-1 (méthyl-3 phényl)-5 prolinate de tert-butyle-(2RS,5SR) et de 0,4 $cm^3$ d'iodotriméthylsilane dans 40 $cm^3$ de chloroforme. Après traitement, on obtient 0,4 g de (méthyl-3 phényl)-5 prolinate de tert-butyle-(2RS,5SR) sous forme d'une huile utilisée telle quelle dans les synthèses ultérieures.

Le tert-butoxycarbonyl-1 (méthyl-3 phényl)-5 prolinate de tert-butyle-(2RS,5SR) peut être préparé comme décrit à l'exemple 90 §A, mais à partir de 2,4 g de dihydrogénophosphate de sodium et de 7,63 g d'amalgame de sodium à 6% (dans le mercure) dans une solution de 2,5 g d'un mélange des deux épimères en 4 du tert-butoxycarbonyl-1 (méthyl-3 phényl)-5 phénylsulfonyl-4 prolinate de tert-butyle-(2RS,5RS) dans un mélange de 20 $cm^3$ de méthanol et de 40 $cm^3$ de tétrahydrofuranne. Après traitement, on obtient 1 g de tert-butoxycarbonyl-1 (méthyl-3 phényl)-5 prolinate de tert-butyle-(2RS,5SR) sous forme d'une huile utilisée telle quelle dans les synthèses ultérieures.

Le tert-butoxycarbonyl-1 (méthyl-3 phényl)-5 phénylsulfonyl-4 prolinate de tert-butyle-(2RS,5RS) peut être préparé de la manière suivante : à une solution de 2,01 g d'un mélange des deux épimères en 4 du (méthyl-3 phényl)-5 phénylsulfonyl-4 prolinate de tert-butyle-(2RS,5RS) dans 50 $cm^3$ de dichlorométhane, on ajoute successivement 0,42 g d'hydrogénocarbonate de sodium puis une solution de 1,09 g de dicarbonate de di-tert-butyle dans 20 $cm^3$ de dichlorométhane. Le mélange réactionnel est agité pendant 72 heures à une température voisine de 20°C, puis on ajoute 50 $cm^3$ d'eau. La phase organique est séparée par décantation, séchée sur sulfate de magnésium et concentrée à sec sous pression réduite. On obtient ainsi 2,5 g de tert-butoxycarbonyl-1 (méthyl-3 phényl)-5 phénylsulfonyl-4 prolinate de tert-butyle-(2RS,5RS, mélange des deux épimères en 4) sous forme d'une huile utilisée telle quelle dans les synthèses ultérieures.

Le (méthyl-3 phényl)-5 phénylsulfonyl-4 prolinate de tert-butyle-(2RS,5RS) peut être préparé comme décrit à l'exemple 89 §A mais à partir de 4,7 g de N-(métaméthylbenzylidène)-glycinate de tert-butyle, de 5 g d'acétate d'argent, de 3,4 g de phénylvinylsulfone et de 2,8 $cm^3$ de triéthylamine. Après traitement, on obtient 6,1 g de (méthyl-3 phényl)-5 phénylsulfonyl-4 prolinate de tert-butyle-(2RS,5RS), mélange des deux épimères en 4, fondant à 139°C.

Le N-(métaméthylbenzylidène)-glycinate de tert-butyle peut être préparé comme décrit à l'exemple 89 §B mais à partir de 2,4 $cm^3$ de métatolualdéhyde, de 3,35 g de chlorhydrate de glycinate de tert-butyle, de 2,8 $cm^3$ de triéthylamine et de 3 g de tamis moléculaire 4Å dans 30 $cm^3$ de dichlorométhane. Après traitement, on obtient 4,7 g de N-(métaméthylbenzylidène)-glycinate de tert-butyle sous forme d'une huile utilisée telle quelle dans les synthèses ultérieures.

**Exemple 103**

On opère d'une manière analogue à celle décrite à l'exemple 9, mais à partir de 1,2 g d'un mélange d'{[(éthoxycarbonyl-3 phényl)-3 uréido]-2 acétyl}-1 phényl-3 prolinate de tert-butyle-(2RS,3SR) et -(2RS,3RS) 85 pour 15 en poids en solution dans 30 $cm^3$ de tétrahydrofuranne et de 2,4 $cm^3$ d'une solution normale d'hydroxyde de sodium diluée par 15 $cm^3$ d'eau. Après traitement, on obtient un mélange de 0,7 g d'acide {[(tert-butoxycarbonyl-2 phényl-3 pyrrolidinyl-1)-2 oxo-2 éthyl]-3 uréido}-3 benzoïque-(2RS,3SR) et -(2RS,3RS) 85 pour 15 en poids fondant à 225°C.

L'{[(éthoxycarbonyl-3 phényl)-3 uréido]-2 acétyl}-1 phényl-3 prolinate de tert-butyle-(2RS,3SR) peut être préparé comme décrit à l'exemple 41 §A, mais à partir de 1 g d'un mélange de phényl-3 prolinate de tert-butyle-(2RS,3SR) et -(2RS,3RS) 85 pour 15 en poids, de 1,1 g d'acide [(éthoxycarbonyl-3 phényl)-3 uréido]-2 acétique et de 0,83 g de N,N'-dicyclohexylcarbodiimide dans 30 $cm^3$ de tétrahydrofuranne. Après traitement, on obtient un mélange de 1,2 g d'{[(éthoxycarbonyl-3 phényl)-3 uréido]-2 acétyl}-1 phényl-3 prolinate de tert-butyle-(2RS,3SR) et -(2RS,3RS) 85 pour 15 en poids fondant à 154°C.

Le phényl-3 prolinate de tert-butyle-(2RS,3SR) et le phényl-3 prolinate de tert-butyle-(2RS,3RS) peuvent être préparés comme décrit à l'exemple 1 §B, mais à partir de 10 g d'un mélange de phényl-3 proline-(2RS,5SR) et -(2RS,5RS), d'isobutène et de 3 $cm^3$ d'acide sulfurique dans 200 $cm^3$ de chloroforme. Après traitement et chromatographie sur silice [éluant cyclohexane-acétate d'éthyle (50-50 en volumes)], on obtient 1 g d'un mélange de phényl-3 prolinate de tert-butyle-(2RS,3SR) et -(2RS,3RS) 85 pour 15 en poids sous forme d'une huile utilisée telle quelle dans les synthèses ultérieures et 2,5 g d'un mélange de phényl-3 prolinate de tert-butyle-(2RS,3RS) et -(2RS,3SR) 85 pour 15 en poids sous forme d'une huile utilisée telle quelle dans les synthèses ultérieures.

Les phényl-3 proline-(2RS,5SR) et -(2RS,5RS) peuvent être préparées selon les méthodes décrites par Y.N. BELOKON et coll., J. Chem. Soc., Perkin Trans., 1, 2075 (1988) et J. RIVIER,, G.R. MARSHALL Peptides, Chemistry, Structure and Biology, Proceedings of the Eleventh American Peptide Symposium, July 9-14 1989, La Jolla, California, USA, ESCOM, Leiden, 1990.

**Exemple 104**

On opère d'une manière analogue à celle décrite à l'exemple 9, mais à partir de 2,5 g d'un mélange d'{[(éthoxycarbonyl-3 phényl)-3 uréido]-2 acétyl}-1 phényl-3 prolinate de tert-butyle-(2RS,3RS) et -(2RS,3SR) 80 pour 20 en poids en solution dans 30 $cm^3$ de tétrahydrofuranne et de 5 $cm^3$ d'une solution normale d'hydroxyde de sodium diluée par 15 $cm^3$ d'eau. Après traitement, on obtient un mélange de 1 g d'acide {[(tert-butoxycarbonyl-2 phényl-3 pyrrolidinyl-1)-2 oxo-2 éthyl]-3 uréido}-3 benzoique-(2RS,3RS) et -(2RS,3SR) 90 pour 10 en poids fondant à 200°C.

L'{[(éthoxycarbonyl-3 phényl)-3 uréido]-2 acétyl}-1 phényl-3 prolinate de tert-butyle-(2RS,3RS) peut être préparé comme décrit à l'exemple 41 §A, mais à partir de 2,5 g d'un mélange de phényl-3 prolinate de tert-butyle-(2RS,3RS) et -(2RS,3SR) 90 pour 10 en poids, de 2,7 g d'acide [(éthoxycarbonyl-3 phényl)-3 uréido]-2 acétique et de 2,1 g de N,N'-dicyclohexylcarbodiimide dans 50 $cm^3$ de tétrahydrofuranne. Après traitement, on obtient un mélange de 1,2 g d'{[(éthoxycarbonyl-3 phényl)-3 uréido]-2 acétyl}-1 phényl-3 prolinate de tert-butyle-(2RS,3RS) et -(2RS,3SR) 80 pour 20 en poids fondant à 141°C.

**Exemple 105**

On opère d'une manière analogue à celle décrite à l'exemple 9, mais à partir de 2,1 g de {[(méthoxycarbonyl-3 phényl)-3 uréido]-2 acétyl}-1 phényl-4 prolinate de tert-butyle-(2S,4S) en solution dans 30 $cm^3$ de méthanol et de 0,5 g d'hydroxyde de potassium dissous dans 15 $cm^3$ d'eau. Après traitement et cristallisation dans un mélange oxyde de diisopropyle-acétate d'isopropyle (90-10 en volumes), on obtient 0,65 g d'acide {[(tert-butoxycarbonyl-2 phényl-4 pyrrolidinyl-1)-2 oxo-2 éthyl]-3 uréido}-3 benzoïque-(2S,4S) fondant à 144°C.

Le {[(méthoxycarbonyl-3 phényl)-3 uréido]-2 acétyl}-1 phényl-4 prolinate de tert-butyle-(2S,4S) peut être préparé comme décrit à l'exemple 41 §A, mais à partir de 1,2 g de phényl-4 prolinate de tert-butyle-(2S,4S), de 1,2 g d'acide [(méthoxycarbonyl-3 phényl)-3 uréido]-2 acétique et de 1 g de N,N'-dicyclohexylcarbodiimide dans 30 $cm^3$ d'acétonitrile. Après traitement, on obtient 2,1 g de {[(méthoxycarbonyl-3 phényl)-3 uréido]-2 acétyl}-1 phényl-4 prolinate de tert-butyle-(2S,4S) sous forme d'une meringue utilisée telle quelle dans les synthèses ultérieures.

Le phényl-4 prolinate de tert-butyle-(2S,4S) peut être préparé comme décrit à l'exemple 1 §B, mais à partir de 3,4 g de phényl-4 proline-(2S,4S), d'isobutène et de 1,5 $cm^3$ d'acide sulfurique dans 150 $cm^3$ de chloroforme. Après traitement, on obtient 1,2 g de phényl-4 prolinate de tert-butyle-(2S,4S) sous forme d'une meringue utilisée telle quelle dans les synthèses ultérieures.

La phényl-4 proline-(2S,4S) peut être préparée selon les méthodes décrites par J.K. THOTTATHIL et J.L. MONIOT Tetrahedron Lett., 27, 151 (1986) et D.R KRONENTHAL et coll. Tetrahedron Lett., 31, 1241 (1990).

**Exemple 106**

On opère d'une manière analogue à celle décrite à l'exemple 9, mais à partir de 1,8 g de {[(méthoxycarbonyl-3 phényl)-3 uréido]-2 acétyl}-1 phényl-4 prolinate de tert-butyle-(2S,4R) en solution dans 30 $cm^3$ de méthanol et de 0,21 g d'hydroxyde de potassium dissous dans 15 $cm^3$ d'eau. Après traitement et cristallisation dans un mélange oxyde de diisopropyle-acétate d'isopropyle (90-10 en volumes), on obtient 0,55 g d'acide {[(tert-butoxycarbonyl-2 phényl-4 pyrrolidinyl-1)-2 oxo-2 éthyl]-3 uréido}-3 benzoique-(2S,4R) fondant à 140°C.

Le {[(méthoxycarbonyl-3 phényl)-3 uréido]-2 acétyl}-1 phényl-4 prolinate de tert-butyle-(2S,4R) peut être préparé comme décrit à l'exemple 41 §A, mais à partir de 1 g de phényl-4 prolinate de tert-butyle-(2S,4R), de 1 g d'acide [(méthoxycarbonyl-3 phényl)-3 uréido]-2 acétique et de 0,85 g de N,N'-dicyclohexylcarbodiimide dans 30 $cm^3$ d'acétonitrile. Après traitement, on obtient 1,8 g de {[(méthoxycarbonyl-3 phényl)-3 uréido]-2 acétyl}-1 phényl-4 prolinate de tert-butyle-(2S,4R) sous forme d'une meringue utilisée telle quelle dans les synthèses ultérieures.

Le phényl-4 prolinate de tert-butyle-(2S,4R) peut être préparé comme décrit à l'exemple 1 §B, mais à partir de 4,4 g de phényl-4 proline-(2S,4R), d'isobutène et de 1,5 $cm^3$ d'acide sulfurique dans 150 $cm^3$ de chloroforme. Après traitement, on obtient 1 g de phényl-4 prolinate de tert-butyle-(2S,4R) fondant à 62°C.

La phényl-4 proline-(2S,4R) peut être préparée selon les méthodes décrites par J.K. THOTTATHIL et J.L. MONIOT Tetrahedron Lett., 27, 151 (1986) et D.R KRONENTHAL et coll. Tetrahedron Lett., 31, 1241 (1990).

**Exemple 107**

On opère d'une manière analogue à celle décrite à l'exemple 9, mais à partir de 0,55 g de {[(benzyloxycarbonyl-3 phényl)-3 uréido]-2 acétyl}-1 (pyridyl-2)-5 prolinate de tert-butyle-(2RS,5SR) en solution dans 20 $cm^3$ de méthanol et de 10 $cm^3$ d'une solution aqueuse 0,1 N d'hydroxyde de potassium. Après traitement on obtient 0,17 g d'acide {{[tert-butoxycarbonyl-2 (pyridyl-2)-5 pyrrolidinyl-1)-2 oxo-2 éthyl]-3 uréido}-3 benzoique-(2RS,5SR} fondant à 174°C.

Le {[(benzyloxycarbonyl-3 phényl)-3 uréido]-2 acétyl}-1 (pyridyl-2)-5 prolinate de tert-butyle-(2RS,5SR) peut être préparé comme décrit à l'exemple 2, mais à partir de 0,5 g d'(amino-2 acétyl)-1 (pyridyl-2)-5 prolinate de tert-butyle-(2RS,5SR) et de 0,45 g d'isocyanato-3 benzoate de benzyle dans 25 $cm^3$ de tétrahydrofuranne. Après traitement, on obtient 0,7 g de {[(benzyloxycarbonyl-3 phényl)-3 uréido]-2 acétyl}-1 (pyridyl-2)-5 prolinate de tert-butyle-(2RS,5SR) sous forme d'une meringue utilisée telle quelle dans les synthèses ultérieures.

L'(amino-2 acétyl)-1 (pyridyl-2)-5 prolinate de tert-butyle-(2RS,5SR) peut être préparé comme décrit à l'exemple 2 §A, mais à partir de 0,7 g de (tert-butoxycarbonylamino-2 acétyl)-1 (pyridyl-2)-5 prolinate de tert-butyle-(2RS,5SR) et de 0,25 $cm^3$ d'iodotriméthylsilane dans 30 $cm^3$ de chloroforme. Après traitement, on obtient 0.5 g d'(amino-2 acétyl)-1 (pyridyl-2)-5 prolinate de tert-butyle-(2RS,5SR) sous forme d'une huile utilisée telle quelle dans les synthèses ultérieures.

Le (tert-butoxycarbonylamino-2 acétyl)-1 (pyridyl-2)-5 prolinate de tert-butyle-(2RS,5SR) peut être préparé comme décrit à l'exemple 90 §A, mais à partir de 4,8 g de dihydrogénophosphate de sodium et de 15,3 g d'amalgame de sodium à 6% (dans le mercure) dans une solution de 5,5 g d'un mélange des deux épimères en 4 du (tert-butoxycarbonylamino-2 acétyl)-1 phénylsulfonyl-4 (pyridyl-2)-5 prolinate de tert-butyle-(2RS,5RS) dans un mélange de 20 $cm^3$ de méthanol et de 60 $cm^3$ de tétrahydrofuranne. Après traitement, on obtient 0,7 g de (tert-butoxycarbonylamino-2 acétyl)-1 (pyridyl-2)-5 prolinate de tert-butyle-(2RS,5SR) sous forme d'une huile utilisée telle quelle dans les synthèses ultérieures.

Le (tert-butoxycarbonylamino-2 acétyl)-1 phénylsulfonyl-4 (pyridyl-2)-5 prolinate de tert-butyle-(2RS,5RS) peut être préparé de la manière suivante : à une solution de 2,7 g d'acide tert-butoxycarbonylamino-2 acétique dans un mélange de 2,2 $cm^3$ de triéthylamine et de 100 $cm^3$ de dichlorométhane maintenue à une température voisine de 0°C, on ajoute 1,5 $cm^3$ de chloroformiate d'éthyle. Le milieu réactionnel est agité pendant trente minutes à une température voisine de 0°C, puis on ajoute une solution de 6 g de phénylsulfonyl-4 (pyridyl-2)-5 prolinate de tert-butyle-(2RS,5RS) dans 50 $cm^3$ de dichlorométhane. Le mélange réactionnel est agité pendant vingt heures à une température voisine de 20°C, puis on ajoute 100 $cm^3$ d'eau.

La phase organique est séparée par décantation, lavée par 25 $cm^3$ d'eau, séchée sur sulfate de magnésium et concentrée sous pression réduite. Le résidu est purifié par chromatographie sur silice [éluant : cyclohexane-acétate d'éthyle (50-50 en volumes)]. Les fractions contenant le produit attendu sont réunies et concentrées

sous pression réduite. On obtient ainsi 5,6 g de (tert-butoxycarbonylamino-2 acétyl)-1 phénylsulfonyl-4 (pyridyl-2)-5 prolinate de tert-butyle-(2RS,5RS, mélange des deux épimères en 4) sous forme d'une huile utilisée telle quelle dans les synthèses ultérieures.

Le phénylsulfonyl-4 (pyridyl-2)-5 prolinate de tert-butyle-(2RS,5RS) peut être préparé comme décrit à l'exemple 89 §A mais à partir de 4,4 g de N-[(pyridyl-2)méthylène]-glycinate de tert-butyle, de 5 g d'acétate d'argent, de 3,4 g de phénylvinylsulfone et de 2,8 $cm^3$ de triéthylamine dans 200 $cm^3$ d'acétonitrile. Après traitement, on obtient 6 g de phénylsulfonyl-4 (pyridyl-2)-5 prolinate de tert-butyle-(2RS,5RS, mélange des deux épimères en 4) sous forme d'une huile utilisée telle quelle dans les synthèses ultérieures.

Le N-[(pyridyl-2)méthylène]-glycinate de tert-butyle peut être préparé comme décrit à l'exemple 89 §B mais à partir de 1,4 $cm^3$ de pyridyl-2 carboxaldéhyde, de 3,35 g de chlorhydrate de glycinate de tert-butyle, de 2,8 $cm^3$ de triéthylamine et de 3 g de tamis moléculaire 4Å dans 30 $cm^3$ de dichlorométhane. Après traitement, on obtient 4,8 g de N-[(pyridyl-2)méthylène]-glycinate de tert-butyle sous forme d'une huile utilisée telle quelle dans les synthèses ultérieures.

**Exemple 108**

A une solution de 0,65 g d'acide {{{[tert-butoxycarbonyl-4 (fluoro-2 phényl)-2 thiazolidinyl-3-(2R,4R)]-2 oxo-2 éthyl}-3 uréido}-3 phényl}-2 propionique (forme A) dans 6 $cm^3$ de méthanol, on ajoute 1,3 g d'oxone$^R$ en solution dans 8 $cm^3$ d'eau distillée. Le milieu réactionnel est agité pendant 12 h à une température voisine de 25° C puis concentré sous pression réduite à 40° C. Le produit obtenu est purifié par chromatographie sur silice [éluant : chlorure de méthylène-méthanol (90/10 en volumes)]. Les fractions contenant le produit attendu sont réunies et concentrées à sec sous pression réduite à 40° C. On obtient ainsi 0,4 g d'acide {{{[tert-butoxycarbonyl-4 (fluoro-2 phényl)-2 oxyde-1 thiazolidinyl-3-(1RS,2R,4R)]-2 oxo-2 éthyl}-3 uréido}-3 phényl}-2 propionique (forme A) sous forme d'un produit amorphe.

**RMN du proton** (200 MHz, DMSO $D_6$, δ en ppm, J en Hz), à 120 C on observe le mélange de deux diastéréoisomères dans les proportions 85-15, déplacements chimiques caractéristiques à 120° C : 1,32 (d, J = 7,5, 3H, $-CH_3$); 1,50 et 1,58 (2s, 9H en totalité,$-C(CH_3)_3$ de l'isomère majoritaire puis de l'isomère minoritaire); 2,85 (t, J = 12,5, 0,85H, 1H du $-S-CH_2$ pour l'isomère majoritaire); 3,4 à 4,4 (m, 6,15H,-CH-COO, l'autre H du $-S-CH_2$ pour l'isomère majoritaire, le $-S-CH_2$ de l'isomère minoritaire et $N-COCH_2-N$); 4,8 (t, J = 8, 0,15H, S-CH-N de l'isomère minoritaire); 4,97 (dd, J = 12,5 et 5,0, 0,85H, S-CH-N de l'isomère majoritaire); 6,3 à 6,45 (m, 1H, -NHCO-); 6,5 et 6,6 (2s, 0,15H et 0,85H, S-CH-N de l'isomère minoritaire et de l'isomère majoritaire); 6,8 (bd, J = 8, 1H, en position 4 sur CO-NH-Ph-); 7,0 à 7,6 (m, 6H, aromatiques); 7,77 et 8,10 (t, J = 8, 0,15H et 0,85H, en position 6 sur S-CH-Ph- pour l'isomère minoritaire et pour l'isomère majoritaire); 8,84 et 8,96 (2s, 0,85H et 0,15H, ArNHCO- pour l'isomère majoritaire et pour l'isomère minoritaire).

**Spectre infra-rouge** (KBr), bandes caractéristiques en $cm^{-1}$: 3390, 3100 à 3000, 2975, 2930, 2875, 2750 à 2300 (bande large), 1735, 1670, 1615, 1595, 1555, 1490, 1460, 1400, 1395, 1370, 1150, 760, 700.

**Exemple 109**

On opère d'une manière analogue à celle décrite à l'exemple **41**, mais à partir de 1,85 g de {{[tert-butoxycarbonyl-4 (naphtyl-1)-2 thiazolidinyl-3]-2 oxo-2 éthyl}-3 uréido}-3 benzoate de triméthylsilyl-2 éthyle-(2R, 4R) et de 5,82 $cm^3$d'une solution de fluorure de tétrabutylammonium 1M. On obtient ainsi 0,47 g d'acide {{[tert-butoxycarbonyl-4 (naphtyl-1)-2 thiazolidinyl-3]-2 oxo-1 éthyl}-2 uréido}-3 benzoïque-(2R, 4R) sous forme d'un solide beige fondant à 210°C.

$$[\alpha]_D^{20} = +222 \pm 5 \ (C = 0{,}5\%;\ CHCl_3).$$

**RMN du proton** (200 MHz, DMSO $D_6$ plus quelques gouttes de $CD_3COOD$, δ en ppm, J en Hz), 2 rotamères à température ambiante, déplacements chimiques caractéristiques à 120° C: 1,53 (s, 9H,$-C(CH_3)_3$); 3,25 (dd, J = 12 et 7, 1H, 1H du $S-CH_2$); 3,49 (dd, J = 12 et 6,5, 1H, l'autre H du $S-CH_2$); 3,7 (bd, J = 17,5, 1H, 1H du $N-COCH_2-N$); 4,08 (d, J = 17,5, 1H, l'autre H du $N-COCH_2-N$); 5,02 (dd, J = 7,0 et 6,5, 1H, N-CH-COO); 7,08 (s,1H, S-CH-N); 7,15 à 8,15 (m, 11 H, aromatiques).

**Spectre infra-rouge** (KBr), bandes caractéristiques en $cm^{-1}$: 3390, 3100 à 3000, 2975, 2930, 1735, 1655, 1595, 1555, 1510, 1485, 1400, 1370, 1150, 785, 770.

Le {{[tert-butoxycarbonyl-4 (naphtyl-1)-2 thiazolidinyl-3]-2 oxo-2 éthyl}-3 uréido}-3 benzoate de triméthylsilyl-2 éthyle-(2R, 4R) peut être préparé d'une manière analogue à celle décrite à l'exemple **34** mais à partir de 2,4 g d'(amino-2 acétyl)-3 (naphtyl-1)-2 thiazolidinecarboxylate-4 de tert-butyle-(2R,4R) et de 2,1 g d'isocyanato-3 benzoate de triméthylsilyl-2 éthyle. Le produit brut est purifié par chromatographie sur silice [éluant : acétate d'éthyle-cyclohexane (30/70 en volumes)]. Les fractions contenant le produit attendu sont réunies et concentrées à sec sous pression réduite à 40° C. On obtient ainsi 1,85 g de {{[tert-butoxycarbonyl-4 (naphtyl-1)-2 thiazolidinyl-3]-2 oxo-2 éthyl}-3 uréido}-3 benzoate de triméthylsilyl-2 éthyle-(4R) (Forme A) sous forme d'une pâte beige utilisée telle quelle dans les synthèses ultérieures.

L'(amino-2 acétyl)-3 (naphtyl-1)-2 thiazolidinecarboxylate-4 de tert-butyle-(2R, 4R) peut être préparé d'une manière analogue à celle décrite à l'exemple **34 A** mais à partir de 3,65 g (tert-butoxycarbonylamino-2 acétyl)-3 (naphtyl-1)-2 thiazolidinecarboxylate-4 de tert-butyle-(2R, 4R) et de 1,13 $cm^3$ de iodotriméthylsilane. On obtient ainsi 2,43 g d'(amino-2 acétyl)-3 (naphtyl-1)-2 thiazolidinecarboxylate-4 de tert-butyle-(2R, 4R) sous forme d'un solide jaune utilisé tel quel dans les synthèses ultérieures.

Le (tert-butoxycarbonylamino-2 acétyl)-3 (naphtyl-1)-2 thiazolidinecarboxylate-4 de tert-butyle-(2R, 4R) peut être préparé d'une manière analogue à celle décrite à l'exemple **34 B** mais à partir de 7,54 g de (naphtyl-1)-2 thiazolidinecarboxylate-4 de tert-butyle-(2RS,4R), de 4,23 g d'acide tert-butoxycarbonylamino-2 acétique et de 4,98 g de N,N'-dicyclohexylcarbodiimide. Le produit brut est purifié par chromatographie sur silice [éluant : acétate d'éthyle-cyclohexane (30/70 en volumes)]. Les fractions contenant le produit attendu sont réunies et concentrées à sec sous pression réduite. On obtient ainsi 13,9 g de (tert-butoxycarbonylamino-2 acétyl)-3 (naphtyl-1)-2 thiazolidinecarboxylate-4 de tert-butyle-(2R, 4R) sous forme d'une huile orange utilisée telle quelle dans les synthèses ultérieures.

Le (naphtyl-1)-2 thiazolidinecarboxylate-4 de tert-butyle-(2RS,4R) peut être préparé d'une manière analogue à celle décrite à l'exemple **34 C** mais à partir de 20,0 g d'acide (naphtyl-1)-2 thiazolidinecarboxylique-4-(2RS,4R) en solution dans 300 $cm^3$ de chloroforme, de 5,8 $cm^3$ d'acide sulfurique concentré et d'un excès d'isobutène. Le produit brut obtenu est purifié par chromatographie sur silice [éluant : acétate d'éthyle-cyclohexane (30/70 en volumes)]. Les fractions contenant le produit attendu sont réunies et concentrées à sec sous pression réduite à 40° C. On obtient ainsi 10,15 g de (naphtyl-1)-2 thiazolidinecarboxylate-4 de tert-butyle-(2RS,4R) sous forme d'une huile orange, mélange des isomères (2R,4R) et (2S,4R), utilisée telle quelle dans les synthèses ultérieures.

L'acide (naphtyl-1)-2 thiazolidinecarboxylique-4-(2RS,4R) peut être préparé comme à l'exemple **34 D** mais à partir de 20,0 g de L-cystéine et de 28,5 g de naphtyl-1 carboxaldéhyde. On obtient ainsi 36,3 g d'acide (naphtyl-1)-2 thiazolidinecarboxylique-4-(2RS,4R) fondant à 208° C, utilisé tel quel dans les synthèses ultérieures.

**Exemple 110**

On opère d'une manière analogue à celle décrite à l'exemple **41**, mais à partir de 0,12 g de {{[tert-butoxycarbonyl-4 (méthyl-5 thiényl-2)-2 thiazolidinyl-3]-2 oxo-2 éthyl}-3 uréido}-3 benzoate de triméthylsilyl-2 éthyle-(4R) (forme A) et de 0,4 $cm^3$d'une solution de fluorure de tétrabutylammonium 1M. On obtient ainsi 0,08 g d'acide {{[tert-butoxycarbonyl-4 (méthyl-5 thiényl-2)-2 thiazolidinyl-3]-2 oxo-1 éthyl}-2 uréido}-3 benzoïque-(4R) (forme A) sous forme d'un solide orange fondant à 85-90° C.

**RMN du proton** (300 MHz, DMSO $D_6$ plus quelques gouttes de $CD_3COOD$, d en ppm, J en Hz), 2 rotamères à température ambiante, déplacements chimiques caractéristiques à 120° C : 1,5 (s, 9H,$-C(CH_3)_3$); 2,46 (s, 3H, $-CH_3$); 3,4 et 3,52 (2dd, J = 12 et 6,5, 1H chacun, $-S-CH_2$); 3,96 (d, J = 17,5, 1H, 1H du $N-COCH_2-N$); 4,05 (d, J = 17,5, 1H, l'autre H du $N-COCH_2-N$); 4,96 (t, J = 6,5, 1H, OOC-CH-N); 6,65 (s,1H, S-CH-N); 6,65 (d, J = 4, 1H, H en position 4 sur le thiényl); 7,12 (d, J = 4, 1H, H en position 3 sur le thiényl); 7,55 et 7,62 (2dd, J = 8 et 2, 1H chacun, H en position 4 et 6 sur CO-NH-Ph-), 8,02 (t, J = 2, 1H, H en position 2 sur CO-NH-Ph-).

**Spectre infra-rouge** (KBr), bandes caractéristiques en $cm^{-1}$: 3390, 3100 à 3000, 2975, 2930, 2830, 2750-2300 (bande large), 1715, 1695, 1650, 1615, 1595, 1560, 1490, 1425, 1395, 1370, 1150, 800, 785, 755.

Le {{[tert-butoxycarbonyl-4 (méthyl-5 thiényl-2)-2 thiazolidinyl-3]-2 oxo-2 éthyl}-3 uréido}-3 benzoate de triméthylsilyl-2 éthyle-(4R) (forme A) peut être préparé d'une manière analogue à celle décrite à l'exemple **41 A** mais à partir de 0,38 g de (méthyl-5 thiényl-2)-2 thiazolidinecarboxylate-4 de tert-butyle-(2RS,4R), de 0,45 g d'acide {[(triméthylsilyl-2 éthoxycarbonyl)-3 phényl]-3 uréido}-2 acétique et de 0,30 g de N,N'-dicyclohexylcarbodiimide. Le produit brut est purifié par chromatographie sur silice [éluant : acétate d'éthyle-cyclohexane (30/70 en volumes)]. Les fractions contenant le produit attendu sont réunies et concentrées à sec sous pression réduite à 40° C. On obtient ainsi 0,12 g de {{[tert-butoxycarbonyl-4 (méthyl-5 thiényl-2)-2 thiazolidinyl-3]-2 oxo-2 éthyl}-3 uréido}-3 benzoate de triméthylsilyl-2 éthyle-(4R) (forme A) sous forme d'un solide jaune -orangé utilisé tel quel dans les synthèses ultérieures.

Le (méthyl-5 thiényl-2)-2 thiazolidinecarboxylate-4 de tert-butyle-(2RS,4R) peut être préparé d'une manière analogue à celle décrite à l'exemple **48B** mais à partir de 1,0 g de tert-butoxycarbonyl-3 (méthyl-5 thiényl-2)-2 thiazolidinecarboxylate-4 de tert-butyle-(4R) (forme A) et de 0,38 $cm^3$ d'iodotriméthylsilane. On obtient ainsi 0,38 g de (méthyl-5 thiényl-2)-2 thiazolidinecarboxylate-4 de tert-butyle-(2RS,4R) sous forme d'une huile jaune utilisée telle quelle dans les synthèses ultérieures.

Le tert-butoxycarbonyl-3 (méthyl-5 thiényl-2)-2 thiazolidinecarboxylate-4 de tert-butyle-(4R) (forme A) peut être préparé d'une manière analogue à celle décrite à l'exemple **48C** mais à partir de 2,97g d'acide tert-butoxycarbonyl-3 (méthyl-5 thiényl-2)-2 thiazolidinecarboxylique-4-(4R) (forme A), de 1,72 g de chlorure de paratoluènesulfonyle et de 0,67 g de tert-butanol. Le produit brut obtenu est purifié par chromatographie sur silice [éluant : acétate d'éthyle-cyclohexane (30/70 en volumes)]. Les fractions contenant le produit attendu sont réunies et concentrées à sec sous pression réduite à 40° C. On obtient ainsi 1,0 g de tert-butoxycarbonyl-3 (méthyl-5 thiényl-2)-2 thiazolidinecarboxylate-4 de tert-butyle-(4R) (forme A) sous forme d'une huile orange utilisée telle quelle dans les synthèses ultérieures.

L'acide tert-butoxycarbonyl-3 (méthyl-5 thiényl-2)-2 thiazolidinecarboxylique-4-(4R) (forme A) peut être préparé d'une manière analogue à celle décrite à l'exemple **48 D** mais à partir de 2,7 g d'acide (méthyl-5 thiényl-2)-2 thiazolidinecarboxylique-4-(2RS,4R), de 11,9 $cm^3$ d'une solution aqueuse 1N d'hydroxyde de sodium et de 2,6 g de dicarbonate de ditert-butyle. On obtient ainsi 3,0 g d'acide tert-butoxycarbonyl-3 (méthyl-5 thiényl-2)-2 thiazolidinecarboxylique-4-(4R) (forme A) sous forme d'un solide jaune utilisé tel quel dans les synthèses ultérieures.

L'acide (méthyl-5 thiényl-2)-2 thiazolidinecarboxylique-4-(2RS,4R) peut être préparé comme à l'exemple **34 D** mais à partir de 10,0 g de L-cystéine et de 11,3 g de (méthyl-5 thiényl-2) carboxaldéhyde. On obtient ainsi 7,86 g d'acide (méthyl-5 thiényl-2)-2 thiazolidinecarboxylique-4-(2RS,4R) fondant à 178° C, utilisé tel quel dans les synthèses ultérieures.

**Exemple 111**

On opère comme à l'exemple 84, mais à partir de 0,88 g de {{[(tert-butoxycarbonyl-2 phényl-5 pyrrolidinyl-1-(2S,5R))-2 oxo-2 éthyl]-3 uréido}-3 phényl}-2 propionate de benzyle (forme B) en solution dans 50 $cm^3$ d'acétate d'éthyle et de 0,22 g de palladium à 10% sur charbon. Après traitement, on obtient 0,5 g d'acide {{[(tert-butoxycarbonyl-2 phényl-5 pyrrolidinyl-1-(2S,5R))-2 oxo-2 éthyl]-3 uréido}-3 phényl}-2 propionique (forme B) fondant à 120° C, $[\alpha]_D^{20}$ = +49°8 ± 0,8° (c=0,53 ; méthanol).

Le {{[(tert-butoxycarbonyl-2 phényl-5 pyrrolidinyl-1-(2S,5R))-2 oxo-2 éthyl]-3 uréido}-3 phényl}-2 propionate de benzyle (forme B) peut être obtenu d'une manière analogue à celle décrite à l'exemple 2, mais à partir de 0,79 g d'(amino-2 acétyl)-1 phényl-5 prolinate de tert-butyle-(2S,5R) et de 0,8 g d'(isocyanato-3 phényl)-2 propionate de benzyle (forme B) dans 40 $cm^3$ de tetrahydrofuranne. Après traitement, on obtient 0.9 g de {{[(tert-butoxycarbonyl-2 phényl-5 pyrrolidinyl-1-(2S,5R))-2 oxo-2 éthyl]-3 uréido}-3 phényl}-2 propionate de benzyle fondant à 75° C.

L'(amino-2 acétyl)-1 phényl-5 prolinate de tert-butyle-(2S,5R) peut être obtenu d'une manière analogue à celle décrite à l'exemple 2A, mais à partir de 1,22 g de (tert-butoxycarbonylamino-2 acétyl)-1 phényl-5 prolinate de tert-butyle -(2S,5R), de 0,45 $cm^3$ de iodotriméthylsilane en solution dans 30 $cm^3$ de chloroforme anhydre. On obtient ainsi 0,79 g d'(amino-2 acétyl)-1 phényl-5 prolinate de tert-butyle-(2S,5R) sous forme d'une huile utilisée telle quelle dans les synthèses ultérieures.

Le (tert-butoxycarbonylamino-2 acétyl)-1 phényl-5 de tert-butyle-(2S,5R) peut être obtenu comme à l'exemple 2B, mais à partir d'une solution contenant 8 g de phényl-5 prolinate de tert-butyle-(2S,5R), 5,7 g d'acide tertbutoxycarbonylamino-2 acétique et 6,7 g de N,N′-dicyclohexylcarbodiimide dans 75 $cm^3$ d'acétonitrile anhydre. On obtient ainsi 10,5 g de (tert-butoxycarbonylamino-2 acétyl)-1 phényl-5 prolinate de tert-butyle-(2S,5R) fondant à 136° C, $[\alpha]_D^{20}$ = + 19°1 ± 0,8° (c = 0,64; méthanol).

**Exemple 112**

On opère d'une manière analogue à celle décrite à l'exemple 2, mais à partir de 2,5 g d'(amino-2 acétyl)-1 phényl-5 prolinate de tert-butyle-(2S,5R), de 5 g d'(isocyanato-3 phényl)-1 éthanesulfonate de tétrabutylammonium-(RS) dans 60 $cm^3$ de tetrahydrofuranne. Après traitement, on obtient 0,23 g de {{[(tert-butoxycarbonyl-2 phényl-5 pyrrolidinyl-1)-2 oxo-2 éthyl]-3 uréido}-3 phényl}-1 éthanesulfonate de potassium-(2S,5R), (mélange des deux épimères en 1) sous forme d'un solide beige amorphe, $[\alpha]_D^{20}$ = +20°7 ±0,9° (c=0,55 ; méthanol).

L'(isocyanato-3 phényl)-1 éthanesulfonate de tétrabutylammonium-(RS) peut être préparé comme décrit à l'exemple 21 mais à partir de 4,5 g d'(amino-3 phényl)-1 éthanesulfonate de tétrabutylammonium-(RS) dans 40 $cm^3$ de toluène, de 0,2 g de charbon et de 1,21 $cm^3$ de chloroformiate de trichlorométhyle. Après traitement, on obtient 5 g d'(isocyanato-3 phényl)-1 éthanesulfonate de tétrabutylammonium-(RS) sous forme d'une huile utilisée telle quelle dans les synthèses ultérieures.

**Exemple 113**

On opère d'une manière analogue à celle décrite à l'exemple 2, mais à partir de 2,6 g d'(amino-2 acétyl)-1 phényl-5 prolinate de tert-butyle-(2S,5R), de 4,8 g d'(isocyanato-3 phényl) méthanesulfonate de tétrabuty-

lammonium dans 60 $cm^3$ de tetrahydrofuranne. Après traitement, on obtient 0,21 g de {{[(tert-butoxycarbonyl-2 phényl-5 pyrrolidinyl-1)-2 oxo-2 éthyl]-3 uréido}-3 phényl}-1 méthanesulfonate de potassium-(2S,5R) sous forme d'un solide beige amorphe, $[\alpha]_D^{20}$ = +18°7 ± 0,8° (c=0,63; méthanol).

L'(isocyanato-3 phényl)méthanesulfonate de tétrabutylammonium peut être préparé comme décrit à l'exemple 21 mais à partir de 10 g d'(amino-3 phényl)méthanesulfonate de tétrabutylammonium-(RS) dans 80 $cm^3$de toluène, de 0,49 g de charbon et de 2,85 $cm^3$ de chloroformiate de trichlorométhyle. Après traitement, on obtient 11 g d'(isocyanato-3 phényl)méthanesulfonate de tétrabutylammonium sous forme d'une huile utilisée telle quelle dans les synthèses ultérieures.

**Exemple 114**

Dans un ballon contenant 1,38 g de {{{[tert-butoxycarbonyl-4 (difluoro-2,3 phényl)-2 thiazolidinyl-3-(2R, 4R)]-2 oxo-2 éthyl}-3 uréido}-3 phényl}-2 propionate de benzyle (Forme B), 0,85 g de formiate d'ammonium et 1,38 g de palladium sur charbon à 10 %, on additionne lentement sous atmosphère inerte 20 $cm^3$ de méthanol. Le milieu réactionnel est chauffé à reflux pendant 1 heure, puis refroidi à une température voisine de 25° C. Le catalyseur est ensuite séparé par filtration et le filtrat est concentré à sec sous pression réduite à 40° C. Le résidu obtenu est dissous dans 20 $cm^3$ d'une solution aqueuse d'hydroxyde de sodium 0,1N et lavé par deux fois 10 $cm^3$ d'oxyde de diéthyle. La phase aqueuse est amenée à pH 2 par addition d'une solution aqueuse d'acide sulfurique 1N. Le produit précipité est séparé par filtration, lavé par 2 fois 10 $cm^3$ d'eau et séché à l'air. On obtient ainsi, 1,0 g d'acide {{{[tert-butoxycarbonyl-4 (difluoro-2,3 phényl)-2 thiazolidinyl-3-(2R, 4R)]-2 oxo-2 éthyl}-3 uréido}-3 phényl}-2 propionique (Forme B) fondant à 130° C.

$[\alpha]_D^{20}$ = + 82° ± 2 (C = 0,707 % ; DMF)

Le {{[tert-butoxycarbonyl-4 (difluoro-2,3 phényl)-2 thiazolidinyl-3-(2R, 4R)]-2 oxo-2 éthyl}-3 uréido}-3 phényl}-2 propionate de benzyle (Forme B) peut être préparé d'une manière analogue à celle décrite à l'exemple 34 mais à partir de 2,5 g d'(amino-2 acéyl)-3 (difluoro-2,3 phényl)-2 thiazolidinecarboxylate-4 de tert-butyle-(2R, 4R) et de 1,96 g d'(isocyanato-3 phényl)-2 propionate de benzyle (Forme B). Le produit brut est purifié par chromatographie sur silice [éluant : acétate d'éthyle / cyclohexane (40/60 en volumes)]. Les fractions contenant le produit attendu sont réunies et concentrées à sec sous pression réduite à 40° C. On obtient ainsi 1,8 g de {{{[tert-butoxycarbonyl-4 (difluoro-2,3 phényl)-2 thiazolidinyl-3-(2R, 4R)]-2 oxo-2 éthyl}-3 uréido}-3 phényl}-2 propionate de benzyle (Forme B) sous forme d'un produit amorphe utilisé tel quel dans les synthèses ultérieures.

**Exemple 115**

Une solution de 2,1 g de {{[(diphényl-2,5 pyrrolidinyl-1)-2 oxo-2 éthyl]-3 uréido}-3 phényl}méthylèneaminoxyacétate de tert-butyle-(cis) en solution dans 10 $cm^3$ d'acide trifluoroacétique est agitée pendant deux heures à une température vosine de 20° C, puis le mieu réctionnel est amené à sec sous pression réduite à une température voisine de 40° C. Le résidu est cristallisé dans 50 $cm^3$ d'eau, filtré, lavé par trois fois 15 $cm^3$ d'eau et séché à une température voisine de 20° C. Le produit ainsi obtenu est purifié par chromatographie sur silice [éluant : dichlorométhane-méthanol (95-5 en volumes)]. Les fractions contenant le produit attendu sont réunies et concentrées à sec sous pression réduite à une température voisine de 35° C. On obtient ainsi, après recristallisation dans l'acétonitrile, 1 g d'acide {{[(diphényl-2,5 pyrrolidinyl-1)-2 oxo-2 éthyl]-3 uréido}-3 phényl}méthylèneaminoxyacétique-(cis) fondant à 194 C.

Le {{[(diphényl-2,5 pyrrolidinyl-1)-2 oxo-2 éthyl]-3 uréido}-3 phényl}méthylèneaminoxyacétate de tert-butyle-(cis) peut être obtenu d'une manière analogue à celle décrite à l'exemple 3, mais à partir de 4,2 g d'(amino-2 acétyl)-1 diphényl-2,5 pyrrolidine-(cis), de 1,78 g de N,N'-diimidazole-carbonyle et de 2,5 g d'(amino-3 phényl)méthylèneaminoxyacétate de tert-butyle dans 60 $cm^3$ de dichloro-1,2 éthane. Après traitement, on obtient 2,1 g de {{[(diphényl-2,5 pyrrolidinyl-1)-2 oxo-2 éthyl]-3 uréido}-3 phényl}méthylèneaminoxyacétate de tert-butyle-(cis) sous forme d'un solide beige amorphe.

L'(amino-3 phényl)méthylèneaminoxyacétate de tert-butyle peut être obtenu de la manière suivante : à une solution de 7,5 g de (nitro-3 phényl)méthylèneaminoxyacétate de tert-butyle dans 100 $cm^3$d'éthanol, on ajoute 0.75 g d'oxyde de platine. La suspension est agitée pendant 1 heure à une température voisine de 20 C sous atmosphère d'hydrogène (130 kPa).

Le catalyseur est séparé par filtration et le filtrat est concentré à sec sous pression réduite à 45° C. Le résidu est dissout dans 50 $cm^3$ d'acétate d'éthyle et la phase organique lavée par 50 $cm^3$ d'eau, séchée sur sulfate de magnésium et concentrée sous pression réduite. On obtient ainsi 6.2 g d'(amino-3 phényl)méthylèneaminoxyacétate de tert-butyle sous forme d'une huile utilisée telle quelle dans les synthèses ultérieures.

Le (nitro-3 phényl)méthylèneaminoxyacétate de tert-butyle peut être obtenu de la manière suivante : une

solution de 4,3 g d'hydrure de sodium à 60% dans la vaseline est degraissée par lavage par deux fois 50 $cm^3$d'hexane. On ajoute ensuite 100 $cm^3$ de tetrahydrofuranne puis la suspension est refroidie à une température voisine de 5° C et on introduit goutte à goutte une solution de 4,98 g de nitro-3 benzaldoxime dans 20 $cm^3$ de tétrahydrofuranne. Le mélange réactionnel est agité pendant une heure à une température voisine de 25° C, on ajoute une solution de 6 g de bromacétate de tert-butyle dans 5 $cm^3$ de tétrahydrofuranne et on poursuit l'agitation pendant une heure à une température voisine de 25° C. Le milieu réactionnel est ensuite versé dans un mélange de 200 $cm^3$ d'eau et de 100 $cm^3$ d'acétate d'éthyle. La phase organnique est séparée par décantation, séchée sur sulfate de magnésium et concentré à sec sous pression réduite à une température voisine de 40° C. On obtient aisni 7,5 g de (nitro-3 phényl)méthylèneaminoxyacétate de tert-butyle fondant à 120° C.

La nitro-3 benzaldoxime peut être préparée selon la méthode décrite par T.M. Opryshko et coll. Izv. Vyssh. Uchebn. Zaved., Khim. Khim. Tekhnol., 31, 53 (1988).

**Exemple 116**

On opère comme à l'exemple 108, mais à partir de 0,5 g d'acide {{{[tert-butoxycarbonyl-4 (fluoro-2 phényl)-2 thiazolidinyl-3-(2R,4R)]-2 oxo-2 éthyl}-3 uréido}-3 phényl}-2 propionique (forme B) et de 1,0 g d'oxone[R]. Le produit brut obtenu est purifié par chromatographie sur silice [éluant : chlorure de méthylène-méthanol (90/10 en volumes)] en recueillant des fractions de 10 $cm^3$. Les fractions 15 à 19 sont réunies et concentrées à sec sous pression réduite à 40° C. On obtient ainsi 0,2 g d'acide {{{[tert-butoxycarbonyl-4 (fluoro-2 phényl)-2 oxyde-1 thiazolidinyl-3-(1RS,2R,4R)]-2 oxo-2 éthyl}-3 uréido}-3 phényl}-2 propionique (forme B) sous forme d'un produit amorphe. Les fractions 9 à 14 sont réunies et concentrées à sec sous pression réduite à 40° C. On obtient ainsi 0,1 g d'un mélange d'acide {{{[tert-butoxycarbonyl-4 (fluoro-2 phényl)-2 oxyde-1 thiazolidinyl-3-(1RS,2R,4R)]-2 oxo-2 éthyl}-3 uréido}-3 phényl}-2 propionique (forme B) et d'acide {{{[tert-butoxycarbonyl-4 (fluoro-2 phényl)-2 dioxyde-1 thiazolidinyl-3(2R,4R)]-2 oxo-2 éthyl}-3 uréido}-3 phényl}-2 propionique (forme B).

**RMN du proton** (200 MHz, DMSO $D_6$, δ en ppm, J en Hz), à 120° C on observe le mélange de deux diastéréoisomères dans les proportions 88-12, déplacements chimiques caractéristiques à 120° C : 1,32 (d,J = 7,5, 3H, $-CH_3$) ; 1,50 et 1,58 (2s, 9H en totalité, $-C(CH_3)_3$ de l'isomère majoritaire puis de l'isomère minoritaire) ; 2,85 (t, J = 12,5, 0,88H, 1H du $-S-CH_2$ pour l'isomère majoritaire) ; 3,4 à 4,4 (m, 6,12H,-CH-COO, l'autre H du $-S-CH_2$ pour l'isomère majoritaire, le $-S-CH_2$ de l'isomère minoritaire et $N-COCH_2-N$); 4,8 (t, J = 8, 0,12H, S-CH-N de l'isomère minoritaire) ; 4,97 (dd, J = 12,5 et 5,0 0,88H, S-CH-N de l'isomère majoritaire) ; 6,3 à 6,45 (m, 1H, -NHCO-) ; 6,5 et 6,6 (2s, 0,12H et 0,88H, S-CH-N de l'isomère minoritaire et de l'isomère majoritaire) ; 6,8 (bd, J = 8, 1H, en position 4 sur CO-NH-Ph-) ; 7,0 à 7,6 (m, 6H, aromatiques) ; 7,77 et 8,10 (t, J = 8, 0,12H et 0,88H, en position 6 sur S-CH-Ph- pour l'isomère minoritaire et pour l'isomère majoritaire) ; 8,84 et 8,96 (2s, 0,88H et 0,12H, ArNHCO- pour l'isomère majoritaire et pour l'isomère minoritaire).

**Spectre infra-rouge** (KBr), bandes caractéristiques en $cm^{-1}$ : 3380, 2975,2930, 2650 à 2250 (bande large), 1735, 1650, 1610, 1595, 1560, 1490, 1455, 1370, 1230, 1150, 1050, 760, 700.

L'acide {{{[tert-butoxycarbonyl-4 (fluoro-2 phényl)-2 dioxyde-1 thiazolidinyl-3-(2R,4R)]-2 oxo-2 éthyl}-3 uréido}-3 phényl}-2 propionique (forme B) a été séparé du mélange d'acide {{{[tert-butoxycarbonyl-4 (fluoro-2 phényl)-2 oxyde-1 thiazolidinyl-3-(1RS,2R,4R)]-2 oxo-2 éthyl}-3 uréido}-3 phényl}-2 propionique (forme B) et d'acide {{{[tert-butoxycarbonyl-4 (fluoro-2 phényl)-2 dioxyde-1 thiazolidinyl-3-(2R,4R)-2 oxo-2 éthyl}-3 uréido}-3 phényl}-2 propionique (forme B) par chromatographie sur silice [éluant : acétate d'éthyle-méthanol (95/5 en volumes)]. Les fractions contenant le produit attendu sont réunies et concentrées à sec sous pression réduite à 30° C. On obtient ainsi 0,02 g d'acide {{{[tert-butoxycarbonyl-4 (fluoro-2 phényl)-2 dioxyde-1 thiazolidinyl-3-(2R,4R)]-2 oxo-2 éthyl}-3 uréido}-3 phényl}-2 propionique (forme B) sous forme d'un produit amorphe.

**RMN du proton** (200 MHz, DMSO $D_6$, δ en ppm): 1,3 (d, 3H, $-CH_3$); 1,5 (s, 9H, $-CH_3)_3$); 3,6 (m, 2H, $CH_2-SO_2$), 4,0 à 4,3 (m, 3H, Ar-CH-COO et $N-COCH_2-N$) ; 4,8 (bt, 1H, N-CH-COO) ; 6,4 (bt, 1H, -NHCO-) ; 6,5 (s, 1H S-CH-N) ; 6,8 (bd, 1H, en position 4 sur CO-NH-Ph-) ; 7,1 à 7,8 (m, 6H, aromatiques) ; 8,1 (bt, 1H, en position 6 sur S-CH-Ph-) ; 8,8 (bs, 1H, ArNHCO-).

**Spectre infra-rouge** (KBr), bandes caractéristiques en $cm^{-1}$ : 3380, 2975, 2930, 2650 à 2250 (bande large), 1735, 1650, 1610, 1595, 1560, 1490, 1455, 1370, 1345, 1230, 1150, 760, 700, 550.

Les médicaments selon l'invention sont constitués par un composé de formule (I) sous forme libre ou sous forme d'un sel d'addition avec un acide pharmaceutiquement acceptable, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules,

des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organ iques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, lotions, collyres, collutoires, gouttes nasales ou aérosols.

En thérapeutique humaine, les composés selon l'invention sont particulièrement utiles dans le traitement et la prévention des désordres liés à la CCK et à la gastrine au niveau du système nerveux et de l'appareil gastrointestinal. Ces composés peuvent donc être utilisés dans le traitement et la prévention des psychoses, des troubles anxieux, de la maladie de Parkinson, de la diskinésie tardive, du syndrome du colon irritable, de la pancréatite aiguë, des ulcères, des désordres de la motilité intestinale, de certaines tumeurs sensibles à la CCK, des troubles de la mémoire, comme analgésiques, comme potentialisateur de l'activité analgésique des médicaments analgésiques narcotiques et non narcotiques et comme régulateur de l'appétit.

Les doses dépendent de l'effet recherché, de la durée du traitement et de la voie d'administration utilisée; elles sont généralement comprises entre 0,05 g et 1 g par jour par voie orale pour un adulte avec des doses unitaires allant de 10 mg à 500 mg de substance active.

D'une façon générale, le médecin déterminera la posologie appropirée en fonction de l'âge, du poids et de tous les autres facteurs propres au sujet à traiter.

Les exemples suivants illustrent des compositions selon l'invention :

EXEMPLE 4

On prépare, selon la technique habituelle, des gélules dosées à 50 mg de produit actif ayant la composition suivante :

| | |
|---|---|
| - Acide {[(tert-butoxycarbonyl-2 phényl-5 pyrrolidinyl-1)-2 oxo-2 éthyl]-3 uréido}-3 phényl-3 benzoïque-(2RS,5SR) | 50 mg |
| - Cellulose | 18 mg |
| - Lactose | 55 mg |
| - Silice colloïdale | 1 mg |
| - Carboxyméthylamidon sodique | 10 mg |
| - Talc | 10 mg |
| - Stéarate de magnésium | 1 mg |

EXEMPLE B

On prépare selon la technique habituelle de comprimés dosés à 50 mg de produit actif ayant la composition suivante :

- Acide {[(tert-butoxycarbonyl-2 phényl-5 pyrrolidinyl-1)-2 oxo-2 éthyl]-3 uréido-3 benzoïque-(2R,5S)................................ 50 mg

- Lactose.......................................................................... 104 mg
- Cellulose........................................................................ 40 mg
- Polyvidone...................................................................... 10 mg
- Carboxyméthylamidon sodique......................................... 22 mg
- Talc................................................................................. 10 mg
- Stéarate de magnésium.................................................... 2 mg
- Silice colloïdale................................................................ 2 mg
- Mélange d'hydroxyméthylcellulose, glycérine, oxyde de titane (72-3,5-24,5) q.s.p. 1 comprimé pelliculé terminé à 245 mg

EXEMPLE C

On prépare une solution injectable contenant 10 mg de produit actif ayant la composition suivante :

- Acide {[(tert-butoxycarbonyl-4 phényl-2 thiazolidinyl-3)-2 oxo-2 éthyl]-3 uréido}-3 phénylacétique-(2R,4R).................. 10 mg
- Acide benzoïque............................................................ 80 mg
- Alcool benzylique.......................................................... 0,06 $cm^3$
- Benzoate de sodium....................................................... 80 mg
- Ethanol à 95 %............................................................... 0,4 $cm^3$
- Hydroxyde de sodium..................................................... 24 mg
- Propylène glycol............................................................ 1,6 $cm^3$
- Eau.........................................................................q.s.p. 4 cm3

**Revendications**

**1 -** Composés de formule :

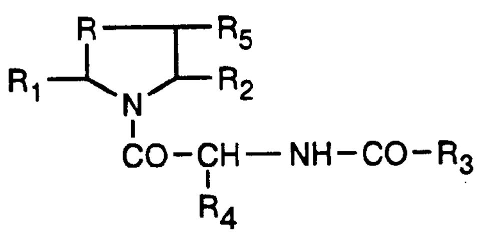

(I)

dans laquelle

- soit R représente un radical méthylène, éthylène, SO, $SO_2$, CHOH ou un atome de soufre, $R_1$ représente un radical pyridyle éventuellement substitué par un ou plusieurs radicaux alkyle, furyle éventuellement substitué par un ou plusieurs radicaux alkyle, thiényle éventuellement substitué par un ou plusieurs radicaux alkyle, quinolyle éventuellement substitué par un ou plusieurs radicaux alkyle, naphtyle éventuellement substitué par un ou plusieurs radicaux alkyle, indolyle éventuellement substitué par un ou plusieurs radicaux alkyle ou phényle éventuellement substitué par un ou plusieurs substituants choisis parmi les atomes d'halogène et les radicaux alkyle, alcoxy, hydroxy, nitro, amino, monoalkylamino, dialkylamino, alcoxycarbonyle, -CO-$NR_7R_8$ -NH-CO-$CH_3$, trifluorométhyle ou trifluorométhoxy et $R_8$ représente un atome d'hydrogène,
- soit R représente un radical méthylène, $R_1$ représente un atome d'hydrogène et $R_5$ représente un radical phényle,
- soit R représente un radical $CHR_8$, $R_1$ et $R_5$ représentent chacun un atome d'hydrogène,
- $R_2$ représente un radical alcoxycarbonyle, cycloalkyloxycarbonyle, cycloalkylalkyloxycarbonyle, -$CONR_9R_{10}$ ou phényle éventuellement substitué par un ou plusieurs substituants choisis parmi les radicaux alkyle, alcoxy ou hydroxy,
- $R_3$ représente un radical phényle (éventuellement substitué par un ou plusieurs substituants choisis parmi les atomes d'halogène et les radicaux alkyle, alcoxy et alkylthio), naphtyle, indolyle, quinolyle ou phénylamino dont le noyau phényle est éventuellement substitué par un ou plusieurs substituants choisis parmi les atomes d'halogène et les radicaux alkyle, alcoxy, alkylthio, trifluorométhyle, carboxy, alcoxycarbonyle, hydroxy, nitro, amino, acyle, cyano, sulfamoyle, carbamoyle, hydroxyiminoalkyle, alcoxyiminoalkyle, hydroxyaminocarbonyle, alcoxyaminocarbonyle, tétrazolyl-5, tétrazolyl-5 alkyle, trifluorométhylsulfonamido, alkylsulfinyle, mono ou polyhydroxyalkyle, sulfo, -alk-O-CO-alk, -alk-COOX, -alk-O-alk, -alk'-COOX, -O-alk-COOX, -CH=CH-COOX, -CO-COOX, -alk-$SO_3H$, sous forme de sel, -CH=CH-alk', -C(=NOH)-COOX, -S-alk-COOX, -O-$CH_2$-alk'-COOX, -CX=N-O-alk-COOX, -alk-N(OH)-CO-alk ou diméthyl-2,2 dioxo-4,6 dioxanne- 1,3-yle-5,
- $R_4$ représente un atome d'hydrogène ou un radical alkyle,
- $R_8$ représente un radical phényle,
- $R_7$ représente un atome d'hydrogène ou un radical alkyle, phénylalkyle ou phényle éventuellement substitué par un ou plusieurs substituants choisis parmi les atomes d'halogène et les radicaux alkyle, alcoxy et alkylthio,
- $R_8$ représente un radical alkyle, phénylalkyle ou phényle éventuellement substitué par un ou plusieurs substituants choisis parmi les atomes d'halogène et les radicaux alkyle, alcoxy et alkylthio,

ou bien $R_7$ et $R_8$ forment avec l'atome d'azote auquel ils sont rattachés un hétérocycle mono ou polycyclique saturé ou insaturé contenant 4 à 9 atomes de carbone et un ou plusieurs hétéroatomes (O, N) et éventuellement substitué par un ou plusieurs radicaux alkyle,

- $R_9$ représente un atome d'hydrogène ou un radical alkyle, cycloalkylalkyle, cycloalkyle, phénylalkyle ou phényle éventuellement substitué par un ou plusieurs substituants choisis parmi les atomes d'halogène et les radicaux alkyle, alcoxy et alkylthio,
- $R_{10}$ représente un radical alkyle, cycloalkylalkyle, cycloalkyle, phénylalkyle ou phényle éventuellement substitué par un ou plusieurs substituants choisis parmi les atomes d'halogène et les radicaux alkyle, alcoxy et alkylthio,

ou bien $R_9$ et $R_{10}$ forment ensemble avec l'atome d'azote auquel ils sont rattachés un hétérocycle mono ou polycyclique saturé ou insaturé contenant 4 à 9 atomes de carbone et un ou plusieurs hétéroatomes (O, N, S) et éventuellement substitué par un ou plusieurs radicaux alkyle,

- X représente un atome d'hydrogène, un radical alkyle ou phénylalkyle,
- alk représente un radical alkyle ou alkylène,
- alk' représente un radical hydroxyalkyle, hydroxyalkylène, alkoxyalkyle ou alkoxyalkylène, étant entendu que les radicaux alkyle, alkylène et alcoxy et les portions alkyle, alkylène et alcoxy contiennent 1 à 4 atomes de carbone en chaîne droite ou ramifiée, les radicaux et portions acyle contiennent 2 à 4 atomes de carbone et les radicaux et portions cycloalkyle contiennent 3 à 6 atomes de carbone, ainsi que leurs sels et leurs racémiques et énantiomères lorsqu'ils comportent au moins un centre asymétrique.

**2 -** Composés de formule (I) selon la revendication 1 pour lesquels R représente un radical méthylène ou un atome de soufre, $R_1$ représente un radical phényle, $R_2$ représente un radical phényle ou alcoxycarbonyle et $R_3$ représente un radical phénylamino dont le noyau phényle est substitué par un radical carboxy, -alk-COOH, -S-alk-COOH, hydroxyalkyle, alk'-COOH ou alk-$SO_3^-$, $R_4$ représente un atome d'hydrogène et $R_5$ représente un atome d'hydrogène.

**3 -** Les composés de formule (I) selon la revendication 1 suivants :

- {[(hydroxy-1 éthyl-(RS))-3 phényl)-3 uréido]-2 acétyl}-1 phényl-5 prolinate de tert-butyle-(2RS,5SR),
- acide {{[tert-butoxycarbonyl-2 phényl-5 pyrrolidinyl-1 (2S,5R))-2 oxo-2 éthyl]-3 uréido}-3 phényl)-2 propionique (forme B)
- acide {{[(tert-butoxycarbonyl-2 phényl-5 pyrrolidinyl-1)-2 oxo-2 éthyl]-3 uréido}-3 phénylthio} acétique-(2RS,5SR),
- acide {[(tert-butoxycarbonyl-4 (fluoro-2 thiazolidinyl-3)-2 oxo-2 éthyl]-3 uréido}-3 phénylacétique-(2R,4R),
- acide {{[(tert-butoxycarbonyl-2 (fluoro-2 phényl)-2 thiazolidinyl-3-(2R,4R))-2 oxo-2 éthyl]-3 uréido}-3 phényl}-2 propionique (forme B),
- {{[(tert-butoxycarbonyl-4 phényl-2 thiazolidinyl-3-(2R,4R)))-2 oxo-2 éthyl]-3 uréido}-3 phényl}-1 éthanesulfonate de potassium-(RS), mélange des formes A et B,
- {{[(tert-butoxycarbonyl-2 phényl-5 pyrrolidinyl-1-(2S,5R))-2 oxo-2 éthyl]-3 uréido}-3 phényl}-1 éthanesulfonate de potassium-(RS),
- {{[(tert-butoxycarbonyl-2 phényl-5 pyrrolidinyl-1)-2 oxo-2 éthyl]-3 uréïdo}-3 phényl-1 méthanesulfonate de potassium-(2S,5R).
- acide {[tert-butoxycarbonyl-2 phényl-5 pyrrolidinyl-1)-2 oxo-2 éthyl]-3 uréido}-3 benzoïque-(2S,5R),
- acide {{[tert-butoxycarbonyl-2 (fluoro-2 phényl)-5 pyrrolidinyl-1]-2 oxo-2 éthyl}-3 uréido}-3 benzoïque-(2RS,5SR),
- acide {[(diphényl-2,5 pyrrolidinyl-1)-2 oxo-2 éthyl]-3 uréido}-3 benzoïque-(cis),
- acide {{[(hydroxy-2 phényl)-2 phényl-5 pyrrolidinyl-1]-2 oxo-2 uréido}-3 phénylacétique-(2RS,5SR),
- acide {[(tert-butoxycarbonyl-4 phényl-2 thiazolidinyl-3)-2 oxo-2 éthyl]-3 uréido}-3 phénylacétique-(2R,4R),
- acide {[(tert-butoxycarbonyl-4 phényl-2 thiazolidinyl-3)-2 oxo-2 éthyl]-3 uréido}-3 benzoïque-(2R,4R),
- acide {{[(tert-butoxycarbonyl-4 (fluoro-2 phényl)-2 oxyde-1 thiazolidinyl-3-(1RS,2R,4R))-2 oxo-2 éthyl}-3 uréido}-3 phényl}-2 propionique (forme A),
- acide {[(tert-butoxycarbonyl-4 (difluoro-2,3 phényl)-2 thiazolidinyl-3)-2 oxo-2 éthyl]-3 uréido}-3 phénylacétique-(2R,4R),
- {{[(hydroxyimino-1 éthyl)-3 phényl-(E)]-3 uréido}-2 acétyl}-1 phényl-5 prolinate de tert-butyle-(2RS,5SR).

**4** - Procédé de préparation des composés de formule (I) selon l'une des revendications 1 à 3 pour lesquels R représente un radical méthylène, éthylène, CHOH, $CHR_6$ ou un atome de soufre et $R_3$ représente un radical phénylamino dont le noyau phényle est éventuellement substitué caractérisé en ce que l'on fait réagir un dérivé réactif de l'acide carbamique, obtenu éventuellement in situ par action d'un dérivé réactif de l'acide carbonique choisi parmi le N,N'-diimidazole carbonyle, le phosgène, le diphosgène et le chloroformiate de p-nitrophényle sur un dérivé de formule :

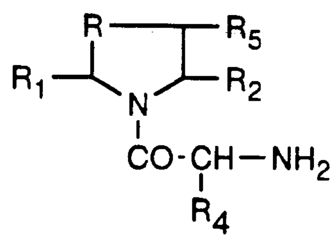

(II)

dans laquelle R représente un radical méthylène, éthylène, CHOH, $CHR_6$ ou un atome de soufre et $R_1$, $R_2$, $R_4$, $R_5$ et $R_5$ ont les mêmes significations que dans la formule (I), sur une aniline dont le noyau phényle est éventuellement substitué par un ou plusieurs substituants choisis parmi les atomes d'halogène et les radicaux alkyle, alcoxy, alkylthio, trifluorométhyle, carboxy, alcoxycarbonyle, hydroxy, nitro, amino, acyle, cyano, sulfamoyle, carbamoyle, hydroxyiminoalkyle, alcoxyiminoalkyle, hydroxyaminocarbonyle, alcoxyamino-carbonyle, tétrazolyl-5, tétrazolyl-5 alkyle, trifluorométhylsulfonamido, alkylsulfinyle, mono ou polyhydroxyalkyle, sulfo, -alk-O-CO-alk, -alk-COOX, -alk-O-alk, -alk'-COOX, -O-alk-COOX, -CH=CH-COOX, -CO-COOX, -alk-$SO_3H$, -CH=CH-alk', -C(=NOH)-COOX, -S-alk-COOX, -O-$CH_2$-alk'-COOX, -CX=N-O-alk-COOX ou -alk-N(OH)-CO-alk ou diméthyl-2,2 dioxo-4,6 dioxa nne-1,3-yl-5, isole le produit obtenu et le transforme éventuellement en sel.

**5** - Procédé selon la revendication 4 caractérisé en ce que l'on opère dans un solvant organique inerte à une température comprise entre 20° C et la température d'ébullition du solvant.

**6** - Procédé de préparation des composés de formule (I) selon l'une des revendications 1 à 3 pour lesquels R représente un radical méthylène, éthylène, CHOH, $CHR_6$ ou un atome de soufre et $R_3$ représente un radical phénylamino dont le noyau phényle est éventuellement substitué par un ou plusieurs substituants choisis parmi

les atomes d'halogène et les radicaux alkyle, alcoxy, alkylthio, trifluorométhyle, nitro, acyle, cyano, sulfamoyle, alcoxycarbonyle, -alk-O-alk, trifluorométhylsulfonamido, -alk-$SO_3H$, sous forme de sel, alk-COOX ou alk'-COOX dans lesquels X est différent d'un atome d'hydrogènecaractérisé en ce que l'on fait réagir un produit de formule (II) sur un phénylisocyanate dont le noyau phényle est éventuellement substitué par un ou plusieurs substituants choisis parmi les atomes d'halogène et les radicaux alkyle, alcoxy, akylthio, trifluorométhyle, nitro, acyle, cyano, sulfamoyle, alcoxycarbonyle, -alk-O-alk, trifluorométhylsulfonamido ou alk-$SO_3H$, isole produit obtenu et le transforme éventuellement en sel.

**7 -** Procédé selon la revendication 6 caractérisé en ce que l'on opère dans un solvant organique inerte à une température comprise entre 10° C et la température d'ébullition du solvant.

**8 -** Procédé de préparation des composés de formule (I) selon l'une des revendications 1 à 3 pour lesquels R représente un radical méthylène, éthylène, CHOH, $CHR_6$ ou un atome de soufre et $R_3$ représente un radical phénylamino dont le noyau phényle est substitué par un radical carboxy, -alk-COOH, -O-alk-COOH, -alk'-COOH, -CH=CH-COOH, -CO-COOH, -S-alk-COOH, -C(=NOH)-COOH, O-$CH_2$-alk'-COOH ou CX=N-O-alk-COOH caractérisé en ce que l'on hydrolyse ou hydrogénolyse un ester correspondant, isole le produit obtenu et le transforme éventuellement en sel.

**9 -** Procédé de préparation des composés de formule (I) selon l'une des revendications 1 à 3 pour lesquels R représente un radical méthylène, éthylène, CHOH, $CHR_6$ ou un atome de soufre et $R_3$ représente un radical phényle éventuellement substitué, naphtyle, indolyle, quinolyle éventuellement substitué ou phénylamino dont le noyau phényle est éventuellement substitué par un ou plusieurs substituants choisis parmi les atomes d'halogène et les radicaux alkyle, alcoxy, alkylthio, trifluorométhyle, nitro, acyle, cyano, sulfamoyle, alcoxycarbonyle, -alk-O-alk, alk-COOX ou alk'-COOX dans lesquels X est différent d'un atome d'hydrogène trifluorométhylsulfonamido caractérisé en ce que l'on fait agir un acide de formule :

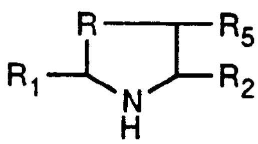

(IV)

dans laquelle R est défini comme ci-dessus et $R_1$ et $R_5$ sont définis comme dans la revendication 1, sur un acide de formule :

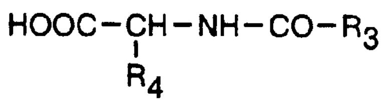

dans laquelle $R_3$ est défini comme ci-dessus et $R_4$ est défini comme dans la revendication 1, ou un dérivé réactif de cet acide, isole le produit obtenu et le transforme éventuellement en sel.

**10 -** Procédé de préparation des composés de formule (I) selon l'une des revendication 1 à 3 pour lesquels R représente SO ou $SO_2$ caractérisé en ce que l'on oxyde un produit correspondant pour lequel R représente un atome de soufre, isole le produit obtenu et le transforme éventuellement en sel.

**11 -** Procédé de séparation des énantiomènes des composés de formule (I) selon l'une des revendications 1 à 3 caractérisé en ce que l'on effectue une chromatographie sur une phase chirale définie par la structure :

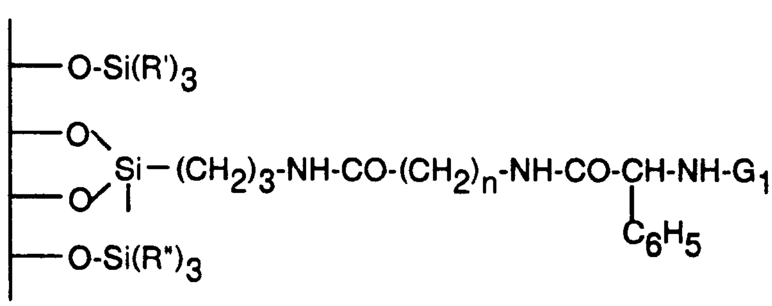

dans laquelle les symboles R', identiques ou différents, et R", identiques ou différents, représentent des radicaux alcoyles contenant 1 à 10 atomes de carbone, $G_1$ représente un groupement électro-attracteur et n représente un nombre entier compris entre 3 et 13 inclusivement.

**12 -** La phase chirale telle qu'elle est définie dans la revendication 11.

**13 -** Médicaments caractérisés en ce qu'ils contiennent comme principe actif au moins un des composés selon l'une des revendications 1 à 3.

**14 -** Médicaments selon la revendication 13 utilisables pour le traitement des désordres liés à la CCK et à la gastrine.

Office européen des brevets

# RAPPORT DE RECHERCHE EUROPEENNE

Numero de la demande

EP 92 40 1904
Page 1

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | EUROPEAN JOURNAL OF MEDICINAL CHEMISTRY vol. 21, no. 6, 1986, PARIS, FRANCE pages 493 - 497; ABOULWAFA ET AL: 'Synthesis of n-glycylthiazolidine derivatives as potential radioprotectors' --- | 1-14 | C07D207/06 C07K5/06 A61K31/40 A61K37/02 C07B57/00 |
| A | EUROPEAN JOURNAL OF MEDICINAL CHEMISTRY vol. 13, no. 2, 1978, PARIS, FRANCE pages 149 - 151; TEROL ET AL: 'Recherche d'agents radioprotecteurs dérivés de la phényl-2 thiazolidine' * Page 150, Tableau I * --- | 1-14 | |
| A | DE-A-3 332 633 (LUITPOLD-WERK CHEMISCH-PHARMAZEUTISCHE FABRIK) 4 Avril 1985 * Revendications * * pages 29-30 * * Pages 91-94 * --- | 1-14 | |
| | | | DOMAINES TECHNIQUES RECHERCHES (Int. Cl.5) |
| A | US-A-4 499 102 (OYA ET AL) 12 Février 1985 * Colonnes 27-31 * --- | 1-14 | C07K C07D B01J |
| A | EP-A-0 059 966 (MERCK&CO. INC.) 15 Septembre 1982 * Page 1-3 * * Pages 23-25, Tableau II * --- | 1-14 | |
| A | CH-A-362 072 (CIBA) 14 Juillet 1962 * Exemple 1 * --- | 1-14 | |
| A | JOURNAL OF CHROMATOGRAPHY vol. 441, no. 2, 1988, AMSTERDAM, PAYS-BAS pages 311 - 322; PIRKLE ET AL: 'An improved chiral stationary phase for the separation of enantiomers' * Pages 311-312 * --- -/-- | 11-12 | |

Le présent rapport a été établi pour toutes les revendications

| Lieu de la recherche | Date d'achèvement de la recherche | Examinateur |
|---|---|---|
| LA HAYE | 01 SEPTEMBRE 1992 | KORSNER S.E. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P0402)

Office européen des brevets

# RAPPORT DE RECHERCHE EUROPEENNE

Numero de la demande

EP 92 40 1904
Page 2

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| A | CHEMICAL ABSTRACTS, vol. 106, 1987, Columbus, Ohio, US; abstract no. 50637, KINOSHITA ET AL: 'Chromatographic separation of amino acid enantiomers' page 687 ;colonne 2 ; * abrégé * & JP-A-6 193 145 (FUNAKOSHI PHARMACEUTICAL CO., LTD.) 12 Mai 1986 | 11-12 | |
| | ----- | | |

DOMAINES TECHNIQUES RECHERCHES (Int. Cl.5 )

Le présent rapport a été établi pour toutes les revendications

| Lieu de la recherche | Date d'achèvement de la recherche | Examinateur |
|---|---|---|
| LA HAYE | 01 SEPTEMBRE 1992 | KORSNER S.E. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)